(19) Europäisches Patentamt
European Patent Office
Office européen des brevets

(11) EP 2 549 991 B1

(12) EUROPÄISCHE PATENTSCHRIFT

(45) Veröffentlichungstag und Bekanntmachung des Hinweises auf die Patenterteilung:
**22.11.2017 Patentblatt 2017/47**

(21) Anmeldenummer: **11710182.4**

(22) Anmeldetag: **22.03.2011**

(51) Int Cl.:
***A61K 31/295*** *(2006.01)* ***A61K 31/4453*** *(2006.01)*
***A61P 7/06*** *(2006.01)* ***A61K 45/06*** *(2006.01)*
***A61K 9/00*** *(2006.01)* ***A61K 31/555*** *(2006.01)*
***C07F 15/02*** *(2006.01)*

(86) Internationale Anmeldenummer:
**PCT/EP2011/054315**

(87) Internationale Veröffentlichungsnummer:
**WO 2011/117225 (29.09.2011 Gazette 2011/39)**

(54) **FE(III)-KOMPLEXVERBINDUNGEN ZUR BEHANDLUNG UND PROPHYLAXE VON EISENMANGELERSCHEINUNGEN UND EISENMANGELANÄMIEN**

FE(III) COMPLEX COMPOUNDS FOR TREATING AND PROPHYLAXIS FOR ANAEMIA SYMPTOMS AND ANAEMIA

LIAISONS DE COMPLEXES DE FE(III) POUR LE TRAITEMENT ET LA PROPHYLAXIE DES CARENCES EN FER ET DES ANÉMIES

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **23.03.2010 EP 10157387**

(43) Veröffentlichungstag der Anmeldung:
**30.01.2013 Patentblatt 2013/05**

(73) Patentinhaber: **Vifor (International) AG**
**9001 St. Gallen (CH)**

(72) Erfinder:
- **BARK, Thomas**
**8050 Zürich (CH)**
- **BUHR, Wilm**
**78465 Konstanz (DE)**
- **BURCKHARDT, Susanna**
**8049 Zürich (CH)**
- **BURGERT, Michael**
**88045 Friedrichshafen (DE)**
- **CANCLINI, Camillo**
**9000 St. Gallen (CH)**
- **DÜRRENBERGER, Franz**
**4143 Dornach (CH)**
- **FUNK, Felix**
**8404 Winterthur (CH)**
- **GEISSER, Peter**
**9014 St. Gallen (CH)**
- **KALOGERAKIS, Aris**
**8400 Winterthur (CH)**
- **MAYER, Simona**
**9055 Bühler (CH)**
- **PHILIPP, Erik**
**9320 Arbon (CH)**
- **REIM, Stefan**
**8404 Stadel Winterthur (CH)**
- **SIEBER, Diana**
**9030 Abtwil (CH)**
- **SCHMITT, Jörg**
**9425 Thal (CH)**
- **SCHWARZ, Katrin**
**9000 St. Gallen (CH)**

(74) Vertreter: **Gille Hrabal**
**Brucknerstrasse 20**
**40593 Düsseldorf (DE)**

(56) Entgegenhaltungen:
**US-A- 6 121 287 US-A1- 2005 192 315**

- **SYAMAL, A.: "Ferric benzoylacetanilides", CANADIAN JOURNAL OF CHEMISTRY , 47(10), 1693-6 CODEN: CJCHAG; ISSN: 0008-4042, 1969, XP002600690, in der Anmeldung erwähnt**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

### Einleitung:

Die Erfindung betrifft Eisen(III)-ß-ketoamid-Komplexverbindungen und deren sie umfassende pharmazeutischen Zusammensetzungen zur Behandlung und/oder zur Prophylaxe von Eisenmangelerscheinungen und Eisenmangelanämien.

### Hintergrund:

Eisen ist ein essentielles Spurenelement für fast alle Lebewesen und ist dabei insbesondere für das Wachstum und die Blutbildung relevant. Die Balance des Eisenhaushaltes wird dabei primär auf der Ebene der Eisenwiedergewinnung aus Hämoglobin von alternden Erythrozyten und der duodenalen Absorption von nahrungsgebundenem Eisen reguliert. Das freigesetzte Eisen wird über den Darm insbesondere durch spezifische Transportsysteme (DMT-1, Ferroportin, Transferrin, Transferrin Rezeptoren) aufgenommen, in den Blutkreislauf transportiert und hierüber in die entsprechenden Gewebe und Organe weitergeleitet.
Das Element Eisen ist im menschlichen Körper unter anderem für den Sauerstofftransport, die Sauerstoffaufnahme, Zellfunktionen, wie den mitochondrialen Elektronentransport und letztlich für den gesamten Energiestoffwechsel von großer Bedeutung.
Der Körper eines Menschen enthält im Durchschnitt 4 bis 5 g Eisen, wobei dies in Enzymen, in Hämoglobin und Myoglobin, sowie als Depot- oder Reserve-Eisen in Form von Ferritin und Hämosiderin vorliegt.
Etwa die Hälfte dieses Eisens ca. 2 g liegt als Häm Eisen gebunden im Hämoglobin der roten Blutkörperchen vor. Da Erythrozyten nur eine begrenzte Lebensdauer aufweisen (75-150 Tage), müssen ständig neue gebildet und alte eliminiert werden (über 2 Mio. Erythrozyten werden pro Sekunde neu gebildet). Diese hohe Neubildungskapazität wird durch Makrophagen erreicht, indem diese die alternden Erythrozyten phagozytotisch aufnehmen, lysieren und so das enthaltene Eisen für den Eisenhaushalt rezyklieren können. Somit wird die täglich für die Erythropoese benötige Eisenmenge von ca. 25 mg grösstenteils bereit gestellt.

Der tägliche Eisenbedarf eines erwachsenen Menschen beträgt zwischen 0,5 und 1,5 mg pro Tag, bei Kleinkindern sowie bei Frauen in der Schwangerschaft liegt der Eisenbedarf bei 2 bis 5 mg pro Tag. Die täglichen Eisenverluste, z.B. durch Abschilferung von Haut- und Epithelzellen ist gering, erhöhte Eisenverluste treten beispielsweise bei der Menstruationsblutung bei Frauen auf. Generell können Blutverluste den Eisenhaushalt beträchtlich verringern, da pro 2 ml Blut etwa 1 mg Eisen verloren gehen. Der normale tägliche Eisenverlust von ca. 1 mg wird üblicherweise bei einem erwachsenen, gesunden Menschen über die tägliche Nahrungsaufnahme wieder ersetzt. Der Eisenhaushalt wird über Resorption reguliert, wobei die Resorptionsquote des in der Nahrung vorhandenen Eisens zwischen 6 und 12 % beträgt, bei Eisenmangel beträgt die Resorptionsquote bis zu 25 %. Die Resorptionsquote wird vom Organismus in Abhängigkeit vom Eisenbedarf und der Eisenspeichergröße reguliert. Dabei nutzt der menschliche Organismus sowohl zweiwertige als auch dreiwertige Eisenionen. Üblicherweise werden Eisen(III)-Verbindungen bei ausreichend saurem pH-Wert im Magen gelöst und damit für die Resorption verfügbar gemacht. Die Resorption des Eisens findet im oberen Dünndarm durch Mukosazellen statt. Dabei wird dreiwertiges nicht Häm Eisen für die Resorption z.B. durch Ferrireduktase (membranständiges, duodenales Cytochrom b) in der Darmzellmembran zunächst zu Fe(II) reduziert, um dann durch das Transportprotein DMT1 (divalent metal transporter 1) in die Darmzellen transportiert werden zu können. Dagegen gelangt Häm Eisen unverändert über die Zellmembran in die Enterozyten. In den Enterozyten wird Eisen entweder als Depot-Eisen in Ferritin gespeichert oder durch das Transportprotein Ferroportin ins Blut abgegeben. In diesem Vorgang spielt Hepcidin eine zentrale Rolle, da es den wesentlichen Regulationsfaktor der Eisenaufnahme darstellt. Das durch das Ferroportin ins Blut transportierte zweiwertige Eisen wird durch Oxidasen (Ceruloplasmin, Hephaestin) in dreiwertiges Eisen überführt, welches dann mittels Transferrin an die relevanten Stellen im Organismus transportiert wird (s. zum Beispiel: "Balancing acts: molecular control of mammalian iron metabolism". M.W. Hentze, Cell 117,2004,285-297.)

Der Organismus von Säugetieren kann Eisen nicht aktiv ausscheiden. Der Eisenmetabolismus wird im Wesentlichen über die zelluläre Freisetzung von Eisen aus Makrophagen, Hepatocyten und Enterozyten durch das Hepcidin gesteuert.

Ein verringerter Serum-Eisenspiegel führt in krankhaften Fällen zu einem verringerten Gehalt an Hämoglobin, verringerter Erythrozytenproduktion und damit zu einer Anämie.

Äussere Symptome von Anämien beinhalten Müdigkeit, Blässe sowie verringerte Aufmerksamkeitskapazitäten. Die klinischen Symptome einer Anämie beinhalten niedrige Serum-Eisengehalte (Hypoferrämie), geringe Hämoglobingehalte, geringe Hämatokritlevel sowie eine reduzierte Anzahl an roten Blutkörperchen, reduzierte Retikulozyten und erhöhte Werte an löslichen Transferrinrezeptoren.

Eisenmangelerscheinungen oder Eisenanämien werden durch Eisenzufuhr behandelt. Dabei erfolgt die Substitution mit Eisen entweder auf dem oralen Weg oder durch intravenöse Eisengabe. Außerdem können zur Ankurbelung

der Bildung von roten Blutkörperchen auch Erythropoetin und andere Erythropoesestimulierende Substanzen bei der Behandlung von Anämien eingesetzt werden.

Anämie kann oft auf Mangelernährung bzw. eisenarme Diäten oder unausgewogene, eisenarme Ernährungsgewohnheiten zurück geführt werden. Außerdem treten Anämien durch verringerte bzw. schlechte Eisenabsorption beispielsweise aufgrund von Gastrektomien oder von Erkrankungen wie Crohn's-Disease auf. Auch kann ein Eisenmangel infolge eines erhöhten Blutverlustes z.B. durch eine Verletzung, starke Menstruationsblutung oder Blutspende auftreten. Auch ist ein erhöhter Eisenbedarf in der Wachstumsphase Heranwachsender und Kinder sowie von Schwangeren bekannt. Da ein Eisenmangel nicht nur zu einer verringerten Bildung von roten Blutkörperchen, sondern damit auch zu einer schlechten Versorgung des Organismus mit Sauerstoff führt, was zu den oben genannten Symptomen wie Müdigkeit, Blässe und Konzentrationsschwäche auch gerade bei Heranwachsenden zu langfristigen negativen Auswirkungen auf die kognitive Entwicklung führen kann, ist eine gut wirksame und gut verträgliche Therapie von besonderem Interesse.

Durch die Verwendung der erfindungsgemäßen Fe(III)-Komplexverbindungen besteht die Möglichkeit, Eisenmangelerscheinungen und Eisenmangelanämien durch orale Applikation effektiv zu behandeln, ohne das grosse Nebenwirkungspotential der klassischen Präparate, der Fe(II)-Eisensalze, wie $FeSO_4$, hervorgerufen durch Oxidativen Stress, in Kauf zu nehmen. Somit wird eine schlechte Compliance vermieden, die oft Grund für die mangelnde Beseitigung des Eisenmangelzustands darstellt.

**Stand der Technik:**

Aus dem Stand der Technik sind eine Vielzahl von Eisenkomplexe für die Behandlung von Eisenmangelzuständen bekannt.
Ein sehr grosser Anteil dieser Komplex-Verbindungen besteht aus polymeren Strukturen. Hierbei handelt es sich bei den meisten Komplex-Verbindungen um Eisen-Polysaccharid-Komplexverbindungen (WO20081455586, WO2007062546, WO20040437865, US2003236224, EP150085). Gerade aus diesem Bereich sind schon Medikamente auf dem Markt verfügbar (wie Maltofer, Venofer, Ferinject, Dexferrum, Ferumoxytol).
Ein anderer grosser Bestandteil der Gruppe der polymeren Komplexverbindungen sind die Eisen-Peptid-Komplexverbindungen (CN101481404, EP939083, JP02083400).

In der Literatur sind auch Fe-Komplexverbindungen beschrieben, die sich von Makromolekülen, wie Hämoglobin, Chlorophyll, Curcumin und Heparin strukturell herleiten (US474670, CN1687089, Biometals, 2009,22,701-710).

Ferner sind in der Literatur auch niedermolekulare Fe-Komplexverbindungen beschrieben. Eine Vielzahl dieser Fe-Komplexverbindungen umfasst Carbonsäuren und Aminosäuren als Liganden. Besonders stehen hier Aspartat (US2009035385) und Citrat (EP308362) als Liganden im Vordergrund. In diesem Zusammenhang werden auch Fe-Komplexverbindungen, die derivatisierte Phenylalanin-Reste als Liganden beinhalten beschrieben (ES2044777).

Im weiteren sind in der Literatur Fe-Komplexverbindungen beschrieben, die aus monomeren Zuckereinheiten oder aus einer Kombination von monomeren und polymeren Einheiten aufgebaut sind (FR19671016).

In der Literatur sind auch Hydroxypyron- und Hydroxypyridon-Fe-Komplexverbindungen beschrieben (EP159194, EP138420, EP107458). Analog hierzu sind auch die entsprechenden 5-Ringsysteme, die Hydroxyfuranon-Fe-Komplexverbindungen beschrieben (WO2006037449).

Ausserdem sind in der Literatur Eisen-Cyclopentadienyl-Komplexverbindungen beschrieben (GB842637).

Im weiteren sind in der Literatur auch β-Ketoamide als Fe-Liganden beschrieben. Die Verbindungen wurden aber nicht als Arzneimittel, inbesondere für eine Behandlung von Eisenmangelzuständen vorgeschlagen oder verwendet. Hierbei handelt es sich um Ketoamidstruktureinheiten, die Bestandteil von Siderophoren sind (JACS, 2008, 130, 2124-2125). Weiterhin wurden hexadentate Ferric aerobactin Komplexverbindungen (Inorg.chem. 2006, 45, 6028-6033) beschrieben. Außerdem sind in der Literatur (Inorg. chem. 1990, 29, 4096-9) tripodale Fe-Liganden mit ß-Ketoamidstruktureinheiten offenbart.

Im weiteren sind in der Literatur β-Ketoamide als Fe-Liganden beschrieben, die aromatische Reste am Amid oder Keton tragen und als Fe(III)-Komplexe vorliegen (Journal of the Chemical Society of Pakistan, 1991,13,79-83; Indian Journal of Chemistry, 1981,20A,372-4; Journal of Inorganic and Nuclear Chemistry, 1973, 35, 1397-400; Can J Chem,1969,47, 1693-6; Indian Journal of Chemistry, 1968, 6, 516-20).

Ausserdem sind in der Literatur β-Ketoamide als Fe(II)-Liganden beschrieben, die Pyridin-Reste (Journal of Inorganic and Nuclear Chemistry, 1967, 29, 2484-6.), Benzyloxazolidinone oder substituierte Sultam-Reste (Polyhedron, 1995, 14, 1397-9) beinhalten.

Die US 2005/0192315 beschreibt Salze von Chinolin-Verbindungen, die gegenüber den Neutralformen der Chinolin-Verbindungen stabilisiert sind (vgl. Abschnitte 2 und 3). Unter den Salzen findet sich das Eisen(III)salz von N-Ethyl-N-phenyl-5-chlor-1,2-dihydro-4-hydroxy-1-methyl-2-oxo-3-chinolincarboxamid. In dem Dokument ist auch die Rede von pharmazeutischen Zusammensetzungen. Allerdings wird im gesamten Dokument keine einzige konkrete medizinische Indikation erwähnt, geschweige denn durch Wirkdaten belegt. Eine medizinische Verwendung von Eisen(III)-ß-ketoamid-Komplexverbindungen wird in diesem Dokument daher nicht gelehrt. Soweit eine medizinische Verwendung des Eisen(III)salzes von N-Ethyl-N-phenyl-5-chlor-1,2-dihydro-4-hydroxy-1-methyl-2-oxo-3-chinolincarboxamid dennoch gelehrt sein sollte, wird dieses von der vorliegenden Erfindung ausgenommen. In Syamal, A.: "Ferric benzoylacetanilides" Canadian Journal of Chemistry, 47 (10), 1969, 1693-6 werden lediglich die Synthese und spektroskopischen Eigenschaften einzelner Benzoylacetanilid-Eisenkomplexverbindungen beschrieben.

In keiner der im vorstehenden Text zitierten Literaturstellen wird die Verwendung dieser Komplexe zur Behandlung und zur Prophylaxe von Eisenmangelerscheinungen und Eisenmangelanämien beschrieben.

Ein andere wichtiger Bestandteil für die Behandlung von Eisenmangelerscheinungen und Eisenmangelanämien stellen die Eisensalze (z.B. Eisen(II)sulfat, Eisen(II)fumarat, Eisen(III)chlorid, Eisen(II)aspartat, Eisen(II)succinat) dar.

Ein grosses Problem dieser Eisensalze stellt ihre teilweise hohe Unverträglichkeit (bis zu 50%) in Form von Übelkeit, Erbrechen, Durchfall aber auch Obstipation und Krämpfe dar. Ausserdem kommt es bei der Verwendung von diesen Eisen(II)-salzen zum Auftreten freier Fe(II)Ionen, die die Bildung (u.a. Fenton Reaktion) von reaktiven Sauerstoff Spezies (ROS) katalysieren. Durch diese ROS kommt es zur Beschädigung von DNA, Lipiden, Proteinen und Kohlenhydraten, was weitreichende Auswirkungen auf Zellen, Gewebe und Organe hat. Diese Problematik ist bekannt und wird in der Literatur weitgehend als Ursache für die hohe Unverträglichkeit angesehen und als Oxidativer Stress bezeichnet.

**Aufgabenstellung:**

Die Aufgabe der vorliegenden Erfindung bestand darin, neue therapeutisch wirksame Verbindungen zu entwickeln, die für eine effektive Therapie zur bevorzugt oralen Behandlung von Eisenmangelerscheinungen und Eisenmangelanämien eingesetzt werden können. Dabei sollten diese Eisenkomplexe gleichzeitig deutlich weniger Nebenwirkungen aufzeigen, als die klassisch verwendeten Fe(II)-Salze. Ausserdem sollten diese Eisenkomplexe im Unterschied zu den bekannten polymeren Eisenkomplexverbindungen möglichst eine definierte Struktur (Stöchiometrie) aufweisen und durch einfache Synthesewege darstellbar sein. Dieses Ziel wurde durch die Entwicklung neuartiger Fe(III)-Komplexverbindungen erreicht.

Weiterhin sollten die neuen Eisenkomplexe so beschaffen sein, das sie direkt über die Membran in die Darmzellen aufgenommen werden, um somit ihr komplexgebundenes Eisen direkt an das Ferritin oder an das Transferrin abzugeben oder direkt als intakter Komplex in die Blutbahn zu gelangen. Diese neuen Komplexe sollten aufgrund ihrer Eigenschaften praktisch nicht zum Auftreten von hohen Konzentrationen an freien Eisenionen führen. Denn gerade durch freie Eisenionen kommt es zum Auftreten von ROS, die letztlich für die auftretenden Nebenwirkungen verantwortlich sind.

Um diese Anforderungen erreichen zu können, entwickelten die Erfinder neue Fe(III)-Komplexverbindungen mit nicht zu grossem Molekulargewicht, mittlerer Lipophilie und optimaler Komplexstabilität.

**Beschreibung der Erfindung:**

Die Erfinder fanden, dass Fe(III)-Komplexverbindungen mit β-Ketoamidliganden sich für die oben beschriebenen Anforderungen besonders eignen. Es konnte gezeigt werden, dass diese Fe-Komplexverbindungen eine hohe Eisenaufnahme zeigen, wodurch ein schneller Therapieerfolg bei der Behandlung der Eisenmangelanämie erreicht werden kann. Gerade im Vergleich mit Eisensalzen zeigen die erfindungsgemäßen Komplexverbindungen eine schnellere und höhere Utilisation. Ausserdem weisen diese neuen Systeme deutlich geringere Nebenwirkungen als die klassisch verwendeten Eisensalze auf, da es hier nicht in nennenswertem Ausmaß zum Auftreten von freien Eisenionen kommt. Die erfindungsgemäßen Komplexverbindungen zeigen nahezu keinen Oxidativen Stress, da es nicht zur Bildung von freien Radikalen kommt. Somit treten bei diesen Komplexverbindungen deutlich weniger Nebenwirkungen als bei den aus dem Stand der Technik bekannten Fe Salzen auf. Die Komplexverbindungen zeigen in unterschiedlichen pH-Wert-Bereichen gute Stabilitäten. Die Komplexverbindungen lassen sich gut herstellen und eignen sich optimal zur Formulierung von Arzneimitteln, insbesondere zur oralen Verabreichung.

Gegenstand der Erfindung sind somit Eisen(III)-ß-ketoamid-Komplexverbindungen oder deren pharmazeutisch verträglichen Salze zur <u>Behandlung und Prophylaxe von Eisenmangelerscheinungen und Eisenmangelanämien sowie der damit einhergehenden Symptome. Gegenstand der Erfindung sind demnach Eisen(III)-ß-ketoamid-Komplexverbindungen oder deren pharmazeutisch verträglichen Salze zur Anwendung in einem erfindungsgemäßen Verfahren zur therapeutischen Behandlung des menschlichen oder tierischen Körpers.</u>

Die erfindungsgemäß verwendeten Eisen(III)-ß-ketoamid-Komplexverbindungen <u>weisen das folgende</u> Strukturelement auf:

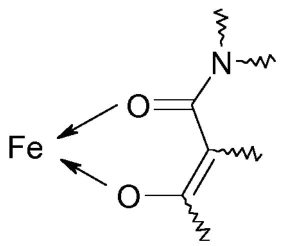

bzw. der mesomeren Grenzformel:

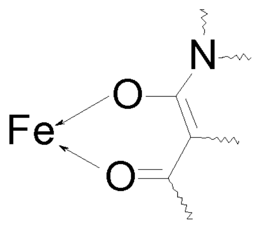

worin 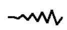 jeweils ein die freie Valenz absättigender Substituent ist und die Pfeile jeweils koordinative Bindungen darstellen. Ein ß-Ketoamid-Ligand trägt somit formal eine negative Ladung und das Eisen eine positive Ladung (d.h., bei drei ß-Ketoamid-Liganden besitzt das Eisen formal die Oxidationsstufe +3). Es ist für den Fachmann weiterhin klar, dass es im ß-Ketoamid-Ligand zu einer Delokalisierung der Elektronen kommt.

Erfindungsgemäß sind auch Eisen(III)-ß-ketoamid-Komplexverbindungen umfasst, bei denen der ß-Ketoamid-Ligand eine Brücke zwischen verschiedenen Eisenatomen bildet:

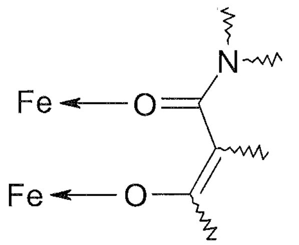

.

Erfindungsgemäß sind insbesondere zweizähnige ß-Ketoamid-Liganden bevorzugt, bei welchen die Bindung zum Eisenatom über die beiden Sauerstoffatome der ß-Keto-Struktureinheit erfolgt. Höherzähnige ß-Ketoamid-Liganden wie drei-, vier-, fünf- oder gar sechs-zähnige ß-Ketoamid-Liganden sind erfindungsgemäß zwar umfasst, jedoch aufgrund ihrer hohen Komplexstabilität (Chelateffekt) weniger bevorzugt, da das Eisen im Körper aufgrund der zu hohen Komplexstabilitäten unter Umständen nicht ausreichend freigesetzt wird. Höherzähnige ß-Ketoamid-Liganden sind insbesondere solche, die neben den beiden Sauerstoffatomen der ß-Ketoamidstruktur weitere funktionelle, koordinierende Gruppen aufweisen, welche etwa in den unten stehend noch erläuterten Substituentengruppen $R_1$ bis $R_4$ vorliegen. Dabei kann es sich beispielsweise um Sauerstoff- oder Stickstoff-haltige funktionelle Gruppen, wie Hydroxy, Amino oder dergleichen handeln.

Die erfindungsgemäßen Eisen(III)-ß-ketoamid-Komplexverbindungen schließen insbesondere solche Komplexverbindungen ein, die mindestens einen, bevorzugt zweizähnigen ß-Ketoamid-Liganden aufweisen, der, wie vorstehend gezeigt, an ein oder zwei verschiedene Eisenatome gebunden ist.

Bevorzugt sind Eisen(III)-ß-ketoamid-Komplexverbindungen, die ausschließlich, bevorzugt zweizähnige ß-Ketoamid-Liganden aufweisen, welche gleich oder verschieden sein können.

Besonders bevorzugt sind weiterhin Eisen(III)-ß-ketoamid-Komplexverbindungen, die ausschließlich die gleichen, bevorzugt zweizähnigen ß-Ketoamid-Liganden aufweisen.

Erfindungsgemäß umfasst sind jedoch auch solche Komplexverbindungen, die neben dem ß-Ketoamid-Liganden bevorzugt einen oder mehrere (wie zwei oder drei) weitere gleiche oder verschiedene ein- oder mehrzähnige Liganden aufweisen, wie zum Beispiel Carbonsäure- bzw. Carboxylat-Liganden (R-COOH bzw. $RCOO^-$), Alkohol-Liganden (R-OH), wie Kohlenhydrat-Liganden, primäre oder sekundäre Amino-Liganden ($R\text{-}NH_2$, R-NHR), Imino-Liganden (R=NH), Oximo-Liganden (R=N-OH), Hydroxyl-Liganden (OH oder $H_2O$), Ether-Liganden, oder Halogen-Liganden. Solche Komplexverbindungen können auch intermediär beim Abbau im Körper also insbesondere in wässriger Lösung auftreten und gegebenenfalls dann auch intermediär koordinativ ungesättigt vorliegen.

In den erfindungsgemäßen Eisen(III)-ß-ketoamid-Komplexverbindungen beträgt die Koordinationszahl der Eisenatome im Allgemeinen sechs (6), wobei im Allgemeinen eine oktaedrische Anordnung der koordinierenden Atome vorliegt.

Erfindungsgemäß sind weiterhin ein- oder mehrkernige Eisen(III)-ß-ketoamid-Komplexverbindungen umfasst, in denen ein oder mehrere (wie 2, 3 oder 4) Eisenatome vorhanden sind. Bevorzugt sind jedoch einkernige Eisen(III)-ß-ketoamid-Komplexverbindungen, in denen ein zentrales Eisenatom vorliegt.

Im allgemeinen können 1-4 Eisenatome und 2-10 Liganden in den Eisen(III)-ß-ketoamid-Komplexverbindungen vorliegen. Bevorzugt sind einkernige Eisen(III)-ß-ketoamid-Komplexverbindungen mit mindestens einem bevorzugt 3, bevorzugt zweizähnigen ß-Ketoamid-Liganden.

Die erfindungsgemäßen Eisen(III)-ß-ketoamid-Komplexverbindungen liegen im Allgemeinen in neutraler Form vor. Es sind jedoch auch salzartige Eisen(III)-ß-ketoamid-Komplexverbindungen eingeschlossen, in denen der Komplex eine positive oder negative Ladung aufweist, die insbesondere durch pharmakologisch verträgliche, im wesentlichen nicht-koordinierende Anionen (wie insbesondere Halogenide, wie Chlorid) oder Kationen (wie insbesondere Alkali- oder Erdalkalimetallionen) ausgeglichen wird. Die erfindungsgemässen Eisen(III)-Komplexverbindungen enthalten mindestens einen Liganden der Formel (I):

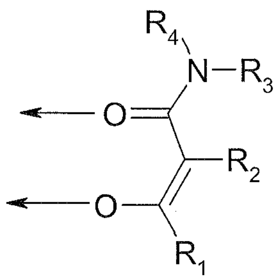

(I)

worin

die Pfeile jeweils eine koordinative Bindung zu einem oder verschiedenen Eisenatomen darstellen,

$R_1$ ausgewählt wird aus der Gruppe, die besteht aus gegebenenfalls substituiertem Alkyl und gegebenenfalls substituiertem Alkoxycarbonyl,

$R_2$ ausgewählt wird aus der Gruppe, die besteht aus Wasserstoff, gegebenenfalls substituiertem Alkyl, Halogen und Cyano, oder

$R_1$ und $R_2$ gemeinsam mit den Kohlenstoffatomen, an die sie gebunden sind, einen gegebenenfalls substituierten 5- oder 6-gliedrigen Ring bilden, der gegebenenfalls ein oder mehrere Heteroatome aufweisen kann,

$R_3$ und $R_4$ gleich oder verschieden sind, und jeweils ausgewählt werden aus der Gruppe, die besteht aus Wasserstoff, gegebenenfalls substituiertem Amino und gegebenenfalls substituiertem Alkyl, oder

$R_3$ und $R_4$ gemeinsam mit dem Stickstoffatom, an das sie gebunden sind, einen gegebenenfalls substituierten 3- bis 6-gliedrigen Ring bilden, der gegebenenfalls ein oder mehrere weitere Heteroatome aufweisen kann,

oder

$R_2$ und $R_3$ gemeinsam einen gesättigten oder ungesättigten gegebenenfalls substituierten 5- oder 6-gliedrigen Ring unter Bildung eines Liganden der Formel (Ia) bilden:

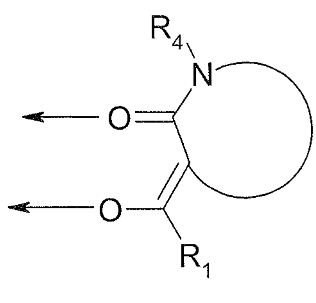

(Ia)

worin $R_1$ und $R_4$ wie zuvor definiert sind,

oder
$R_2$ und $R_3$ gemeinsam einen gesättigten oder ungesättigten gegebenenfalls substituierten 5- oder 6-gliedrigen Ring und $R_1$ und $R_2$ gemeinsam einen gesättigten oder ungesättigten gegebenenfalls substituierten 5- oder 6-gliedrigen Ring unter Bildung eines Liganden der Formel (Ib) bilden:

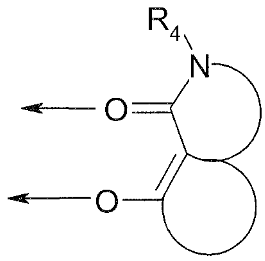

(Ib),

worin $R_4$ wie zuvor definiert ist,
oder pharmazeutisch verträgliche Salze davon.

Besonders bevorzugt sind erfindungsgemäß weiterhin Eisen(III)-Komplexverbindungen enthaltend mindestens einen Liganden der Formel (I):

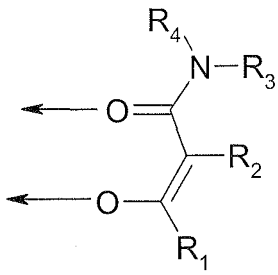

(I)

worin

die Pfeile jeweils eine koordinative Bindung zu einem oder verschiedenen Eisenatomen darstellen,
$R_1$ gegebenenfalls substituiertes Alkyl ist,
$R_2$ ausgewählt wird aus der Gruppe, die besteht aus Wasserstoff, gegebenenfalls substituiertem Alkyl, Halogen und Cyano, oder
$R_1$ und $R_2$ gemeinsam mit den Kohlenstoffatomen, an die sie gebunden sind, einen gegebenenfalls substituierten 5- oder 6-gliedrigen Ring bilden, der gegebenenfalls ein oder mehrere Heteroatome aufweisen kann,
$R_3$ und $R_4$ gleich oder verschieden sind, und jeweils ausgewählt werden aus der Gruppe, die besteht aus Wasserstoff und gegebenenfalls substituiertem Alkyl, oder
$R_3$ und $R_4$ gemeinsam mit dem Stickstoffatom, an das sie gebunden sind, einen gegebenenfalls substituierten 3- bis 6-gliedrigen Ring bilden, der gegebenenfalls ein oder mehrere weitere Heteroatome aufweisen kann,
oder in einer weiteren bevorzugten Ausführungsform:

$R_2$ und $R_3$ gemeinsam einen gesättigten oder ungesättigten gegebenenfalls substituierten 5- oder 6-gliedrigen Ring unter Bildung eines Liganden der Formel (Ia) bilden:

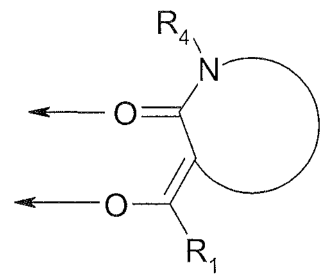

(Ia)

worin $R_1$ und $R_4$ wie zuvor definiert sind,

oder in einer weiteren bevorzugten Ausführungsform:

$R_2$ und $R_3$ gemeinsam einen gesättigten oder ungesättigten gegebenenfalls substituierten 5- oder 6-gliedrigen Ring und $R_1$ und $R_2$ gemeinsam einen gesättigten oder ungesättigten gegebenenfalls substituierten 5- oder 6-gliedrigen Ring unter Bildung eines Liganden der Formel (Ib) bilden:

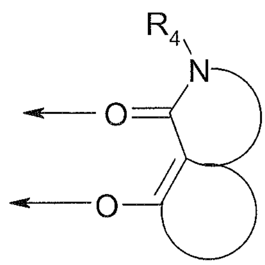

(Ib),

worin $R_4$ wie zuvor definiert ist,

oder pharmazeutisch verträgliche Salze davon.

In einer bevorzugten Ausführungsform der Erfindung betrifft diese Eisen(III)-Komplexverbindungen enthaltend mindestens einen Liganden der Formel (I):

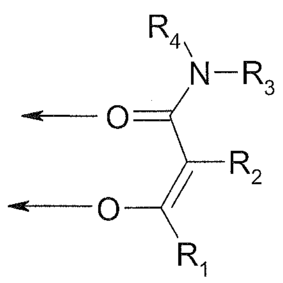

(I)

worin

die Pfeile jeweils eine koordinative Bindung zu einem oder verschiedenen Eisenatomen darstellen,
$R_1$ ausgewählt wird aus der Gruppe, die besteht aus gegebenenfalls substituiertem Alkyl und gegebenenfalls substituiertem Alkoxycarbonyl, bevorzugt gegebenenfalls substituiertes Alkyl ist,
$R_2$ ausgewählt wird aus der Gruppe, die besteht aus Wasserstoff, gegebenenfalls substituiertem Alkyl, Halogen und Cyano, oder
$R_1$ und $R_2$ gemeinsam mit den Kohlenstoffatomen, an die sie gebunden sind, einen gegebenenfalls substituierten 5- oder 6-gliedrigen Ring bilden, der gegebenenfalls ein oder mehrere Heteroatome aufweisen kann,
$R_3$ und $R_4$ gleich oder verschieden sind, und jeweils ausgewählt werden aus der Gruppe, die besteht aus Wasserstoff,

gegebenenfalls substituiertem Amino und gegebenenfalls substituiertem Alkyl, bevorzugt Wasserstoff und gegebenenfalls substituiertem Alkyl, oder
$R_3$ und $R_4$ gemeinsam mit dem Stickstoffatom, an das sie gebunden sind, einen gegebenenfalls substituierten 3- bis 6-gliedrigen Ring bilden, der gegebenenfalls ein oder mehrere weitere Heteroatome aufweisen kann.

Dabei schließt gegebenenfalls substituiertes Alkyl im Rahmen der gesamten Erfindung insbesondere für die Substituenten $R_1$ bis $R_4$ bevorzugt ein: Geradkettiges oder verzweigtes Alkyl mit 1 bis 8, bevorzugt 1 bis 6, Kohlenstoffatomen, Cycloalkyl mit 3 bis 8, bevorzugt 5 oder 6 Kohlenstoffatomen, oder Alkyl mit 1 bis 4 Kohlenstoffatomen, welches mit Cycloalkyl substituiert ist, wobei diese Alkylgruppen gegebenenfalls substituiert sein können.

Die genannten Alkylgruppen können gegebenenfalls jeweils bevorzugt 1 bis 3 Substituenten tragen.

Diese Substituenten werden bevorzugt aus der Gruppe ausgewählt, die besteht aus: Hydroxy, gegebenenfalls substituiertem Aryl, insbesondere wie nachstehend definiert, gegebenenfalls substituiertem Heteroaryl, insbesondere wie nachstehend definiert, gegebenenfalls substituiertem Alkoxy insbesondere wie nachstehend definiert, gegebenenfalls substituiertem Alkoxycarbonyl, insbesondere wie nachstehend definiert, gegebenenfalls substituiertem Acyl, insbesondere wie nachstehend definiert, Halogen, insbesondere wie nachstehend definiert, gegebenenfalls substituierten Amino, insbesondere wie nachstehend definiert, gegebenenfalls substituierten Aminocarbonyl, insbesondere wie nachstehend definiert, und Cyano.

Eisen(III)-Komplexverbindungen, in denen $R_1$, $R_2$, $R_3$ und/oder $R_4$ Aryl- oder Heteroaryl-substituierte Alkylgruppen darstellen, sind erfindungsgemäß weniger bevorzugt.

Halogen schließt hier und im Rahmen der vorliegenden Erfindung Fluor, Chlor, Brom und Iod, bevorzugt Fluor oder Chlor, ein.

In den vorstehend definierten Alkylgruppen können des weiteren gegebenenfalls ein oder mehrere, bevorzugter 1 bis 3 Kohlenstoffatome durch heteroanaloge Gruppen, die Stickstoff, Sauerstoff oder Schwefel enthalten, ersetzt sein. Dies bedeutet insbesondere, dass beispielsweise eine oder mehrere, bevorzugt 1 bis 3, noch bevorzugter eine (1) Methylengruppe ($-CH_2-$) in den Alkylresten durch -NH-, $-NR_5-$, -O- oder -S- ersetzt sein können, worin $R_5$ gegebenenfalls substituiertes Alkyl, wie vorstehend definiert, bevorzugt gegebenenfalls mit 1 bis 3 Substituenten, wie Fluor, Chlor, Hydroxy, Alkoxy, substituiertes C1-C6 Alkyl, wie Methyl oder Ethyl ist.

Beispiele von Alkylresten mit 1 bis 8 Kohlenstoffatomen schließen ein: eine Methylgruppe, eine Ethylgruppe, eine n-Propylgruppe, eine i-Propylgruppe, eine n-Butylgruppe, eine i-Butylgruppe, eine sec-Butylgruppe, eine t-Butylgruppe, eine n-Pentylgruppe, eine i-Pentylgruppe, eine sec-Pentylgruppe, eine t-Pentylgruppe, eine 2-Methylbutylgruppe, eine n-Hexylgruppe, eine 1-Methylpentylgruppe, eine 2-Methylpentylgruppe, eine 3-Methylpentylgruppe, eine 4-Methylpentylgruppe, eine 1-Ethylbutylgruppe, eine 2-Ethylbutylgruppe, eine 3-Ethylbutylgruppe, eine 1,1-Dimethylbutylgruppe, eine 2,2-Dimethylbutylgruppe, eine 3,3-Dimethylbutylgruppe, eine 1-Ethyl-1-methylpropylgruppe, eine n-Heptylgruppe, eine 1-Methylhexylgruppe, eine 2-Methylhexylgruppe, eine 3-Methylhexylgruppe, eine 4-Methylhexylgruppe, eine 5-Methylhexylgruppe, eine 1-Ethylpentylgruppe, eine 2-Ethylpentylgruppe, eine 3-Ethylpentylgruppe, eine 4-Ethylpentylgruppe, eine 1,1-Dimethylpentylgruppe, eine 2,2-Dimethylpentylgruppe, eine 3,3-Dimethylpentylgruppe, eine 4,4-Dimethylpentylgruppe, eine 1-Propylbutylgruppe, eine n-Octylgruppe, eine 1-Methylheptylgruppe, eine 2-Methylheptylgruppe, eine 3-Methylheptylgruppe, eine 4-Methylheptylgruppe, eine 5-Methylheptylgruppe, eine 6-Methylheptylgruppe, eine 1-Ethylhexylgruppe, eine 2-Ethylhexylgruppe, eine 3-Ethylhexylgruppe, eine 4-Ethylhexylgruppe, eine 5-Ethylhexylgruppe, eine 1,1-Dimethylhexylgruppe, eine 2,2-Dimethylhexylgruppe, eine 3,3-Dimethylhexylgruppe, eine 4,4-Dimethylhexylgruppe, eine 5,5-Dimethylhexylgruppe, eine 1-Propylpentylgruppe, eine 2-Propylpentylgruppe, usw. Bevorzugt sind solche mit 1 bis 6 Kohlenstoffatomen. Am meisten bevorzugt sind Methyl, Ethyl, n-Propyl und n-Butyl.

Beispiele von Alkylgruppen, die durch Austausch mit einer oder mehreren heteroanalogen Gruppe, wie -O-, -S-, -NH- oder $-N(R_5)-$ hervorgehen, sind bevorzugt solche, in denen eine oder mehrere Methylengruppen ($-CH_2-$) durch -O- unter Bildung einer Ethergruppe ersetzt sind, wie Methoxymethyl, Ethoxymethyl, 2-Methoxyethyl usw. Daher schließt die Definition von Alkyl auch beispielsweise Alkoxyalkylgruppen, wie nachstehend definiert, ein, welche durch Austausch einer Methylengruppe durch -O- aus den genannten Alkylgruppen hervorgehen. Sind erfindungsgemäß darüber hinaus Alkoxygruppen als Substituenten von Alkyl zugelassen, können auch auf diese Weise mehrere Ethergruppen gebildet werden (wie zum Beispiel eine $-CH_2-O-CH_2-OCH_3$-Gruppe). Erfindungsgemäß sind somit auch Polyethergruppen von der Definition von Alkyl umfasst.

Beispiele von Thio-haltigen Alkyl-Resten, insbesondere als $R_4$ sind:

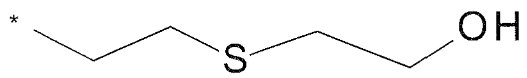

(*= Bindungsstelle)

Cycloalkylreste mit 3 bis 8 Kohlenstoffatomen schließen bevorzugt ein: eine Cyclopropylgruppe, eine Cyclobutylgruppe, eine Cyclopentylgruppe, eine Cyclohexylgruppe, eine Cycloheptylgruppe, eine Cyclooctylgruppe usw. ein. Bevorzugt sind eine Cyclopropylgruppe, eine Cyclobutylgruppe, eine Cyclopentylgruppe und eine Cyclohexylgruppe. Die Cycloalkylreste können gegebenenfalls substituiert sein, bevorzugt mit 1 bis 2 Substituenten, wie Hydroxy, wie beispielsweise im Falle des 4-Hydroxycyclohexyls, oder C1-C6-Alkoxycarbonyl, wie beispielsweise im Falle der Reste:

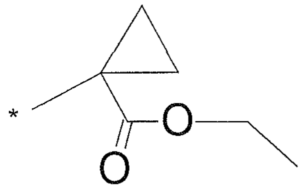

(*=Bindungstelle),

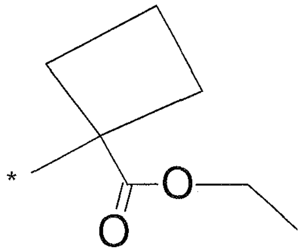

(*=Bindungstelle),

oder

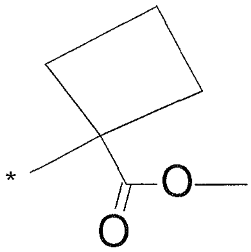

(*=Bindungstelle),

Die Definition der gegebenenfalls substituierten Alkylgruppen schließt auch Alkylgruppen ein, welche durch vorstehend definierte Cycloalkylgruppen substituiert sind, wie beispielsweise Cyclopropylmethyl, Cyclobutylmethyl, Cyclopentylmethyl oder Cyclohexylmethyl.

Heterocyclische Alkylreste sind erfindungsgemäß insbesondere solche, die durch Austausch von Methylen durch Heteroanaloge Gruppen aus Cycloalkyl gebildet werden, und schließen beispielsweise gesättigte 5- oder 6-gliedrige heterocyclische Reste ein, welche über ein Kohlenstoffatom oder ein Stickstoffatom angebunden sein können, und welche bevorzugt 1 bis 3, bevorzugt 2 Heteroatome wie insbesondere O, N aufweisen können, wie Tetrahydrofuryl, Azetidin-1-yl, substituiertes Azetidinyl, wie 3-Hydroxyazetidin-1-yl, Pyrrolidinyl, wie Pyrrolidin-1-yl, substituiertes Pyrrolidinyl, wie 3-Hydroxypyrrolidin-1-yl, 2-Hydroxypyrrolidin-1-yl, 2-Methoxycarbonylpyrrolidin-1-yl, 2-Ethoxycarbonylpyrrolidin-1-yl, 2-Methoxypyrrolidin-1-yl, 2-Ethoxypyrrolidin-1-yl, 3-Methoxycarbonylpyrrolidin-1-yl, 3-Ethoxycarbonylpyrrolidin-1-yl, 3-Methoxypyrrolidin-1-yl, 3-Ethoxypyrrolidin-1-yl, Piperidinyl, wie Piperidin-1-yl, Piperidin-4-yl, substituiertes Piperidinyl, wie 4-Methyl-1-piperidyl, 4-Hydroxy-1-piperidyl, 4-Methoxy-1-piperidyl, 4-Ethoxy-1-piperidyl, 4-Methoxycarbonyl-1-piperidyl, 4-Ethoxycarbonyl-1-piperidyl, 4-Carboxy-1-piperidyl, 4-Acetyl-1-piperidyl, 4-Formyl-1-piperidyl, 1-Methyl-4-piperidyl, 4-Hydroxy-2,2,6,6-tetramethyl-1-piperidyl, 4-(Dimethylamino)-1-piperidyl, 4-(Diethylamino)-1-piperidyl, 4-Amino-1-piperidyl, 2-(Hydroxymethyl)-1-piperidyl, 3-(Hydroxymethyl)-1-piperidyl, 4-(Hydroxymethyl)-1-piperidyl, 2-Hydroxy-1-piperidyl, 3-Hydroxy-1-piperidyl, 4-Hydroxy-1-piperidyl, Morpholin-4-yl, substituiertes Morpholinyl, wie 2,6-Dimethylmorpholin-4-yl, Piperazinyl, wie Piperazin-1-yl, substituiertes Piperazinyl, wie 4-Methylpiperazin-1-yl, 4-Ethylpiperazin-1-yl, 4-Ethoxycarbonylpiperazin-1-yl, 4-Methoxycarbonylpiperazin-1-yl, oder Tetrahydropyranyl, wie Tetrahydropyran-4-yl, und welche gegebenenfalls mit aromatischen Ringen kondensiert sein können, und welche gegebenenfalls substituiert sein können, wie mit 1 bis 2 Substituenten, wie Hydroxy, Halogen, C1-C6-Alkyl etc. Die Definition der gegebenenfalls substituierten Alkylgruppen schließt somit auch Alkylgruppen ein, welche durch vorstehend definierte heterocyclische Gruppen substituiert sind, wie beispielsweise 3-(1-Piperidyl)propyl, 3-Pyrrolidin-1-ylpropyl, 3-Morpholinopropyl, 2-Morpholinoethyl, 2-Tetrahydropyran-4-ylethyl, 3-Tetrahydropyran-4-ylpropyl, 3-(Azetidin-1-yl)propyl etc..

Beispiele eines mit Halogen substituierten linearen oder verzweigten Alkylrestes mit 1 bis 8 Kohlenstoffatomen schließen insbesondere ein:

eine Fluormethylgruppe, eine Difluormethylgruppe, eine Trifluormethylgruppe, eine Chlormethylgruppe, eine Dichlormethylgruppe, eine Trichlormethylgruppe, eine Brommethylgruppe, eine Dibrommethylgruppe, eine Tribrommethylgruppe, eine 1-Fluorethylgruppe, eine 1-Chlorethylgruppe, eine 1-Bromethylgruppe, eine 2-Fluorethylgruppe, eine 2-Chlorethylgruppe, eine 2-Bromethylgruppe, eine 1,2-Difluorethylgruppe, eine 1,2-Dichlorethylgruppe, eine 1,2-Dibromethylgruppe, eine 2,2,2-Trifluorethylgruppe, eine Heptafluorethylgruppe, eine 1-Fluorpropylgruppe, eine 1-Chlorpropylgruppe, eine 1-Brompropylgruppe, eine 2-Fluorpropylgruppe, eine 2-Chlorpropylgruppe, eine 2-Brompropylgruppe, eine 3-Fluorpropylgruppe, eine 3-Chlorpropylgruppe, eine 3-Brompropylgruppe, eine 1,2-Difluorpropylgruppe, eine 1,2-Dichlorpropylgruppe, eine 1,2-Dibrompropylgruppe, eine 2,3-Difluorpropylgruppe, eine 2,3-Dichlorpropylgruppe, eine 2,3-Dibrompropylgruppe, eine 3,3,3-Trifluorpropylgruppe, eine 2,2,3,3,3-Pentafluorpropylgruppe, eine 2-Fluorbutylgruppe, eine 2-Chlorbutylgruppe, eine 2-Brombutylgruppe, eine 4-Fluorbutylgruppe, eine 4-Chlorbutylgruppe, eine 4-Brombutylgruppe, eine 4,4,4-Trifluorbutylgruppe, eine 2,2,3,3,4,4,4-Heptafluorbutylgruppe, eine Perfluorbutylgruppe, eine 2-Fluorpentylgruppe, eine 2-Chlorpentylgruppe, eine 2-Brompentylgruppe, eine 5-Fluorpentylgruppe, eine 5-Chlorpentylgruppe, eine 5-Brompentylgruppe, eine Perfluorpentylgruppe, eine 2-Fluorhexylgruppe, eine 2-Chlorhexylgruppe, eine 2-Bromhexylgruppe, eine 6-Fluorhexylgruppe, eine 6-Chlorhexylgruppe, eine 6-Bromhexylgruppe, eine Perfluorhexylgruppe, eine 2-Fluorheptylgruppe, eine 2-Chlorheptylgruppe, eine 2-Bromheptylgruppe, eine 7-Fluorheptylgruppe, eine 7-Chlorheptylgruppe, eine 7-Bromheptylgruppe, eine Perfluorheptylgruppe, usw.

Beispiele eines mit Hydroxy substituierten Alkylrestes schließen die oben genannten Alkylreste ein, die 1 bis 3 Hydroxylreste aufweisen, wie zum Beispiel Hydroxymethyl, 2-Hydroxyethyl, 3-Hydroxypropyl, 4-Hydroxybutyl, 5-Hydroxypentyl, 6-Hydroxyhexyl, etc. und die gegebenenfalls auch weitere Substituenten, wie Alkoxycarbonyl, oder Heteroatome, wie Schwefel, aufweisen können, wie zum Beispiel:

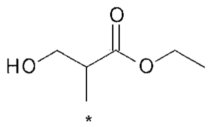

(* = Bindungstelle),

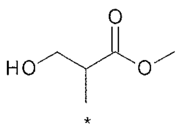

(* = Bindungstelle),

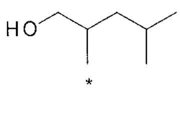

(* = Bindungstelle),

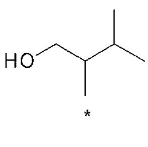

(* = Bindungstelle),

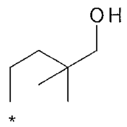

(* = Bindungstelle),

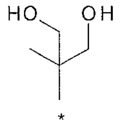

(* = Bindungstelle),

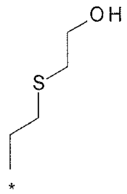

(* = Bindungstelle),

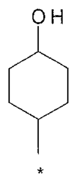

(* = Bindungstelle),

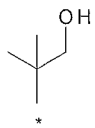

(* = Bindungstelle),

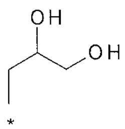

(* = Bindungstelle),

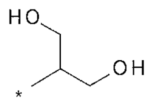

(* = Bindungstelle),

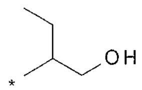

(* = Bindungstelle),

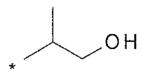

(* = Bindungstelle),

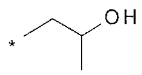

(* = Bindungstelle),

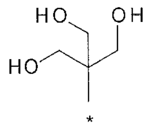

(* = Bindungstelle),

welche sämtlich auch Beispiel für $R_3$ und/oder $R_4$ sind.

Gegebenenfalls substituiertes Aryl schließt erfindungsgemäß bevorzugt aromatische Kohlenwasserstoffreste mit 6 bis 14 Kohlenstoffatomen (ohne Heteroatome im aromatischen Ringsystem) ein, wie beispielweise: Phenyl, Naphthyl, Phenanthrenyl und Anthracenyl. Die genannten aromatischen Reste können gegebenenfalls bevorzugt einen oder mehrere, bevorzugt einen Substituenten aufweisen, insbesondere Halogen, Hydroxy, Alkyl, Alkoxy, jeweils wie vorstehend oder nachstehend erläutert. Bevorzugter aromatischer Rest ist Phenyl. Bevorzugtes mit einer aromatischen Gruppe substituiertes Alkyl (Arylalkyl) ist Benzyl.

Gegebenenfalls substituiertes Aryl schließt erfindungsgemäß weiterhin gegebenenfalls substituiertes Heteroaryl also heteroaromatische Reste ein, wie beispielsweise: Pyridyl, Pyridyl-N-oxid, Pyrimidyl, Pyridazinyl, Pyrazinyl, Thienyl, Furyl, Pyrrolyl, Pyrazolyl, Imidazolyl, Thiazolyl, Oxazolyl oder Isoxazolyl, Indolizinyl, Indolyl, Benzo[b]thienyl, Benzo[b]furyl, Indazolyl, Chinolyl, Isochinolyl, Naphthyridinyl, Chinazolinyl. 5- oder 6-gliedrige aromatische Heterocyclen wie z.B. Pyridyl, Pyridyl-N-oxid, Pyrimidyl, Pyridazinyl, Furyl und Thienyl sind bevorzugt. Die genannten heteroaromatische Reste können bevorzugt einen oder mehrere, bevorzugt einen Substituenten aufweisen, insbesondere Halogen, Hydroxy, Alkyl, Alkoxy, jeweils wie vorstehend oder nachstehend erläutert. Bevorzugte Beispiele einer mit einer heteroaromatischen Gruppe substituiertes Alkyl (Hetarylalkyl) sind Methyl, Ethyl oder Propyl jeweils substituiert mit einer heteroaromatischen Gruppe, wie Thienylmethyl, Pyridylmethyl etc.

Gegebenenfalls substituiertes Alkoxy (RO-) schließt erfindungsgemäß beispielsweise lineare oder verzweigte Alkoxyreste bevorzugt mit bis zu 6 Kohlenstoffatomen ein, wie eine Methoxygruppe, eine Ethoxygruppe, eine n-Propyloxygruppe, eine i-Propyloxygruppe, eine n-Butyloxygruppe, eine i-Butyloxygruppe, eine sec-Butyloxygruppe, eine t-Butyloxygruppe, eine n-Pentyloxygruppe, eine i-Pentyloxygruppe, eine sec-Pentyloxygruppe, eine t-Pentyloxygruppe, eine 2-Methylbutoxygrupe, eine n-Hexyloxygruppe, eine i-Hexyloxygruppe, eine t-Hexyloxygruppe, eine sec-Hexyloxygruppe, eine 2-Methylpentyloxygruppe, eine 3-Methylpentyloxygruppe, eine 1-Ethylbutyloxygruppe, eine 2-Ethylbutyloxygruppe, eine 1,1-Dimethylbutyloxygruppe, eine 2,2-Dimethylbutyloxygruppe, eine 3,3-Dimethylbutyloxygruppe, eine 1-Ethyl-1-methylpropyloxygruppe, usw. ein. Bevorzugt sind eine Methoxygruppe, eine Ethoxygruppe, eine n-Propyloxygruppe, eine i-Propyloxygruppe, eine n-Butyloxygruppe, eine i-Butyloxygruppe, eine sec-Butyloxygruppe, eine t-Butyloxygruppe, usw. Die Alkoxygruppen können gegebenenfalls substituiert sein, wie beispielsweise mit den für Alkyl genannten möglichen Substituenten.

Bevorzugtes Alkoxy ist Methoxy, Ethoxy, n-Propoxy, n-Butoxy etc..

Gegebenenfalls substituierte Alkoxycarbonyl (RO-CO-)-Gruppen leiten sich aus den vorstehend definierten Alkylgruppen formal durch Hinzufügen eines -O-C(O)-Restes ab unter Bildung eines gegebenenfalls substituierten Alkyloxycarbonyl-Restes ab. Insoweit kann auf die Definition der vorstehend beschriebenen Alkylgruppen verwiesen werden. Alternativ leiten sich gegebenenfalls substituierte Alkoxycarbonyl (RO-CO-)-Gruppen aus den vorstehend genannten Alkoxygruppen entsprechend formal durch Hinzufügen einer Carbonylgruppe ab. Bevorzugte Alkoxycarbonyl-Gruppen sind Methoxycarbonyl, Ethoxycarbonyl, n-Propoxycarbonyl, n-Butoxycarbonyl, tert.-Butoxycarbonyl etc., welche sämtlich wie vorstehend definierte Alkylgruppen substituiert sein können.

Gegebenenfalls substituiertes Amino schließt erfindungsgemäß bevorzugt ein: Amino ($-NH_2$), gegebenenfalls substituiertes Mono- oder Dialkylamino (RHN-, $(R)_2N$-) wobei hinsichtlich der Definition von gegebenenfalls substituiertem Alkyl auf vorstehende Definition verwiesen werden kann. Ferner sind gegebenenfalls substituierte Mono- oder Diarylamino-Reste eingeschlossen oder gemischte gegebenenfalls substituierte Alkylarylamino-Reste, wobei hinsichtlich der Definition von gegebenenfalls substituiertem Alkyl oder Aryl auf vorstehende Definition verwiesen werden kann. Derartige Gruppen schließen beispielsweise Methylamino, Dimethylamino, Ethylamino, Hydroxyethylamino, wie 2-Hydroxyethylamino, Diethylamino, Phenylamino, Methylphenylamino etc. ein. Gegebenenfalls substituiertes Amino schließt weiterhin gegebenenfalls substituiertes cyclisches Amino ein, wie gegebenenfalls substituiertes 5 oder 6-gliedriges cyclisches Amino, dass gegebenenfalls weitere Heteroatome wie beispielsweise N, O, S, bevorzugt O enthalten kann. Beispiele von solchen cyclischen Aminogruppen schließen ein die vorstehend genannten stickstoffenthaltenden heterocyclischen Gruppen, die über ein Stickstoffatom angebunden sind, wie Piperidin-1-yl, 4-Hydroxy-piperidin-1-yl, 2-(Methoxycarbonyl)pyrrolidin-1-yl, Pyrrolidin-1-yl, Morpholin-4-yl, etc.

Gegebenenfalls substituiertes Acyl schließt im Rahmen der Erfindung aliphatisches und aromatisches Acyl ein, wobei aliphatisches Acyl insbesondere Formyl sowie gegebenenfalls substituiertes Alkylcarbonyl ist, wobei hinsichtlich der Definition des gegebenenfalls substituierten Alkyls auf die vorstehenden Erläuterungen verwiesen werden kann. Aromatisches Acyl schließt demzufolge gegebenenfalls substituiertes Arylcarbonyl ein, wobei hinsichtlich der Definition des gegebenenfalls substituierten Aryls auf die vorstehenden Erläuterungen verwiesen werden kann. Bevorzugte Acylgruppen stellen erfindungsgemäß beispielsweise dar: Formyl (-C(=O)H), Acetyl, Propionyl, Butanoyl, Pentanoyl, Hexanoyl und jeweils die Isomeren davon, Benzoyl. Substituenten von Acyl schließen die vorstehend für Alkyl und Aryl genannten Substituenten ein, so dass insoweit zu den diesbezüglichen Substituenten verwiesen werden kann.

Gegebenenfalls substituiertes Aminocarbonyl leitet sich im Rahmen der Erfindung formal von vorstehend definiertem gegebenenfalls substituierten Amino durch Hinzufügen eines Carbonylrestes ab ($(R)_2N-C(=O)-$), sodass insoweit auf vorstehende Definition von gegebenenfalls substituiertem Amino verwiesen werden kann. Beispiele schließen demnach ein Carbamoyl ($H_2NC(=O)-$), gegebenenfalls substituiertes Mono- oder Dialkylaminocarbonyl (RHNC(=O)-, $(R)_2NC(=O)-$) wobei hinsichtlich der Definition von gegebenenfalls substituiertem Alkyl auf vorstehende Definition verwiesen werden kann. Ferner sind gegebenenfalls substituierte Mono- oder Diarylaminocarbonyl-Reste eingeschlossen oder gemischte gegebenenfalls substituierte Alkylarylaminocarbonyl-Reste, wobei hinsichtlich der Definition von gegebenenfalls substituiertem Alkyl oder Aryl auf vorstehende Definition verwiesen werden kann. Derartige Gruppen schließen beispielsweise Methylaminocarbonyl, Dimethylaminocarbonyl, Ethylaminocarbonyl, Diethylaminocarbonyl, Phenylaminocarbonyl, Methylphenylaminocarbonyl etc. ein.

Erfindungsgemäß bevorzugt sind Eisen(III)-Komplexverbindungen, enthaltend mindestens einen Liganden der Formel (I):

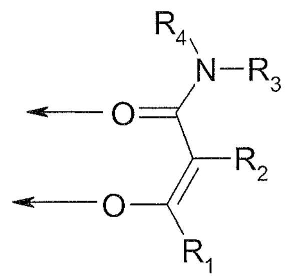

(I)

worin

die Pfeile jeweils eine koordinative Bindung zu einem oder verschiedenen Eisenatomen darstellen,

$R_1$ Alkyl ist, dass gegebenenfalls durch 1 bis 3 Substituenten substituiert sein kann, die aus der Gruppe ausgewählt sind, die besteht aus Hydroxy, Alkoxy, wie vorstehend definiert, wie insbesondere Methoxy, Ethoxy, Halogen, Cyano, Alkoxycarbonyl, wie vorstehend definiert, wie insbesondere Methoxycarbonyl, Ethoxycarbonyl, und Aminocarbonyl, wie vorstehend definiert, wie insbesondere Carbamoyl, Dimethylaminocarbonyl, oder
$R_1$ Alkoxycarbonyl ist, dass gegebenenfalls durch 1 bis 3 Substituenten substituiert sein kann, die aus der Gruppe ausgewählt sind, die besteht aus Hydroxy, C1-C6-Alkoxy und Halogen, wie insbesondere Methoxycarbonyl, Ethoxycarbonyl, Propoxycarbonyl etc.
$R_2$ ausgewählt wird aus der Gruppe, die besteht aus

- Wasserstoff,
- Alkyl, dass gegebenenfalls durch 1 bis 3 Substituenten substituiert sein kann, die aus der Gruppe ausgewählt sind, die besteht aus Hydroxy, Alkoxy, Halogen, Cyano und Alkoxycarbonyl,
- Halogen, wie Chlor, Fluor, besonders bevorzugt Fluor und
- Cyano, oder

$R_1$ und $R_2$ gemeinsam mit den Kohlenstoffatomen, an die sie gebunden sind, einen gegebenenfalls substituierten 5- oder 6-gliedrigen Ring bilden, wie einen Cyclopentanring oder einen Cyclohexanring, der gegebenenfalls auch ein oder mehrere Heteroatome aufweisen kann, und weitere Substituenten tragen kann, wie die für Alkyl genannten,
$R_3$ und $R_4$ gleich oder verschieden sind, und jeweils ausgewählt werden aus der Gruppe, die besteht aus Wasserstoff und Alkyl, dass gegebenenfalls durch 1 bis 3 Substituenten substituiert sein kann, die aus der Gruppe ausgewählt sind, die besteht aus Hydroxy, Alkoxy, Halogen, Cyano, gegebenenfalls substituiertem Amino und Alkoxycarbonyl, und wobei Alkyl ein oder mehrere Heteroatome ausgewählt aus -O- oder -S- anstelle von $-CH_2-$ aufweisen kann, und/oder
$R_3$ und $R_4$ ausgewählt werden aus gegebenenfalls substituiertem Amino, wie vorstehend erwähnt, wie insbesondere Hydroxyethylamino, 4-Morpholinyl, 1-Piperidyl, 4-Hydroxy-1-piperidyl, Piperazin-1-yl, 4-Methylpiperazin-1-yl.

In einer bevorzugten Ausführungsform ist nur einer von $R_3$ oder $R_4$ Wasserstoff.
Oder $R_3$ und $R_4$ bilden gemeinsam mit dem Stickstoffatom, an das sie gebunden sind, einen gegebenenfalls substituierten 3- bis 6-gliedrigen Ring, der gegebenenfalls ein oder mehrere weitere Heteroatome aufweisen kann, wie die vorstehend erwähnten gegebenenfalls substituierten über Stickstoff gebundenen Heterocyclen.
Oder in einer weiteren bevorzugten Ausführungsform bilden
$R_2$ und $R_3$ gemeinsam einen gesättigten oder ungesättigten gegebenenfalls substituierten 5- oder 6-gliedrigen Ring unter Bildung eines Liganden der Formel (Ia):

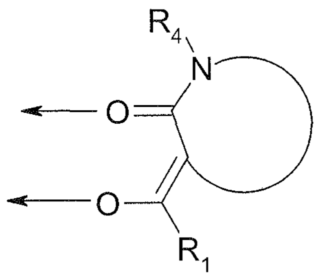

(Ia)

worin $R_1$ und $R_4$ wie zuvor definiert sind, wie beispielsweise in den nachfolgenden Verbindungen realisiert:

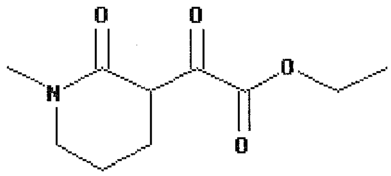

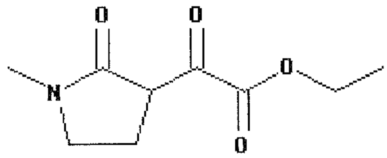

,

wobei die 5- oder 6-gliedrigen Ringsysteme gegebenenfalls substituiert sein können, wie durch ein bis drei Substituenten wie Oxo, Halogen, wie in nachfolgender Bespielverbindung gezeigt:

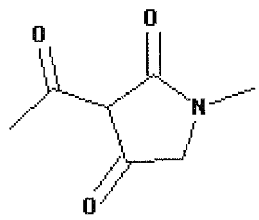

,

welche Oxo als Substituenten trägt, oder in einer weiteren bevorzugten Ausführungsform $R_2$ und $R_3$ gemeinsam einen gesättigten oder ungesättigten gegebenenfalls substituierten 5- oder 6-gliedrigen Ring und $R_1$ und $R_2$ gemeinsam einen gesättigten oder ungesättigten gegebenenfalls substituierten 5- oder 6-gliedrigen Ring unter Bildung eines Liganden der Formel (Ib) bilden:

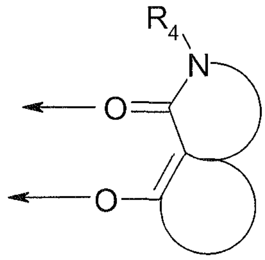

(Ib),

worin $R_4$ wie zuvor definiert ist,
oder pharmazeutisch verträgliche Salze davon.

Besonders bevorzugt sind insbesondere die Eisen(III)-Komplexverbindungen der allgemeinen Formel (II):

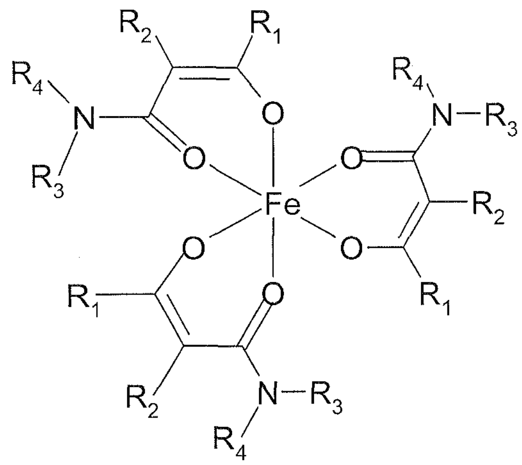

(II)

worin $R_1$, $R_2$, $R_3$ und $R_4$ jeweils wie oben oder bevorzugt wie nachstehend definiert sind.

In einer bevorzugten Ausführungsform der Erfindung wird $R_1$ ausgewählt aus der Gruppe, die besteht aus:

- $C_{1-6}$-Alkyl, bevorzugt wie vorstehend erläutert, gegebenenfalls substituiert mit $C_{1-4}$-Alkoxy, wie vorstehend erläutert, oder mit Dialkylaminocarbonyl, ebenfalls wie vorstehend erläutert,
- $C_{3-6}$-Cycloalkyl, bevorzugt wie vorstehend erläutert,
- $C_{3-6}$-cycloalkyl-$C_{1-4}$-alkyl, bevorzugt wie vorstehend erläutert,
- $C_{1-4}$-Alkoxy-$C_{1-4}$-alkyl, bevorzugt wie vorstehend erläutert.
- Hydroxy-$C_{1-4}$-alkyl, bevorzugt wie vorstehend erläutert, und
- Halogen-$C_{1-4}$-alkyl, bevorzugt wie vorstehend erläutert, oder
- $C_{1-4}$-Alkoxycarbonyl, bevorzugt wie vorstehend erläutert.

Besonders bevorzugt ist $R_1$ $C_{1-6}$-Alkyl, bevorzugt wie vorstehend erläutert, insbesondere Methyl, Ethyl, Propyl,

insbesondere n-Propyl, und Butyl, insbesondere n-Butyl. Am meisten bevorzugt ist $R_1$ Methyl, Ethyl und n-Butyl, welche gegebenenfalls durch $C_{1-6}$-Alkoxy, wie Methoxy oder durch Di-$C_{1-6}$-alkylaminocarbonyl wie Dimethylaminocarbonyl substituiert sind, oder $R_1$ ist $C_{1-4}$-Alkoxycarbonyl, wie insbesondere Ethoxycarbonyl oder Methoxycarbonyl.

In einer bevorzugten Ausführungsform der Erfindung wird $R_2$ ausgewählt aus der Gruppe, die besteht aus:

- Wasserstoff,
- Halogen, bevorzugt wie vorstehend erläutert,
- $C_{1-6}$-Alkyl, bevorzugt wie vorstehend erläutert,
- $C_{3-6}$-Cycloalkyl, bevorzugt wie vorstehend erläutert,
- Halogen-$C_{1-4}$-alkyl, bevorzugt wie vorstehend erläutert, und
- Cyano.

Besonders bevorzugt ist $R_2$ Wasserstoff, Halogen, $C_{1-6}$-Alkyl oder Cyano, jeweils bevorzugt wie vorstehend erläutert, noch bevorzugter Wasserstoff, Methyl und Halogen, insbesondere Chlor oder Fluor, am meisten bevorzugt Wasserstoff oder Fluor.

In einer Ausführungsform der Erfindung können $R_1$ und $R_2$ gemeinsam mit den Kohlenstoffatomen, an die sie gebunden sind, einen gegebenenfalls substituierten 5- oder 6-gliedrigen Ring bilden, der gegebenenfalls ein oder mehrere (wie insbesondere 2) Heteroatome aufweisen kann. Dann liegen ß-Ketoamid-Liganden der folgenden Formel vor:

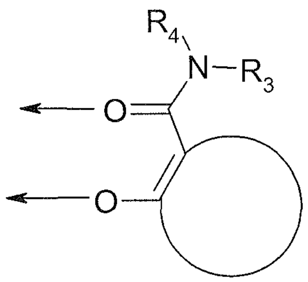

worin $R_3$ und $R_4$ wie vorstehend oder nachstehend beschrieben sind. Diese Ausführungsform ist allerdings weniger bevorzugt.

In dieser Ausführungsform stellen $R_1$ und $R_2$ bevorzugt zusammen eine Propylen($-CH_2-CH_2-CH_2-$)- oder eine Butylen ($-CH_2-CH_2-CH_2-CH_2-$)-Gruppe dar, in denen jeweils eine Methylengruppe ($-CH_2-$) durch -O-, -NH-, oder $-NR_5$-ersetzt sein kann, worin $R_5$ gegebenenfalls substituiertes Alkyl ist, und worin die von $R_1$ und $R_2$ gebildeten Gruppen weiterhin jeweils durch einen bis drei Substituenten, ausgewählt aus der Gruppe, die besteht aus Hydroxy, $C_{1-4}$-Alkoxy, Amino und Mono- oder Di-($C_{1-4}$-alkyl)amino, substituiert sein können. Beispielhafte Liganden sind die folgenden:

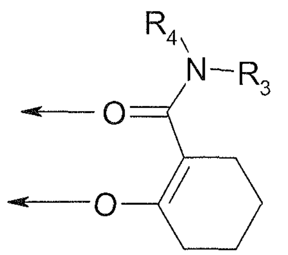

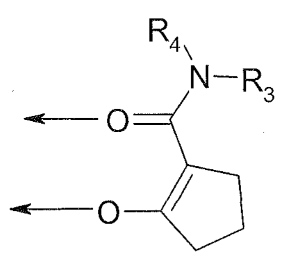

worin $R_3$ und $R_4$ jeweils wie oben definiert sind, und worin der Ring gegebenenfalls 1 oder 2 Substituenten tragen kann, wie beispielsweise Oxo, Alkyl oder Halogen.

In der Erfindung sind $R_3$ und $R_4$ gleich oder verschieden, und werden jeweils ausgewählt aus der Gruppe, die besteht aus Wasserstoff, gegebenenfalls substituiertem Amino und gegebenenfalls substituiertem Alkyl, bevorzugt aus Wasserstoff und gegebenenfalls substituiertem Alkyl, jeweils wie vorstehend definiert.

Oder $R_3$ und $R_4$ bilden gemeinsam mit dem Stickstoffatom, an das sie gebunden sind, einen gegebenenfalls substituierten 3- bis 6-gliedrigen Ring, der gegebenenfalls ein oder mehrere weitere Heteroatome aufweisen kann, jeweils wie vorstehend definiert.

Bevorzugt sind $R_3$ und $R_4$ gleich oder verschieden, und werden jeweils ausgewählt aus der Gruppe, die besteht aus:

- Wasserstoff, und
  substituierten oder unsubstituierten Alkylgruppen, wie ausgewählt aus:
- $C_{1-6}$-Alkyl, bevorzugt wie vorstehend erläutert,
- Di($C_{1-6}$-alkyl)amino-$C_{1-6}$-alkyl, wie Dimethyl- oder Diethylamino-$C_{1-6}$-alkyl, bevorzugt wie vorstehend erläutert,
- Di($C_{1-6}$-alkyl)aminocarbonyl-$C_{1-6}$-alkyl, wie Aminocarbonyl-$C_{1-6}$-alkyl, oder Dimethyl- oder Diethylaminocarbonyl-$C_{1-6}$-alkyl, bevorzugt wie vorstehend erläutert,
- $C_{3-6}$-Cycloalkyl, bevorzugt wie vorstehend erläutert,
- $C_{3-6}$-cycloalkyl-$C_{1-4}$-alkyl, bevorzugt wie vorstehend erläutert,
- $C_{1-4}$-Alkoxy-$C_{1-4}$-alkyl, bevorzugt wie vorstehend erläutert,
- $C_{1-3}$-Alkoxycarbonyl-$C_{3-6}$-cycloalkyl, bevorzugt wie vorstehend erläutert,
- $C_{1-3}$-Alkoxycarbonyl-$C_{1-6}$-alkyl, bevorzugt wie vorstehend erläutert,
- Hydroxy-$C_{1-4}$-alkyl, bevorzugt wie vorstehend erläutert, und
- Halogen-$C_{1-4}$-alkyl, bevorzugt wie vorstehend erläutert,
  wobei gegebenenfalls auch mehrere Substituenten gleichzeitig am Alkyl vorliegen können, wie Hydroxy und $C_{1-3}$-Alkoxycarbonyl, oder mehrere Hydroxygruppen, wie 2 bis 3 Hydroxygruppen, $C_{3-6}$-Cycloalkyl auch $C_{3-6}$-Heterocyclyl einschließt, und gegebenenfalls $-CH_2-$ durch -S- ersetzt sein kann, jeweils wie vorstehend erläutert, oder

$R_3$ und $R_4$ bilden zusammen eine Ethylen($-CH_2-CH_2-$)-, Propylen ($-CH_2-CH_2-CH_2-$)-, Isopropylen($-CH_2-CH(CH_3)-$)-, Butylen ($-CH_2-CH_2-CH_2-CH_2-$)-, Isobutylen-, Pentylen($-CH_2-CH_2-CH_2-CH_2-CH_2-$)-, oder Isopentylen-Gruppe, worin jeweils eine Methylengruppe ($-CH_2-$) durch -O-, -NH-, oder $-NR_5-$ ersetzt sein kann, worin $R_5$ gegebenenfalls substituiertes Alkyl ist, und worin die von $R_3$ und $R_4$ gebildeten Gruppen weiterhin jeweils durch einen bis drei Substituenten, ausgewählt aus der Gruppe, die besteht aus Hydroxy, $C_{1-4}$-Alkoxy, Amino ($-NH_2$) und Mono- oder Di($C_{1-4}$-alkyl)amino, substituiert sein können. Das bedeutet, dass $R_3$ und $R_4$ in diesem Fall einen gegebenenfalls substituierten Stickstoff-haltigen 5- bis 6-gliedrigen Heterocyclus bilden, wie die vorstehend oder die nachstehend genannten.

Besonders bevorzugt sind $R_3$ und $R_4$ gleich oder verschieden und werden ausgewählt aus

- Wasserstoff,
- $C_{1-6}$-Alkyl, wie vorstehend erläutert, bevorzugt Methyl, Ethyl, Propyl, insbesondere n-Propyl, Butyl, insbesondere n-Butyl, Pentyl, insbesondere n-Pentyl und Hexyl, insbesondere n-Hexyl, und
- Hydroxy-$C_{1-4}$-alkyl, wie vorstehend erläutert, und bevorzugt Hydroxymethyl, Hydroxyethyl,
- $C_{1-3}$-Alkoxycarbonyl-$C_{1-6}$-alkyl, wie vorstehend erläutert, und bevorzugt Methoxycarbonyl, Ethoxycarbonyl
- $C_{1-4}$-Alkoxy-$C_{1-4}$-alkyl, wie vorstehend erläutert.

Weiterhin bilden $R_3$ und $R_4$ in einer bevorzugten Ausführungsform zusammen eine Pentylen ($-CH_2-CH_2-CH_2-CH_2-CH_2-$)-Gruppe, worin jeweils eine Methylengruppe ($-CH_2-$) durch -O-, -NH-, oder $-NR_5-$ (wie oben definiert) ersetzt sein kann, und die jeweils durch einen Substituenten ausgewählt aus Hydroxy, $C_{1-4}$-Alkoxy, Amino ($-NH_2$) und Mono- oder Di($C_{1-4}$-alkyl)amino, substituiert sein kann. Beispiele von Gruppen, die aus $R_3$ und $R_4$ und dem Stickstoffatom an das sie gebunden sind, hervorgehen, sind beispielsweise die vorstehend erwähnten über Stickstoff gebundenen Heterocyclen, welche gegebenenfalls 1 bis 3, wie 1 oder 2 Heteroatome wie insbesondere O, N aufweisen können, wie Azetidin-1-yl, substituiertes Azetidinyl, wie 3-Hydroxyazetidin-1-yl, Pyrrolidinyl, wie Pyrrolidin-1-yl, substituiertes Pyrrolidinyl, wie 3-Hydroxypyrrolidin-1-yl, 2-Hydroxypyrrolidin-1-yl, 2-Methoxycarbonylpyrrolidin-1-yl, 2-Ethoxycarbonylpyrrolidin-1-yl, 2-Methoxypyrrolidin-1-yl, 2-Ethoxypyrrolidin-1-yl, 3-Methoxycarbonylpyrrolidin-1-yl, 3-Ethoxycarbonylpyrrolidin-1-yl, 3-Methoxypyrrolidin-1-yl, 3-Ethoxypyrrolidin-1-yl, Piperidinyl, wie Piperidin-1-yl, substituiertes Piperidinyl, wie 4-Methyl-1-piperidyl, 4-Hydroxy-1-piperidyl, 4-Methoxy-1-piperidyl, 4-Ethoxy-1-piperidyl, 4-Methoxycarbonyl-1-piperidyl, 4-Ethoxycarbonyl-1-piperidyl, 4-Carboxy-1-piperidyl, 4-Acetyl-1-piperidyl, 4-Formyl-1-piperidyl, 4-Hydroxy-2,2,6,6-tetramethyl-1-piperidyl, 4-(Dimethylamino)-1-piperidyl, 4-(Diethylamino)-1-piperidyl, 4-Amino-1-piperidyl, 2-(Hydroxymethyl)-1-piperidyl, 3-(Hydroxymethyl)-1-piperidyl, 4-(Hydroxymethyl)-1-piperidyl, 2-Hydroxy-1-piperidyl, 3-Hydroxy-1-piperidyl, Morpholin-4-yl, substituiertes Morpholinyl, wie 2,6-Dimethylmorpholin-4-yl, Piperazinyl, wie Piperazin-1-yl, substituiertes Piperazinyl, wie 4-Methylpiperazin-1-yl, 4-Ethylpiperazin-1-yl, 4-Ethoxycarbonylpiperazin-1-yl, 4-Methoxycarbonylpiperazin-1-yl.

Besonders bevorzugt sind Piperidin-1-yl, Piperazin-1-yl, Morpholin-4-yl, die gegebenenfalls substituiert sein können, wie durch Hydroxy, wie zum Beispiel 4-Hydroxy-piperidin-1-yl. Bevorzugter sind 4-Hydroxy-piperidin-1-yl und Piperidin-1-yl.

In einer weiteren weniger bevorzugten Ausführungsform der Erfindung bilden $R_2$ und $R_3$ gemeinsam einen gesättigten oder ungesättigten gegebenenfalls substituierten 5- oder 6-gliedrigen Ring unter Bildung eines ß-Ketoamid der Formel (Ia):

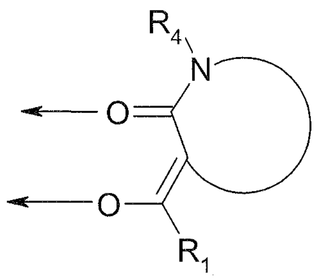

(Ia)

worin $R_1$ und $R_4$ wie zuvor definiert sind. Beispiele derartiger Liganden sind Verbindungen in denen $R_2$ und $R_3$ zusammen eine Ethylen ($-CH_2-CH_2-$)- oder Propylen($-CH_2-CH_2-CH_2-$)-Gruppe bilden:

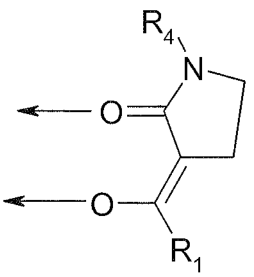

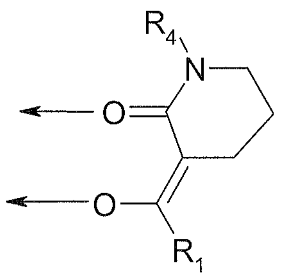

.

Liganden diesen Typs sind beispielsweise beschrieben in Korte et al., Chemische Berichte, 95, 2424 und Wamhoff et al., Liebigs Ann. Chem. 715, 23-34 (1968).

In einer weiteren ebenfalls weniger bevorzugten Ausführungsform der Erfindung bilden $R_2$ und $R_3$ gemeinsam einen gesättigten oder ungesättigten gegebenenfalls substituierten 5- oder 6-gliedrigen Ring, und $R_1$ und $R_2$ bilden gemeinsam einen gesättigten oder ungesättigten gegebenenfalls substituierten 5- oder 6-gliedrigen Ring unter Bildung eines ß-Ketoamid der Formel (Ib:

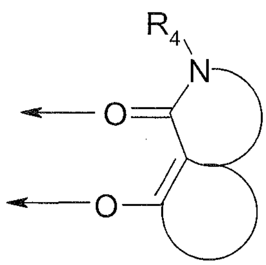

(Ib),

worin $R_4$ wie zuvor definiert ist.

Beispiele derartiger Liganden schließen ein:

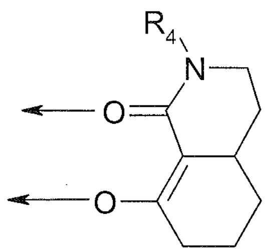

.

Es ist für den Fachmann klar, dass die erfindungsgemäßen Liganden

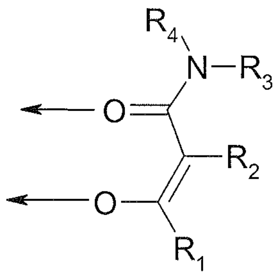

aus den entsprechenden ß-Ketoamidverbindungen hervorgehen:

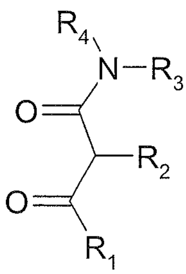

,

in denen bekanntermaßen eine Keto-Enol-Tautomerie vorliegt:

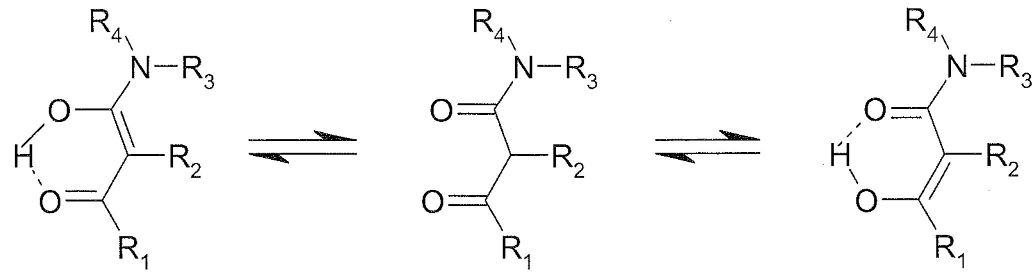

A B C

.

Die mesomeren Grenzformen A und C sind analytisch nicht unterscheidbar. Im Rahmen der vorliegenden Erfindung sind in jedem Fall sämtliche Formen eingeschlossen, wobei jedoch im Rahmen der vorliegenden Erfindung bei den Liganden im Allgemeinen nur die Ketoform gezeichnet ist.

Der Ligand geht formal durch Abspalten eines Protons aus den entsprechenden ß-Ketoamidverbindungen hervor:

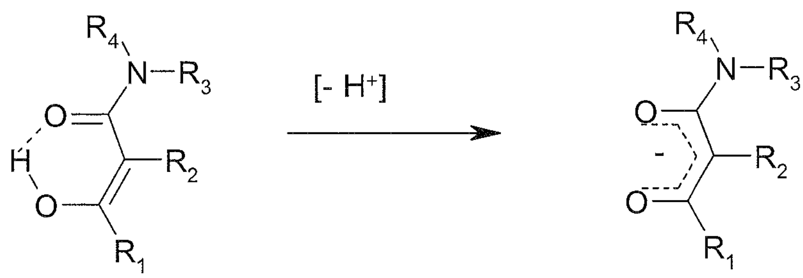


,

trägt also formal eine einfach negative Ladung. Auch bei den EisenKomplexverbindungen ist im Rahmen der vorliegenden Erfindung stets lediglich eine der beiden lokalisierten Grenzformeln gezeigt:

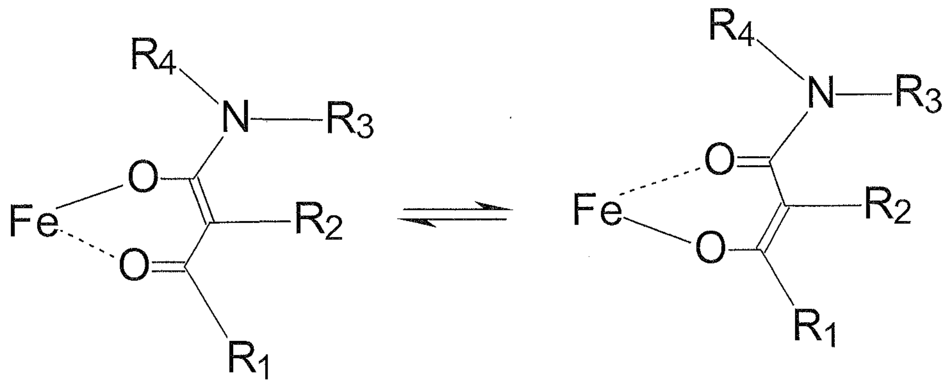

A C

wobei aufgrund der geringeren Elektronendichte am amidischen Sauerstoffatom zu erwarten ist, dass die Grenzformel C vorherrschen sein sollte. Eine analytische Unterscheidung der Grenzformeln A und C ist jedoch wie gesagt nicht möglich.

Beispiele von erfindungsgemäß verwendeten ß-Ketoamid-Liganden werden nachfolgend dargestellt:

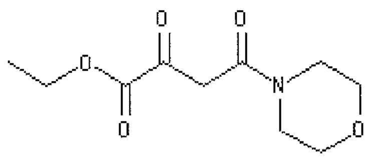

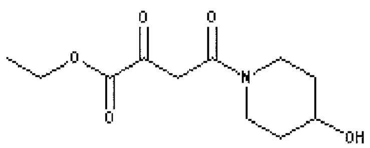

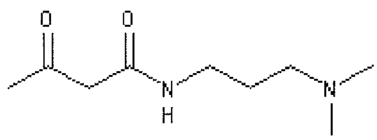

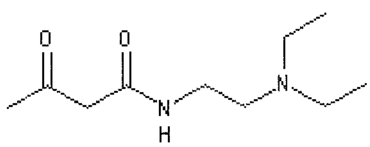

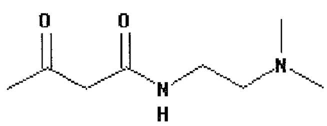

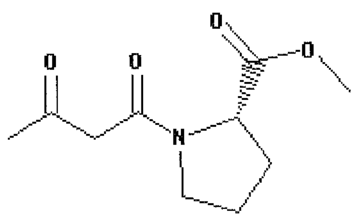

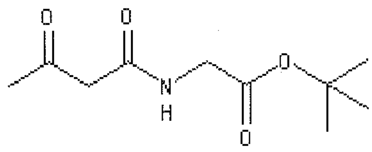

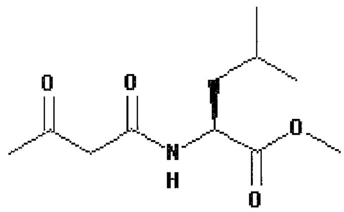

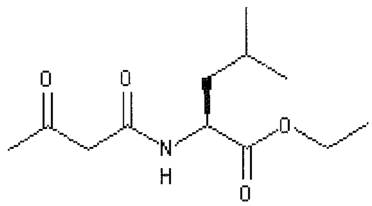

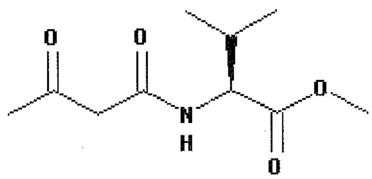

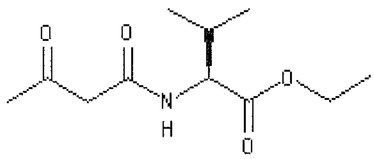

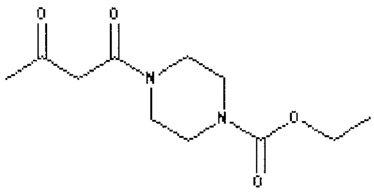

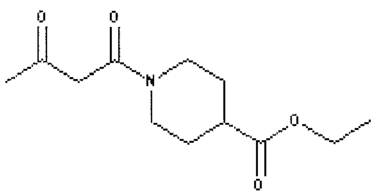

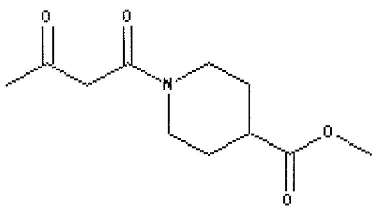

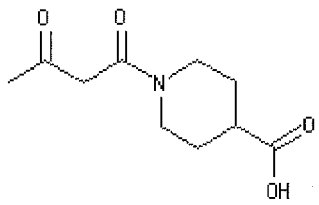

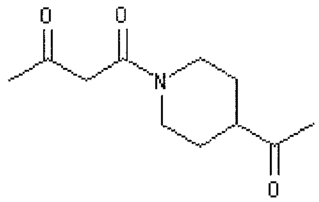

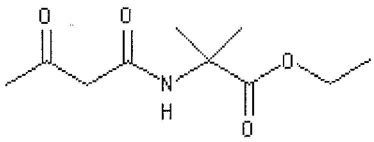

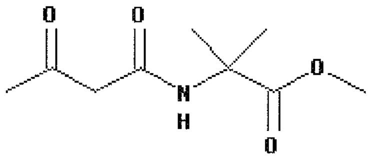

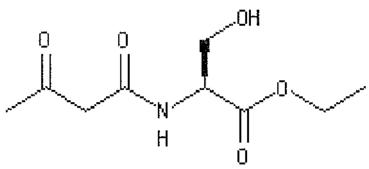

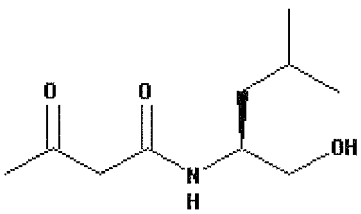

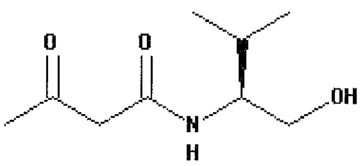

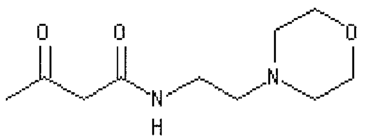

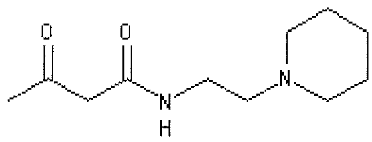

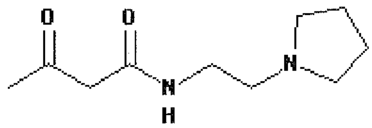

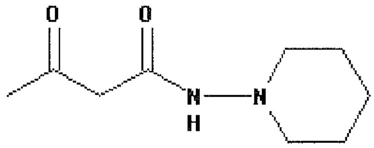

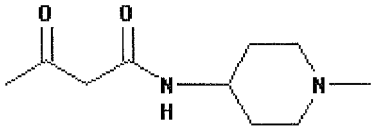

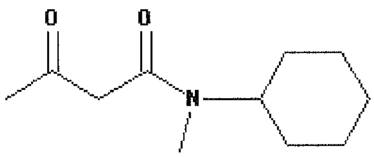

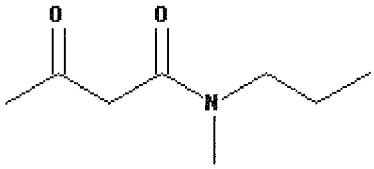

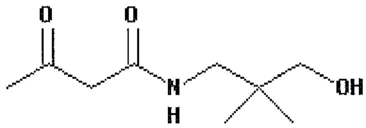

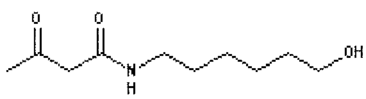

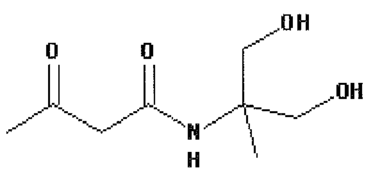

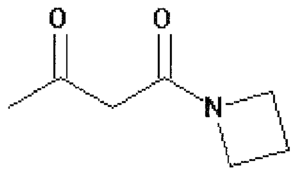

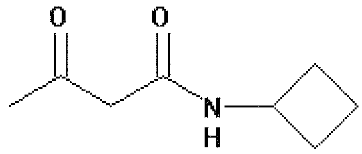

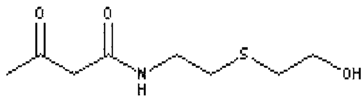

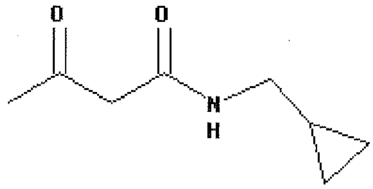

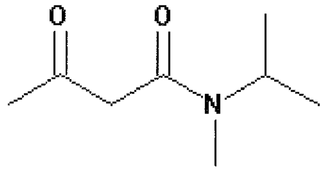

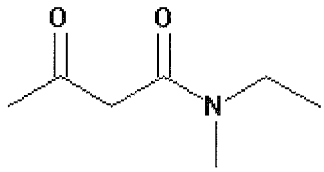

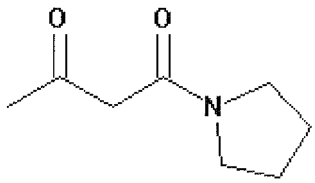

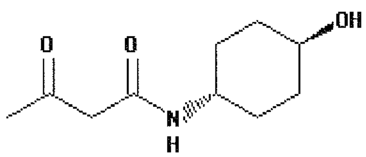

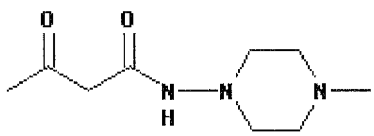

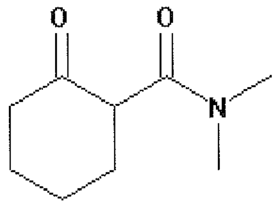

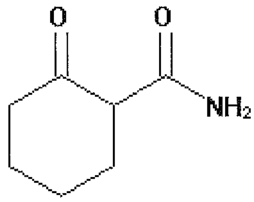

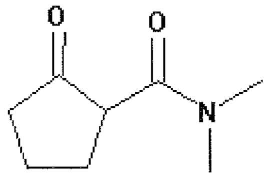

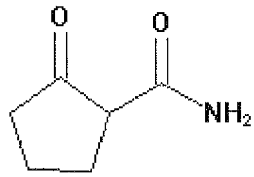

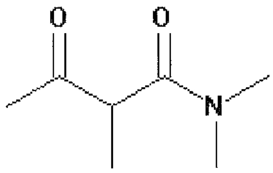

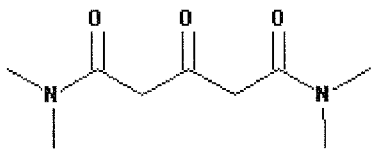

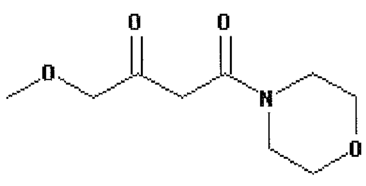

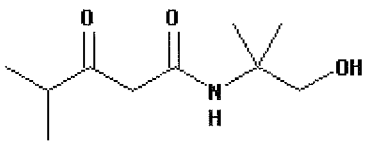

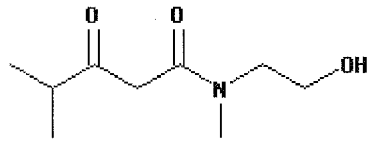

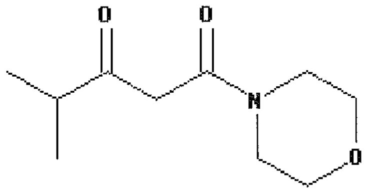

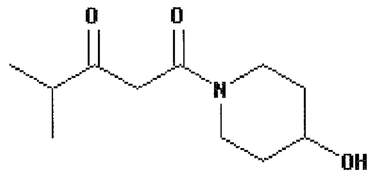

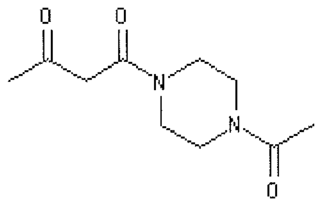

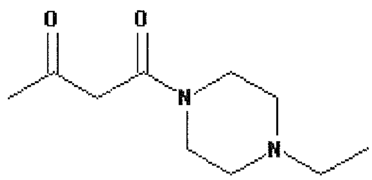

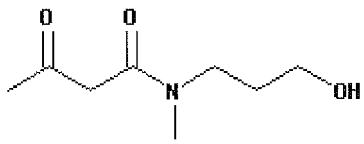

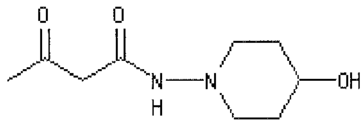

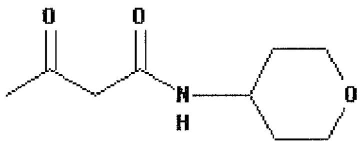

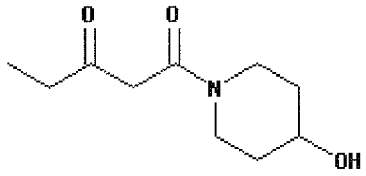

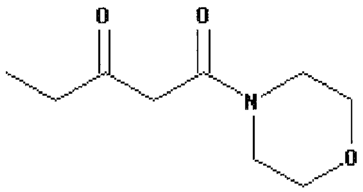

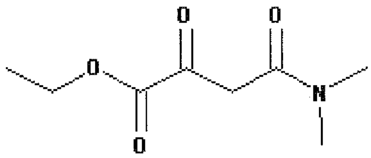

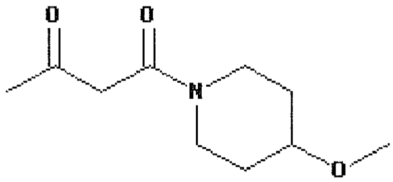

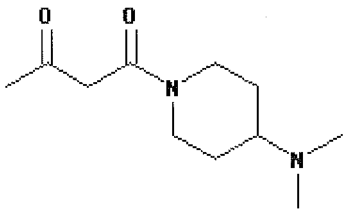

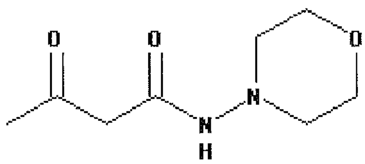

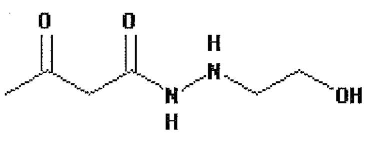

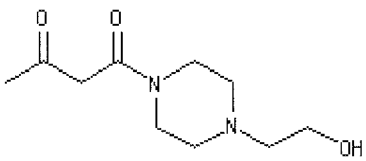

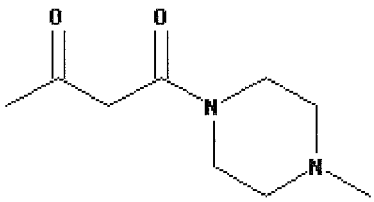

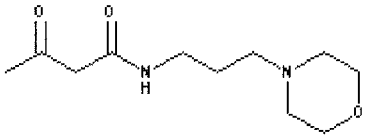

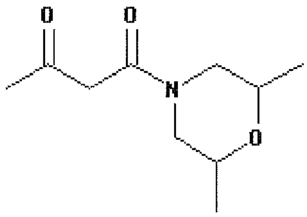

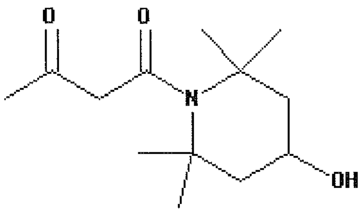

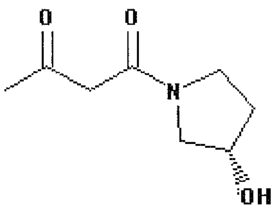

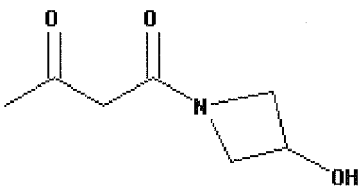

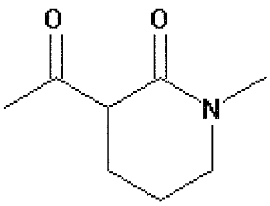

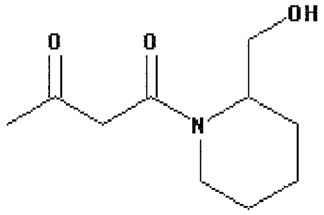

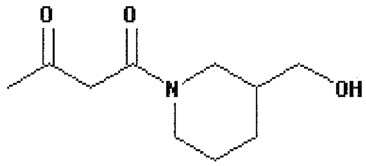

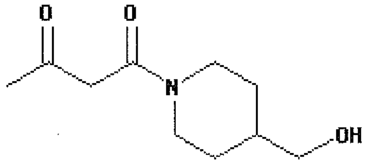

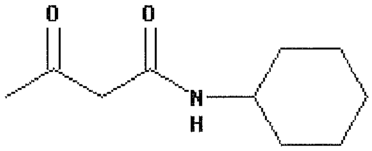

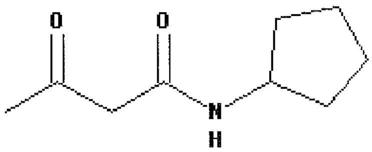

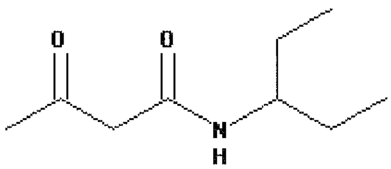

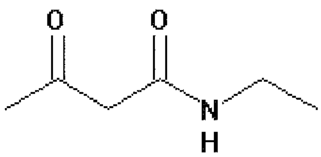

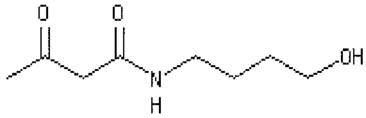

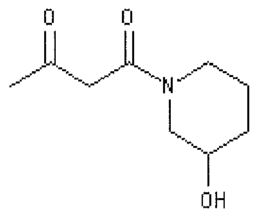

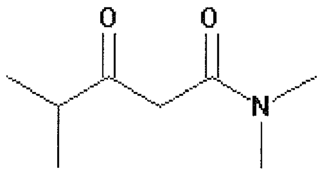

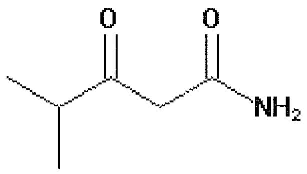

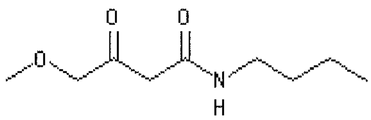

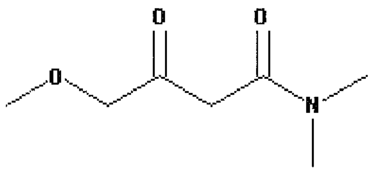

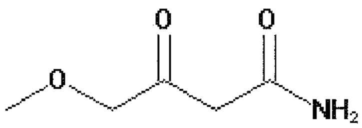

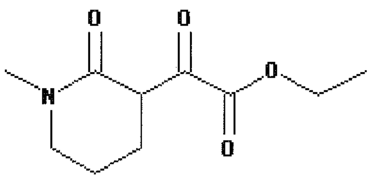

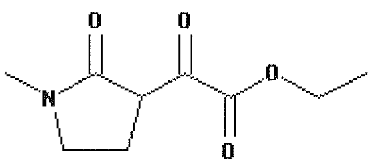

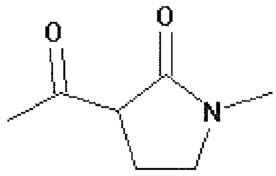

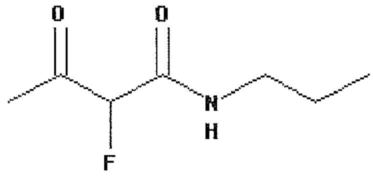

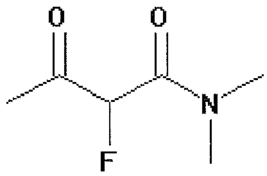

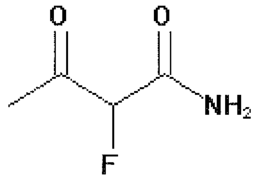

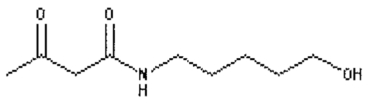

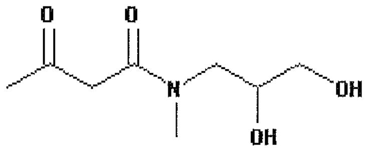

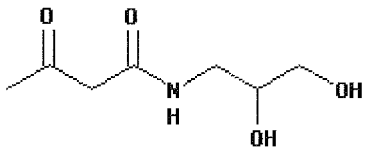

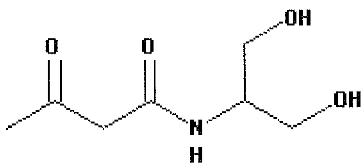

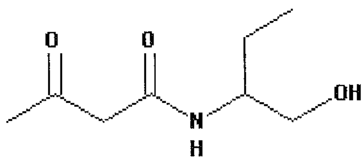

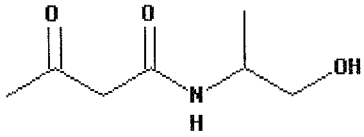

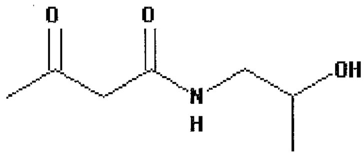

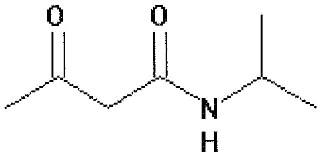

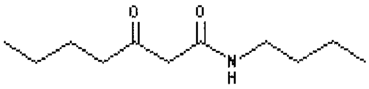

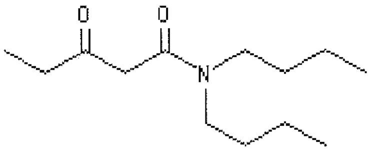

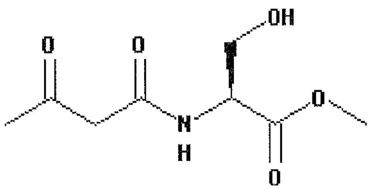

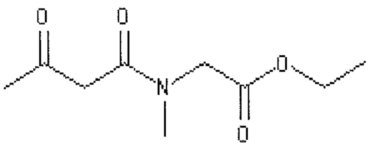

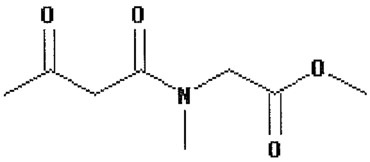

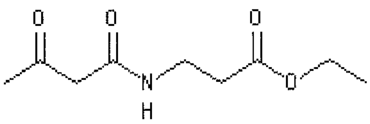

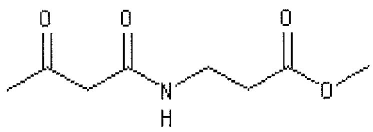

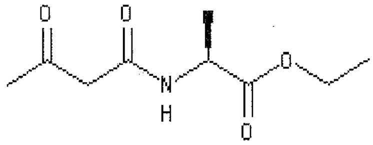

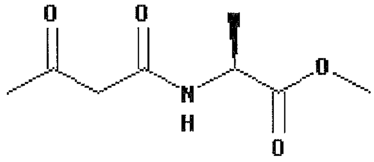

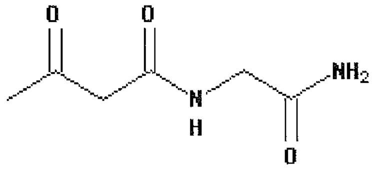

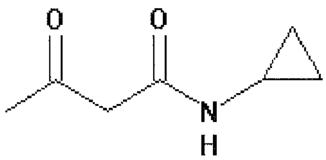

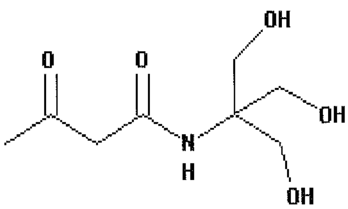

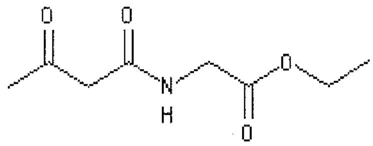

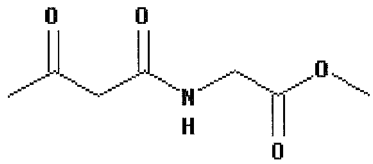

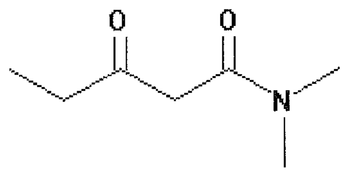

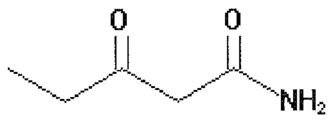

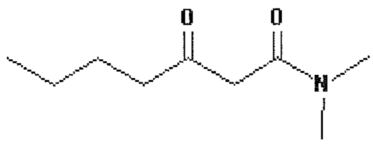

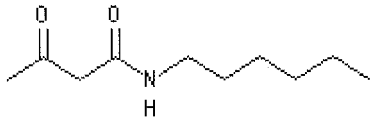

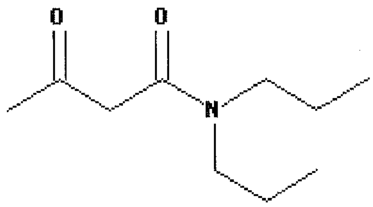

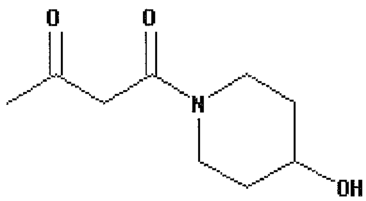

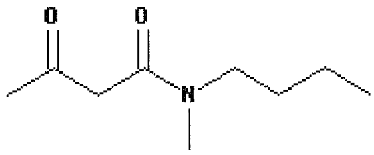

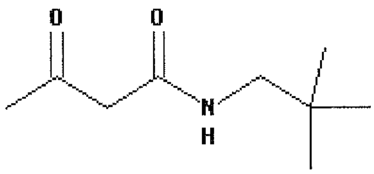

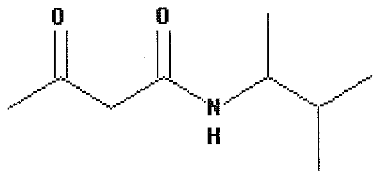

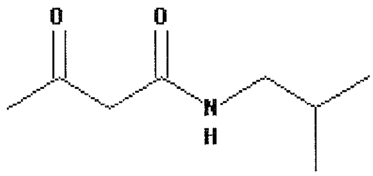

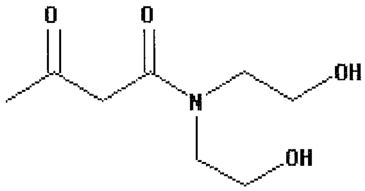

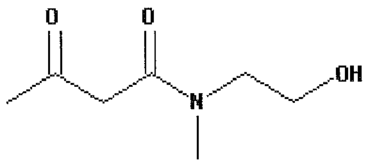

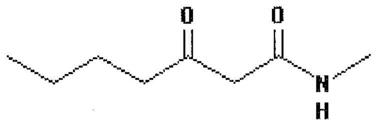

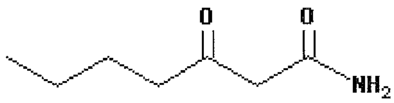

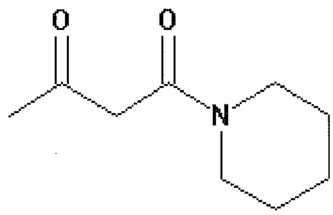

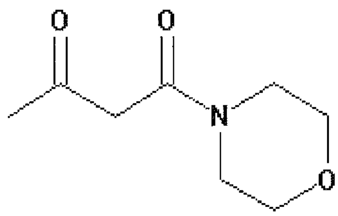

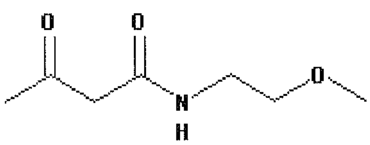

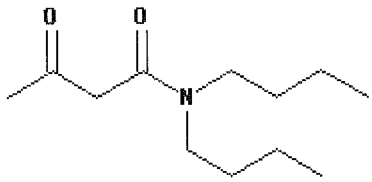

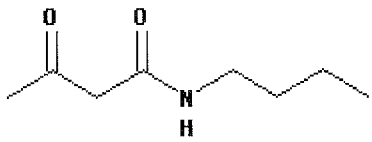

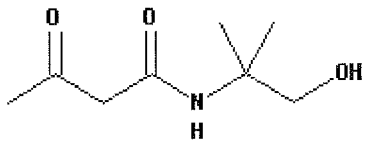

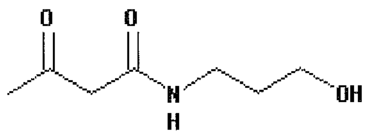

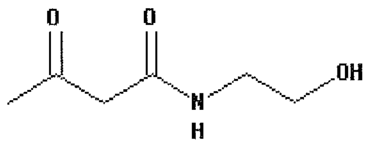

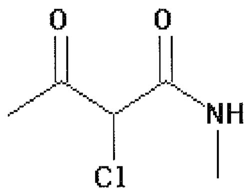

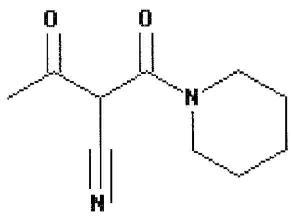

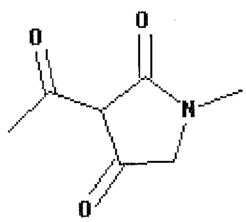

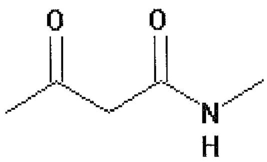

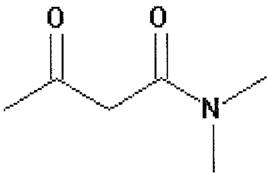

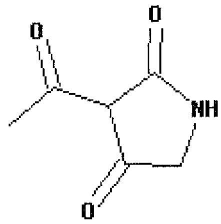

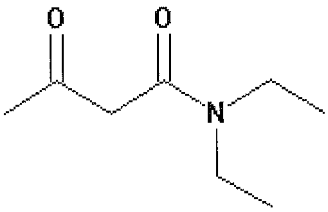

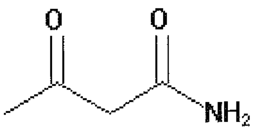

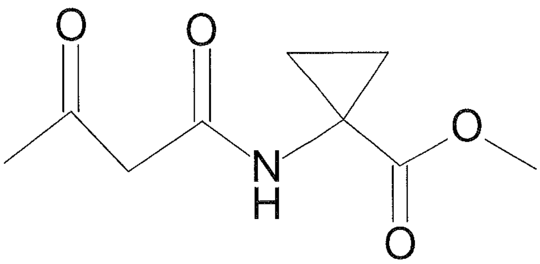

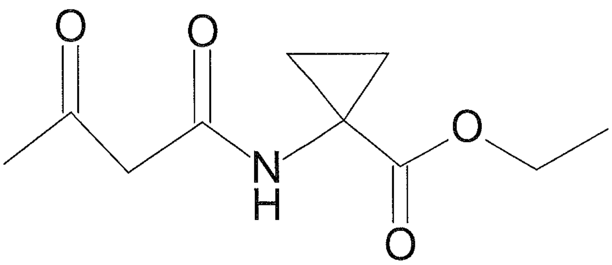

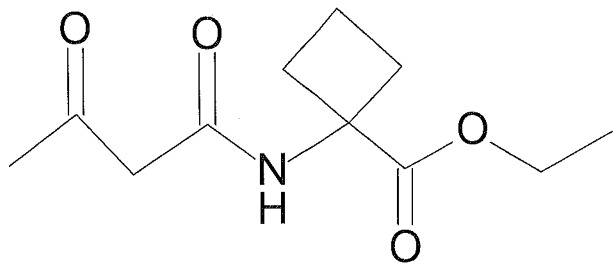

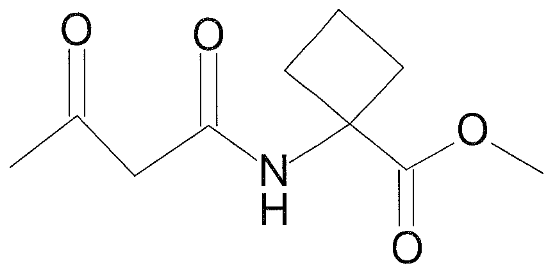

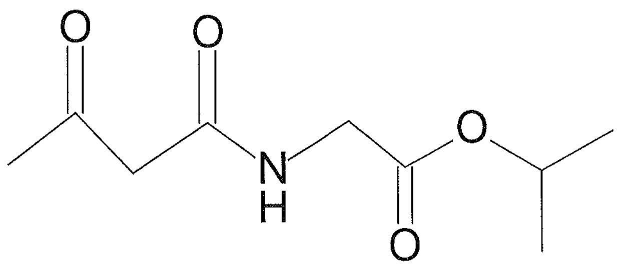

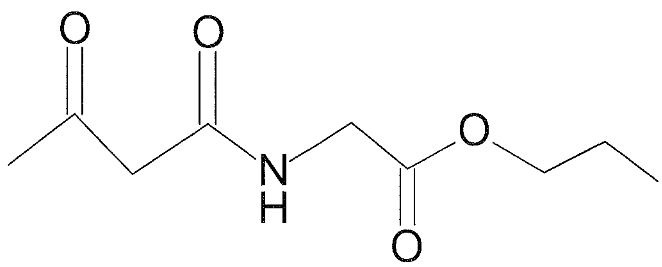

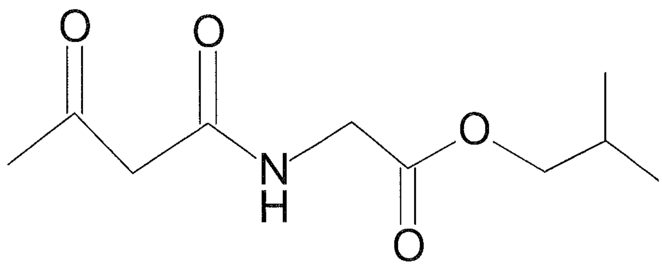

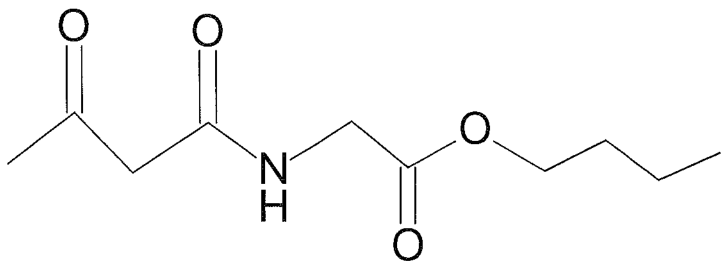

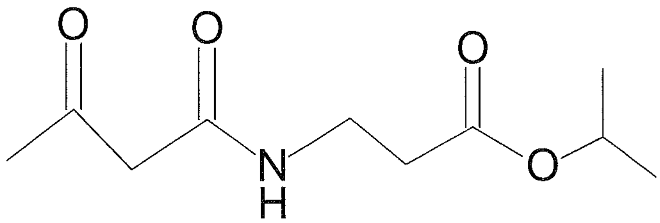

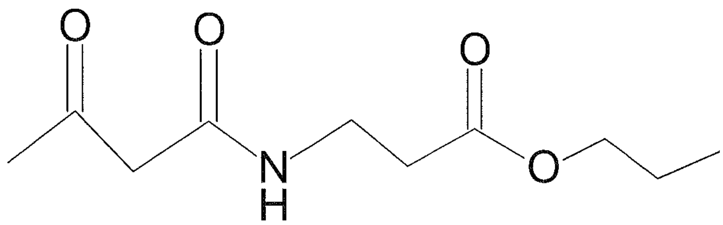

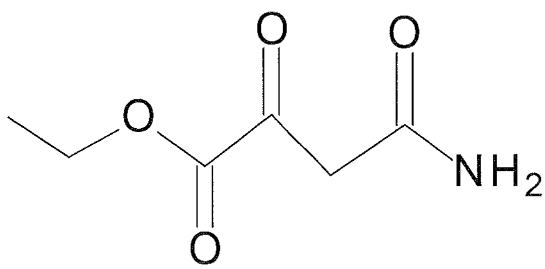

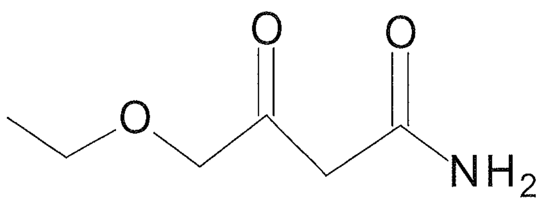

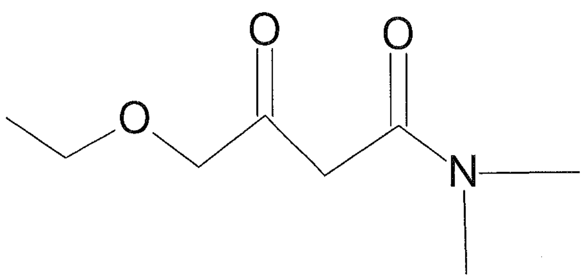

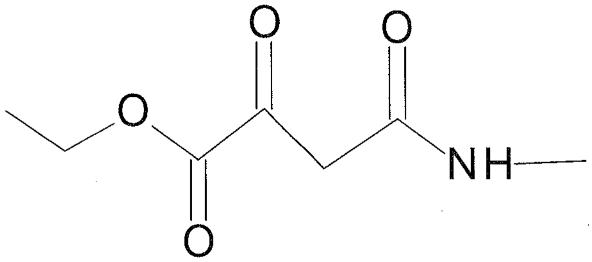

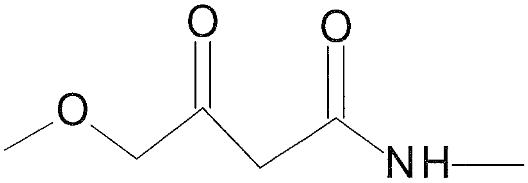

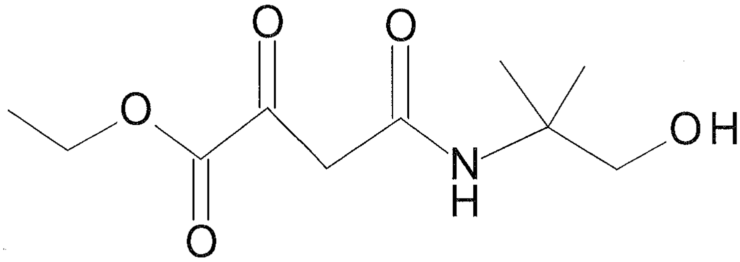

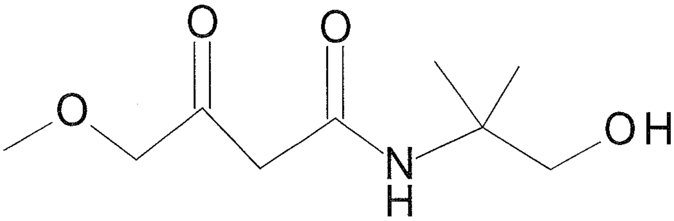

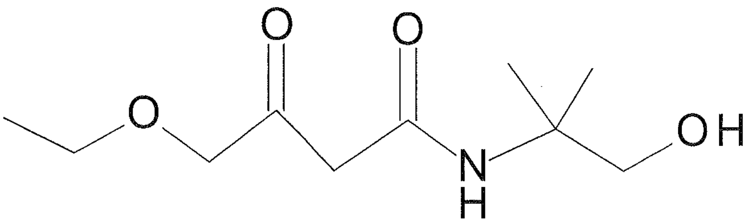

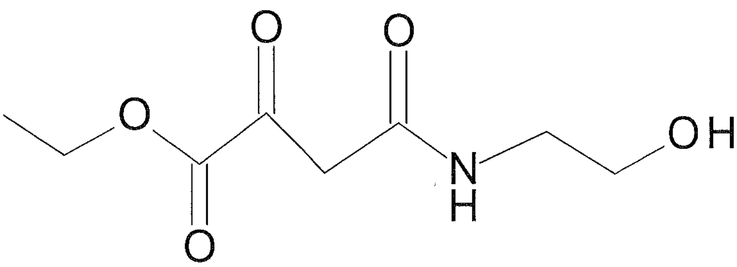

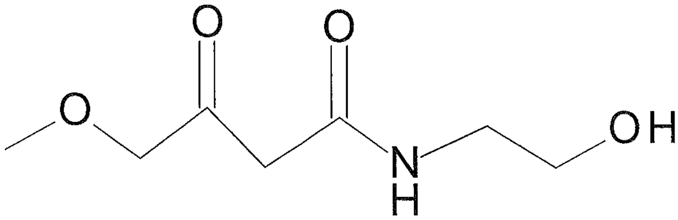

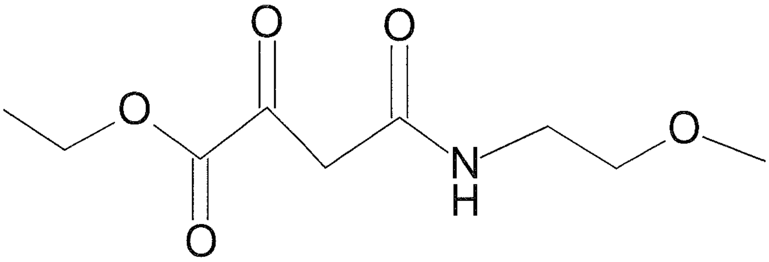

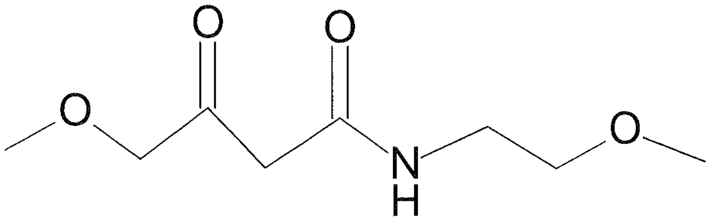

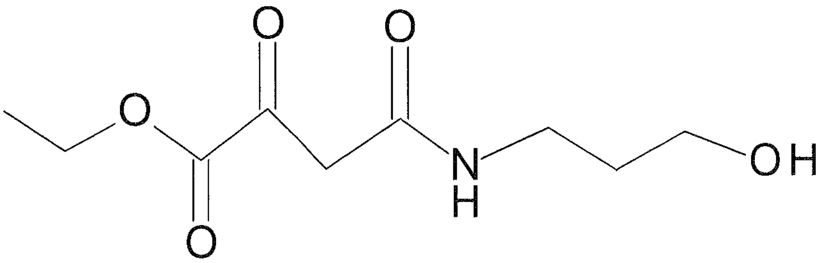

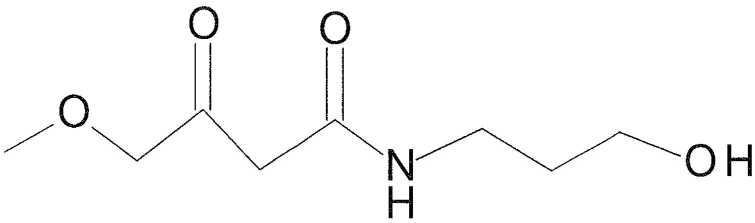

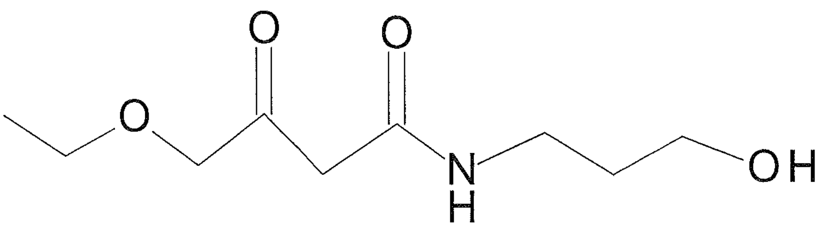

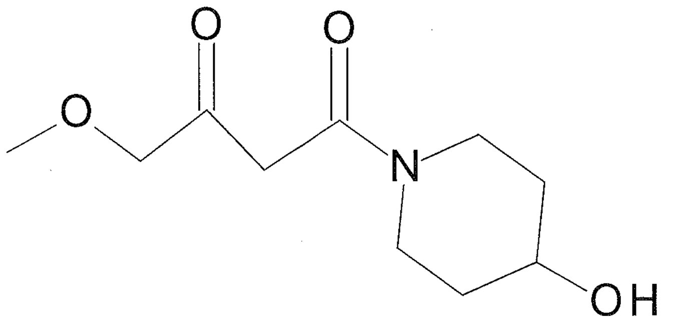

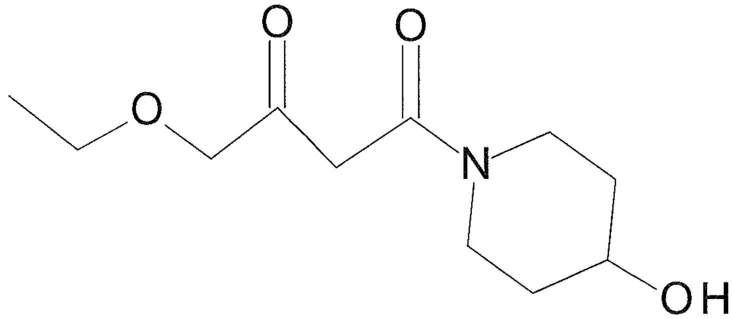

Die erfindungsgemäßen Eisen(III)-ß-ketoamid-Komplexverbindungen insbesondere die der allgemeinen Formel (II) können in Form verschiedener Isomere vorliegen. Isomere Formen schließen beispielsweise Regioisomere ein, die sich durch die Lage der Liganden zueinander unterscheiden, einschließlich sogenannter optischer Isomere, die sich wie Bild und Spiegelbild zueinander verhalten. Weiterhin können die Liganden bei Vorliegen asymmetrischer Kohlenstoffatome in Form optischer Isomere vorliegen, die sich wie Bild und Spiegelbild zueinander verhalten, und reine Enantiomere, Mischungen der Enantiomere, insbesondere Racemate umfassen. Enantiomerenreine Liganden können wie dem Fachmann bekannt ist durch optische Auflösungsverfahren, wie Umsetzung mit chiralen Reagenzien zu Diastereomeren, Trennung der Diastereomeren und Freisetzen der Enantiomere erhalten werden.
Bevorzugte Ausführungsformen der Erfindung sind weiterhin insbesondere die Folgenden:
(In der vorliegenden Erfindung bedeuten die Ziffern 1-6 in "1-6C" oder "C1-6", bzw. "1-4" in "1-4C" oder "C1-4" etc. jeweils die Anzahl der Kohlenstoffatome der sich daran anschließenden Kohlenwasserstoffreste-Bezeichnungen).

$R_1$ wird ausgewählt aus der Gruppe, die besteht aus:

- 1-6C-alkyl, (also Alkyl mit 1 bis 6 Kohlenstoffatomen),
- 3-6C-cycloalkyl,
- 3-6C-cycloalkyl-1-4C-alkyl,
- 1-4C-alkoxy-1-4C-alkyl,
- hydroxy-1-4C-alkyl,
- fluoro-1-4C-alkyl;

$R_2$ wird ausgewählt aus der Gruppe, die besteht aus:

- H,
- 1-6C-alkyl,
- 3-6C-cycloalkyl,
- fluor-1-4C-alkyl,
- halogen,
- cyano;

oder $\mathbf{R_1}$ und $\mathbf{R_2}$ bilden zusammen eine Propylen ($-CH_2-CH_2-CH_2$), Butylen ($-CH_2-CH_2-CH_2-CH_2-$), Azabutylen oder Oxabutylen- Gruppe;

$\mathbf{R_3}$ und $\mathbf{R_4}$ sind gleich oder verschieden, und werden jeweils ausgewählt aus der Gruppe, die besteht aus:

- H,
- 1-6C-alkyl,
- 3-6C-cycloalkyl,
- 3-6C-cycloalkyl-1-4C-alkyl,
- 1-4C-alkoxy-1-4C-alkyl,
- 1-3C-alkoxy-carbonyl-1-6C-alkyl,
- hydroxy-1-4C-alkyl,
- fluoro-1-4C-alkyl;

oder **$R_3$** und **$R_4$** bilden zusammen eine Ethylen(-$CH_2$-$CH_2$-)-, Propylen-(-$CH_2$-$CH_2$-$CH_2$-)-, Hydroxypropylen-, bevorzugt 2-Hydroxypropylen-, 3-Methylpropylen-, Butylen- (-$CH_2$-$CH_2$-$CH_2$-$CH_2$-), 2-Hydroxybutylen-, 2-Methoxybutylen-, Isobutylen-, Pentylen-(-$CH_2$-$CH_2$-$CH_2$-$CH_2$-$CH_2$-), Hydroxypentylen-, bevorzugt 3-Hydroxypentylen, Methoxypentylen-, bevorzugt 3-Methoxypentylen-, Ethoxypentylen-, bevorzugt 3-Ethoxypentylen-, Propoxypentylen-, bevorzugt 3-Propoxypentylen-, Isopropoxypentylen-, bevorzugt 3-Isopropoxypentylen-, Cyclopropoxypentylen-, bevorzugt 3-Cyclopropoxypentylen- , Azapentylen- insbesondere -$CH_2$-$CH_2$-NH-$CH_2$-$CH_2$-, oder eine Oxapentylen-Gruppe, insbesondere -$CH_2$-$CH_2$-O-$CH_2$-$CH_2$-
oder pharmazeutisch verträgliche Salze davon.

Bevorzugt werden die vorstehend genannten Substituentengruppen wie folgt definiert:

**1-6C-Alkyl** beinhaltet bevorzugt geradkettige oder verzweigte Alkylgruppen mit 1 bis 6 Kohlenstoffatomen. Beispiele hierfür können Methyl, Ethyl, n-Propyl, isoPropyl, n-Butyl, iso-Butyl, sec-Butyl, tert-Butyl n-Pentyl, iso-Pentyl, neo-Pentyl, n-Hexyl, iso-Hexyl und neo-Hexyl sein.

**3-6C-Cycloalkyl** beinhaltet bevorzugt Cycloalkyl mit 1 bis 6 Kohlenstoffatomen, wie Cyclopropyl, Cyclobutyl, Cyclopentyl, Cyclohexyl.

**3-6C-Cycloalkyl-1-4C-alkyl** beinhaltet bevorzugt eine oben beschriebene 1-6C-Alkyl Gruppe, substituiert mit einer oben beschriebenen 3-6C-Cycloalkyl Gruppe. Beispiele hierfür können eine Cyclopropylmethyl, Cyclopentylmethyl und Cyclohexylmethyl Gruppe sein.

**1-3C-alkoxy-carbonyl-1-6C-alkyl,** beinhaltet bevorzugt eine oben beschriebene 1-6C-Alkyl Gruppe, die mit einer Carbonylgruppe, die mit einer 1-3C Alkoxy Gruppe als Carbonsäureester vorliegt, verknüpft ist. Beispiele hierfür können ein Methoxycarbonylmethyl, Ethoxycarbonylmethyl, Methoxycarbonylethyl, Ethoxycarbonylethyl und Isopropoxycarbonylmethyl.

**1-4C-Alkoxy** beinhaltet bevorzugt eine 1-4C-Alkoxy Gruppe, bei der ein Sauerstoffatom mit einer geraden oder verzweigten Alkylkette mit 1-4 Kohlenstoffatome verbunden ist. Beispiele dieser Gruppe können Methoxy, Ethoxy, Propoxy und Isobutoxy sein.

**1-4C-Alkoxy-1-4C-alkyl** beinhaltet bevorzugt eine oben beschriebene 1-4C-Alkoxy Gruppe, die mit einer oben beschriebenen 1-4C-alkyl Gruppe verknüpft ist. Beispiele dieser Gruppe können Methoxyethyl, Ethoxypropyl, Methoxypropyl, Isobutoxymethyl sein.

**Hydroxy-1-4C-alkyl** beinhaltet eine oben beschriebene 1-4C-Alkyl Gruppe, die mit einer Hydroxygruppe substituiert ist. Beispiele hier können Hydroxyethyl, Hydroxybutyl und Hydroxyisopropyl sein.

**Fluor-1-4C-alkyl** beinhaltet eine oben beschriebene 1-4C-Alkyl Gruppe, die mit eins bis drei Fluoratomen substituiert ist. Beispiele hier können Trifluormethyl und Trifluorethyl sein.

**Halogen** bedeutet F, Cl, Br, I.

Die Gruppen und Reste können auch chirale Zentren enthalten. Hierbei sind dann alle möglichen Enantiomerenmischungen sowie die reinen Enantiomere im Umfang mit enthalten.

Besonders bevorzugt wird:

**$R_1$** ausgewählt aus der Gruppe, die besteht aus:

- 1-6C-alkyl,

- 1-4C-alkoxy-1-4C-alkyl,
- hydroxy-1-4C-alkyl;

$\mathbf{R_2}$ ausgewählt aus der Gruppe, die besteht aus:

- H,
- 1-6C-alkyl;

und

$\mathbf{R_3}$ und $\mathbf{R_4}$ sind gleich oder verschieden, und werden jeweils ausgewählt aus der Gruppe, die besteht aus:

- H,
- 1-6C-alkyl,
- 1-4C-alkoxy-1-4C-alkyl,
- 1-3C-alkoxy-carbonyl-1-6C-alkyl,
- hydroxy-1-4C-alkyl;

oder $\mathbf{R_3}$ und $\mathbf{R_4}$ bilden zusammen eine Butylen($-CH_2-CH_2-CH_2-CH_2-$)-, Pentylen ($-CH_2-CH_2-CH_2-CH_2-CH_2-$), Hydroxypentylen, Azapentylen oder Oxapentylen-Gruppe.

Besonders bevorzugt wird:

$\mathbf{R_1}$ ausgewählt aus der Gruppe, die besteht aus:

- 1-6C-alkyl;

$\mathbf{R_2}$ ausgewählt wird aus der Gruppe, die besteht aus:

- H,
- 1-6C-alkyl;

und

$\mathbf{R_3}$ und $\mathbf{R_4}$ sind gleich oder verschieden, und werden jeweils ausgewählt aus der Gruppe, die besteht aus:

- H,
- 1-6C-alkyl,
- 1-4C-alkoxy-1-4C-alkyl,
- 1-3C-alkoxy-carbonyl-1-6C-alkyl,
- hydroxy-1-4C-alkyl;

oder $\mathbf{R_3}$ und $\mathbf{R_4}$ bilden zusammen eine Pentylen- ($-CH_2-CH_2-CH_2-CH_2-CH_2-$), Hydroxypentylen, bevorzugt 3-Hydroxypentylen oder Oxapentylen-Gruppe, wie zuvor beschrieben.

Besonders bevorzugte Komplex-Verbindungen der allgemeinen Formel (II) sind in den Beispielen beschrieben.

Die Erfindung betrifft weiterhin ein Verfahren zur Herstellung der erfindungsgemäßen Eisen(III) -Komplexverbindungen, welches die Umsetzung eines ß-Ketoamids mit einem Eisen(III)-salz umfasst.

ß-Ketoamide schließen insbesondere diejenigen der Formel (III) ein:

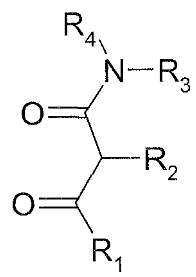

(III)

worin $R_1$ bis $R_4$ wie oben definiert sind.

Beispiele geeigneter Eisen(III)-salze schließen ein: Eisen(III)-chlorid, Eisen(III)-acetat, Eisen(III)-sulfat, Eisen(III)-nitrat und Eisen(III)-acetylacetonat, worunter Eisen(III)-chlorid bevorzugt ist.

Ein bevorzugtes Verfahren ist im folgenden Schema gezeigt:

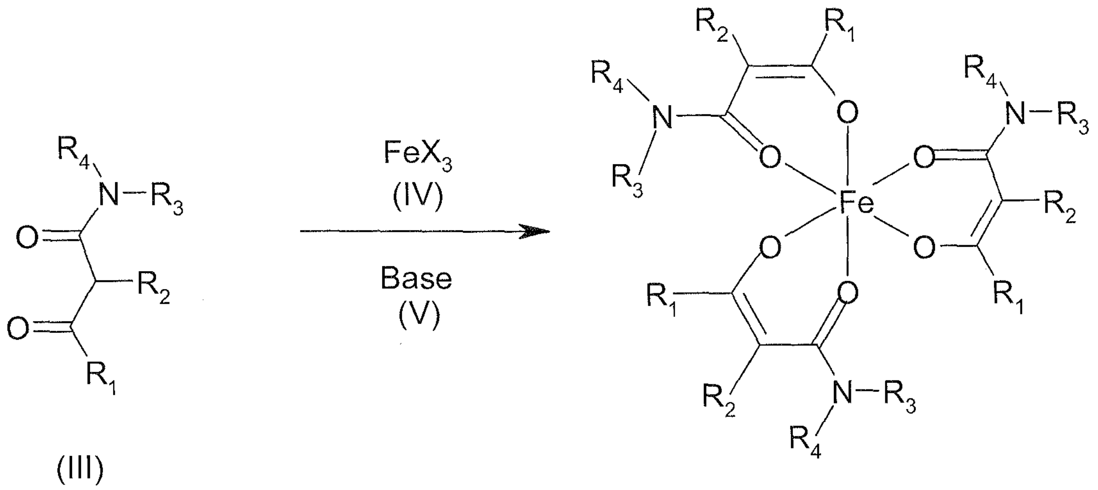


(III)

(II) ,

worin $R_1$ bis $R_4$ wie oben definiert sind, X ein Anion wie Halogenid, wie Chlorid, ein Carboxylat, wie Acetat, Sulfat, Nitrat und Acetylacetonat ist und Base eine übliche organische oder anorganische Base ist.

Beim erfindungsgemäßen Verfahren werden bevorzugt 3-5 eq Ligand (III) unter Verwendung von geeigneten Eisen(III)-salzen (IV) (besonders eignen sich hier Fe(III)-chlorid, Fe(III)-acetat, Fe(III)-sulfat und Fe(III)-acetylacetonat) zu den entsprechenden Komplexen der allgemeinen Formel (II) unter Standardbedingungen umgesetzt. Hierbei wird die Synthese unter den für die Komplexbildung optimalen pH-Bedingungen durchgeführt. Der optimale pH-Wert wird durch Zugabe von Base (V), besonders eignet sich hier die Verwendung von Natrium-Carbonat, Natrium-Hydrogencarbonat, Natrium-Methanolat, Kaliumcarbonat, Kalium-Hydrogencarbonat oder Kalium-Methanolat, eingestellt.

Die für die Darstellung der Komplexe benötigten Liganden (III) sind entweder käuflich erhältlich oder wurden nach folgender Synthesemethode dargestellt. Hierzu wurden zwei unterschiedliche Synthesewege beschritten. Für Liganden der allgemeinen Formel $R_1$ = Methyl, $R_2$ =H wurde das käufliche Diketon (3) mit dem entsprechenden Amin (4) unter Standardbedingungen zum Liganden der allgemeinen Formel (III) umgesetzt.

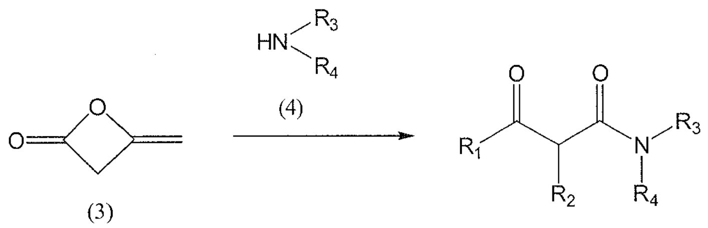

(4)

(3)

(III)

Für die anderen Liganden der allgemeinen Formel (III) wurde der entsprechende Ketoester (5) mit dem entsprechen Amin (6) unter Standardbedingungen umgesetzt.

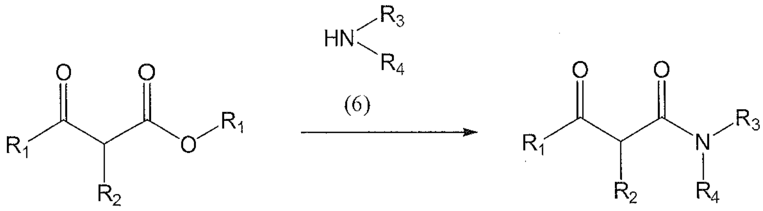

(5) (III)

worin $R_1$ bis $R_4$ jeweils wie oben definiert sind.

Pharmazeutisch annehmbare Salze der erfindungsgemäßen Verbindungen, in denen der Eisen(III)-Komplex formal eine positive Ladung trägt, schließen beispielsweise Salze mit geeigneten Anionen ein, wie Carboxylate, Sulfonate, Sulfate, Chloride, Bromide, Iodide, Phosphate, Tartrate, Methansulfonate, Hydroxyethansulfonate, Glycinate, Maleate, Propionate, Fumarate, Toluolsulfonate, Benzolsulfonate, Trifluoracetate, Naphthalindisulfonate-1,5, Salicylate, Benzoate, Lactate, Salze der Äpfelsäure, Salze der 3-Hydroxy-2-naphthoesäure-2, Citrate und Acetate ein.

Pharmazeutisch annehmbare Salze der erfindungsgemäßen Verbindungen, in denen der Eisen(III)-Komplex formal eine negative Ladung trägt, schließen beispielsweise Salze mit geeigneten pharmazeutisch annehmbaren Basen ein, wie z.B. Salze mit Alkali- oder Erdalkalihydroxiden, wie NaOH, KOH, $Ca(OH)_2$, $Mg(OH)_2$ etc., Aminverbindungen, wie Ethylamin, Diethylamin, Triethylamin, Ethyldiisopropylamin, Ethanolamin, Diethanolamin, Triethanolamin, Methylglucamin, Dicyclohexylamin, Dimethylaminoethanol, Prokain, Dibenzylamin, N-Methylmorpholin, Arginin, Lysin, Ethylendiamin, N-Methylpiperidin 2-Amino-2-methyl-propanol-(1), 2-Amino-2-methyl-propandiol-(1,3), 2-Amino-2-hydroxylmethyl-propandiol-(1,3) (TRIS) etc..

Die Wasserlöslichkeit bzw. die Löslichkeit in physiologischer Kochsalzlösung und damit gegebenenfalls auch die Wirksamkeit der erfindungsgemäßen Verbindungen kann durch Salzbildung generell, speziell auch durch die Wahl des Gegenions signifikant beeinflusst werden.

Bevorzugt stellen die erfindungsgemäßen Verbindungen neutrale Komplex-Verbindungen dar.

**Vorteilhafte pharmakologische Effekte:**

Die Erfinder fanden überraschend, dass die Eisen(III)-ß-ketoamid-Komplexverbindungen, die Gegenstand der vorliegenden Erfindung sind, und die insbesondere durch die allgemeine Strukturformel (II) dargestellt werden, stabile bioverfügbare Eisenkomplexe sind und zur Verwendung als Arzneimittel zur Behandlung und Prophylaxe von Eisenmangelerscheinungen und Eisenmangelanämien sowie der damit einhergehenden Symptome geeignet sind.

Die erfindungsgemäßen Verbindungen enthaltende Arzneimittel eignen sich zum Einsatz in der Human- und der Veterinärmedizin.

Die erfindungsgemäßen Verbindungen eignen sich somit auch zur Herstellung eines Medikamentes zur Behandlung von Patienten, die unter Symptomen einer Eisenmangelanämie wie zum Beispiel: Ermüdung, Antriebslosigkeit, Konzentrationsschwäche, geringe kognitive Effizienz, Schwierigkeiten beim Finden der richtige Worte, Vergesslichkeit, unnatürliche Blässe, Reizbarkeit, Beschleunigung der Herzfrequenz (Tachykardie), wunde oder geschwollene Zunge, vergrößerte Milz, Schwangerengelüste (Pica), Kopfschmerzen, Appetitlosigkeit, erhöhte Infektionsanfälligkeit oder einer depressive Verstimmungen leiden.

Die erfindungsgemäßen Eisen(III)-Komplexverbindungen eignen sich weiterhin zur Behandlung der Eisenmangelanämie bei Schwangeren, der latenten Eisenmangelanämie bei Kindern und Heranwachsenden, der Eisenmangelanämie infolge gastrointestinaler Abnormalitäten, der Eisenmangelanämie infolge von Blutverlusten, wie durch gastrointestinale Blutungen (z.B. infolge von Geschwüren, Karzinomen, Hämorriden, entzündlichen Störungen, Einnahme von Acetylsalicylsäure), der Eisenmangelanämie infolge von Menstruation, der Eisenmangelanämie infolge von Verletzungen, der Eisenmangelanämie infolge von Psilosis (sprue), der Eisenmangelanämie infolge verminderter Eisenaufnahme bei der Ernährung, insbesondere bei selektiv essenden Kindern und Heranwachsenden, der Immunschwäche hervorgerufen durch Eisenmangelanämie, der Beeinträchtigung der Hirnleistung hervorgerufen durch Eisenmangelanämien, des Restless Leg Syndrom hervorgerufen durch Eisenmangelanämien, Eisenmangelanämien bei Krebs, Eisenmangelanämien ausgelöst durch Chemotherapien, Eisenmangelanämien ausgelöst durch Inflammation (AI), Eisenmangelanämien bei kongestiver Herzinsuffizienz (CHF;congestive heart failure), Eisenmangelanämien bei chronischer Niereninsuffizienz Stadium 3 -5 (CDK 3-5; chronic kidney diseases stage 3-5), Eisenmangelanämien ausgelöst durch chronische Inflammation (ACD), Eisenmangelanämien bei rheumatischer Arthritis (RA; rheumatoid arthritis), Eisenmangelanämien bei systemischem Lupus erythemotodes (SLE; systemic lupus erythematosus) und Eisenmangelanämien bei inflammatorischen Darmerkrankungen (IBD; inflammatory bowel diseases).

Die Verabreichung kann über einen Zeitraum von mehreren Monaten bis zur Verbesserung des Eisenstatus,

reflektiert beispielsweise durch den Hämoglobin-Wert, die Transferrin-Sättigung und den Serum Ferritin-Wert der Patienten, oder zur gewünschten Verbesserung einer durch Eisenmangelanämie hervorgerufenen Beeinträchtigung des Gesundheitszustandes erfolgen.

Das erfindungsgemäße Präparat kann von Kindern, Jugendlichen und Erwachsenen eingenommen werden.

Die erfindungsgemäßen Verbindungen angewendeten Verbindungen können dabei sowohl oral als auch parenteral verabreicht werden. Bevorzugt ist die orale Verabreichung.

Die erfindungsgemäßen Verbindungen sowie die vorgenannten Kombinationen der erfindungsgemäßen Verbindungen mit weiteren Wirkstoffen oder Arzneimitteln können somit insbesondere zur Herstellung von Medikamenten zur Behandlung der Eisenmangelanämie verwendet werden, wie Eisenmangelanämien bei Schwangeren, der latenten Eisenmangelanämie bei Kindern und Heranwachsenden, der Eisenmangelanämie infolge gastrointestinaler Abnormalitäten, der Eisenmangelanämie infolge von Blutverlusten, wie durch gastrointestinale Blutungen (z.B. infolge von Geschwüren, Karzinomen, Hämorriden, entzündlichen Störungen, Einnahme von Acetylsalicylsäure), Menstruation, Verletzungen, Eisenmangelanämie infolge von Psilosis (sprue), Eisenmangelanämie infolge verminderter Eisenaufnahme bei der Ernährung, insbesondere bei selektiv essenden Kindern und Heranwachsenden, Immunschwäche hervorgerufen durch Eisenmangelanämie, Beeinträchtigung der Hirnleistung hervorgerufen durch Eisenmangelanämie, Restless Leg Syndrom.

Die erfindungsgemäße Anwendung führt zu einer Verbesserung der Eisen-, Hämoglobin-, Ferritin- und Transferrinwerte, welche insbesondere bei Heranwachsenden und Kindern aber auch bei Erwachsenen mit einer Verbesserung im Kurzzeitgedächtnistests (STM), im Langzeitgedächtnistest (LTM), im Test der progressiven Matrices nach Raven, in der Welschers Erwachsenenintelligenzskala (WAIS) und/oder im emotionalen Koeffizienten (Baron EQ-i, YV-Test; Jugendversion), oder zu einer Verbesserung der Neutrophilen-Niveaus, der Antikörperniveaus und/oder der Lymphozytenfunktion einhergeht.

Die vorliegende Erfindung betrifft weiterhin pharmazeutische Zusammensetzungen, enthaltend eine oder mehrere der erfindungsgemäßen Verbindungen, insbesondere nach Formel (II), sowie gegebenenfalls eine oder mehrere weitere pharmazeutisch wirksame Verbindung sowie gegebenenfalls einen oder mehrere pharmakologisch verträgliche Träger und/oder Hilfsstoffe und/oder Lösungsmittel.

Dabei handelt es sich um übliche pharmazeutische Träger, Hilfsstoff oder Lösungsmittel. Genannte pharmazeutische Zusammensetzungen sind beispielsweise geeignet zur intravenösen, intraperitonealen, intramuskulären, intravaginalen, intrabuccalen, perkutanen, subkutanen, mucokutanen, oralen, rektalen, transdermalen, topikalen, intradermalen, intragastralen oder intrakutanen Applikation und liegen beispielsweise in der Form von Pillen, Tabletten, magensaftresistenten Tabletten, Filmtabletten, Schichttabletten, Retardformulierungen zur oralen, subkutanen oder kutanen Verabreichung (insbesondere als Pflaster), Depotformulierung, Dragees, Zäpfchen, Gelen, Salben, Sirup, Granulaten, Suppositorien, Emulsionen, Dispersionen, Mikrokapseln, Mikroformulierungen, Nanoformulierungen, liposomalen Formulierungen, Kapseln, magensaftresistente Kapseln, Pulvern, Inhalationspulvern, mikrokristallinen Formulierungen, Inhalationssprays, Puder, Tropfen, Nasentropfen, Nasensprays, Aerosolen, Ampullen, Lösungen, Säften, Suspensionen, Infusionslösungen oder Injektionslösungen etc. vor.

Bevorzugt werden die erfindungsgemäßen Verbindungen sowie pharmazeutische Zusammensetzungen, enthaltend solche Verbindungen oral appliziert obwohl auch andere Formen wie parenteral, insbesondere intravenös möglich sind.

Dazu liegen die erfindungsgemäßen Verbindungen bevorzugt in pharmazeutischen Zusammensetzungen in Form von Pillen, Tabletten, magensaftresistenten Tabletten, Filmtabletten, Schichttabletten, Retardformulierungen zur oralen Verabreichung, Depotformulierungen, Dragees, Granulaten, Emulsionen, Dispersionen, Mikrokapseln, Mikroformulierungen, Nanoformulierungen, liposomalen Formulierungen, Kapseln, magensaftresistente Kapseln, Pulvern, mikrokristallinen Formulierungen, Puder, Tropfen, Ampullen, Lösungen, Suspensionen, Infusionslösungen oder Injektionslösungen vor.

Die erfindungsgemäßen Verbindungen können in pharmazeutischen Zusammensetzung verabreicht werden, die verschiedene organische oder anorganische Träger- und/oder Hilfsmaterialien enthalten können, wie sie üblicherweise für pharmazeutische Zwecke insbesondere für feste Arzneimittelformulierungen verwendet werden, wie beispielsweise Exzipienten (wie Saccharose, Stärke, Mannit, Sorbit, Lactose, Glucose, Cellulose, Talk, Calciumphosphat, Calciumcarbonat), Bindemittel (wie Cellulose, Methylcellulose, Hydroxypropylcellulose, Polypropylpyrrolidon, Gelatine, Gummiarabicum, Polyethylenglykol, Saccharose, Stärke), Desintegrationsmittel (wie Stärke, hydrolysierte Stärke, Carboxymethylcellulose, Calciumsalz von Carboxymethylcellulose, Hydroxypropylstärke, Natriumglycolstärke, Natriumbicarbonat, Calciumphosphat, Calciumcitrat), Gleit- bzw. Schmiermittel (wie Magnesiumstearat, Talk, Natriumlaurylsulfat), ein Geschmacksbildner (wie Citronensäure, Menthol, Glycin, Orangenpulver), Konservierungsmittel (wie Natriumbenzoat, Natriumbisulfit, Methylparaben, Propylparaben), Stabilisatoren (wie Citronensäure, Natriumcitrat, Essigsäure, und Multicarbonsäuren aus der Titriplex Reihe wie z.B. Diethylentriaminpentaessigsäure (DTPA), Suspendiermittel (wie Methylcellulose, Polyvinylpyrrolidon, Aluminiumstearat), Dispergiermittel, Verdünnungsmittel (wie Wasser, organische Lösungsmittel), Bienenwachs, Kakaobutter, Polyethylenglykol, weißes Petrolatum etc..

Flüssige Arzneimittelformulierungen, wie Lösungen, Suspensionen und Gele enthalten üblicherweise einen flüssigen Träger, wie Wasser und/oder pharmazeutisch verträgliche organische Lösungsmittel. Weiterhin können derartige flüssigen Formulierungen auch pH-einstellende Mittel, Emulgatoren oder dispergierende Agenzien, puffernde Agenzien, Konservierungsmittel, Netzmittel, Geliermittel (beispielsweise Methylcellulose), Färbemittel und/oder Aromastoffe enthalten. Die Zusammensetzungen können isotonisch sein, das heisst diese können den gleichen osmotischen Druck wie Blut haben. Die Isotonie der Zusammensetzung kann durch die Verwendung von Natriumchlorid oder anderer pharmazeutisch annehmbare Agenzien wie beispielsweise Dextrose, Maltose, Borsäure, Natriumtartrat, Propylenglykol oder andere anorganische oder organisch lösliche Substanzen eingestellt werden. Die Viskosität der flüssigen Zusammensetzungen kann unter Verwendung eines pharmazeutisch annehmbaren Verdickungsmittels, wie Methylcellulose eingestellt werden. Andere geeignete Verdickungsmittel umfassen beispielsweise Xanthan, Carboxymethylcellulose, Hydroxypropylcellulose, Carbomer und dergleichen. Die bevorzugte Konzentration des Verdickungsmittels wird von dem ausgewählten Agens abhängen. Pharmazeutisch annehmbare Konservierungsmittel können verwendet werden, um die Haltbarkeit der flüssigen Zusammensetzung zu erhöhen. Benzylalkohol kann geeignet sein, obwohl eine Vielzahl von Konservierungsmitteln einschließlich beispielsweise Paraben, Thimerosal, Chlorbutanol oder Benzalkoniumchlorid ebenfalls verwendet werden können.

Der Wirkstoff kann beispielsweise mit einer Einheitsdosis von 0,001 mg/kg bis 500 mg/kg Körpergewicht beispielweise bis zu 1 bis 4 mal am Tag verabreicht werden. Die Dosierung kann jedoch je nach Alter, Gewicht, Zustand des Patienten, Schwere der Erkrankung oder Art der Verabreichung erhöht oder herabgesetzt werden.

## BEISPIELE

Die Erfindung wird durch die folgenden Beispiele näher veranschaulicht. Die Beispiele stellen lediglich Exemplifizierungen dar, und der Fachmann ist in der Lage, die spezifischen Beispiele auf weitere beanspruchte Verbindungen auszudehnen. Die Bezeichnung der Beispielnamen wurde mit Hilfe des Computerprogrammes ACD/Name Version 12 bestimmt und festgelegt.

### AUSGANGSVERBINDUNGEN:

Die in den Beispielen verwendeten Ausgangsverbindungen wurden wie folgt erhalten.

### A. *N,N*-Diethyl-3-oxobutanamid

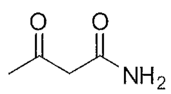

Kommerziell erhältlich: Fluka 00405

### B. *N,N*-Diethyl-3-oxobutanamid

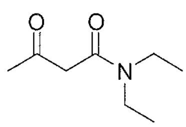

Kommerziell erhältlich: Aldrich 165093

### C. *N,N*-Dimethyl-3-oxobutanamid

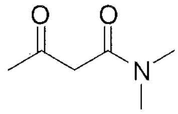

Kommerziell erhältlich: Aldrich 407054

**D. *N*-Methyl-3-oxobutanamid**

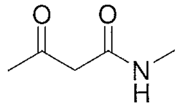

Kommerziell erhältlich: Acros 25544

**E. 2-Chlor-*N*-methyl-3-oxobutanamid**

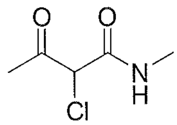

Kommerziell erhältlich: Aurora Fine Chemicals Ltd. Kafd-00164

**F. 1-(Piperidin-1-yl)-butan-1,3-dion**

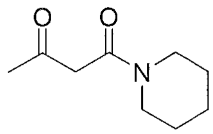

Eine Lösung von 25 g (0,294 mol) Piperidin in 100 ml *tert.*-Butyl-methylether wurde zu einer Lösung von 26,25 g (0,310 mol) Diketen in 200 ml *tert.*-Butylmethylether tropfenweise bei -5 bis 0°C zugegeben. Nach 1 Stunde Rühren bei - 1°C wurde dünnschichtchromatographisch kein Edukt mehr detektiert Das Reaktionsgemisch wurde abrotiert und der Rückstand im Vakuum destilliert. Man erhielt 49,55 g Produkt als leicht gelbliche Flüssigkeit.
IR (in Substanz, $cm^{-1}$): 3001, 2936, 2856, 1719, 1631, 1584, 1486, 1441, 1389, 1355, 1302, 1255, 1219, 1159, 1137.
Elementaranalyse: C 63,51; H 8,94; N 8,36.
LC-MS: 170 (M+H), 192 (M+Na)
$^1H$-NMR ($CDCl_3$, 400 MHz): $\delta$ [ppm] = 35 (4H), 3.3 (2H), 2.2 (3H), 1.6 (2H), 1.5 (4H).

**G. *N,N*-Bis-(2-hydroxyethyl)-3-oxobutanamid**

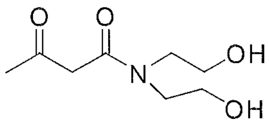

Eine Lösung von 52,53 g (0,50 mol) Diethanolamin in 100 ml MeOH wurde zu einer Lösung von 40,00 g (0,48

mol) Diketen in 100 ml MeOH tropfenweise bei 0°C zugegeben. Nach 1 Stunde Rühren bei 0°C wurde dünnschichtchromatographisch kein Edukt mehr detektiert. Das Reaktionsgemisch wurde abrotiert und der Rückstand mittels Säulechromatographie gereinigt. Man erhielt 81,32 g Produkt als leicht gelbes Öl. IR (in Substanz, $cm^{-1}$): 3381, 2941, 2885, 1718, 1626, 1481, 1432, 1361, 1312, 1212, 1053.
Elementaranalyse: C 50,62; H 8,05; N 7,28.
$^1$H-NMR ($CDCl_3$, 400 MHz): $\delta$ [ppm] = 2.2 (3H), 3.5 (4H), 3.7 (6H), 4.15 (1H), 4.35 (1 H).

**H. *N*-Butyl-*N*-methyl-3-oxobutanamid**

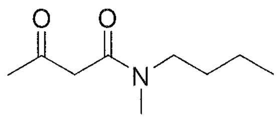

14,1 ml (0,12 mol) N-n-Butylmethylamin wurden zu einer Lösung von 10,00 g (0,12 mol) Diketen in 500 ml MeOH tropfenweise bei 0°C zugegeben. Nach 6 Stunden Rühren bei 0°C wurde dünnschichtchromatographisch kein Edukt mehr detektiert. Das Reaktionsgemisch wurde abrotiert und der Rückstand mittels Säulenchromatographie gereinigt. Man erhielt 11,61 g Produkt als leicht gelbes Öl. IR (in Substanz, $cm^{-1}$): 2958, 2932, 2873, 1721,.1635, 1593, 1493, 1381, 1358, 1307, 1228, 1209, 1144.

LC-MS: 172(M+H).
$^1$H-NMR ($CDCl_3$, 400 MHz): $\delta$ [ppm] = 0.9 (3H), 1.3 (2H), 1.5 (2H), 1.9 (1H), 2.2 (2H), 2.9 (3H), 3.2 (1H), 3.3 (1H), 3.5 (1H).

**I. 1-(4-Hydroxypiperidin-1-yl)-butan-1,3-dion**

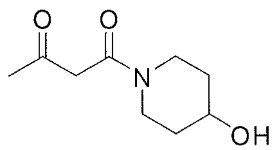

Eine Lösung von 25,30 g (0,25 mol) 4-Hydroxy-piperidin in 50 ml MeOH wurde zu einer Lösung von 20,00 g (0,24 mol) Diketen in 50 ml MeOH tropfenweise bei 0°C zugegeben. Nach 1 Stunden Rühren bei 0°C wurde dünnschichtchromatographisch kein Edukt mehr detektiert. Das Reaktionsgemisch wurde abrotiert und der Rückstand mittels Säulenchromatographie gereinigt. Man erhielt 42,54 g (96% Ausbeute) leicht gelbes Öl.
IR (in Substanz, $cm^{-1}$): 3401, 2928, 2869, 1717, 1617, 1448, 1360, 1307, 1268, 1206, 1158, 1074, 1026.
CHN-Elementaranalyse: C 57,98; H 8,69; N 7,32.
LC-MS: 186(M+H).
$^1$H-NMR ($CDCl_3$, 400 MHz): $\delta$ [ppm] = 1.5 (2H), 1.8 (2H), 2.2 (3H), 3.2 (2H), 3.6 (3H), 3.9 (2H).

**J. 3-Oxo-*N,N*-dipropylbutanamid**

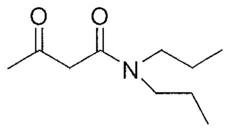

Eine Lösung von 27,80 g (0,28 mol) N,N-Di-n-propylamin wurde zu einer Lösung von 20,00 g (0,24 mol) Diketen in 100 ml MeOH tropfenweise bei 0°C zugegeben. Nach 3 Stunden Rühren bei 0°C wurde dünnschichtchromatographisch kein Edukt mehr detektiert. Das Reaktionsgemisch wurde abrotiert und der Rückstand mittels Säulenchromatographie gereinigt. Man erhielt 38,05 g Produkt als leicht gelbes Öl.

IR (in Substanz, $cm^{-1}$): 2964, 2934, 2876, 1721, 1634, 1589, 1490, 1456, 1430, 1392, 1381, 1359, 1301, 12444, 1101.
Elementaranalyse: C 64,28; H 10,17; N 7,36.
$^1$H-NMR ($CDCl_3$, 400 MHz): δ [ppm] = 0.9 (6H), 1.6 (4H), 1.9 (1H), 2.3 (2H), 3.1 (2H), 3.3 (2H), 3.5 (2H).

**K. *N*-Hexyl-3-oxobutanamid**

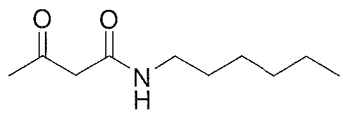

Eine Lösung von 27,83 g (0,28 mol) N-n-Hexylamin wurde zu einer Lösung von 20,00 g (0,24 mol) Diketen in 100 ml MeOH tropfenweise bei 0°C zugegeben. Nach 4 Stunden Rühren bei 0°C wurde dünnschichtchromatographisch kein Edukt mehr detektiert. Das Reaktionsgemisch wurde abrotiert und der erhaltene Feststoff in 200 ml n-Hexan aufgenommen, abfiltriert und im Vakuum über Nacht getrocknet. Man erhielt 22,53 g Produkt als weissen Feststoff.
IR (in Substanz, $cm^{-1}$): 3272, 3098, 2956, 2921, 2872, 2853, 1728, 1709, 1644, 1563, 1467, 1419, 1363, 1347, 1336, 1190, 1167.
Elementaranalyse: C 64,85; H 10,28; N 7,62.
$^1$H-NMR ($CDCl_3$, 400 MHz): δ [ppm] = 0.9 (3H), 1.3 (6H), 1.5 (2H), 2.2 (3H), 3.2 (2H), 3.4 (2H), 6.9 (1H).

**L. Ethyl- *N*-(3-Oxobutanoyl)glycinat**

**(3-Oxo-butyrylamino)essigsäurethylester)**

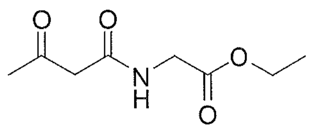

Eine Lösung von 20,00 g (0,24 mol) Diketen in 250 ml Toluol wurde tropfenweise bei 0°C zu einer Lösung von 33,21 g (0,24 mol) Glycinethylester-Hydrochlorid (Aminoessigsäureethylester-Hydrochlorid) zugegeben. Dann wurden 40,00 g (0,48 mol) $NaHCO_3$ zugegeben und das Reaktionsgemisch über Nacht bei Raumtemperatur weitergerührt. Da dünnschichtchromatographisch kein Edukt mehr detektiert werden konnte, wurde das Reaktionsgemisch abfiltriert, das Filtrat abrotiert, der erhaltene Feststoff in 100 ml Diethylether aufgenommen, kurz gerührt, abfiltriert und im Vakuum über Nacht getrocknet. Man erhielt 40,00 g (0,21 mol, 89 % Ausbeute) Produkt als weissen Feststoff.
IR (in Substanz, $cm^{-1}$): 3346, 1750, 1708, 1669, 1538, 1399, 1360, 1320, 1278, 1198, 1165, 1024.
Elementaranalyse: C 51,57; H 7,05; N 7,55.
LC-MS: 210 (M+Na), 188 (M+H).
$^1$H-NMR ($CDCl_3$, 400 MHz): δ [ppm] = 1.2 (3H), 2.2 (3H), 3.4 (2H), 4.0 (2H), 4.2 (2H), 7.4 (1H).

**M. 3-Oxoheptanamid**

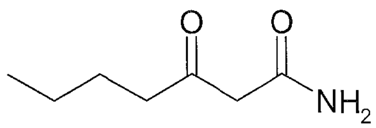

0,4 mol (63,7 ml) 3-Oxo-heptansäuremethylester und 400 ml 7 N Ammoniaklösung wurden im Druckreaktor 3 Stunden bei 100°C gerührt. Nach dem Abkühlen wurden die Lösungsmittel am Rotationsverdampfer abdestilliert, das Rohprodukt in 200 ml Wasser und 40 ml Ethanol aufgenommen und mit 40 ml 32% HCl auf ca. pH 3 eingestellt. Das Reaktionsgemisch wurde 4 Stunden auf 90°C geheizt und erneut am Rotationsverdampfer zur Trockne eingeengt, in 600 ml Dichlormethan aufgenommen und 3x mit 100 ml Wasser gewaschen. Die organische Phase wurde abdestilliert

und das Rohprodukt aus 100 ml Toluol umkristallisiert. Das Produkt wurde abfiltriert und im Vakuumtrockenschrank bei 50°C getrocknet. Man erhielt 3,5 g der Titelverbindung.
IR (in Substanz, $cm^{-1}$): 3368, 3178, 2956, 2932, 2872, 1703, 1651, 1619, 1466, 1439, 1408, 1376, 1345, 1306, 1221, 1183, 1125, 1058, 716, 663. Elementaranalyse: C 58,38; H 9,12; N 9,90.
LC-MS: 144 (M+H).
$^1$H-NMR (DMSO-$d_6$, 400 MHz): δ [ppm] = 14.5 (s, 1H), 7.45 (s, 1H), 7.01 (s, 1H), 4.92 (s, 1H), 2.06 (t, 2H), 1.47-1.38 (m, 3H), 1.31-1.18 (m, 2H), 0.88-0.81 (m, 3H); Keto-Tautomer (86%), δ = 7.45 (s, 1H), 7.01 (s, 1H), 3.23 (s, 2H), 2.49 (t, 2H), 1.47-1.38 (m, 2H), 1.31-1.18 (m, 2H), 0.88-0.81 (m, 3H).

**N. *N*-Methyl-3-oxoheptanamid**

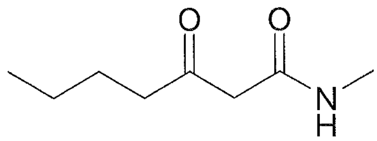

0,378 mol (60,2 ml) 3-Oxo-heptansäuremethylester, 400 ml 33% Methylamin Lösung in Ethanol und 40 ml Wasser wurden im Rundkolben mit Rückflusskühler 48 Stunden bei 80°C gerührt. Nach dem Abkühlen wurden die Lösungsmittel am Rotationsverdampfer abdestilliert, das Rohprodukt in 200 ml Wasser und 40 ml Ethanol aufgenommen und mit 40 ml 32% HCl auf ca. pH 3 eingestellt. Das Reaktionsgemisch wurde 24 Stunden auf 90°C geheizt und erneut am Rotationsverdampfer zur Trockne eingeengt. Das Rohprodukt wurde mit 150 ml Toluol auf Rückfluss geheizt, heiss filtriert und das Filtrat abkühlen gelassen. Das Produkt wurde abfiltriert und im Vakuumtrockenschrank bei 50°C getrocknet. Man erhielt 5,9 g der Titelverbindung.
IR (in Substanz, $cm^{-1}$): 3368, 3178, 2956, 2932, 2872, 1703, 1651, 1619, 1466, 1439, 1408, 1376, 1345, 1306, 1221, 1183, 1125, 1058, 716, 663. Elementaranalyse: C 60,22; H 9,69; N 8,75.
$^1$H-NMR (DMSO-$d_6$, 400 MHz): δ [ppm] = Keto-Tautomer (ca. 90%), Enol-Tautomer* (ca. 10%), = 8.21*/7.81* (m, 1H), 7.94 (s, 1H), 6.12*/4.91* (s, 1H), 3.25 (s, 2H), 2.69*/2.61* (d, 3H), 2.57 (d, 3H), 2.50-2.46 (m, 2 H), 2.06* (t, 2H), 1.45-1.38 (m, 2H), 1.31-1.18 (m, 2H), 0.88-0.81 (m, 3H)
(*Enol-Tautomer vermutlich als E/Z Isomere der Amidbindung vorliegend)

**O. *N,N*-Dimethyl-3-oxoheptanamid**

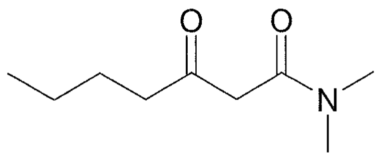

0,632 mol (100 ml) Methyl-3-oxoheptanoat (3-Oxo-heptansäuremethylester) und 227 ml 33% Dimethylaminlösung in Ethanol (ca. 1,26 mol Dimethylamin) wurden im Druckreaktor 4 Stunden bei 110°C gerührt. Nach dem Abkühlen wurden die Lösungsmittel am Rotationsverdampfer abdestilliert, das Rohprodukt in 40 ml Wasser, 30 ml 32%-ige HCl und 40 ml Ethanol aufgenommen und 24 Stunden bei 100°C gerührt. Das Gemisch wurde erneut zur Trockne eingedampft, in 300 ml Essigsäureethylester wieder aufgenommen und filtriert, anschliessend wurden die Lösungsmittel am Rotationsverdampfer abdestilliert. 26 g Rohprodukt wurden über 500 g Kieselgel 60 gereinigt (Laufmittel: Essigsäureethylester : Hexan 1:1, Fluss: 50 ml/min, Fraktionsgrösse: 100 ml, Produkt: Fraktionen 20-35), das Produkt wurde als leichtbewegliches Öl erhalten. Man erhielt 17,8 g der Titelverbindung.
IR (in Substanz, $cm^{-1}$): 2957, 2932, 2873, 1717, 1639, 1597, 1501, 1465, 1396, 1366, 1302, 1262, 1197, 1142, 1058, 933, 776, 726, 692, 645, 612. Elementaranalyse: C 62,12; H 9,965; N 8,17.
$^1$H-NMR (DMSO-$d_6$, 400 MHz): δ [ppm] = Enol-Tautomer (28%), δ = 15.1 (s, 1H), 5.32 (s, 1H), 3.3-2.4 (m, 6H), 2.13 (t, 2H), 1.51-1.39 (m, 2H), 1.34-1.18 (m, 2H), 0.89-0.82 (m, 3H); Keto-Tautomer (72%), δ = 3.57 (s, 2H), 2.88 (s, 3H), 2.80 (s, 3H), 2.50-2.48 (m, 2H), 1.51-1.39 (m, 2H), 1.34-1.18 (m, 2H), 0.89-0.82 (m, 3H).

**P. 3-Oxopentanamid**

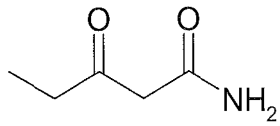

0,8 mol (100 ml) Methyl-3-oxovalerat (3-Oxo-pentansäuremethylester) und 220 ml 7 N Ammoniaklösung in Methanol wurden im Druckreaktor 4 Stunden bei 110°C gerührt. Nach dem Abkühlen wurden die Lösungsmittel am Rotationsverdampfer abdestilliert, das Rohprodukt in 100 ml Ethanol aufgenommen und filtriert. Das Filtrat wurde eingeengt, der Rückstand in 40 ml Diethylether gelöst und 2 Tage bei +5°C stehen gelassen. Das in feinen Nadeln kristallisierte Produkt wurde abfiltriert und im Vakuumtrockenschrank bei 50°C getrocknet. Man erhielt 7,10 g der Titelverbindung.
IR (in Substanz, $cm^{-1}$): 3370, 3177, 2974, 2937, 2878, 1705, 1651, 1620, 1440, 1384, 1355, 1301, 1268, 1189, 1109, 1043, 999, 911, 863, 804, 740, 659. Elementaranalyse: C 51,48; H 7,924; N 12,28.
$^1$H-NMR (DMSO-$d_6$, 400 MHz): $\delta$ [ppm] = Enol-Tautomer (12%), $\delta$ = 14.5 (s, 1H), 7.45 (s, 1H), 7.01 (s, 1H), 4.93 (s, 1H), 2.09 (q, 2H), 1.00 (t, 3H); Keto-Tautomer (88%), $\delta$ = 7.45 (s, 1H), 7.01 (s, 1H), 3.24 (s, 2H), 2.5 (q überlagert, 2H), 0.90 (t, 3H).

**Q. *N,N*-Dimethyl-3-oxopentanamid**

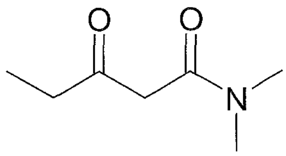

0,8 mol (100 ml) Methyl-3-oxovalerat (3-Oxo-pentansäuremethylester) und 250 ml 33% Dimethylaminlösung in Ethanol (ca. 1,4 mol Dimethylamin) wurden im Druckreaktor 4 Stunden bei 110°C gerührt. Nach dem Abkühlen wurden die Lösungsmittel am Rotationsverdampfer abdestilliert, das Rohprodukt in 40 ml Wasser, 30 ml 32% HCl und 40 ml Ethanol aufgenommen und 24 Stunden bei 100°C gerührt. Das Gemisch wurde erneut zur Trockne eingedampft, in 300 ml Essigsäureethylester wieder aufgenommen und filtriert. Das Filtrat wurde zur Trockne eingedampft und das Produkt am Ölpumpenvakuum fertig getrocknet. Man erhielt 25,8 g der Titelverbindung.
IR (in Substanz, $cm^{-1}$): 2976, 2939, 1717, 1637, 1500, 1459, 1396, 1376, 1357, 1298, 1262, 1198, 1142, 1107, 1055, 967, 912, 775, 647, 611. Elementaranalyse: C 54,78; H 8,724; N 9,20.
$^1$H-NMR (DMSO-$d_6$, 400 MHz): $\delta$ [ppm] = Enol-Tautomer (12%), $\delta$ = 15.1 (s, 1H), 5.31 (s, 1H), 2.89 (s, 3H), 2.81 (s, 3H), 2.15 (q, 2H), 1.04 (t, 3H); Keto-Tautomer (87%), $\delta$ = 3.57 (s, 2H), 2.89 (s, 3H), 2.81 (s, 3H), 2.49 (q, 2H), 0.91 (t, 3H).

**R. 3-Oxo-2-(piperidin-1-ylcarbonyl)butannitril**

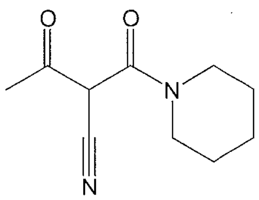

In einer Stickstoffatmosphäre wurden 45,6 g (0.30 mol) 1-Cyanoacetylpiperidin in 800 ml THF (trocken) vorgelegt. Es wurden 18,7 g (0,47 mol) NaH (60 % Mineralölsuspension) so zugegeben, dass die Innentemperatur 10 °C nicht überstieg (Kühlung mit Eis/NaCl-Bad). Anschliessend wurde 30 min bei 0 - 10 °C nachgerührt. 20 ml (0,28 mol)

Acetylchlorid wurden so zudosiert, dass die Innentemperatur 10 °C nicht überstieg. Anschliessend wurde die Reaktionsmischung 1 Stunden bei 45 °C nachgerührt. Danach wurden langsam 40 ml Essigsäure zugefügt, die Reaktionsmischung auf 2,0 l Eiswasser gegeben und 50 ml HCl (20 % m/m) zudosiert. Die Mischung wurde mit 500 ml Toluol ausgeschüttelt, die org. Phase separiert, über 120 g $Na_2SO_4$ getrocknet und filtriert. Die Lösung wurde bei 40 °C / 80 mbar am Rotationsverdampfer bis zu einem Öl eingeengt und mit einer Eis/Kochsalzmischung gekühlt. Der ausgefallene Feststoff wurde abfiltriert, mit 50 ml kaltem Toluol nachgewaschen und für mind. 15 Stunden bei 50 °C / < 100 mbar getrocknet. Man erhielt 35,1 g Produkt als Feststoff.
IR (in Substanz, $cm^{-1}$): 3009, 2943, 2863, 2206, 2147, 1763, 1659, 1573, 1483, 1455, 1275, 1229, 1131, 1022, 969.
Elementaranalyse: C 60,22; H 9,69; N 8,75.
$^1H$-NMR ($CDCl_3$, 400 MHz): $\delta$ [ppm] = 1.56 - 1.67 (m, 6H), 2.27 (s, 3H), 3.66 (t, 4H), 17.17 (br. s, 1H).

**S. *N*-Butyl-3-oxobutanamid**

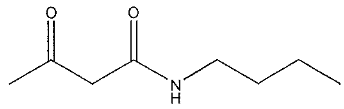

14,64 g (0,2 mol) n-Butylamin und 200 mg DMAP wurden in 60 ml THF (trocken) vorgelegt und 16.8 g (0,2 mol) Diketen bei - 5 bis 5 °C zudosiert. Danach wurde die Reaktionsmischung 2 Stunden bei 20 - 25 °C nachgerührt und anschliessend am Rotationsverdampfer aufkonzentriert. Der Rückstand wurde mind. 15 Stunden bei 50 °C / < 100 mbar getrocknet. Man erhielt 16,0 g Produkt.
IR (in Substanz, $cm^{-1}$): 3271, 3097, 2959, 2928, 2867, 1714, 1644, 1561, 1459, 1421, 1357, 1227, 1163, 1000, 968, 759, 724, 656, 619.
$^1H$-NMR ($CDCl_3$, 400 MHz): $\delta$ [ppm] = 0.86 - 0.94 (m, 3H), 1.26 - 1.71 (m, 4H), 2.23 (s, 3H), 3.23 (q, 2H), 3.36 (s, 2H), 7.00 (br. s, 1H).

**T. *N,N*-Dibutyl-3-oxobutanamid**

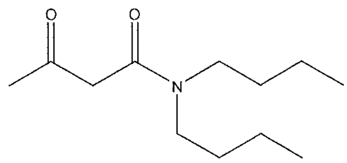

16,8 g (0.20 mol) Diketen wurden in 60 ml THF unter Stickstoff vorgelegt und die Lösung mittels eines Eis/NaCl-Gemisches auf ca. - 10 °C gekühlt. Anschliessend wurden 25,6 g (0.20 mol) Di-n-butylamin so zudosiert, dass die Innentemperatur 0 °C nicht überstiegen wurde. Die Reaktionsmischung wurde 1 Stunden bei - 10 bis 0 °C und anschliessend 2 Stunden bei 20 - 25 °C nachgerührt. Am Rotationsverdampfer wurde das Lösungsmittel der Reaktionsmischung bei 50 °C / < 100 mbar abdestilliert. Man erhielt 45,9 g Produkt als Öl.
Je 10 g dieses Rohprodukts wurden auf 200 g Kieselgel gegeben und mit 2 l Essigsäureethylester / n-Hexan (2 : 1 v/v) eluiert. Das Eluat wurde am Rotationsverdampfer bei 50 °C / < 100 mbar zu einem Öl eingeengt und für mind. 15 Stunden am Hochvakuum bei 20 - 25 °C weiter getrocknet. Man erhielt 31.5 g Produkt als Öl.
IR (in Substanz, $cm^{-1}$): 2958, 2932, 2873, 1722, 1635, 1590, 1490, 1458, 1431, 1392, 1372, 1292, 1226, 1144, 1113, 1037, 1007, 931, 774, 732, 683.
$^1H$-NMR ($CDCl_3$, 400 MHz): $\delta$ [ppm] / Keto-Tautomer $\delta$ = 0.87 - 0.92 (m, 3H), 1.25 - 1.31 (m, 2H), 1.47 - 1.50 (m, 2H), 2.23 (s, 3H), 3.26 - 3.30 (m, 2H), 3.45 (s, 2H); Enol-Tautomer $\delta$ = 0.87 - 0.92 (m, 3H), 1.25 - 1.31 (m, 2H), 1.47 - 1.50 (m, 2H), 1.91 (m, 3H), 3.13 - 3.17 (m, 2H), 5.00 (s, 1H), 14.95 (br. s, 1H).

**U. (nicht vergeben)**

**V. *N,N*-Dibutyl-3-oxopentanamid**

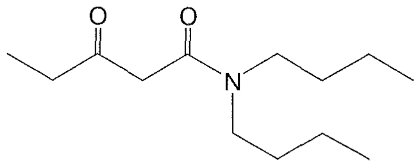

0,16 mol (20 ml) Methyl-3-oxovalerat (3-Oxo-pentansäuremethylester) und 0,415 mol (70 ml) Dibutylamin wurden 3 h bei 130°C gerührt, wobei entstehendes Methanol über eine Destillationsbrücke abgetrennt wurde. Danach wurde überschüssiges Dibutylamin am Rotationsverdampfer abdestilliert, das Rohprodukt in 40 ml Wasser und 90 ml Ethanol aufgenommen, mit 3 ml 20% HCl auf pH 3 eingestellt und 1 h bei 50°C gerührt. Das Gemisch wurde erneut zur Trockne eingedampft, in 200 ml Essigsäureethylester wieder aufgenommen und 3x mit 100 ml Wasser gewaschen. Die organische Phase wurde mit $Na_2SO_4$ getrocknet und nach dem Filtrieren am Rotationsverdampfer zur Trockne eingeengt. Nach dem Trocknen wurden 31.8 g der Titelverbindung erhalten.
IR (in Substanz, $cm^{-1}$): 2959, 2933, 2874, 1721, 1631, 1589, 1490, 1458, 1429, 1393, 1374, 1320, 1292, 1250, 1223, 1188, 1144, 1111, 1061, 940, 914, 800, 775, 732, 689, 635.
CHN-Elementaranalyse: C, 68.22; H, 11.336; N, 6.51.
LC-MS: $M+H^+$ 228.5; $M+Na^+$ 250.5.
1H-NMR (DMSO-$d_6$, 400 MHz): Enol-Tautomer (40%), $\delta$ = 15.1 (s, 1H), 5.31 (s, 1H), 2.89 (s, 3H), 2.81 (s, 3H), 2.15 (q, 2H), 1.04 (t, 3H); Keto-Tautomer (60%), $\delta$ = 3.57 (s, 2H), 2.89 (s, 3H), 2.81 (s, 3H), 2.49 (q, 2H), 0.91 (t, 3H).

**W. 2-Fluoro-*N,N*-dimethyl-3-oxobutanamid**

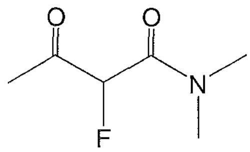

0,2 mol (25 ml) Ethyl-2-fluoroacetoacetat und 107 ml 33% Dimethylaminlösung in Ethanol (ca. 0,6 mol Dimethylamin) wurden 3 h bei 70°C gerührt. Das Reaktionsgemisch wurde zur Trockne eingedampft und 27 g Rohprodukt über 350 g Kieselgel mit Ethylacetat/Methanol 9/1 chromatographiert. Es wurden 13,4 g der Titelverbindung erhalten.
IR (in Substanz, $cm^{-1}$): 2942, 1729, 1650, 1501, 1403, 1358, 1260, 1217, 1176, 1151, 1072, 962, 829, 705, 681, 632, 611.
CHN-Elementaranalyse: C, 47.99; H, 6.70; N, 9.05.
LC-MS: $M+H^+$ 148.2; $M+Na^+$ 170.2.
1H-NMR (DMSO-$d_6$, 400 MHz): $\delta$= 5.96 (d (J = 48 Hz), 1H), 3.03 (d (J = 1.4 Hz), 3H), 2.86 (d (J = 1.3 Hz), 3H), 2.18 (d (J = 4.0 Hz), 3H).

**X. 2-Fluoro-3-oxo-*N*-propylbutanamid**

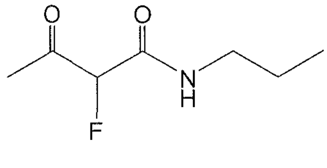

0,8 mol (65,8 ml) *n*-Propylamin wurden innerhalb von 0,5 h zu 0,2 mol (25 ml) Ethyl-2-fluoroacetoacetat getropft. Das Reaktionsgemisch erwärmte sich und wurde anschliessend noch 2 h bei 60°C gehalten. Dann wurde es zur Trockne

eingedampft, in 50 ml Wasser und 60 ml Ethanol gelöst und mit 32% HCl auf pH 3 gestellt. Es wurde erneut eingedampft, in 300 ml Ethylacetat gelöst, filtriert und erneut zur Trockne eingedampft. 17 g Rohprodukt wurden über 800 g Kieselgel mit Ethylacetat/Hexan 1/1 chromatografiert. Nach dem Trocknen wurden 14 g der Titelverbindung erhalten.
IR (in Substanz, $cm^{-1}$): 3325, 2967, 2938, 2878, 1736, 1665, 1535, 1460, 1441, 1421, 1359, 1277, 1235, 1210, 1179, 1150, 1089, 963, 892, 818, 617. CHN-Elementaranalyse: C, 50.61; H, 7.19; N, 8.58.
LC-MS: $M+H^+$, 162.7; $M+Na^+$ 184.6.
1H-NMR (DMSO-$d_6$, 400 MHz): $\delta$= 8.47 (s (breit), 1H), 5.49 (d (J = 49 Hz), 1H), 3.05 (m, 2H), 2.22 (d (J = 3.1 Hz), 3H), 2.18 (d (J = 4.0 Hz), 3H); 1.42 (sextett, 2H); 0.81 (t, 3H).

**Y. 4-Methoxy-*N,N*-dimethyl-3-oxobutanamid**

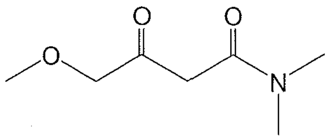

0,17 mol (25 g) Methyl-4-methoxy-acetoacetat und 91 ml 33% Dimethylaminlösung in Ethanol (ca. 0,51 mol Dimethylamin) wurden im Druckreaktor 4 h bei 110°C gerührt. Das Reaktionsgemisch wurde anschliessend zur Trockne eingedampft und 27 g Rohprodukt über 350 g Kieselgel mit Ethylacetat/Methanol 9/1 chromatografiert. Nach dem Trocknen wurden 12,4 g der Titelverbindung erhalten.
LC-MS: $M+H^+$, 160.7; $M+Na^+$, 182.6.
1H-NMR (DMSO-$d_6$, 400 MHz): Enol-Tautomer (21%), $\delta$ = 15.1 (s, 1H), 5.48 (s, 1H), 3.90 (s, 2H), 3.30 (s, 3H), 3.0-2.8 (m, 6H); Keto-Tautomer (79%), $\delta$ = 4.10 (s, 2H), 3.55 (s, 2H), 3.27 (s, 3H), 2.90 (s, 3H), 2.81 (s, 3H).

**Z. *N,N*,4-Trimethyl-3-oxopentanamid**

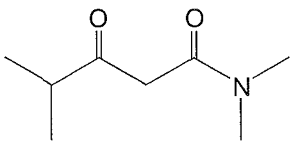

0,69 mol (100 g) 4-Methyl-3-oxovaleriansäuremethylester und 309 ml 33% Dimethylaminlösung in Ethanol (ca. 1.73 mol Dimethylamin) wurden im Druckreaktor 2 h bei 110°C gerührt. Nach dem Abkühlen wurden die Lösungsmittel am Rotationsverdampfer abdestilliert. Nach dem Trocknen wurden 93 g der Titelverbindung erhalten.
IR (in Substanz, $cm^{-1}$): 2969, 2934, 2875, 1714, 1630, 1597, 1502, 1467, 1396, 1384, 1359, 1322, 1263, 1207, 1162, 1142, 1073, 1047, 972, 927, 892, 782, 725, 701, 650, 614.
CHN-Elementaranalyse: C, 59.51; H, 9.60; N, 8.47.
LC-MS: $M+H^+$, 158.5; $M+Na^+$, 180.2.
1H-NMR (DMSO-$d_6$, 400 MHz): Enol-Tautomer (19%), $\delta$ = 15.18 (s, 1H), 5.28 (s, 1H), 2.87 (s, 3H), 2.80 (s, 3H), 2.35 (heptett, 1H), 1.05 (d, 6H); Keto-Tautomer (81%), $\delta$ = 3.62 (s, 2H), 2.87 (s, 3H), 2.80 (s, 3H), 2.68 (heptett, 1H), 1.00 (d, 6H).

**AA. 4-Methyl-1-(morpholin-4-yl)-pentan-1,3-dion**

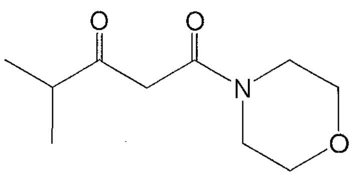

0,351 mol (49,6 ml) 4-Methyl-3-oxovaleriansäuremethylester und 0,369 mol (32,3 ml) Morpholin wurden 14 h bei 110°C am Wasserabscheider gerührt. Es wurden 8 ml Methanol aufgefangen. Anschliessend wurden die überschüssigen Startmaterialien 3 h am Rotationsverdampfer abdestilliert (80°C Wasserbad, 2 mbar). Das Rohprodukt wurde in 500 ml Toluol gelöst, mit 50 ml 1 M NaOH und 3x 50 ml Wasser extrahiert, und die organische Phase wurde am Rotationsverdampfer zur Trockne eingeengt. Nach dem Trocknen wurden 62,2 g der Titelverbindung erhalten.
IR (in Substanz, $cm^{-1}$): 2969, 2929, 2858, 1713, 1636, 1587, 1460, 1436, 1385, 1361, 1301, 1272, 1243, 1190, 1166, 1113, 1069, 1050, 1036, 984, 964, 929, 886,850,779,733,700,660,619.
CHN-Elementaranalyse: C, 58.04; H, 8.24; N, 6.95.
LC-MS: $M+H^+$, 200 9; $M+Na^+$, 222.8.
1H-NMR (DMSO-$d_6$, 400 MHz): Enol-Tautomer (16%), δ = 14.7 (s, 1H), 5.37 (s, 1H), 3.57-3.28 (m, 8H), 2.36 (heptett, 1H), 1.06 (d, 6H); Keto-Tautomer (84%), δ = 3.70 (s, 2H), 3.57-3.28 (m, 8H), 2.67 (heptett, 1H), 1.00 (d, 6H).

**AB. 4-Methoxy-1-(morpholin-4-yl)butan-1,3-dion**

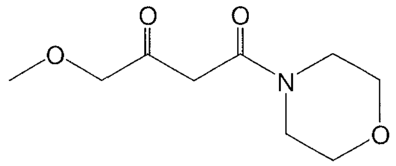

0,346 mol (50,5 g) Methyl-4-methoxy-acetoacetat und 0,363 mol (31,6 g) Morpholin wurden 20 h bei 110°C am Wasserabscheider gerührt. Es wurden nochmals 0,069 mol (6,02 g) Morpholin zugegeben und es wurde weitere 3 h gerührt. Anschliessend wurden die überschüssige Startmaterialien am Rotationsverdampfer abdestilliert. 67,3 g Rohprodukt wurden über 600 g Kieselgel mit Ethylacetat/Ethanol 9/1 chromatografiert. Es wurden 36,9 g der Titelverbindung erhalten.
IR (in Substanz, $cm^{-1}$): 2921, 2857, 2361, 1729, 1633, 1590, 1438, 1362, 1303, 1272, 1244, 1199, 1111, 1069, 1038, 1019, 984, 965, 939, 920, 849, 778, 718, 681,631.
LC-MS: $M+H^+$, 202.7; $M+Na^+$, 224.6.
1H-NMR (DMSO-$d_6$, 400 MHz): δEnol-Tautomer (14%), δ = 14.8 (s, 1H), 5.55 (s, 1H), 3.91 (s, 2H), 3.60-3.32 (m, 8H), 3.30 (s, 3H); Keto-Tautomer (86%), δ = 4.11 (s, 2H), 3.60 (s, 2H), 3.60-3.32 (m, 8H), 3.28 (s, 3H).

**AC. 3-Acetyl-1-methylpyrrolidin-2-on**

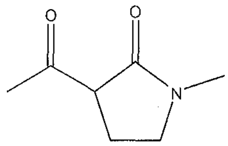

20,40 g (0.20 mol) Diisopropylamin wurden in 200 ml trockenem THF unter Stickstoffatmosphäre vorlegt und bei -78°C 80 ml n-Butyllithium in Hexan (2,5 M) zugetropft. Man liess für 20 Minuten rühren und tropfte anschliessend 20,00 g (0,20 mol) 1-Methyl-2-pyrrolidinon bei -78°C zu. Man rührte die Reaktionsmischung 1 Stunde und gab anschliessend bei -78°C 17,64 g (0.20 mol) trockenes Ethylacetat zu. Die Reaktionsmischung wurde auf Raumtemperatur erwärmt und für 14 Stunden gerührt. Das THF wurde am Rotationsverdampfer unter vermindertem Druck entfernt und der Rückstand in 80ml 6 N HCl aufgenommen. Man sättigte die saure, wässrige Phase mit NaCl und extrahiert 5x mit 300ml Ethylacetat. Die vereinten organischen Phasen wurden über $Na_2SO_4$ getrocknet und das Lösungsmittel am Rotationsverdampfer entfernt. Das zurückgebliebene gelbe Öl wurde säulenchromatographisch gereinigt. Man erhielt 5.10 g Produkt als leicht gelbes Öl.
IR (in Substanz, $cm^{-1}$): 3495, 2929, 2885m 1714, 1674, 1500, 1433, 1403, 1358, 1297, 1262, 1166, 1107, 989, 763, 712.
$^1$H-NMR ($CDCl_3$, 400 MHz): δ [ppm] = 1.95 (1H), 2.30 (3H), 2.45 (1H), 2.75 (3H), 3.25 (1H), 3.35 (1H), 3.5 (1H).

**AD. Ethyl (1-methyl-2-oxopyrrolidin-3-yl)(oxo)acetat**

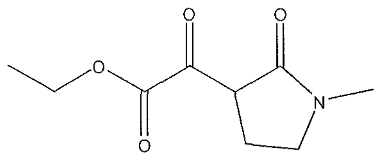

13,20 g 60%iges NaH (in Paraffinöl) (0,33 mol) wurden mit 100ml trockenem Diethylether versetzt. Unter starkem Rühren gab man 19,83g (0,20 mol) N-Methylpyrrolidon zu. Dann tropft man innerhalb einer Stunde 121,30g (0,83 mol) Oxalsäurediethylester zu. Nach beendeter Zugabe erwärmte man die Suspension auf 40°C und rührte für 21 Stunden. Die Reaktionsmischung wurde dann im Eisbad gekühlt, mit 66ml 5M HCl versetzt und die entstandene 2 Phasenmischung einmal filtriert. Danach trennt man die beiden Phasen und extrahierte die wässrige Phase noch zweimal mit je 100ml Diethylether. Die vereinten etherischen Phasen wurden über $Na_2SO_4$ getrocknet und am Rotationsverdampfer zur Trockene eingeengt. Der ölige Rückstand wurde auf -80°C gekühlt und mit 200ml Diethylether versetzt. Der entstandene braune Feststoff wurde abfiltriert und nochmals aus Heptan kristallisiert. Man erhielt 5,2g Produkt in Form weisser Nadeln.
IR (in Substanz, $cm^{-1}$): 3342, 2977, 2939, 1723, 1658, 1581, 1498, 1469, 1448, 1373, 1344, 1307, 1269, 1196, 1160, 1117, 1094, 1024, 977, 903, 876, 819, 783, 737, 701, 675.
$^1$H-NMR ($CDCl_3$, 400 MHz): $\delta$ [ppm] = 1.26 (3H), 2.83 (3H), 2.94 (2H), 3.37 (2H), 4.21 (2H), 11.80 (1H).

**AE. Ethyl (1-methyl-2-oxopiperidin-3-yl)(oxo)acetat**

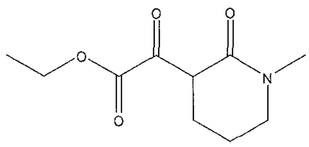

20,4 g Diisopropylamin (0,1 mol) wurden in 200ml trockenem THF unter Stickstoff vorgelegt und in einer Kältemischung abgekühlt. Man tropfte 80ml n-Buthyllithium in Hexan (2,5M, 0,2 mol) langsam zu. Nach erfolgter Zugabe liess man 60 min rühren und tropfte anschliessend 22,6 g N-Methyl-2-piperidon (0,2 mol) zu. Die Reaktionsmischung wurde für weitere 20 min in der Kältemischung gerührt. In einem zweiten Kolben wurden 87,7 g Diethyloxalat (0,6 mol) in 100 ml trockenem THF in einer Kältemischung abgekühlt und die gekühlte Reaktionsmischung unter Rühren in kleinen Portionen dazu kanüliert. Man liess über Nacht unter Rühren auf Raumtemperatur erwärmen. Das THF wurde am Rotationsverdampfer entfernt und der Rückstand in 80 ml halbkonzentrierter Salzsäure aufgenommen. Man extrahierte die wässrige Phase fünfmal mit je 300ml Ethylacetat, trocknete die vereinten organischen Phasen über Natriumsulfat und engte am Rotationsverdampfer bis zur Trockene ein. Das überschüssige Diethyloxalat wurde abdestilliert (2-3 mbar, 80°C) und man erhielt 45g Rohprodukt. 10,4 g Produkt wurde in Form weisser Kristalle mit Schmelzpunkt 23°C nach Kristallisation aus Diethylether/n-Hexan erhalten.
IR (in Substanz, $cm^{-1}$): 2981, 2935, 1721, 1610, 1503, 1449, 1393, 1371, 1331, 1304, 1247, 1223, 1193, 1095, 1081, 1028, 995, 944, 895, 860, 794, 758, 722, 671, 628.
$^1$H-NMR ($CDCl_3$, 400 MHz): $\delta$ [ppm] = 14.00 (1H), 4.20 (2H), 3.25 (2H), 2.90 (3H), 2.65 (2H), 1.73 (2H), 1.23 (3H).

**AF. 3-Acetyl-1-methyl-piperidin-2-on**

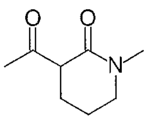

20,6 g Diisopropylamin (0.2 mol) wurden in 200ml trockenem THF unter Stickstoff vorgelegt und auf -80 °C

abgekühlt. Man tropfte 80ml n-Buthyllithium in Hexan (2,5M, 0,2 mol) langsam zu. Nach erfolgter Zugabe liess man 15 min rühren und tropfte anschliessend 22,6 g N-Methyl-2-piperidon (0,2 mol) zu. Die Reaktionsmischung wurde für 30 min gerührt und anschliessend 17,6 g trockenes Ethylacetat (0,2 mol) zu gegeben. Man liess die Reaktionsmischung unter Rühren über Nacht auf Raumtemperatur erwärmen. Das THF wurde am Rotationsverdampfer entfernt und der Rückstand in 80ml 6M HCl aufgenommen. Man extrahierte die wässrige Phase fünfmal mit 100 ml Ethylacetat. Die vereinten organischen Phasen wurden über Natriumsulfat getrocknet und das Lösungsmittel am Rotationsverdampfer entfernt. Das Produkt (Ketoform:Enolform = 1:1) wurde nach fraktionierter Destillation bei 0.6 mbar als gelbliche Flüssigkeit (Sdp. 70°C) erhalten.
IR (in Substanz, $cm^{-1}$): 3506, 2940, 2865, 1715, 1632, 1597, 1498, 1463, 1443, 1401, 1386, 1354, 1330, 1310, 1252, 1203, 1160, 1118, 1081, 983, 952, 887, 762, 686, 651, 606.
Enolform:
$^1H$-NMR ($CDCl_3$, 400 MHz): $\delta$ [ppm] = 1.81 (2H), 1.92 (3H), 2.38 (2H), 2.96 (3H), 3.30 (2H), 14.8 (1H).
Ketoform:
$^1H$-NMR ($CDCl_3$, 400 MHz): $\delta$ [ppm] = 1.81 (3H), 2.14 (1H), 2.34 (3H), 2.99 (3H), 3.30 (2H), 3.48 (1H).

**AG. Ethyl-4-(dimethylamino)-2,4-dioxobutanoat**

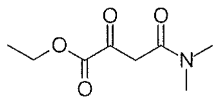

10,2 g Diisopropylamin (0,1 mol) wurden in 100ml trockenem THF unter Stickstoff vorgelegt und in einer Kältemischung abgekühlt. Man tropfte 40ml n-Buthyllithium in Hexan (2,5M, 0,1 mol) langsam zu. Nach erfolgter Zugabe liess man 60 min rühren und tropfte anschliessend 8,7 g N,N-Dimethylacetamid (0,1 mol) zu. Die Reaktionsmischung wurde für weitere 20 min in der Kältemischung gerührt. In einem zweiten Kolben wurden 43,7 g Diethyloxalat (0,3 mol) in 50 ml trockenem THF in einer Kältemischung abgekühlt und die gekühlte Reaktionsmischung unter Rühren in kleinen Portionen dazu kanüliert. Man liess über Nacht unter Rühren auf Raumtemperatur erwärmen. Das THF wurde am Rotationsverdampfer entfernt und der Rückstand in 80 ml halbkonzentrierter Salzsäure aufgenommen. Man extrahierte die wässrige Phase fünfmal mit je 150ml Ethylacetat, trocknete die vereinten organischen Phasen über Natriumsulfat und engte am Rotationsverdampfer bis zur Trockene ein. Das überschüssige Diethyloxalat wurde abdestilliert (2-3 mbar, 80°C) und man erhielt 17g Rohprodukt. 5,5 g Produkt wurde in Form weisser Kristalle mit Schmelzpunkt 28°C nach Kristallisation aus n-Hexan erhalten.
IR (in Substanz, $cm^{-1}$): 2984, 2941, 1738, 1620, 1507, 1467, 1393, 1370, 1355, 1313, 1260, 1170, 1126, 1016, 927, 860, 823, 772, 723, 628.
$^1H$-NMR ($CDCl_3$, 400 MHz): $\delta$ [ppm] = 1.38 (3H), 3.06 (6H), 4.33 (2H), 6.25 (1H)

AH. 1-(Morpholin-4-yl)butan-1,3-dion

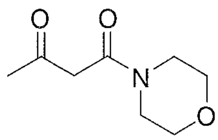

33 ml (0,38 mol) Morpholin wurden zu einer Lösung von 30 g Diketen (0,36 mol) in 300 ml Tetrahydrofuran tropfenweise bei -5 bis 0°C zugegeben. Nach 1 h Rühren bei 0°C wurde dünnschichtchromatographisch kein Edukt mehr detektiert. Das Reaktionsgemisch wurde abrotiert und der Rückstand säulenchromatographisch gereinigt. Man erhielt 49,48 g (0,29 mol, 81% Ausbeute) weissen Feststoff.
IR (in Substanz, $cm^{-1}$): 2965, 2918, 2858, 1718, 1633, 1587, 1488, 1436, 1359, 1303, 1273, 1245, 1220, 1112.
CHN-Elementaranalyse: C 56.03, H 7.62, N 8.12.
LC-MS: 172 (M+H), 194 (M+Na).
$^1H$-NMR ($CDCl_3$, 400 MHz): $\delta$ [ppm] = 3.6 bis 3.3 (10H), 2.2 (3H).

**AI.** N-(2-Methoxyethyl)-3-oxobutanamid

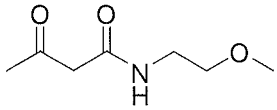

33 ml (0,38 mol) Methoxyethylamin wurden zu einer Lösung von 30 g Diketen (0,36 mol) in 300 ml Tetrahydrofuran tropfenweise bei -5 bis 0°C zugegeben. Nach 1 h Rühren bei 0°C wurde dünnschichtchromatographisch kein Edukt mehr detektiert. Das Reaktionsgemisch wurde abrotiert und der Rückstand säulenchromatographisch gereinigt. Man erhielt 47,15 g (0,30 mol, 83% Ausbeute) weissen Feststoff.
IR (in Substanz, $cm^{-1}$): 3291, 3095, 2978, 2922, 2887, 2851, 1709, 1643, 1560, 1417, 1356, 1344, 1297, 1195, 1166, 1122, 1092.
$^1$H-NMR ($CDCl_3$, 400 MHz): δ [ppm] = 7.1 (1H), 3.4 bis 3.3 (9H), 2.2 (3H).

**AJ.** N-Cyclopropyl-3-oxobutanamid

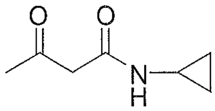

26 ml (0,38 mmol) Cyclopropylamin wurden zu einer Lösung von 30 g Diketen (0,36 mol) in 300 ml Tetrahydrofuran tropfenweise bei -5 bis 0°C zugegeben. Nach 1 h Rühren bei 0°C wurde dünnschichtchromatographisch kein Edukt mehr detektiert. Das Reaktionsgemisch wurde abrotiert und der Rückstand säulenchromatographisch gereinigt. Man erhielt 43,83 g (0,31 mol, 87% Ausbeute) weissen Feststoff.
IR (in Substanz, $cm^{-1}$): 3266, 3082, 3017, 2953, 2922, 1724, 1666, 1636, 1450, 1421, 1358, 1338, 1221, 1193, 1161, 1014.
LC-MS: 140 (M-H).
$^1$H-NMR ($CDCl_3$, 400 MHz): δ [ppm] = 7.2 (1H), 3.4 (2H), 2.7 (1H), 2.2 (3H), 0.7 (2H), 0.5 (2H).

**AK. 3-Oxo-N-(propan-2-yl)butanamid**

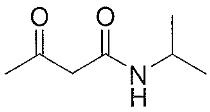

32 ml (0,38 mmol) Isopropylamin wurden zu einer Lösung von 30 g Diketen (0,36 mol) in 300 ml Tetrahydrofuran tropfenweise bei -5 bis 0°C zugegeben. Nach 1 h Rühren bei 0°C wurde dünnschichtchromatographisch kein Edukt mehr detektiert. Das Reaktionsgemisch wurde abrotiert und der Rückstand säulenchromatographisch gereinigt. Man erhielt 40.36 g (0.28 mol, 79% Ausbeute) weissen Feststoff.
IR (in Substanz, $cm^{-1}$): 3254, 3088, 2975, 2925, 1725, 1633, 1561, 1459, 1422, 1350, 1334, 1314, 1289, 1190, 1160, 1133.
$^1$H-NMR ($CDCl_3$, 400 MHz): δ [ppm] = 6.9 (1H), 4.0 (1H), 3.3 (2H), 2.2 (3H), 1.1 (6H).

**AL. N-(2-Hydroxyethyl)-3-oxobutanamid**

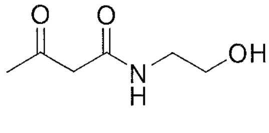

23 ml (0,38 mmol) Ethanolamin wurden zu einer Lösung von 30 g Diketen (0,36 mol) in 300 ml Tetrahydrofuran tropfenweise bei -5 bis 0°C zugegeben. Nach 1 h Rühren bei 0°C wurde dünnschichtchromatographisch kein Edukt mehr detektiert. Das Reaktionsgemisch wurde abrotiert und der Rückstand säulenchromatographisch gereinigt. Man erhielt 41,44 g (0,29 mol, 80% Ausbeute) weissen Feststoff.
IR (in Substanz, $cm^{-1}$): 3273, 3097, 2973, 2938, 2880, 1710, 1646, 1558, 1494, 1465, 1420, 1362, 1347, 1312, 1297, 1215, 1190, 1166, 1052, 1038. CHN-Elementaranalyse: C 49.13, H 7.76, N 9.71.
LC-MS: 145 (M), 127 (M-H2O).
$^1$H-NMR ($CDCl_3$, 400 MHz): δ [ppm] = 7.5 (1H), 3.7 (2H), 3.4 (4H), 2.2 (3H).

**AM. N-(3-hydroxypropyl)-3-oxobutanamid**

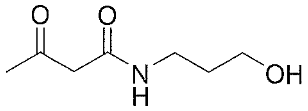

28 ml (0,38 mmol) 3-Amino-1-propanol wurden zu einer Lösung von 30 g Diketen (0,36 mol) in 300 ml Tetrahydrofuran tropfenweise bei -5 bis 0°C zugegeben. Nach 1 h Rühren bei 0°C wurde dünnschichtchromatographisch kein Edukt mehr detektiert. Das Reaktionsgemisch wurde abrotiert und der Rückstand säulenchromatographisch gereinigt. Man erhielt 47,15 g (0,30 mol, 83% Ausbeute) weissen Feststoff.
IR (in Substanz, $cm^{-1}$): 3295, 3090, 2940, 2877, 1715, 1643, 1545, 1470, 1416, 1358, 1324, 1213, 1160, 1056, 963.
LC-MS: 182 (M+Na), 160 (M+H), 142 (M-OH).
$^1$H-NMR ($CDCl_3$, 400 MHz): δ [ppm] = 7.4 (1H), 3.6 (3H), 3.3 (4H), 2.2 (3H), 1.6 (2H).

**AN. N-(4-Hydroxybutyl)-3-oxobutanamid**

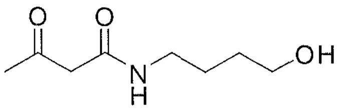

Eine Lösung von 22 ml (0,24 mol) 4-Amino-1-butanol in 50 ml Tetrahydrofuran wurde zu einer Lösung von 20 g Diketen (0,24 mol) in 200 ml Tetrahydrofuran tropfenweise bei -5 bis 0°C zugegeben. Nach 1 h Rühren bei 0°C wurde dünnschichtchromatographisch kein Edukt mehr detektiert. Das Reaktionsgemisch wurde abrotiert und der Rückstand säulenchromatographisch gereinigt. Man erhielt 35,43 g (0,21 mol, 86% Ausbeute) weissen Feststoff.
IR (in Substanz, $cm^{-1}$): 3273, 3098, 2933, 2866, 1712, 1644, 1555, 1418, 1361, 1344, 1322, 1188, 1167, 1060.
$^1$H-NMR ($CDCl_3$, 400 MHz): δ [ppm] = 7.4 (1H), 3.6 (2H), 3.3 (2H), 3.2 (2H), 3.1 (1H), 2.22 (3H), 1.5 (4H).

**AO-1. N-(5-Hydroxypentyl)-3-oxobutanamid**

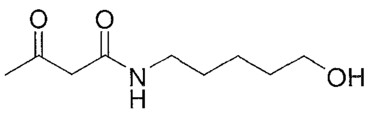

Eine Lösung von 39 g (0,38 mol) 5-Amino-1-pentanol in 300 ml Tetrahydrofuran wurde zu einer Lösung von 30 g Diketen (0.36 mol) in 300 ml Tetrahydrofuran tropfenweise bei -5 bis 0°C zugegeben. Nach 1 h Rühren bei 0°C wurde dünnschichtchromatographisch kein Edukt mehr detektiert. Das Reaktionsgemisch wurde abrotiert und der Rückstand säulenchromatographisch gereinigt. Man erhielt 52,70 g (0,28 mol, 79% Ausbeute) weissen Feststoff.
IR (in Substanz, $cm^{-1}$): 3269, 3096, 2928, 2855, 1725, 1711, 1643, 1418, 1361, 1340, 1190, 1166, 1060. 1007.
LCMS: 188 (M+H).
$^1$H-NMR ($CDCl_3$, 400 MHz): δ [ppm] = 7.2 (1H), 3.6 (2H), 3.4 (2H), 3.2 (2H), 2.8 (1H), 2.2 (3H), 1.5 (4H), 1.3 (2H).

**AO-2. N-(1-Hydroxy-2-methylpropan-2-yl)-3-oxobutanamid**

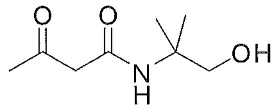

Eine Lösung von 33,40 g (0,38 mol) 2-Amino-2-methyl-1-propanol in 300 ml Tetrahydrofuran wurde zu einer Lösung von 30 g Diketen (0,36 mol) in 300 ml Tetrahydrofuran tropfenweise bei -5 bis 0°C zugegeben. Nach 1 h Rühren bei 0°C wurde dünnschichtchromatographisch kein Edukt mehr detektiert. Das Reaktionsgemisch wurde abrotiert und der Rückstand säulenchromatographisch gereinigt. Man erhielt 50.00 g (0.29 mol, 81% Ausbeute) weissen Feststoff.
IR (in Substanz, $cm^{-1}$): 3308, 2973, 2932, 1714, 1646, 1545, 1456, 1412, 1359, 1331, 1267, 1160, 1057.
CHN-Elementaranalyse: C 54.99, H 8.67, N 8.42.
LCMS: 172 (M-H).
$^1$H-NMR ($CDCl_3$, 400 MHz): $\delta$ [ppm] = 7.1 (1H), 4.6 (1H), 3.5 (2H), 3.3 (2H), 2.2 (3H), 1.3 (3H).

**AP. N-(2-Hydroxyethyl)-N-methyl-3-oxobutanamid**

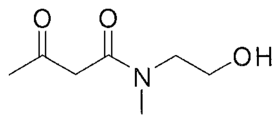

Eine Lösung von 30 ml (0,38 mol) 2-(Methylamino)ethanol in 300 ml Tetrahydrofuran wurde zu einer Lösung von 30 g Diketen (0.36 mol) in 300 ml Tetrahydrofuran tropfenweise bei -5 bis 0°C zugegeben. Nach 1 h Rühren bei 0°C wurde dünnschichtchromatographisch kein Edukt mehr detektiert. Das Reaktionsgemisch wurde abrotiert und der Rückstand säulenchromatographisch gereinigt. Man erhielt 41.46 g (0.26 mol, 73% Ausbeute) weissen Feststoff.
IR (in Substanz, $cm^{-1}$): 3407, 2934, 1717, 1620, 1491, 1401, 1357, 1308, 1161, 1121, 1050, 928, 861, 778.
LCMS: 159 (M), 141 (M-H2O).
$^1$H-NMR ($CDCl_3$, 400 MHz): $\delta$ [ppm] = 3.7 (3H), 3.5 (2H), 3.3 (1H), 3.0 (2H), 2.9 (1H), 2.2 (3H).

**AQ. N-(2-Hydroxypropyl)-3-oxobutanamid**

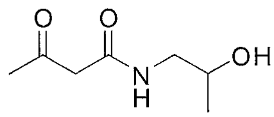

19 ml (0,24 mol) 1-Amino-2-propanol wurden zu einer Lösung von 20 g Diketen (0,24 mol) in 200 ml Tetrahydrofuran tropfenweise bei -5 bis 0°C zugegeben. Nach 1 h Rühren bei 0°C wurde dünnschichtchromatographisch kein Edukt mehr detektiert. Das Reaktionsgemisch wurde abrotiert und der Rückstand säulenchromatographisch gereinigt. Man erhielt 29,50 g (0,19 mol, 78% Ausbeute) farbloses Öl.
IR (in Substanz, $cm^{-1}$): 3307, 3088, 2972, 2930, 1714, 1643, 1545, 1415, 1359, 1326, 1161, 1134, 1090.
$^1$H-NMR ($CDCl_3$, 400 MHz): $\delta$ [ppm] = 7.4 (1H), 3.9 (1H), 3.8 (1H), 3.3 (2H), 3.1 (2H), 2.2 (3H), 1.1 (3H).

**AR. N-(1-Hydroxypropan-2-yl)-3-oxobutanamid**

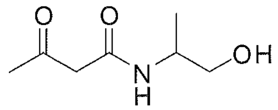

19 ml (0,24 mol) 2-Amino-1-propanol wurden zu einer Lösung von 20 g Diketen (0,24 mol) in 200 ml Tetrahydrofuran tropfenweise bei -5 bis 0°C zugegeben. Nach 1 h Rühren bei 0°C wurde dünnschichtchromatographisch kein Edukt mehr detektiert. Das Reaktionsgemisch wurde abrotiert und der Rückstand säulenchromatographisch gereinigt. Man erhielt 27,12 g (0,17 mol, 71% Ausbeute) farbloses Öl.
IR (in Substanz, $cm^{-1}$): 3295, 3083, 2974, 2935, 2877, 1714, 1640, 1544, 1454, 1413, 1358, 1324, 1220, 1160, 1095, 1050, 992.
$^1$H-NMR ($CDCl_3$, 400 MHz): δ [ppm] = 7.2 (1H), 4.0 (1H), 3.9 (1H), 3.6 (1H), 3.4 (2H), 2.2 (3H), 1.1 (3H).

**AS. N-(1-Hydroxybutan-2-yl)-3-oxobutanamid**

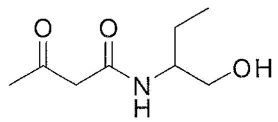

34 ml (0,36 mol) 2-Amino-1-butanol wurden zu einer Lösung von 30 g Diketen (0.36 mol) in 300 ml Tetrahydrofuran tropfenweise bei -5 bis 0°C zugegeben. Nach 1 h Rühren bei 0°C wurde dünnschichtchromatographisch kein Edukt mehr detektiert. Das Reaktionsgemisch wurde abrotiert und der Rückstand säulenchromatographisch gereinigt. Man erhielt 46,97 g (0,27 mol, 76% Ausbeute) farbloses Öl.
IR (in Substanz, $cm^{-1}$): 3294, 3085, 2986, 2936, 2878, 1714, 1640, 1544, 1461, 1414, 1358, 1218, 1160, 1089, 1052.
$^1$H-NMR ($CDCl_3$, 400 MHz): δ [ppm] = 7.2 (1H), 3.8 (1H), 3.6 (1H), 3.5 (1H), 3.4 (1H), 3.3 (1H), 2.2 (3H), 1.5 (1H), 1.4 (1H), 0.8 (3H).

**AT. N-(2,3-Dihydroxypropyl)-3-oxobutanamid**

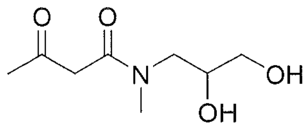

Eine Lösung von 22,5 g (0,21 mol) 3-Methylamino-1,2-propandiol in 200 ml Tetrahydrofuran wurde zu einer Lösung von 18 g Diketen (0,21 mol) in 200 ml Tetrahydrofuran tropfenweise bei -5 bis 0°C zugegeben. Nach 1 h Rühren bei 0°C wurde dünnschichtchromatographisch kein Edukt mehr detektiert. Das Reaktionsgemisch wurde abrotiert und der Rückstand säulenchromatographisch gereinigt. Man erhielt 32.00 g (0.17 mol, 79% Ausbeute) farbloses Öl.
IR (in Substanz, $cm^{-1}$): 3385, 2931, 1716, 1616, 1492, 1403, 1358, 1310, 1162, 1103, 1040, 925.
LC-MS: 212 (M+Na), 190 (M+H), 172 (M-OH).
$^1$H-NMR ($CDCl_3$, 400 MHz): δ [ppm] = 3.9 (1H), 4.8 bis 3.3 (8H), 3.1 (3H), 2.3 (3H).

**AU. 1-(3-Hydroxypiperidin-1-yl)butan-1,3-dion**

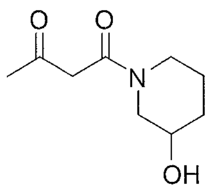

Eine Lösung von 36,10 g (0,36 mol) 3-Hydroxypiperidin in 300 ml Tetrahydrofuran wurde zu einer Lösung von 30 g Diketen (0.36 mol) in 200 ml Tetrahydrofuran tropfenweise bei -5 bis 0°C zugegeben. Nach 1 h Rühren bei 0°C wurde dünnschichtchromatographisch kein Edukt mehr detektiert. Das Reaktionsgemisch wurde abrotiert und der Rückstand säulenchromatographisch gereinigt. Man erhielt 53,53 g (0,29 mol, 81% Ausbeute) weissen Feststoff.
IR (in Substanz, $cm^{-1}$): 3340, 1716, 1618, 1596, 1485, 1462, 1437, 1410, 1360, 1343, 1319, 1279, 1263, 1185, 1159, 1138, 1001. 921, 858.
$^1$H-NMR ($CDCl_3$, 400 MHz): δ [ppm] = 3.8 (2H), 3.6 bis 3.2 (5H), 2.9 (1H), 2.2 (3H), 1.8 (2H), 1.6 (1H), 1.4 (1H).

**AV. 1-[4-(Hydroxymethyl)piperidin-1-yl]butan-1,3-dion**

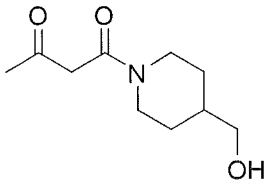

Eine Lösung von 25 g (0,22 mol) 4-Piperidinmethanol in 200 ml Tetrahydrofuran wurde zu einer Lösung von 18 g Diketen (0.2 mol) in 200 ml Tetrahydrofuran tropfenweise bei -5 bis 0°C zugegeben. Nach 1 h Rühren bei 0°C wurde dünnschichtchromatographisch kein Edukt mehr detektiert. Das Reaktionsgemisch wurde abrotiert und der Rückstand säulenchromatographisch gereinigt. Man erhielt 35,40 g (0,18 mol, 83% Ausbeute) farbloses Öl.
IR (in Substanz, $cm^{-1}$): 3401, 3003, 2918, 2860, 1718, 1618, 1447, 1358, 1313, 1270, 1200, 1158, 1089, 1036, 987, 956..
$^1$H-NMR ($CDCl_3$, 400 MHz): δ [ppm] = 4.5 (1H), 3.7 (1H), 3.4 (2H), 3.3 (2H), 2.9 (1H), 2.6 (1H), 2.5 (1H), 2.2 (3H), 1.7 (3H), 1.1 (2H).

**AW. 1-[3-(Hydroxymethyl)piperidin-1-yl]butan-1,3-dion**

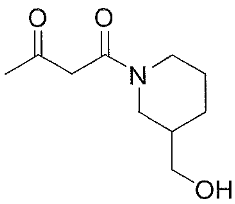

Eine Lösung von 25 g (0,22 mol) 3-Piperidinmethanol in 200 ml Tetrahydrofuran wurde zu einer Lösung von 18 g Diketen (0,2 mol) in 200 ml Tetrahydrofuran tropfenweise bei -5 bis 0°C zugegeben. Nach 1 h Rühren bei 0°C wurde dünnschichtchromatographisch kein Edukt mehr detektiert. Das Reaktionsgemisch wurde abrotiert und der Rückstand säulenchromatographisch gereinigt. Man erhielt 31,50 g (0,16 mol, 74% Ausbeute) farbloses Öl.
IR (in Substanz, $cm^{-1}$): 3411, 2926, 2859, 1717, 1618, 1442, 1358, 1308, 1262, 1222, 1183, 1160, 1036, 855.
$^1$H-NMR ($CDCl_3$, 400 MHz): δ [ppm] = 4.1 (1H), 3.6 bis 3.2 (7H), 2.9 (1H), 2.2 (3H), 1.7 (3H), 1.3 (2H).

**AX. 1-[2-(Hydroxymethyl)piperidin-1-yl]butan-1,3-dion**

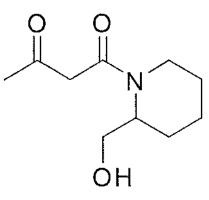

Eine Lösung von 25 g (0,22 mol) 2-Piperidinmethanol in 200 ml Tetrahydrofuran wurde zu einer Lösung von

18 g Diketen (0,2 mol) in 200 ml Tetrahydrofuran tropfenweise bei -5 bis 0°C zugegeben. Nach 1 h Rühren bei 0°C wurde dünnschichtchromatographisch kein Edukt mehr detektiert. Das Reaktionsgemisch wurde abrotiert und der Rückstand säulenchromatographisch gereinigt. Man erhielt 32,40 g (0,17 mol, 76 % Ausbeute) farbloses Öl.
IR (in Substanz, $cm^{-1}$): 3403, 2936, 1717, 1612, 1442, 1357, 1309, 1267, 1226, 1158, 1139, 1049.
$^1$H-NMR ($CDCl_3$, 400 MHz): $\delta$ [ppm] = 4.7 (1H), 4.4 (1H), 3.9 bis 3.7 (2H), 3.6 bis 3.3 (3H), 2.2 (3H), 1.7 bis 1.3 (6H).

**AY. 1-[(3S)-3-Hydroxypyrrolidin-1-yl]butan-1,3-dion**

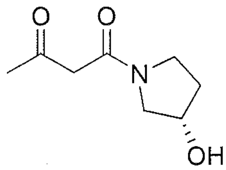

Eine Lösung von 21,8 g (0,25 mol) (S)-3-Pyrrolidinol in 200 ml Tetrahydrofuran wurde zu einer Lösung von 20 g Diketen (0,24 mol) in 200 ml Tetrahydrofuran tropfenweise bei -5 bis 0°C zugegeben. Nach 1 h Rühren bei 0°C wurde dünnschichtchromatographisch kein Edukt mehr detektiert. Das Reaktionsgemisch wurde abrotiert und der Rückstand säulenchromatographisch gereinigt. Man erhielt 31,35 g (0,18 mol, 77 % Ausbeute) farbloses Öl.
IR (in Substanz, $cm^{-1}$): 3390, 2948, 1717, 1616, 1438, 1383, 1357, 1224, 1188, 1160, 1102, 988, 871.
$^1$H-NMR ($CDCl_3$, 400 MHz): $\delta$ [ppm] = 4.4 (1H), 4.1 (1H), 3.6 bis 3.3 (5H), 2.2 (3H), 2.0 bis 1.8 (3H).

**AZ. 1-[4-(2-Hydroxyethyl)piperazin-1-yl]butan-1,3-dion**

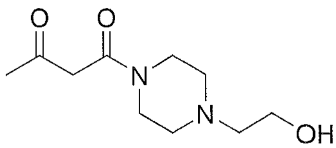

Eine Lösung von 32,5 g (0,25 mol) 1-(2-Hydroxyethyl)piperazin in 100 ml in Tetrahydrofuran wurde zu einer Lösung von 20 g Diketen (0,24 mol) in 200 ml Tetrahydrofuran tropfenweise bei -5 bis 0°C zugegeben. Nach 1 h Rühren bei 0°C wurde dünnschichtchromatographisch kein Edukt mehr detektiert. Das Reaktionsgemisch wurde abrotiert und der Rückstand säulenchromatographisch gereinigt. Man erhielt 36,10 g (0,17 mol, 71 % Ausbeute) farbloses Öl.
IR (in Substanz, $cm^{-1}$): 3412, 2922, 2812, 1717, 1628, 1441, 1357, 1305, 1156, 1051, 1001, 875.
LC-MS: 237 (M+Na), 215 (M+H).
$^1$H-NMR ($CDCl_3$, 400 MHz): $\delta$ [ppm] = 3.6 (4H), 3.5 (2H), 3.4 (2H), 2.7 (1H), 2.6 (2H), 2.5 (4H), 2.2 (3H).

**AAA. 1-(4-Methylpiperazin-1-yl)butan-1,3-dion**

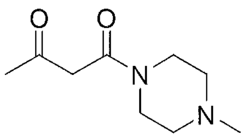

28 ml (0,25 mol) 1-(2-Hydroxyethyl)piperazin wurden zu einer Lösung von 20 g Diketen (0,24 mol) in 300 ml Tetrahydrofuran tropfenweise bei -5 bis 0°C zugegeben. Nach 1 h Rühren bei 0°C wurde dünnschichtchromatographisch kein Edukt mehr detektiert. Das Reaktionsgemisch wurde abrotiert und der Rückstand säulenchromatographisch gereinigt. Man erhielt 33,70 g (0,18 mol, 77 % Ausbeute) farbloses Öl.
IR (in Substanz, $cm^{-1}$): 2938, 2847, 2792, 1719, 1633, 1586, 1488, 1440, 1338, 1358, 1291, 1257, 1141, 1050, 1001, 778.
LC-MS: 207 (M+Na), 185 (M+H).

$^1$H-NMR ($CDCl_3$, 400 MHz): δ [ppm] = 3.6 (2H), 3.5 (2H), 3.4 (2H), 2.4 (4H), 2.3 (3H), 2.2 (3H).

**AAB. N-(3-Hydroxypropyl)-N-methyl-3-oxobutanamid**

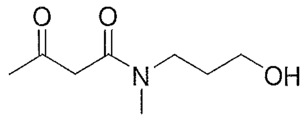

Eine Lösung von 20 g (0,23 mol) 3-Methylamino-1-propanol in 50 ml Tetrahydrofuran wurde zu einer Lösung von 18 g Diketen (0,21 mol) in 200 ml Tetrahydrofuran tropfenweise bei -5 bis 0°C zugegeben. Nach 1 h Rühren bei 0°C wurde dünnschichtchromatographisch kein Edukt mehr detektiert. Das Reaktionsgemisch wurde abrotiert und der Rückstand säulenchromatographisch gereinigt. Man erhielt 29,2 g (0,17 mol, 79% Ausbeute) farbloses Öl.
IR (in Substanz, $cm^{-1}$): 3407, 2936, 1718, 1622, 1493, 1402, 1358, 1310, 1161, 1128, 1058, 945.
LC-MS: 156 (M-OH), 174 (M+H), 196 (M+Na).
$^1$H-NMR ($CDCl_3$, 400 MHz): δ [ppm] = 3.8 bis 3.4 (7H), 2.9 (3H), 2.2 (3H), 1.7 (2H).

**AAC. N-(trans-4-Hydroxycyclohexyl)-3-oxobutanamid**

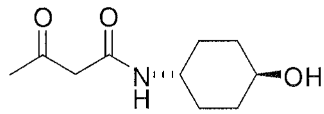

Eine Lösung von 28,8 g (0,25 mol) trans-4-Aminocyclohexanol in 200 ml Tetrahydrofuran wurde zu einer Lösung von 20 g Diketen (0,24 mol) in 200 ml Tetrahydrofuran tropfenweise bei -5 bis 0°C zugegeben. Nach 1 h Rühren bei 0°C wurde dünnschichtchromatographisch kein Edukt mehr detektiert. Das Reaktionsgemisch wurde abrotiert und der Rückstand aus 200 ml Ethylacetat umkristallisiert. Man erhielt 33,6 g (0,17 mol, 71% Ausbeute) farblosen Feststoff.
IR (in Substanz, $cm^{-1}$): 3274, 2940, 2859, 1716, 1639, 1543, 1450, 1423, 1338, 1221, 1136, 1098, 1058, 1009, 963, 948, 900.
LC-MS: 200 (M+H), 222 (M+Na).
$^1$H-NMR ($CDCl_3$, 400 MHz): δ [ppm] = 6.9 (1H), 3.7 (1H), 3.6 (1H), 3.3 (2H), 2.5 (1H), 2.2 (3H), 1.9 (4H), 1.4 bis 1.1 (4H).

**AAD. N-(3-Hydroxy-2,2-dimethylpropyl)-3-oxobutanamid**

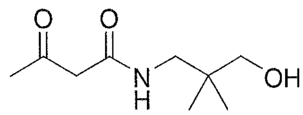

Eine Lösung von 32,2 g (0,31 mol) 3-Amino-2,2-dimethylpropan-1-ol in 60 ml Tetrahydrofuran wurde zu einer Lösung von 25 g Diketen (0,30 mol) in 200 ml Tetrahydrofuran tropfenweise bei -5 bis 0°C zugegeben. Nach 1 h Rühren bei 0°C wurde dünnschichtchromatographisch kein Edukt mehr detektiert. Das Reaktionsgemisch wurde abrotiert und der Rückstand aus 150 ml Ethylacetat umkristallisiert. Man erhielt 40,6 g (0,21 mol, 73% Ausbeute) farblosen Feststoff.
IR (in Substanz, $cm^{-1}$): 3253, 3099, 2957, 2873, 1724, 1637, 1581, 1476, 1453, 1428, 1358, 1325, 1162, 1035, 995, 785.
LC-MS: (M+H), (M+Na).
$^1$H-NMR ($CDCl_3$, 400 MHz): δ [ppm] = 7.5 (1H), 3.8 (1H), 3.4 (2H), 3.1 (4H), 2.3 (3H), 0.8 (6H).

**AAE. 1-[4-(Dimethylamino)piperidin-1-yl]butan-1,3-dion**

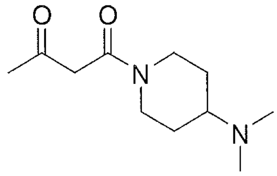

20 g (0,16 mol) 4-Dimethylamin-piperidin wurden zu einer Lösung von 12,5 g Diketen (0,15 mol) in 200 ml Tetrahydrofuran tropfenweise bei -5 bis 0°C zugegeben. Nach 1 h Rühren bei 0°C wurde dünnschichtchromatographisch kein Edukt mehr detektiert. Das Reaktionsgemisch wurde abrotiert und der Rückstand säulenchromatographisch gereinigt. Man erhielt 23,9 g (0,11 mol, 76% Ausbeute) farbloses Öl.
IR (in Substanz, $cm^{-1}$): 3270, 2930, 2863, 2721, 1719, 1633, 1585, 1492, 1448, 1389, 1360, 1270, 1238, 1198, 1154, 1060, 1040, 958, 873, 775.
LC-MS: 213 (M+H), 235 (M+Na).
$^1$H-NMR ($CDCl_3$, 400 MHz): δ [ppm] = 4.5 (1H), 3.7 (1H), 3.5 (2H), 3.0 (1H), 2.6 (1H), 2.3 (1H), 2.2 (9H), 1.8 (2H), 1.3 (2H).

**AAF. 1-(4-Methoxypiperidin-1-yl)butan-1,3-dion**

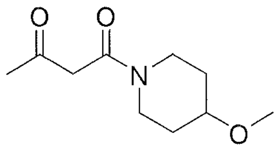

15 g (0,13 mol) 4-Methoxy-piperidin wurden zu einer Lösung von 10.4 g Diketen (0,12 mol) in 200 ml Tetrahydrofuran tropfenweise bei -5 bis 0°C zugegeben. Nach 1 h Rühren bei 0°C wurde dünnschichtchromatographisch kein Edukt mehr detektiert. Das Reaktionsgemisch wurde abrotiert und der Rückstand säulenchromatographisch gereinigt. Man erhielt 17,0 g (0,085 mol, 71% Ausbeute) farbloses Öl.
IR (in Substanz, $cm^{-1}$): 2930, 2863, 1720, 1635, 1586, 1488, 1445, 1390, 1359, 1270, 1188, 1097, 1077, 1067, 1024, 939, 904.
LC-MS: 200 (M+H), 223 (M+Na).
$^1$H-NMR ($CDCl_3$, 400 MHz): δ [ppm] = 3.8 bis 3.2 (8H), 2.2 (3H), 1.8 (4H), 1.5 (2H).

**AAG. 1-(2,6-Dimethylmorpholin-4-yl)butan-1,3-dion**

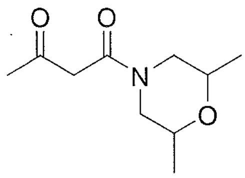

23 ml (0,19 mol) 2,6-Dimethylmorpholin wurden zu einer Lösung von 15 g Diketen (0,18 mol) in 200 ml Tetrahydrofuran tropfenweise bei -5 bis 0°C zugegeben. Nach 1 h Rühren bei 0°C wurde dünnschichtchromatographisch kein Edukt mehr detektiert. Das Reaktionsgemisch wurde abrotiert und der Rückstand säulenchromatographisch gereinigt. Man erhielt 26,5 g (0,13 mol, 74% Ausbeute) farbloses Öl.
IR (in Substanz, $cm^{-1}$): 3683, 2974, 2863, 1720, 1635, 1440, 1377, 1359, 1322, 1246, 1223, 1171, 1139, 1083, 1035, 966, 839.
LC-MS: 200 (M+H), 223 (M+Na).
$^1$H-NMR ($CDCl_3$, 400 MHz): δ [ppm] = 4.4 (1H), 4.0 (1H), 3.6 bis 3.3 (3H), 2.8 (1H), 2.4 (1H), 2.3 (1H), 2.2 (3H), 1.1 (6H).

**AAH. N-(Morpholin-4-yl)-3-oxobutanamid**

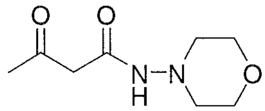

18 ml (0,19 mol) 4-Aminomorpholin wurden zu einer Lösung von 15 g Diketen (0,18 mol) in 200 ml Tetrahydrofuran tropfenweise bei -5 bis 0°C zugegeben. Nach 1 h Rühren bei 0°C wurde dünnschichtchromatographisch kein Edukt mehr detektiert. Das Reaktionsgemisch wurde abrotiert und der Rückstand säulenchromatographisch gereinigt. Man erhielt 26,1 g (0,14 mol, 78% Ausbeute) farbloses Öl.
IR (in Substanz, $cm^{-1}$): 3095, 2940, 2841, 1711, 1677, 1625, 1591, 1504, 1448, 1380, 1358, 1302, 1263, 1193, 1163, 1109, 1073, 1038, 912, 869.
LC-MS: 187 (M+H), 210 (M+Na).
$^1$H-NMR (DMSO, 400 MHz): $\delta$ [ppm] = 3.8 bis 3.3 (6H), 2.8 bis 2.5 (4H), 2.2 (3H), 2.1 (1H).

**AAI. 1-(Morpholin-4-yl)pentan-1,3-dion**

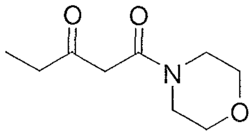

50,0 g (384 mmol) 3-Oxovaleriansäuremethylester und 35,1 g (403 mmol) Morpholin wurden für 9 h auf 110 °C erhitzt, wobei der entstandene Methanol über eine Destillationsbrücke abgeführt wurde. Der Reaktionsansatz wurde danach am Rotationsverdampfer von flüchtigen Bestandteilen befreit und durch Säulenchromatographie (Kieselgel, Laufmittel: Gradient von Ethylacetat auf Ethanol) gereinigt. Die reinen Produktfraktionen wurden am Rotationsverdampfer vom Lösungsmittel befreit und lieferten 39,7 g (60%) eines zähen, farblosen, klaren Öls.
IR (in Substanz, $cm^{-1}$): 2973, 2858, 1716, 1633, 1437, 1359, 1301, 1272, 1247, 1171, 1111, 1068, 1032, 961, 850, 584.
Elementaranalyse: C, 57.26; H, 8.00; N, 7.96.
LC-MS: 186.7 ($M+H^+$), 208.7 ($M+Na^+$).
$^1$H-NMR ($CDCl_3$, 400 MHz): 3.59-3.46 (8H), 3.33 (2H), 2.48 (2H), 0.98 (3H).

**AAJ. 1-(4-Hydroxypiperidin-1-yl)pentan-1,3-dion**

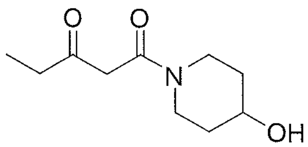

59,0 g (583 mmol) 4-Hydroxypiperidin und 79,7 g (612 mmol) 3-Oxovaleriansäuremethylester wurden 9 h auf 110°C erhitzt und der entstandene Methanol über eine Destillationsbrücke abgeführt. Der Reaktionsrückstand wurde am Rotationsverdampfer von flüchtigen Bestandteilen befreit, in Ethanol aufgenommen und durch 600 ml stark sauren Ionentauscher (Dowex® HCR-W2) filtriert, wieder eingeengt und durch Säulenchromatographie gereinigt (Kieselgel, Eluentengradient von Ethylacetat auf Ethanol). Die reinen Produktfraktionen wurden am Rotationsverdampfer vom Lösungsmittel befreit und ergaben 63,6 g (55%) eines zähen, klaren, farblosen Öls.
IR (in Substanz, $cm^{-1}$): 2940, 2877, 1715, 1616, 1449, 1369, 1299, 1171, 1108, 1077, 1059, 1018, 978, 930, 583.
LC-MS: 186.8 ($M+H^+$); 208.7 ($M+Na^+$).
$^1$H-NMR ($CDCl_3$, 400 MHz): 3.85 (2H), 3.60-3.51 (1H), 3.47 (2H), 3.23 (1H), 3.18-3.07 (2H), 2.47 (2H), 1.75 (2H), 1.42 (2H), 0.96 (3H).

**AAK. *N*-Butyl-3-oxoheptanamid**

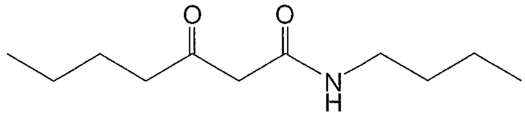

0,9 mol (150 ml) Methyl-3-oxoheptanoat und 2,7 mol (270 ml) Butylamin wurden 5 h bei 100°C im Druckbehälter gerührt. Danach wurde überschüssiges Butylamin am Rotationsverdampfer abdestilliert, das Rohprodukt in Wasser/Ethanol aufgenommen und mit konz. HCl auf pH 3 eingestellt. Das Gemisch wurde erneut zur Trockne eingedampft, in Essigsäureethylester wieder aufgenommen und filtriert. Das Filtrat wurde mit 1M HCl und Wasser gewaschen. Die organische Phase wurde mit $Na_2SO_4$ getrocknet und nach dem Filtrieren am Rotationsverdampfer zur Trockne eingeengt. Das Rohprodukt wurde in 100 ml Diethylether gelöst und bei 5°C kristallisiert. Das Produkt wurde abfiltriert und getrocknet. Es wurden 36,0 g der Titelverbindung erhalten.
IR (in Substanz, $cm^{-1}$): 3268, 3098, 2958, 2931, 2873, 1723, 1707, 1641, 1563, 1466, 1455, 1434, 1416, 1377, 1350, 1337, 1255, 1227, 1176, 1159, 1125, 1098, 1065, 1013, 969, 904, 881, 774, 734, 716, 650.
CHN-Elementaranalyse: C, 66.77; H, 10.62; N, 6.93.
LC-MS: $M+H^+$, 200.6; $M+Na^+$, 222.7.
$^1$H-NMR (DMSO-$d_6$, 400 MHz): Enol-Tautomer (12%), δ [ppm] = 14.16 (s, 1H), 7.82 (m, 1H), 4.92 (s, 1H), 3.1-3.0 (m, 2H), 2.06 (t, 2H), 1.5-1.3 (m, 4H), 1.3-1.2 (m, 4H), 0.88-0.82 (m, 6H); Keto-Tautomer (88%), δ = 7.97 (m, 1H), 3.24 (s, 2H), 3.02 (q, 2H), 2.47 (t, 2H), 1.5-1.3 (m, 4H), 1.3-1.2 (m, 4H), 0.88-0.81 (m, 6H).

**AAL. 1-(4-Hydroxypiperidin-1-yl)-4-methylpentan-1,3-dion**

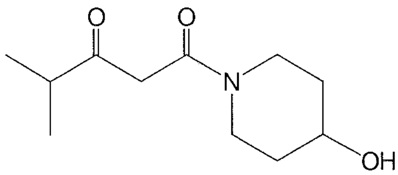

0,351 mol (49,6 ml) 4-Methyl-3-oxovaleriansäuremethylester und 0,369 mol (37,3 g) 4-Hydroxypiperidin wurden 6 h bei 120°C am Wasserabscheider gerührt. Es wurden 12 ml Methanol aufgefangen. Anschliessend wurden die überschüssigen Startmaterialien 3 h am Rotationsverdampfer abdestilliert (80°C Wasserbad, 2 mbar). Es wurden 71 g der Titelverbindung erhalten.
IR (in Substanz, $cm^{-1}$): 2934, 2873, 1712, 1619, 1449, 1384, 1364, 1330, 1300, 1267, 1225, 1187, 1166, 1117, 1071, 1025, 979, 955, 930, 846, 808, 777, 733, 693.
CHN-Elementaranalyse: C, 50.13; H, 7.514; N, 5.89.
LC-MS: $M+H^+$, 214.9; $M+Na^+$, 239.9.
$^1$H-NMR (DMSO-$d_6$, 400 MHz): Enol-Tautomer (15%), δ [ppm] = 5.39 (s, 1H), 4.49 (s (breit), 1H), 3.9-2.9 (m, 5H), 2.6-2.5 (m, 1H), 1.75-1.11 (m, 4H), 1.06 (d, 6H); Keto-Tautomer (85%), δ = 4.73 (s (breit), 1H), 3.67 (s, 2H), 3.9-2.9 (m, 5H), 2.68 (heptett, 1H), 1.75-1.11 (m, 4H), 1.00 (d, 6H).

**AAM. *N*-(2-Hydroxyethyl)-*N*,4-dimethyl-3-oxopentanamid**

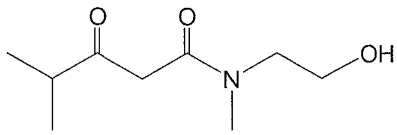

0,351 mol (50 ml) 4-Methyl-3-oxovaleriansäuremethylester und 0,369 mol (29,5 ml) 2-(Methylamino)ethanol wurden 15 h bei 120°C am Wasserabscheider gerührt. Es wurden 12 ml Methanol aufgefangen. Anschliessend wurden die überschüssigen Startmaterialien 2 h am Rotationsverdampfer abdestilliert (80°C Wasserbad, 1 mbar). Es wurden

64 g der Titelverbindung erhalten.
IR (in Substanz, $cm^{-1}$): 2969, 2934, 2875, 1713, 1621, 1491, 1466, 1401, 1384, 1358, 1303, 1265, 1211, 1120, 1072, 1048, 996, 926, 892, 862, 784, 726, 692, 615.
CHN-Elementaranalyse: C, 52.00; H, 6.84; N, 6.01.
LC-MS: $M+H^+$, 188.7; $M+Na^+$, 210.6.
$^1$H-NMR (DMSO-$d_6$, 400 MHz): E/Z-Isomerie im Verhältnis ca. 45/55; Enol-Tautomer (20%), $\delta$ [ppm] = 5.31, 5.28 (s, 1H), 3.55-3.42 (m, 2H), 3.42-3.25 (m, 2H), 3.0-2.8 (m, 3H), 2.4-2.3 (m, 1H), 1.07 (d, 6H); Keto-Tautomer (80%), $\delta$ = 3.70, 3.66 (s, 2H), 3.55-3.42 (m, 2H), 3.42-3.25 (m, 2H), 2.94, 2.83 (s, 3H), 2.71, 2.70 (heptett, 1H), 1.02, 1.01 (d, 6H).

**AAN. *N,N*,2-Trimethyl-3-oxobutanamid**

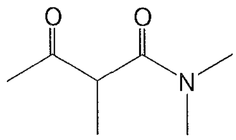

0,318 mol (50 ml) 2-Methylacetessigsäure-ethylester (techn. 90%) und 143 ml 33% Dimethylaminlösung in Ethanol (ca. 0,795 mol Dimethylamin) wurden im Druckreaktor 8 h bei 130°C gerührt. Nach dem Abkühlen wurde die Reaktionsmischung am Rotationsverdampfer eingeengt, der Rückstand wurde in 400 ml Ethylacetat aufgenommen und 5x mit je 150 ml Wasser extrahiert. Die vereinigten Wasserphasen wurden am Rotationsverdampfer zur Trockne eingedampft. Es wurden 18,8 g der Titelverbindung erhalten.
IR (in Substanz, $cm^{-1}$): 2984, 2938, 1721, 1632, 1497, 1451, 1396, 1355, 1312, 1265, 1214, 1180, 1145, 1078, 1041, 952, 795, 770, 734, 689, 635, 623. CHN-Elementaranalyse: C, 71.21; H, 7.81; N, 6.39; O, 14.59.
LC-MS: $M+H^+$, 144.6; $M+Na^+$, 166.6.
$^1$H-NMR (DMSO-$d_6$, 400 MHz): $\delta$ [ppm] = 3.92 (q, 1H); 3.03 (s, 3H); 2.84 (s, 3H); 2.06 (s, 3H); 1.12 (d, 3H).

**AAO. *N,N*-Dimethyl-2-oxocyclohexancarboxamid**

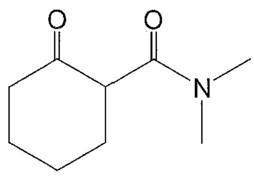

0,313 mol (50 ml) Cyclohexanon-2-carbonsäureethylester und 140 ml 33% Dimethylaminlösung in Ethanol (ca. 0,78 mol Dimethylamin) wurden im Druckreaktor 12 h bei 110°C gerührt. Nach dem Abkühlen wurde die Reaktionsmischung am Rotationsverdampfer eingeengt, der Rückstand wurde in 500 ml Ethylacetat aufgenommen und die organische Phase mit 200 ml 1 M NaOH und 3x 50 ml Wasser gewaschen. Die organische Phase wurde dann am Rotationsverdampfer zur Trockne eingedampft. 46 g Rohprodukt wurden in 100 ml tert.-Butylmethylether gelöst und auf 0°C gekühlt. Ausgefallenes Produkt wurde abfiltiert und getrocknet, es wurden 3,6 g der Titelverbindung erhalten.
IR (in Substanz, $cm^{-1}$): 2939, 2867, 1691, 1639, 1496, 1454, 1442, 1429, 1409, 1396, 1342, 1316, 1287, 1261, 1208, 1155, 1130, 1096, 1067, 1038, 1012, 959, 923,897,870,853,808,769,696,659,623.
CHN-Elementaranalyse: C, 71.21; H, 7.81; N, 6.39; O, 14.59.
LC-MS: $M+H^+$, 170.6; $M+Na^+$, 192.8.
$^1$H-NMR (DMSO-$d_6$, 400 MHz): $\delta$ [ppm] = 3.86 (dd, 1H), 2.82 (s, 3H), 2.81 (s, 3H), 2.54-2.22 (m, 2H), 2.0-1.5 (m, 6H).

**AAP. *N,N*-Dimethyl-2-oxocyclopentancarboxamid**

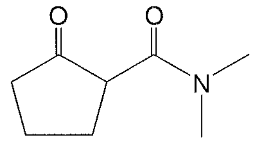

0,337 mol (50 ml) 2-Oxocyclopentancarbonsäureethylester und 152 ml 33% Dimethylaminlösung in Ethanol wurden im Druckreaktor 4 h bei 110°C gerührt. Das Reaktionsgemisch wurde anschliessend zur Trockne eingedampft und das Rohprodukt über 400 g Kieselgel mit Ethylacetat/Hexan 1/1 gereinigt. Es wurden 17,7 g der Titelverbindung erhalten.
IR (in Substanz, $cm^{-1}$): 2955, 2884, 1735, 1634, 1494, 1455, 1396, 1320, 1260, 1198, 1167, 1140, 1101, 1059, 1025, 1004, 983, 946, 915, 904, 833, 752, 689, 623.
LC-MS: $M+H^+$, 156.7; $M+Na^+$, 178.6.
1H-NMR (DMSO-$d_6$, 400 MHz): δ [ppm] = 3.72 (t, 1H); 3.03 (s, 3H); 2.83 (s, 3H); 2.3-1.4 (m, 6H).

**AAQ. N-Cyclopentyl-3-oxobutanamid**

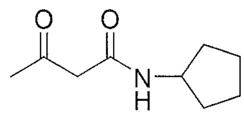

Eine Lösung von 21,3 g (0,25 mol) Cyclopentylamin in 50 ml Tetrahydrofuran wurde zu einer Lösung von 20 g Diketen (0,24 mol) in 200 ml Tetrahydrofuran tropfenweise bei -5 bis 0°C zugegeben. Nach 1 h Rühren bei 0°C wurde dünnschichtchromatographisch kein Edukt mehr detektiert. Das Reaktionsgemisch wurde abrotiert und der Rückstand aus 100 ml Ethylacetat umkristallisiert. Man erhielt 31,4 g (0,19 mol, 78% Ausbeute) weissen Feststoff. IR (in Substanz, $cm^{-1}$): 3256, 3083, 2947, 2919, 2868, 1719, 1647, 1626, 1556, 1422, 1357, 1197, 1161, 999.
LC-MS: 170 (M+H).
$^1$H-NMR ($CDCl_3$, 400 MHz): δ [ppm] = 6.9 (1H), 4.2 (1H), 3.3 (2H), 2.2 (3H), 1.9 (2H), 1.6 (4H), 1.4 (2H).

**AAR. 1-(Pyrrolidin-1-yl)butan-1,3-dion**

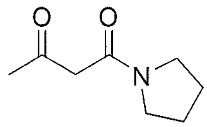

Eine Lösung von 21 ml (0,25 mol) Pyrrolidin in 50 ml Tetrahydrofuran wurde zu einer Lösung von 20 g Diketen (0,24 mol) in 200 ml Tetrahydrofuran tropfenweise bei -5 bis 0°C zugegeben. Nach 1 h Rühren bei 0°C wurde dünnschichtchromatographisch kein Edukt mehr detektiert. Das Reaktionsgemisch wurde abrotiert und der Rückstand säulenchromatographisch gereinigt. Man erhielt 29,9 g (0,19 mol, 81% Ausbeute) Öl.
IR (in Substanz, $cm^{-1}$): 2972, 2875, 1719, 1633, 1589, 1481, 1433, 1381, 1356, 1226, 1193, 1159, 934, 776.
LC-MS: 156 (M+H), 178 (M+Na).
$^1$H-NMR ($CDCl_3$, 400 MHz): δ [ppm] = 3.2 (5H), 2.1 (3H), 1.7 (5H).

**AAS. Methyl-N-(3-oxobutanoyl)-L-serinat**

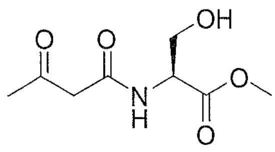

10,8 g (0,13 mol) Diketen, 20 g L-Serin methyl ester-hydrochlorid (0,13 mol) und 21,6 g (0,26 mol, 2 äq) $NaHCO_3$ wurden in 1000 ml THF bei 0°C gemischt, dann wurde das Eisbad entfernt und weiter bei Raumtemperatur gerührt. Nach 24 h Rühren bei Raumtemperatur wurde das Reaktionsgemisch abfiltriert, und der Rückstand aus 100 ml Ethylacetat umkristallisiert. Man erhielt 21,14 g (0.10 mol, 81 % Ausbeute) weissen Feststoff.
IR (in Substanz, $cm^{-1}$): 3499, 3297, 2962, 1734, 1712, 1642, 1556, 1430, 1416, 1351, 1316, 1246, 1209, 1190, 1170, 1137, 1081, 1040, 978, 704.
LC-MS: 226 (M+Na), 187 (M-CH3-H), .
$^1$H-NMR ($CDCl_3$, 400 MHz): $\delta$ [ppm] = 8.4 (1H), 5.1 (1H), 4.4 (1H), 3.7 bis 3.4 (7H), 2.1 (3H).

**AAT. 1-(4-Acetylpiperazin-1-yl)butan-1,3-dion**

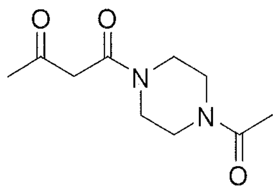

Eine Lösung von 20,8 g (0,17 mol) 1-Acetylpiperazin in 50 ml Tetrahydrofuran wurden zu einer Lösung von 13 g Diketen (0,16 mol) in 200 ml Tetrahydrofuran tropfenweise bei -5 bis 0°C zugegeben. Nach 1 h Rühren bei 0°C wurde dünnschichtchromatographisch kein Edukt mehr detektiert. Das Reaktionsgemisch wurde abrotiert und der Rückstand säulenchromatographisch gereinigt. Man erhielt 27,2 g (0,13 mol, 83% Ausbeute) farbloses Öl.
IR (in Substanz, $cm^{-1}$): 2919, 2864, 1718, 1631, 1422, 1358, 1307, 1284, 1248, 1159, 1063, 993.
LC-MS: 213 (M+H), 235 (M+Na).
$^1$H-NMR ($CDCl_3$, 400 MHz): $\delta$ [ppm] = 3.6 (6H), 3.4 (3H), 3.3 (1H), 2.2 (3H), 2.1 (3H).

**AAU. N-Cyclohexyl-3-oxobutanamid**

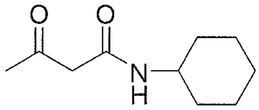

Eine Lösung von 23,6 g (0,24 mol) Cyclohexylamin in 50 ml Tetrahydrofuran wurde zu einer Lösung von 20 g Diketen (0,24 mol) in 200 ml Tetrahydrofuran tropfenweise bei -5 bis 0°C zugegeben. Nach 1 h Rühren bei 0°C wurde dünnschichtchromatographisch kein Edukt mehr detektiert. Das Reaktionsgemisch wurde abrotiert und der Rückstand aus 100 ml Ethylacetat umkristallisiert. Man erhielt 30,0 g (0,16 mol, 69 % Ausbeute) weissen Feststoff.
IR (in Substanz, $cm^{-1}$): 3259, 3088, 2929, 2852, 1716, 1648, 1627, 1560, 1449, 1427, 1359, 1346, 1193, 1162, 1105, 1001, 892, 751, 718.
LC-MS: 184 (M+H), 206 (M+Na).
$^1$H-NMR ($CDCl_3$, 400 MHz): $\delta$ [ppm] = 6.9 (1H), 3.7 (1H), 3.3 (2H), 2.2 (3H), 1.9 (2H), 1.7 (2H), 1.6 (1H), 1.2 (2H), 1.1 (3H).

**AAV. Methyl-N-(3-oxobutanoyl)glycinat**

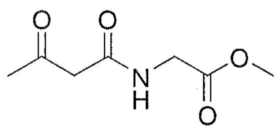

29,87 g (0,24 mol) Glycinmethylester-Hydrochlorid wurden zu einer Lösung von 20,00 g (0,24 mol) Diketen in 250 ml Toluol bei 0°C zugeben. Dann wurden 40,00 g (0,48 mol) $NaHCO_3$ zugegeben und das Reaktionsgemisch über Nacht bei Raumtemperatur weitergerührt. Da dünnschichtchromatographisch kein Edukt mehr detektiert, wurde das Reaktionsgemisch abfiltriert, abrotiert und der Rückstand säulenchromatographisch gereinigt. Man erhielt 33,4 g (0.19 mol, 81% Ausbeute) Öl.
IR (in Substanz, $cm^{-1}$): 3346, 1750, 1708, 1669, 1538, 1399, 1360, 1320, 1278, 1198, 1165, 1024.
LC-MS: 174 (M+H), 196 (M+Na).
$^1$H-NMR ($CDCl_3$, 400 MHz): $\delta$ [ppm] = 7.5 (1H), 4.0 (2H), 3.7 (3H), 3.4 (2H), 2.2 (3H).

**AAW. N-(2-Methylpropyl)-3-oxobutanamid**

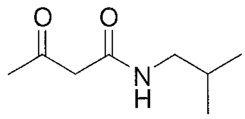

Eine Lösung von 18,20 g (0,25 mol) Isobutylamin in 50 ml Tetrahydrofuran wurde zu einer Lösung von 20 g Diketen (0,24 mol) in 200 ml Tetrahydrofuran tropfenweise bei -5 bis 0°C zugegeben. Nach 1 h Rühren bei 0°C wurde dünnschichtchromatographisch kein Edukt mehr detektiert. Das Reaktionsgemisch wurde abrotiert und der Rückstand aus 100 ml Ethylacetat umkristallisiert. Man erhielt 26,93 g (0.17 mol, 72 % Ausbeute) weissen Feststoff.
IR (in Substanz, $cm^{-1}$): 3269, 3098, 2964, 2875, 1711, 1640, 1574, 1473, 1414, 1355, 1328, 1271, 1190, 1160, 976, 823, 764, 727.
LC-MS: 158 (M+H), 180 (M+Na).
$^1$H-NMR ($CDCl_3$, 400 MHz): $\delta$ [ppm] = 7.1 (1H), 4.3 (2H), 3.1 (2H), 2.2 (3H), 1.7 (1H), 0.9 (6H).

**AAX. N-(Cyclopropylmethyl)-3-oxobutanamid**

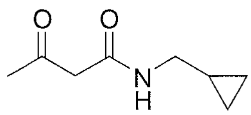

Eine Lösung von 15,1 g (0,22 mol) Cyclopropylmethylamin in 50 ml Tetrahydrofuran wurde zu einer Lösung von 17 g Diketen (0,20 mol) in 200 ml Tetrahydrofuran tropfenweise bei -5 bis 0°C zugegeben. Nach 1 h Rühren bei 0°C wurde dünnschichtchromatographisch kein Edukt mehr detektiert. Das Reaktionsgemisch wurde abrotiert und der Rückstand aus 100 ml Ethylacetat umkristallisiert. Man erhielt 19,7 g (0,13 mol, 63 % Ausbeute) weissen Feststoff.
IR (in Substanz, $cm^{-1}$): 3255, 3083, 3008, 2932, 1712, 1640, 1415, 1356, 1326, 1276, 1191, 1160, 1079, 1017, 830, 800, 775, 729.
LC-MS: 156 (M+H), 178 (M+Na).
$^1$H-NMR ($CDCl_3$, 400 MHz): $\delta$ [ppm] = 7.0 (1H), 3.4 (2H), 3.1 (2H), 2.3 (3H), 0.9 (1H), 0.5 (2H), 0.1 (2H).

**AAY. Ethyl 4-(morpholin-4-yl)-2,4-dioxobutanoat**

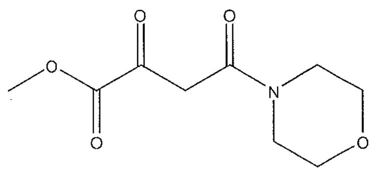

10,2 g Diisopropylamin (0,1 mol) wurden in 100 ml trockenem THF unter Stickstoff vorgelegt und in einer Kältemischung abgekühlt. Man tropfte 40 ml n-Buthyllithium in Hexan (2,5 M, 0,1 mol) langsam zu. Nach erfolgter Zugabe liess man 60 min rühren und tropfte anschliessend 12,9 g Acetylmorpholin (0,1 mol) zu. Die Reaktionsmischung wurde für weitere 20 min in der Kältemischung gerührt. In einem zweiten Kolben wurden 43,7 g Diethyloxalat (0,3 mol) in 50 ml trockenem THF in einer Kältemischung abgekühlt und die gekühlte Reaktionsmischung unter Rühren in kleinen Portionen dazu kanüliert. Man liess über Nacht unter Rühren auf Raumtemperatur erwärmen. Das THF wurde am Rotationsverdampfer entfernt und der Rückstand in 80 ml halbkonzentrierter Salzsäure aufgenommen. Man extrahierte die wässrige Phase fünfmal mit je 150ml Ethylacetat, trocknete die vereinten organischen Phasen über Natriumsulfat und engte am Rotationsverdampfer bis zur Trockene ein. Das überschüssige Diethyloxalat wurde abdestilliert (2-3 mbar, 80°C). 5,0 g Produkt wurden in Form weisser Kristalle mit Schmelzpunkt 60°C nach Kristallisation aus PE (Petrolether) erhalten.
IR (in Substanz, $cm^{-1}$): 2998, 2980, 2915, 2873, 1722, 1632, 1585, 1489, 1446, 1396, 1375, 1271, 1236, 1136, 1113, 1072, 1054, 1019, 981, 917, 874, 858, 837, 825, 766, 723, 639.
$^1$H-NMR ($CDCl_3$, 400 MHz): δ [ppm] = 1.35 (3H), 3.50-3.73 (8H), 4.33 (2H), 6.20 (1H), 14.32 (1H).

**AAZ. Ethyl-N-(3-oxobutanoyl)-L-alaninat**

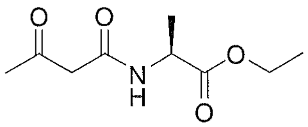

20,00 g (0,13 mol) Ethyl-(2S)-2-aminopropanoat-hydrochlorid wurden zu einer Lösung von 10,95 g (0,13 mol) Diketen in 500 ml Toluol bei 0°C zugeben. Dann wurden 21,90 g (0,26 mol) $NaHCO_3$ zugegeben und das Reaktionsgemisch über Nacht bei Raumtemperatur weitergerührt. Da dünnschichtchromatographisch kein Edukt mehr detektiert, wurde das Reaktionsgemisch abfiltriert, abrotiert und der Rückstand säulenchromatographisch gereinigt. Man erhielt 19,36 g (0,096 mol, 74% Ausbeute) farbloses Öl.
IR (in Substanz, $cm^{-1}$): 3308, 2985, 1720, 1647, 1537, 1453, 1360, 1205, 1155, 1056, 1021.
$^1$H-NMR ($CDCl_3$, 400 MHz): δ [ppm] = 7.4 (1H), 4.5 (1H), 4.1 (2H), 3.4 (2H), 2.2 (3H), 1.3 (3H), 1.2 (3H).

**ABA. N-Cyclobutyl-3-oxobutanamid**

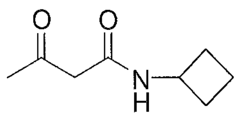

Eine Lösung von 20,00 g (0,28 mol) Cyclobutylamin in 50 ml Tetrahydrofuran wurde zu einer Lösung von 22,00 g Diketen (0,26 mol) in 200 ml Tetrahydrofuran tropfenweise bei -5 bis 0°C zugegeben. Nach 1 h Rühren bei 0°C wurde dünnschichtchromatographisch kein Edukt mehr detektiert. Das Reaktionsgemisch wurde abrotiert und der Rückstand aus 100 ml Ethylacetat umkristallisiert. Man erhielt 24,60 g (0,16 mol, 61 % Ausbeute) weissen Feststoff. IR (in Substanz, $cm^{-1}$): 3251, 3080, 2974, 2949, 1721, 1649, 1626, 1554, 1423, 1356, 1341, 1243, 1216, 1162, 994, 748, 717.
LC-MS: 156 (M+H), 178 (M+Na).
$^1$H-NMR ($CDCl_3$, 400 MHz): δ [ppm] = 7.2 (1H), 4.3 (1H), 3.3 (2H), 2.3 (5H), 2.8 (2H), 2.6 (2H).

**ABB. 1-(Azetidin-1-yl)-butan-1,3-dion**

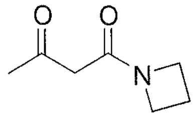

Eine Lösung von 18,00 g (0,31 mol) Azetidin in 50 ml Tetrahydrofuran wurde zu einer Lösung von 25,00 g Diketen (0,30 mol) in 200 ml Tetrahydrofuran tropfenweise bei -5 bis 0°C zugegeben. Nach 1 h Rühren bei 0°C wurde dünnschichtchromatographisch kein Edukt mehr detektiert. Das Reaktionsgemisch wurde abrotiert und der Rückstand aus 100 ml Ethylacetat umkristallisiert. Man erhielt 26,45 g (0,19 mol, 63 % Ausbeute) weissen Feststoff. IR (in Substanz, $cm^{-1}$): 3107, 2983, 2958, 2885, 1718, 1631, 1462, 1443, 1410, 1367, 1306, 1298, 1237, 1188, 1168, 1154, 1112, 1014, 851, 736.
LC-MS: 142 (M+H), 164 (M+Na).
$^1$H-NMR ($CDCl_3$, 400 MHz): $\delta$ [ppm] = 4.1 (2H), 3.9 (2H), 3.1 (2H), 2.1 (5H).

**ABC. Ethyl-1-(3-oxobutanoyl)-piperidin-4-carboxylat**

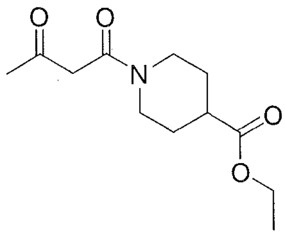

Eine Lösung von 18,70 g (0,12 mol) Ethyl-4-piperidincarboxylat in 50 ml Tetrahydrofuran wurde zu einer Lösung von 10,00 g Diketen (0,12 mol) in 200 ml Tetrahydrofuran tropfenweise bei -5 bis 0°C zugegeben. Nach 1 h Rühren bei 0°C wurde dünnschichtchromatographisch kein Edukt mehr detektiert. Das Reaktionsgemisch wurde abrotiert und der Rückstand säulenchromatographisch gereinigt. Man erhielt 19,52 g (0,081 mol, 68 % Ausbeute) farbloses Öl.
IR (in Substanz, $cm^{-1}$): 2957, 2932, 2863, 1721, 1634, 1446, 1314, 1272, 1250, 1173, 1158, 1110, 1097, 1039, 939.
$^1$H-NMR ($CDCl_3$, 400 MHz): $\delta$ [ppm] = 4.3 (1H), 4.1 (2H), 3.7 (1H), 3.5 (2H), 3.1 (1H), 2.9 (1H), 2.5 (1H), 2.2 (3H), 1.9 (2H), 1.6 (2H), 1.2 (3H).

**ABD. 1-(4-Hydroxypiperidin-1-yl)-4-methoxybutan-1,3-dion**

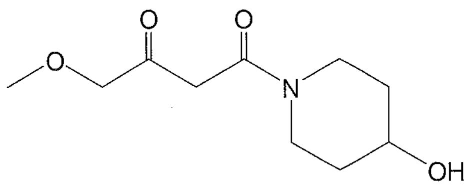

0,34 mol (48,7 g) Methyl-4-methoxy-acetoacetat und 0.36 mol (36,0 g) 4-Hydroxypiperidin wurden 5 h bei 120°C am Wasserabscheider gerührt. 45 g des Rohprodukts wurden über 1 kg Kieselgel mit Ethylacetat/Ethanol 9/1 gereinigt. Es wurden 23,7 g der Titelverbindung erhalten.
IR (in Substanz, $cm^{-1}$): 2931, 1729, 1618, 1448, 1365, 1310, 1266, 1201, 1132, 1101, 1072, 1025, 977, 935, 809, 776, 716, 681.
LC-MS: $M+H^+$, 216.8; $M+Na^+$, 238.7.
1H-NMR (DMSO-$d_6$, 400 MHz): Enol-Tautomer (14%), $\delta$ [ppm] = 15.10 (s, 1H), 5.56 (s, 1H), 4.76 (d (J = 4 Hz), 1H), 3.90 (s, 2H), 3.9-2.9 (m, 5H), 3.29 (s, 3H), 1.8-1.1 (m, 4H); Keto-Tautomer (86%), $\delta$ [ppm] = 4.73 (d (J = 4 Hz), 1H), 4.10

(s, 2H), 3.9-2.9 (m, 5H), 3.57 (s, 2H), 3.27 (s, 3H), 1.8-1.1 (m, 4H).

**TESTMETHODE:**

Die hervorragenden Fe-Utilisationen, die durch die erfindungsgemäßen Fe-Komplexe, erreicht werden können, wurden durch folgendes Mäuse-Modell gemessen.

Männliche NMRI (SPF)-Mäuse (ca. 3 Wochen alt) wurden ca. 3 Wochen mit eisenarmer Diät (ca. 5 ppm Eisen) gefüttert. Dann wurde ihnen während 2 mal 5 Tagen mit einer Unterbrechung von 2 Tagen (Tage 1 - 5 und 8 - 12) die Eisenkomplexe mittels Schlundsonde verabreicht (2 mg Eisen/kg Körpergewicht/Tag). Die Utilisation am Tag 15 wurde berechnet aus der Hämoglobinzunahme und der Körpergewichtszunahme nach der Formel

$$\text{Utilisation (\%)} = \frac{\Delta\,\text{Eisenutilisation} * 100}{\text{Fe Dos.}} = \frac{(\text{Fe ut.} - \text{Fe ut.Control}) * 100}{\text{Fe Dos.}}$$

$$= [(Hb_{2(3)} * BW_{9(14)} - Hb_1 * BW_4) * 0{,}07 * 0{,}0034 - (Hb_{2(3)\,Control} * BW_{9(14)\,Control} - Hb_{1\,Control} * BW_{4\,Control}) * 0{,}07 * 0{,}0034)] * 100 / \text{Fe Dos.}$$

$$= [(Hb_{2(3)} * BW_{9(14)} - Hb_1 * BW_4) * 0{,}000238 - (Hb_{2(3)\,Control} * BW_{9(14)\,Control} - Hb_{1\,Control} * BW_{4\,Control}) * 0{,}000238] * 100 / \text{Fe Dos.}$$

$$= (Hb_{2(3)} * BW_{9(14)} - Hb_1 * BW_4 - Hb_{2(3)\,Control} * BW_{9(14)\,Control} + Hb_{1\,Control} * BW_{4\,Control}) * 0{,}0238 / \text{Fe Dos.}$$

0.07 = Faktor für 70 ml Blut per kg Körpergewicht (BW)
0.0034 = Faktor für 0,0034 g Fe/g Hb
$Hb_1$ = Hämoglobin-Wert (g/l) am Tag 1
$Hb_{2(3)}$ = Hämoglobin-Wert (g/l) am Tag 8 (or 15)
$BW_4$ = Körpergewicht (g) am Tag 1
$BW_{9(14)}$ = Körpergewicht (g) am Tag 8 (or 15)
$Hb_{1\,Control}$ = Durchschnitts-Hämoglobinwert (g/l) am Tag 1 in der Kontrollgruppe,
$Hb_{2(3)\,Control}$ = Durchschnitts-Hämoglobinwert (g/l) am Tag 8 (oder 15) in der Kontrollgruppe,
$BW_{4\,Control}$ = Durchschnittskörpergewicht (g) am Tag 1 in der Kontrollgruppe,
$BW_{9(14)\,Control}$ = Durchschnittskörpergewicht (g) am Tag 8 (oder 15) in der Kontrollgruppe,
Fe Dos. = Gesamtes verabreichtes Eisen (mg Fe) während 5 oder 10 Tage,
Fe ut. = $(Hb_{2(3)} * BW_{9(14)} - Hb_1 * BW_4) * 0.07 * 0.0034$ (mg Fe)
Δ Utilisation = Fe tot. utilisiert (Untersuchte Gruppe) - Fe ut. Kontrollgruppe, utilisiert aus Nahrung, (mg Fe)

Tabelle:

| Beispiel-Nr. | Utilisation n 15 d (abs. %) |
|---|---|
| 1 | 61 |
| 2 | 41 |
| 3 | 95 |
| 4 | 90 |
| 5 | 61 |
| 6 | 57 |
| 7 | 41 |
| 8 | 69 |
| 9 | 54 |
| 10 | 45 |

(fortgesetzt)

| Beispiel-Nr. | Utilisation n 15 d (abs. %) |
|---|---|
| 11 | 75 |
| 12 | 35 |
| 13 | 71 |
| 14 | 68 |
| 15 | 68 |
| 16 | 60 |
| 17 | 48 |
| 18 | 81 |
| 19 | 73 |
| 20 | 77 |
| 21 | - |
| 22 | 78 |
| 23 | 88 |
| 24 | 70 |
| 25 | 60 |
| 26 | 64 |
| 27 | 88 |
| 28 | 73 |
| 29 | 79 |
| 30 | - |
| 31 | 71 |
| 32 | 81 |
| 33 | 69 |
| 34 | 32 |
| 35 | 44 |
| 36 | 79 |
| 37 | 34 |
| 38 | 77 |
| 39 | 66 |
| 40 | 87 |
| 41 | 68 |
| 42 | 30 |
| 43 | 75 |
| 44 | 73 |
| 45 | - |
| 46 | 78 |
| 47 | 61 |
| 48 | 53 |

(fortgesetzt)

| Beispiel-Nr. | Utilisation n 15 d (abs. %) |
|---|---|
| 49 | - |
| 50 | - |
| 51 | 78 |
| 52 | 84 |
| 53 | - |
| 54 | 76 |
| 55 | 84 |
| 56 | - |
| 57 | - |
| 58 | - |
| 59 | 80 |
| 60 | 80 |
| 61 | 73 |
| 62 | 68 |
| 63 | 71 |
| 64 | 79 |
| 65 | 73 |
| 66 | - |
| "-" bedeutet: nicht bestimmt. | |

**HERSTELLUNGSBEISPIELE:**

**Beispiel 1**

**Tris-(3-oxobutanamid)-eisen(III)-Komplex:**

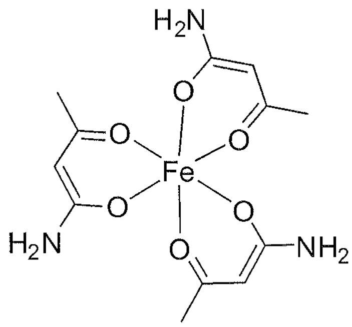

Zu einer Lösung von 45,00 g (0,45 mol) 3-Oxobutanamid in 400 ml Ethanol wurde eine Lösung von 24,00 g (0,15 mol) Eisen(III)-chlorid (wasserfrei) in 400 ml Ethanol tropfenweise zugegeben. Dann wurden 37,46 g (0,45 mol) Natriumbicarbonat portionsweise zugegeben. Nach 4 Stunden Rühren bei Raumtemperatur wurde das Reaktionsgemisch abfiltriert, das Filtrat abrotiert und der Rückstand über Nacht im Trockenschrank bei 50°C getrocknet. Man erhielt 51,63 g Produkt als schwarzroten Feststoff.
IR (in Substanz, $cm^{-1}$): 3317, 3187, 1618, 1569, 1507, 1415, 1328, 1194, 1092, 1034,978,935,780.

Elementaranalyse: C 37,41; H 5,78; N 8,80.
Fe-Gehalt 15,80 % [m/m].

**Beispiel 2**

**Tris-(*N*,*N*-diethyl-3-oxobutanamid)-eisen(III)-Komplex**

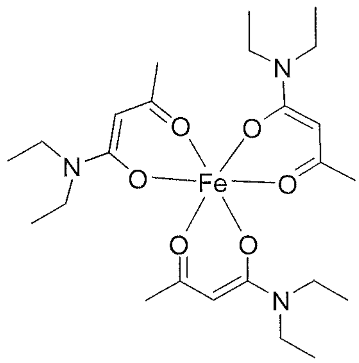

Zu einer Lösung von 2,90 g (18,50 mmol) *N*,*N*-Diethyl-3-oxobutanamid in 30 ml Ethanol wurde eine Lösung von 1,00 g (6,17 mmol) Eisen(III)-chlorid (wasserfrei) in 10 ml Ethanol tropfenweise zugegeben. Dann wurden 3,11 g (37,00 mmol) Natriumbicarbonat portionsweise zugegeben. Nach 2 Stunden Rühren bei Raumtemperatur wurde das Reaktionsgemisch abfiltriert, das Filtrat abrotiert und der Rückstand über Nacht im Trockenschrank bei 50°C getrocknet. Man erhielt 3,50 g Produkt als schwarzrotes Öl.
IR (in Substanz, $cm^{-1}$): 2973, 2932, 1597, 1557, 1511, 1492, 1454, 1434, 1374, 1356, 1309, 1274, 1204, 1163, 1082, 1004, 962.
Elementaranalyse: C 53,54; H 8,00; N 7,88.
Fe-Gehalt: 9,63 % [m/m].

**Beispiel 3:**

**Tris-(*N*,*N*-dimethyl-3-oxobutanamid)-eisen(III)-Komplex**

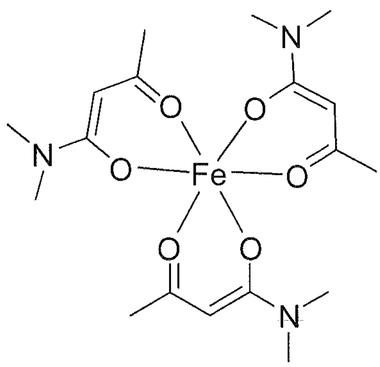

Zu einer Lösung von 8,96 g (55,50 mmol, 80% in Wasser) *N*,*N*-Dimethyl-3-oxobutanamid in 50 ml Ethanol wurde eine Lösung von 3,00 g (18,50 mmol) Eisen(III)-chlorid (wasserfrei) in 50 ml Ethanol tropfenweise zugegeben. Dann wurden 4,66 g (55,50 mmol) Natriumbicarbonat portionsweise zugegeben. Nach 4 Stunden Rühren bei Raumtemperatur wird das Reaktionsgemisch abfiltriert, mit 10 ml EtOH gewaschen, das Filtrat abrotiert und der Rückstand über Nacht im Trockenschrank bei 50°C getrocknet. Man erhielt 8,37 g Produkt als schwarzrotes Öl.
IR (in Substanz, $cm^{-1}$): 2918, 1720, 1643, 1557, 1523, 1490, 1433, 1401, 1347, 1260, 1211, 1177, 1061, 1032, 989, 955.
Elementaranalyse: C 47,39; H 6,70; N 9,22.

Fe-Gehalt: 11,13 % [m/m].

**Beispiel 4:**

**Tris-(*N*-methyl-3-oxobutanamid)-eisen(III)-Komplex**

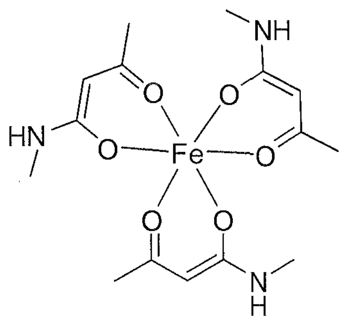

Zu einer Lösung von 9,13 g (55,50 mmol, 80% in Wasser) N-Methyl-3-oxobutanamid in 50 ml Ethanol wurde eine Lösung von 3,00 g (18,50 mmol) Eisen(III)-chlorid (wasserfrei) in 50 ml Ethanol tropfenweise zugegeben. Dann wurden 4,66 g (55,50 mmol) Natriumbicarbonat portionsweise zugegeben. Nach 2 Stunden Rühren bei Raumtemperatur wurde das Reaktionsgemisch abfiltriert, mit 10 ml EtOH gewaschen, das Filtrat abrotiert und der Rückstand über Nacht im Trockenschrank bei 50°C getrocknet. Man erhielt 7,98 g Produkt als schwarzroten Feststoff.
IR (in Substanz, $cm^{-1}$): 3336, 3119, 2913, 1558, 1500, 1448, 1411, 1275, 1191, 1157, 1104, 1010, 959, 935, 779.
Elementaranalyse: C 42,23; H 5,78; N 9,70.
Fe-Gehalt: 12,46 % [m/m].

**Beispiel 5**

**Tris(3-Oxo-2-(piperidin-1-ylcarbonyl)butannitril)-eisen(III)-Komplex**

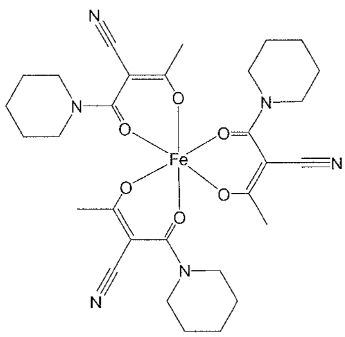

1,4 g (8,5 mmol) $FeCl_3$ (wasserfrei) gelöst in 20 ml Ethanol 99% wurden zu einer Lösung von 5,0 g (25,7 mmol) 2,4-Dioxo-3-cyanobutylpiperidin in 40 ml EtOH 99% gegeben. Anschliessend wurden 2,2 g (25,6 mmol) $NaHCO_3$ zugegeben und 1 Stunden bei 20 - 25 °C nachgerührt. Die Reaktionsmischung wird filtriert, das Filtrat am Rotationsverdampfer bei 50 °C / < 100 mbar zur Trockene eingeengt und der Rückstand mind. 15 Stunden bei 50 °C / < 100 mbar im Vakuumtrockenschrank getrocknet. Der erhaltene Feststoff wurde in 200 ml Wasser mit einem Ultraturax für ca. 30 min suspendiert, abfiltriert und erneut für mind. 15 Stunden bei 50 °C / < 100 mbar im Vakuumtrockenschrank getrocknet. Man erhielt 5,3 g Produkt als orangen Feststoff.
IR (in Substanz, $cm^{-1}$): 2936, 2858, 2197, 1594, 1532, 1506, 1439, 1387, 1270, 1217, 1169, 1145, 1072, 1020, 973, 905, 854, 755, 700, 653.
Elementaranalyse: C 57,0; H 6,40; N 12,60.
Fe-Gehalt: 8,30 % [m/m].

Beispiel 6

**Tris-(2-chlor-*N*-methyl-3-oxobutanamid)-eisen(III)-Komplex**

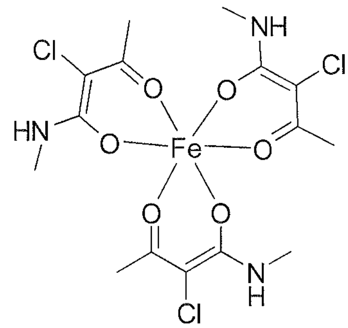

Zu einer Lösung von 2,77 g (18,50 mmol) 2-Chlor-*N*-methyl-3-oxobutanamid in 30 ml Ethanol wurde eine Lösung von 1,00 g (6,17 mmol) Eisen(III)-chlorid wasserfrei in 10 ml Ethanol tropfenweise zugegeben. Dann wurden 1,55 g (18,50 mmol) Natriumbicarbonat portionsweise zugegeben. Nach 2 Stunden Rühren bei Raumtemperatur wurde das Reaktionsgemisch abfiltriert, mit 10 ml EtOH gewaschen, das Filtrat abrotiert und der Rückstand über Nacht im Trockenschrank bei 50°C getrocknet. Man erhielt 3,19 g Produkt als violetten Feststoff.
IR (in Substanz, $cm^{-1}$): 3359, 2942, 1545, 1468, 1406, 1375, 1260, 1155, 1118, 1028,935,796,748.
Elementaranalyse: C 33,42; H 4,23; N 7,74.
Fe-Gehalt: 10,72 % [m/m].

**Beispiel 7**

**Tris(*N*-butyl-3-oxobutanamid)-eisen(III)-Komplex**

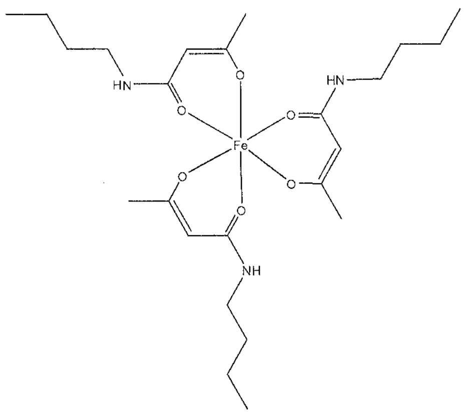

1,4 g (8,6 mmol) $FeCl_3$ (wasserfrei) gelöst in 10 ml Methanol wurden zu einer Lösung von 5 g (31,8 mmol) *N*-n-Butyl-3-oxo-butanamid in 40 ml Toluol gegeben. Anschliessend wurden 4,4 g (52,4 mmol) $NaHCO_3$ sowie 2,2 g (15,5 mmol) $Na_2SO_4$ (wasserfrei) zugegeben und 1 Stunden bei 20 - 25 °C nachgerührt. Die Reaktionsmischung wurde filtriert, das Filtrat am Rotationsverdampfer bei 50 °C / < 100 mbar zur Trockne eingeengt und der Rückstand mindestens 15 Stunden bei 50 °C / < 100 mbar im Vakuumtrockenschrank getrocknet. Man erhielt 5,0 g Produkt als roten Feststoff.
IR (in Substanz, $cm^{-1}$): 2935, 1645, 1592, 1533, 1508, 1439, 1387, 1270, 1219, 1143, 1073, 1020, 973, 909, 855, 822, 699, 652.
Fe-Gehalt: 9,0 % [m/m].

**Beispiel 8**

**Tris(*N,N*-dibutyl-3-oxobutanamid)-eisen(III)-Komplex**

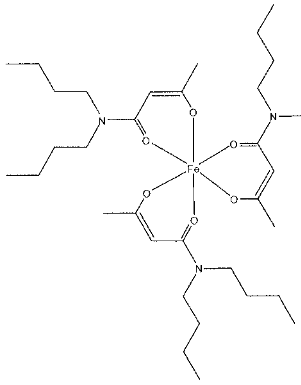

48,5 g (0,23 mol) N,N-Dibutyl-3-oxo-butanamid und 20,6 g (0,08 mol) $FeCl_3$ x $6H_2O$ wurden in 245 ml Ethanol 96% vorgelegt. 14,1 g (0,13 mol) $Na_2CO_3$ (wasserfrei) wurden in mehreren Portionen innerhalb von ca. 5 min zugegeben. Das Reaktionsgefäss wird dabei mit einem Wasserbad von ca. 20 °C gekühlt. Die Reaktionsmischung wurde 6 Stunden bei 20 - 25 °C gerührt, anschliessend über eine Glasfritte filtriert und am Rotationsverdampfer bei 50 °C / < 100 mbar zur Trockne eingeengt. Das ölige Produkt wurde für mind. 15 Stunden unter Hochvakuum bei 20 - 25 °C getrocknet. Man erhielt 50 g Produkt als rotbraunes Öl.
IR (in Substanz, $cm^{-1}$): 2957, 2931, 2872, 1596, 1558, 1513, 1492, 1461, 1431, 1366, 1292, 1257, 1229, 1199, 1155, 1112, 1007, 956, 764. CHN-Elementaranalyse: C 61,10; H 9,60; N 6,00.
Fe-Gehalt: 8,00 % [m/m].

**Beispiel 9**

**Tris-(1-(piperidin-1-yl)-butan-1,3-dion)-eisen(III)-Komplex**

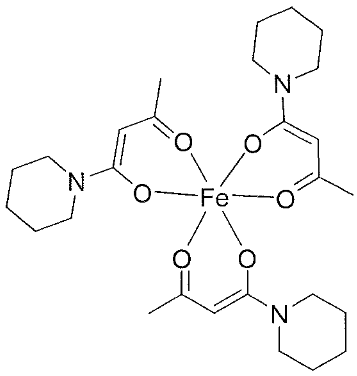

Zu einer Lösung von 3,13 g (18,50 mmol) 1-(Piperidin-1-yl)-butan-1,3-dion in 40 ml Ethanol wurde eine Lösung von 1,00 g (6,17 mmol) Eisen(III)-chlorid (wasserfrei) in 10 ml Ethanol tropfenweise zugegeben. Dann wurden 6,26 g (74,00 mmol) Natriumbicarbonat portionsweise zugegeben. Nach 2 Stunden Rühren bei Raumtemperatur wurde das Reaktionsgemisch abfiltriert, mit 10 ml EtOH gewaschen, das Filtrat abrotiert und der Rückstand über Nacht im Trockenschrank bei 50°C getrocknet. Man erhielt 3,72 g Produkt als schwarzes Öl.
IR (in Substanz, $cm^{-1}$): 2930, 2852, 1594, 1556, 1509, 1483, 1461, 1442, 1374, 1347, 1256, 1231, 1205, 1022, 958.
Elementaranalyse: C 55,33; H 7,49; N 7,01.
Fe-Gehalt: 9,30 % [m/m].

**Beispiel 10**

**Tris(3-oxoheptanamid)-eisen(III)-Komplex**

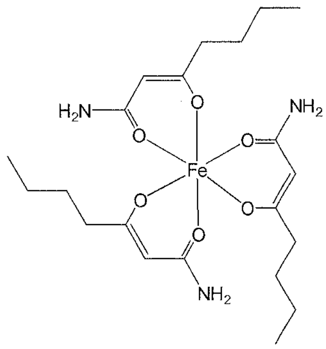

Im 100 ml Erlenmeyerkolben mit Trockenrohr wurden 15 mmol (2,147 g) 3-Oxoheptanamid in 50 ml Ethanol (wasserfrei) gelöst und 5 mmol (0,811 g) $FeCl_3$ (wasserfrei) zugegeben. Nach 5 min wurden 20 mmol (1,68 g) $NaHCO_3$ zugegeben und weitere 2 Stunden gerührt. Die pH-Kontrolle ergab nach 2 Stunden pH 3,78 (Probe wurde filtriert und mit Wasser verdünnt; nach 0,5 Stunden pH 2,78, nach 1 Stunde 3,19, nach 1.5 Stunden 3,56). Das Reaktionsgemisch wurde filtriert, das Filtrat am Rotationsverdampfer eingeengt und das Produkt im Ölpumpenvakuum getrocknet. Man erhielt 2,25 g Produkt.
IR (in Substanz, $cm^{-1}$): 3318, 3195, 2956, 2930, 2870, 1715, 1617, 1564, 1507, 1458, 1355, 1192, 1093, 946, 780, 663.
Elementaranalyse: C 49,06; H 7,45; N 8,28.
Fe-Gehalt: 10,91 % [m/m].

**Beispiel 11**

**Tris(*N*-methyl-3-oxoheptanamid)-eisen(III)-Komplex**

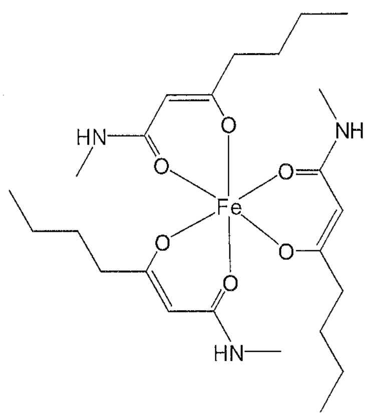

Im 100 ml Erlenmeyerkolben mit Trockenrohr wurden 8,3 mmol (1,37 g) 3-Oxoheptansäuremethylamid in 40 ml Ethanol (wasserfrei) gelöst und 2,8 mmol (0,447 g) $FeCl_3$ (wasserfrei) zugegeben. Es wurden 11 mmol (0,927 g) $NaHCO_3$ zugegeben und 2,5 Stunden gerührt. Die pH-Kontrolle ergab nach 2,5 Stunden pH 3,8 (Probe filtriert und mit Wasser verdünnt). Das Reaktionsgemisch wurde filtriert, das Filtrat am Rotationsverdampfer eingeengt und das Produkt im Vakuumtrockenschrank bei 50°C getrocknet. Man erhielt 1,20 g Produkt.
IR (in Substanz, $cm^{-1}$): 3283, 3117, 2956, 2932, 2871, 1718, 1561, 1502, 1420, 1377, 1271, 1157, 1108, 1082, 967, 937, 886, 779, 649.
Elementaranalyse: C 52,2; H 7,65; N 7,61.

Fe-Gehalt: 9,14 % [m/m].

**Beispiel 12**

**Tris-(*N*,*N*-bis-(2-hydroxyethyl)-3-oxobutanamid)-eisen(III)-Komplex**

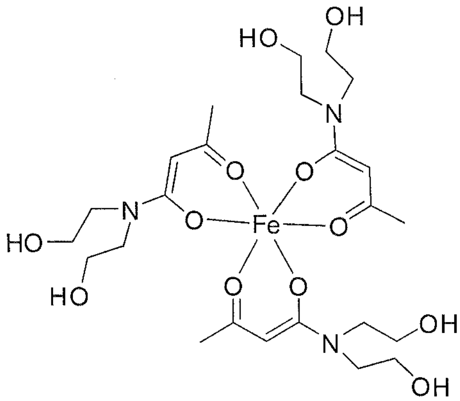

Zu einer Lösung von 3,50 g (18,50 mmol) *N*,*N*-Bis-(2-hydroxyethyl)-3-oxobutanamid in 30 ml Ethanol wurde eine Lösung von 1,00 g (6,17 mmol) Eisen(III)-chlorid (wasserfrei) in 10 ml Ethanol tropfenweise zugegeben. Dann wurden 1,56 g (18,50 mmol) Natriumbicarbonat portionsweise zugegeben. Nach 4 Stunden Rühren bei Raumtemperatur wurde das Reaktionsgemisch abfiltriert, mit 10 ml EtOH gewaschen, das Filtrat abrotiert und der Rückstand über Nacht im Trockenschrank bei 50°C getrocknet. Man erhielt 4,19 g Produkt als schwarzes Öl.
IR (in Substanz, $cm^{-1}$): 3327, 2974, 2934, 2880, 1632, 1567, 1516, 1494, 1438, 1361, 1301, 1228, 1206, 1170, 1052.
Elementaranalyse: C 43,61; H 7,46; N 5,34.
Fe-Gehalt: 8,14 % [m/m].

**Beispiel 13**

**Tris-(*N*-butyl-*N*-methyl-3-oxobutanamid)-eisen(III)-Komplex**

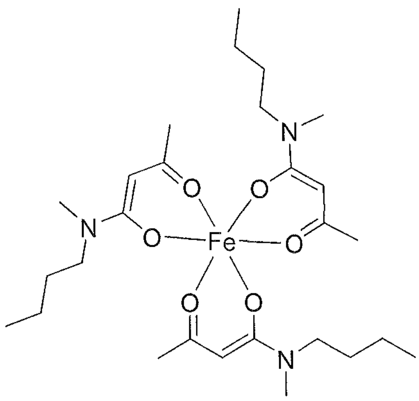

Zu einer Lösung von 1,58 g (9,25 mmol) *N*-n-Butyl-*N*-methyl-3-oxobutyramid in 15 ml Ethanol wurde eine Lösung von 0,50 g (3,08 mmol) Eisen(III)-chlorid (wasserfrei) in 5 ml Ethanol tropfenweise zugegeben. Dann wurden 0,78 g (9,25 mmol) Natriumbicarbonat portionsweise zugegeben. Nach 4 Stunden Rühren bei Raumtemperatur wurde das Reaktionsgemisch abfiltriert, mit 10 ml EtOH gewaschen, das Filtrat abrotiert und der Rückstand über Nacht im Trockenschrank bei 50°C getrocknet. Man erhielt 1,80 g Produkt als schwarzes Öl.
IR (in Substanz, $cm^{-1}$): 2956, 2930, 2871, 1598, 1557, 1515, 1492, 1464, 1353, 1308, 1230, 1211, 1088, 957.
Elementaranalyse: C 55,26; H 8,25; N 7,21.

Fe-Gehalt: 9,36 % [m/m].

**Beispiel 14**

**Tris-(1-(4-hydroxypiperidin-1-yl)-butan-1,3-dion)-eisen(III)-Komplex**

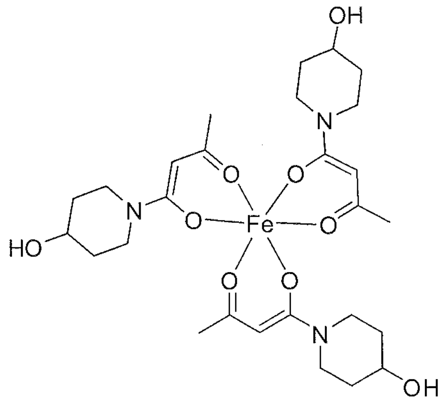

Zu einer Lösung von 3,43 g (18,51 mmol) 1-(4-Hydroxypiperidin-1-yl)-butan-1,3-dion in 30 ml Ethanol wurde eine Lösung von 1,00 g (6,17 mmol) Eisen(III)-chlorid (wasserfrei) in 10 ml Ethanol tropfenweise zugegeben. Dann wurden 3,11 g (37,00 mmol) Natriumbicarbonat portionsweise zugegeben. Nach 4 Stunden Rühren bei Raumtemperatur wurde das Reaktionsgemisch abfiltriert, mit 10 ml EtOH gewaschen, das Filtrat abrotiert und der Rückstand über Nacht im Trockenschrank bei 50°C getrocknet. Man erhielt 3,98 g Produkt als braunen Feststoff.
IR (in Substanz, $cm^{-1}$): 3334, 2923, 2859, 1589, 1554, 1509, 1485, 1443, 1362, 1265, 1227, 1205, 1054, 1023, 954.
Elementaranalyse: C 51,47; H 7,26; N 6,37.
Fe-Gehalt: 8,28 % [m/m].

**Beispiel 15**

**Tris-(3-oxo-*N,N*-dipropylbutanamid)-eisen(III)-Komplex**

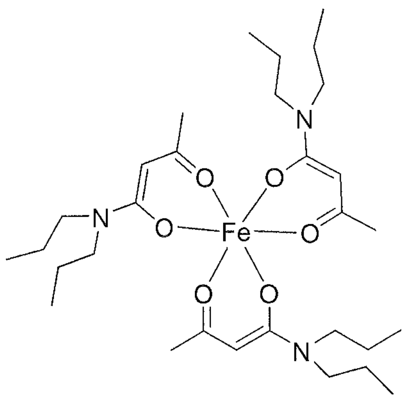

Zu einer Lösung von 1.71 g (9,25 mmol) 3-Oxo-*N,N*-di-n-propylbutanamid in 20 ml Ethanol wurde eine Lösung von 0,5 g (3,08 mmol) Eisen(III)-chlorid (wasserfrei) in 5 ml Ethanol tropfenweise zugegeben. Dann wurden 1,56 g (18,50 mmol) Natriumbicarbonat portionsweise zugegeben. Nach 2 Stunden Rühren bei Raumtemperatur wurde das Reaktionsgemisch abfiltriert, mit 10 ml EtOH gewaschen, das Filtrat abrotiert und der Rückstand über Nacht im Trockenschrank bei 50°C getrocknet. Man erhielt 1,94 g Produkt als braunes Öl.
IR (in Substanz, $cm^{-1}$): 2962, 2931, 2874, 1597, 1557, 1510, 1491, 1468, 1456, 1431, 1367, 1299, 1246, 1202, 1164, 1102, 1058, 996, 958.

Elementaranalyse: C 56,93; H 8,73; N 6,50.
Fe-Gehalt: 8,15 % [m/m].

**Beispiel 16**

**Tris-(*N*-hexyl-3-oxobutanamid)-eisen(III)-Komplex**

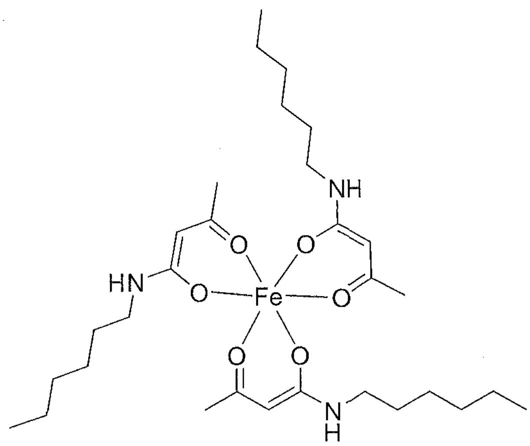

Zu einer Lösung von 3,43 g (18,50 mmol) *N*-n-Hexyl-3-oxobutanamid in 25 ml Ethanol wurde eine Lösung von 1,0 g (6,17 mmol) Eisen(III)-chlorid (wasserfrei) in 5 ml Ethanol tropfenweise zugegeben. Dann wurden 3,12 g (37,00 mmol) Natriumbicarbonat portionsweise zugegeben. Nach 4 Stunden Rühren bei Raumtemperatur wurde das Reaktionsgemisch abfiltriert, mit 10 ml EtOH gewaschen, das Filtrat abrotiert und der Rückstand über Nacht im Trockenschrank bei 50°C getrocknet. Man erhielt 3,86 g Produkt als braunes Öl.
IR (in Substanz, $cm^{-1}$): 3289, 2956, 2926, 2857, 1719, 1656, 1589, 1556, 1503, 1453, 1434, 1410, 1274, 1188, 1037, 1017, 960, 946.
Fe-Gehalt: 8,34 % [m/m].

**Beispiel 17**

**Tris(*N*,*N*-dimethyl-3-oxoheptanamid)-eisen(III)-Komplex**

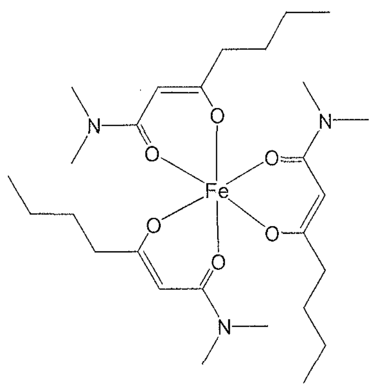

Analog zu den vorhergehenden Beispielen wurde der Tris(*N*,*N*-dimethyl-3-oxoheptanamid)-eisen(III)-Komplex ausgehend von Eisen(III)-chlorid und 3-Oxoheptansäuredimethylamid hergestellt.

**Beispiel 18**

**Tris(3-oxopentanamid)-eisen(III)-Komplex**

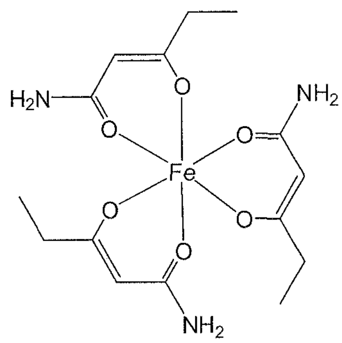

Im 250 ml Rundkolben mit Trockenrohr wurden 15,0 mmol (1,73 g) 3-Oxopentanamid in 100 ml Ethanol (wasserfrei) gelöst und 5,0 mmol (0,811 g) $FeCl_3$ (wasserfrei) zugegeben. Es wurden 20 mmol (1,68 g) $NaHCO_3$ zugegeben und 3 Stunden gerührt. Die pH-Kontrolle ergab pH 5,2 (Probe filtriert und mit Wasser verdünnt). Das Reaktionsgemisch wurde filtriert, das Filtrat am Rotationsverdampfer eingeengt und das Produkt im Vakuumtrockenschrank bei 50°C getrocknet. Man erhielt 2,1 g Produkt.
IR (in Substanz, $cm^{-1}$): 3318, 3189, 2974, 2938, 2361, 1715, 1625, 1567, 1509, 1458, 1341, 1299, 1242, 1184, 1107, 1077, 1020, 952, 917, 804, 785. Elementaranalyse: C 39,86; H 5,821; N 9,28.
Fe-Gehalt: 11,85 % [m/m].

**Beispiel 19**

**Tris(*N*,*N*-dimethyl-3-oxopentanamid)-eisen(III)-Komplex**

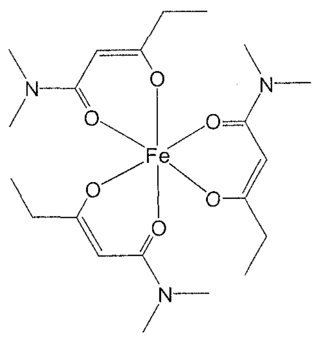

Im 100 ml Rundkolben mit Trockenrohr wurden 15,0 mmol (2,15 g) *N*,*N*-Dimethyl-3-oxopentanamid in 75 ml Ethanol (wasserfrei) gelöst und 5,0 mmol (0,811 g) $FeCl_3$ (wasserfrei) zugegeben. Es wurden 20 mmol (1,68 g) $NaHCO_3$ zugegeben und 1,5 Stunden gerührt. Die pH-Kontrolle ergab pH 4,2 (Probe filtriert und mit Wasser verdünnt). Das Reaktionsgemisch wurde filtriert, das Filtrat am Rotationsverdampfer eingeengt und das Produkt im Vakuumtrockenschrank bei 50°C getrocknet. Man erhielt 2,4 g Produkt.
IR (in Substanz, $cm^{-1}$): 2965, 2933, 2871, 2362, 1720, 1596, 1553, 1526, 1492, 1428, 1402, 1371, 1352, 1311, 1259, 1197, 1175, 1061, 1020, 983, 932, 835, 798,766,718,688.
Elementaranalyse: C 50,35; H 7,307; N 8,31.
Fe-Gehalt: 10,50 % [m/m].

**Beispiel 20**

**Tris-(ethyl *N*-(3-oxobutanoyl)glycinat)-eisen(III)-Komplex**

**(Tris((3-Oxo-butyrylamino)essigsäureethylester)eisen)**

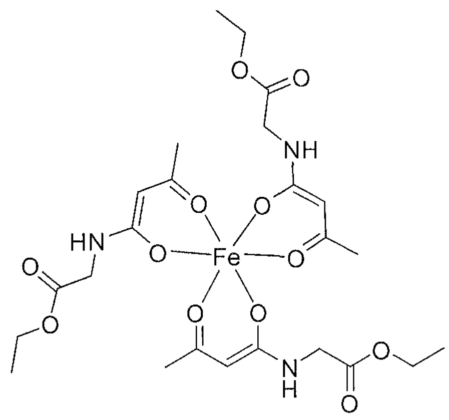

Zu einer Lösung von 1,73 g (9,25 mmol) Ethyl *N*-(3-oxobutanoyl)glycinate (bzw. (3-Oxo-butyrylamino)essigsäureethylester) in 20 ml Ethanol wurde eine Lösung von 0,5 g (3,08 mmol) Eisen(III)-chlorid (wasserfrei) in 5 ml Ethanol tropfenweise zugegeben. Dann wurden 1,56 g (18,50 mmol) Natriumbicarbonat portionsweise zugegeben. Nach 2 Stunden Rühren bei Raumtemperatur wurde das Reaktionsgemisch abfiltriert, mit 10 ml EtOH gewaschen, das Filtrat abrotiert und der Rückstand über Nacht im Trockenschrank bei 50°C getrocknet. Man erhielt 2,11 g Produkt als braunes Öl.
IR (in Substanz, $cm^{-1}$): 3304, 2982, 2936, 1736, 1661, 1586, 1543, 1497, 1453, 1411, 1374, 1284, 1184, 1134, 1051, 1026.
Elementaranalyse: C 44,14; H 6,11; N 6,18.
Fe-Gehalt: 8,47 % [m/m].

**Beispiel 21**

**Tris-(*N*,*N*-Dibutyl-3-oxopentanamid)-eisen(III)-komplex**

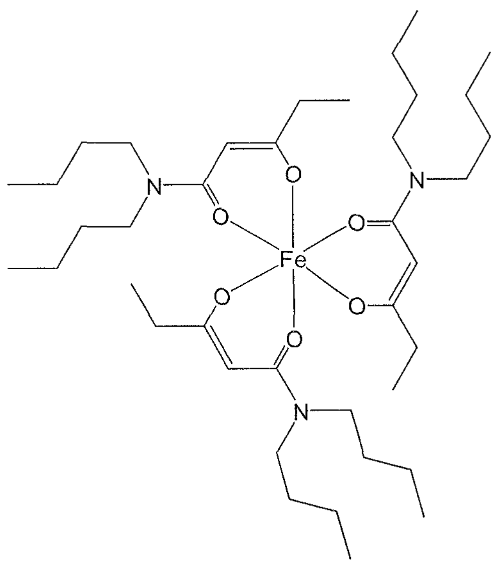

Es wurden 18 mmol (4,09 g) *N,N*-Dibutyl-3-oxopentanamid in 90 ml Ethanol (wasserfrei) gelöst und 6 mmol (0,811 g) $FeCl_3$ (wasserfrei) zugegeben. Dann wurden 5,93 ml Natriumethylat-Lösung (21% m/m, ca. 18 mmol Natriumethylat) zugegeben und 1 h gerührt. Das Reaktionsgemisch wurde filtriert und das Filtrat am Rotationsverdampfer eingeengt. Der Rückstand wurde in 120 ml Dichlormethan aufgenommen, erneut filtriert, das Filtrat eingedampft und das Produkt im Vakuumtrockenschrank bei 50°C getrocknet. Man erhielt 4,0 g der Titelverbindung.
IR (in Substanz, $cm^{-1}$) 2957, 2931, 2872, 2359, 1721, 1634, 1596, 1556, 1513, 1491, 1461, 1429, 1393, 1366, 1316, 1291, 1254, 1225, 1190, 1155, 1112, 1079, 1062,990,926,799,767,733,711,657.
CHN-Elementaranalyse: C, 62.57; H, 9.64; N, 5.66.
Fe-Gehalt: 7.3% [m/m]

**Beispiel 22**

**Tris-(2-Fluoro-*N,N*-dimethyl-3-oxobutanamid)-eisen(III)-komplex**

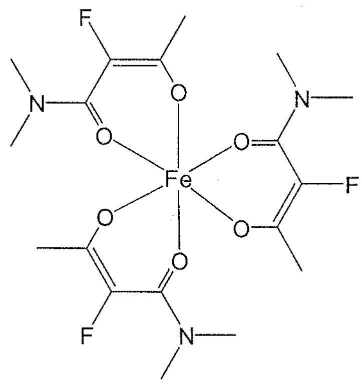

Es wurden 15 mmol (2,32 g) 2-Fluoro-*N,N*-dimethyl-3-oxobutanamid in 125 ml Ethanol (wasserfrei) gelöst und 4,86 ml Natriumethylat-Lösung (21% m/m, ca. 15 mmol Natriumethylat) zugegeben. Dann wurden 5 mmol (0,811 g) $FeCl_3$ (wasserfrei) zugegeben und 2 h bei 50°C gerührt. Das Reaktionsgemisch wurde nach Abkühlen filtriert, das Filtrat am Rotationsverdampfer eingeengt und das Produkt im Vakuumtrockenschrank bei 50°C getrocknet. Man erhielt 2,5 g der Titelverbindung.
IR (in Substanz, $cm^{-1}$): 2937, 2361, 2341, 1737, 1656, 1573, 1477, 1419, 1402, 1361, 1332, 1259, 1209, 1145, 1051, 1020, 977, 894, 870, 736. CHN-Elementaranalyse: C, 38.59; H, 5.23; N, 7.15.
Fe-Gehalt: 9.96% [m/m]

Beispiel 23

**Tris-(2-Fluoro-3-oxo-*N*-propylbutanamid)-eisen(III)-komplex**

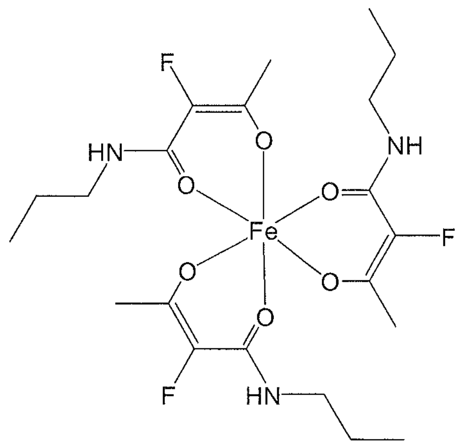

18 mmol (2,9 g) 2-Fluoro-3-oxo-*N*-propylbutanamid und 6 mmol (0,972 g) $FeCl_3$ (wasserfrei) wurden in 90 ml Ethanol (wasserfrei) gelöst und 2,78 ml Natriummethylat-Lösung (30% m/m, ca. 15 mmol Natriummethylat) zugegeben. Es wurde 1 h nachgerührt und die Reaktionslösung filtriert. Das Filtrat wurde am Rotationsverdampfer eingeengt und das Produkt im Vakuumtrockenschrank bei 50°C getrocknet. Man erhielt 3,14 g der Titelverbindung.
IR (in Substanz, $cm^{-1}$): 3306, 3090, 2966, 2936, 2876, 1736, 1674, 1602, 1549, 1522, 1459, 1438, 1382, 1275, 1246, 1160, 1114, 1088, 1050, 992, 958, 899, 821,774,744,645.
CHN-Elementaranalyse: C, 44.36; H, 6.08; N, 7.22.
Fe-Gehalt: 9.34% [m/m]

**Beispiel 24**

**Tris-(4-Methoxy-*N,N*-dimethyl-3-oxobutanamid)-eisen(III)-komplex**

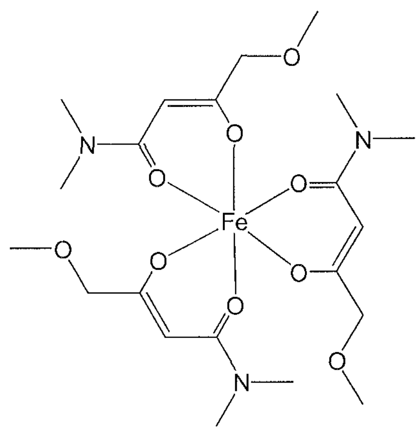

57 mmol (9,1 g) 4-Methoxy-*N,N*-dimethyl-3-oxobutanamid und 19 mmol (3,08 g) $FeCl_3$ (wasserfrei) wurden in 260 ml Ethanol (wasserfrei) gelöst und 9,84 ml Natriummethylat-Lösung (30% m/m, ca. 53 mmol Natriummethylat) zugegeben. Es wurde 1 h nachgerührt und die Reaktionslösung filtriert. Das Filtrat wurde am Rotationsverdampfer eingeengt und das Produkt im Vakuumtrockenschrank bei 50°C getrocknet. Man erhielt 10,5 g der Titelverbindung.
IR (in Substanz, $cm^{-1}$): 2930, 2822, 1730, 1603, 1568, 1497, 1424, 1402, 1368, 1331, 1260, 1200, 1174, 1109, 1059, 1023, 992, 959, 924, 862, 769, 736, 681. CHN-Elementaranalyse: C, 44.94; H, 6.66; N, 7.06.
Fe-Gehalt: 9.49% [m/m]

**Beispiel 25**

**Tris-(*N*,*N*,4-Trimethyl-3-oxopentanamid)-eisen(III)-komplex**

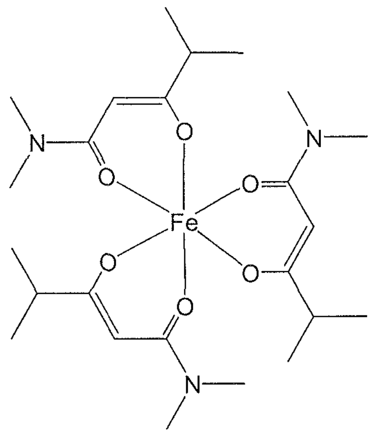

16 mmol (2,36 g) *N*,*N*,4-Trimethyl-3-oxopentanamid und 5 mmol (0,811 g) $FeCl_3$ (wasserfrei) wurden in 100 ml Ethanol (wasserfrei) gelöst und 2,78 ml Natriummethylat-Lösung (30% m/m, ca. 15 mmol Natriummethylat) zugegeben. Es wurde 1 h nachgerührt und die Reaktionslösung filtriert. Das Filtrat wurde am Rotationsverdampfer eingeengt und das Produkt im Vakuumtrockenschrank bei 50°C getrocknet. Man erhielt 2,6 g der Titelverbindung.
IR (in Substanz, $cm^{-1}$): 2958, 2927, 2868, 1720, 1593, 1556, 1526, 1502, 1487, 1402, 1378, 1359, 1311, 1260, 1198, 1176, 1157, 1083, 1006, 946, 891, 801, 773, 722, 678, 655.
CHN-Elementaranalyse: C, 52.88; H, 7.91; N, 7.50.
Fe-Gehalt: 10.40% [m/m]

**Beispiel 26**

**Tris-(4-Methyl-1-(morpholin-4-yl)-pentan-1,3-dion)-eisen(III)-komplex**

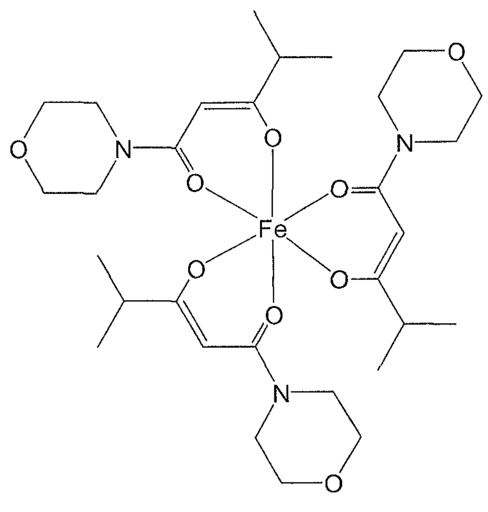

18 mmol (3,59 g) 4-Methyl-1-(morpholin-4-yl)-pentan-1,3-dion und 6 mmol (0,973 g) $FeCl_3$ (wasserfrei) wurden in 50 ml Ethanol (wasserfrei) gelöst und 3,00 ml Natriummethylat-Lösung (30% m/m, ca. 16 mmol Natriummethylat) zugegeben. Es wurde 1 h nachgerührt und die Reaktionslösung filtriert. Das Filtrat wurde am Rotationsverdampfer eingeengt und das Produkt im Vakuumtrockenschrank bei 50°C getrocknet. Man erhielt 4,0 g der Titelverbindung.
IR (in Substanz, $cm^{-1}$): 2964, 2924, 2857, 1714, 1678, 1640, 1549, 1516, 1478, 1459, 1439, 1384, 1371, 1357, 1313,

1300, 1273, 1244, 1189, 1159, 1114, 1087, 1065, 1018, 990, 947, 885, 851, 773, 717, 680.
CHN-Elementaranalyse: C, 48.70; H, 6.84; N, 6.01.
Fe-Gehalt: 7.89% [m/m]

**Beispiel 27**

**Tris-(4-Methoxy-1-(morpholin-4-yl)butan-1,3-dion)-eisen(III)-komplex**

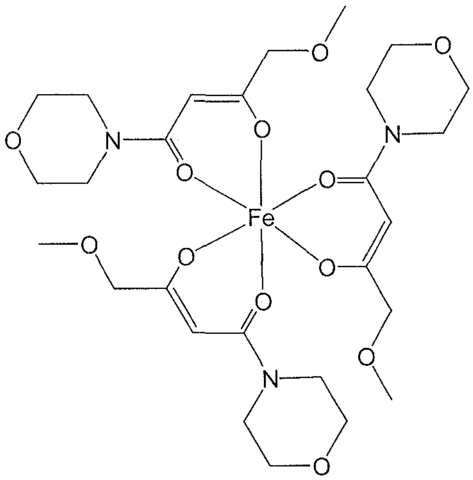

15 mmol (3,02 g) 4-Methoxy-1-(morpholin-4-yl)butan-1,3-dion und 5 mmol (0,811 g) $FeCl_3$ (wasserfrei) wurden in 65 ml Ethanol (wasserfrei) gelöst und 2,59 ml Natriummethylat-Lösung (30% m/m, ca. 14 mmol Natriummethylat) zugegeben. Es wurde 1 h nachgerührt und die Reaktionslösung filtriert. Das Filtrat wurde am Rotationsverdampfer eingeengt und das Produkt im Vakuumtrockenschrank bei 50°C getrocknet. Man erhielt 3,66 g der Titelverbindung.
IR (in Substanz, $cm^{-1}$): 2969, 2897, 2856, 1732, 1640, 1597, 1563, 1514, 1441, 1383, 1301, 1273, 1246, 1197, 1110, 1065, 1017, 992, 961, 922, 863, 765, 730, 676.
CHN-Elementaranalyse: C, 47.62; H, 6.90; N, 5.44.
Fe-Gehalt: 7.13% [m/m]

**Beispiel 28**

**Tris-(3-acetyl-1-methylpyrrolidin-2-on)-eisen(III)-Komplex**

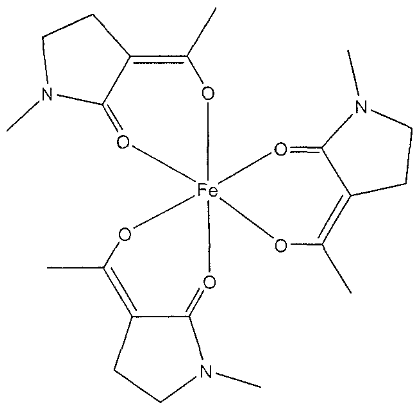

2,48 g (15,30 mmol) Eisen(III)chlorid wurden in 700ml Ethanol gelöst und 6,91 g (49,00 mmol) 3-Acetyl-1-methylpyrrolidin-2-on zugegeben. 9,76 g 25%ige Natriummethanolat-Lösung (45,17 mmol) wurden mit 80ml Ethanol

verdünnt und zur Eisen(III)chlorid-Lösung getropft. Die Reaktionslösung wurde noch für 30 min gerührt und anschliessend abfiltriert. Das Filtrat wurde am Rotationsverdampfer bis zur Trockene eingeengt und der Rückstand über Nacht bei 50°C im Feinvakuum getrocknet. Man erhielt 7,4 g Produkt als violettes Pulver.
IR (in Substanz, $cm^{-1}$): 2915, 2866, 1677, 1601, 1553, 1497, 1476, 1443, 1401, 1365, 1344, 1267, 1182, 996, 964, 908, 746, 612.
Fe-Gehalt: 11.74 % [m/m].

**Beispiel 29**

**Tris-(Ethyl(1-methyl-2-oxopyrrolidin-3-yl)(oxo)acetat)-eisen(III)-Komplex**

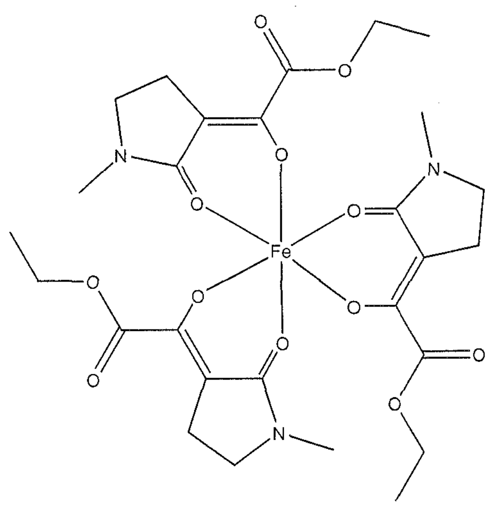

9,72 g einer Eisenethoxid-Lösung (2,22% Fe [m/m], 3,86 mmol) in Ethanol wurden unter einer Stickstoffatmosphäre mit 40 ml trockenem Ethanol verdünnt. Man gab 2,30 g (11,55 mmol) Ethyl (1-methyl-2-oxopyrrolidin-3-yl)(oxo)acetat zu und rührte die Lösung bei Raumtemperatur über Nacht. Man engte anschliessend die Lösung am Rotationsverdampfer zur Trockene ein und trocknete den Rückstand bei 50°C über Nacht im Feinvakuum. Man erhielt 2,6 g Produkt in Form eines roten Feststoffs.
IR (in Substanz, $cm^{-1}$): 2933, 2896, 1719, 1704, 1607, 1500, 1477, 1455, 1407, 1395, 1367, 1350, 1303, 1270, 1207, 1170, 1107, 1031, 1008, 914, 869, 780, 743, 718, 633.
Elementaranalyse: C 47.90% H 5.43% N 6.11%
Fe-Gehalt: 8.48 % [m/m].

**Beispiel 30**

**Tris-(ethyl(1-methyl-2-oxopiperidin-3-yl)(oxo)acetat)-eisen(III)-Komplex**

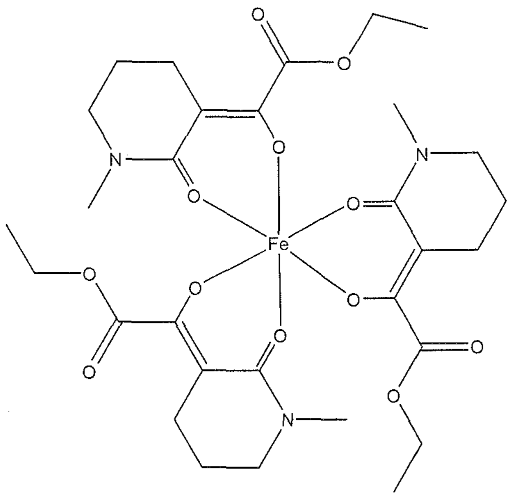

0,65 g (4,00 mmol) Eisen(III)chlorid wurden in 25 ml THF gelöst und 2,56 g (12,00 mmol) Ethyl(1-methyl-2-oxopiperidin-3-yl)(oxo)acetat zugegeben. Nach 40min Rührzeit gab man 1,21 g (12,00 mmol) Triethylamin zu und liess für weitere 60min bei Raumtemperatur rühren. Es wurden anschliessend 2,59 g 25%ige Natriummethanolat-Lösung (12,00 mmol) zugetropft und die Lösung für 2 Stunden weiter gerührt. Das ausgefallene Salz wurde abfiltriert und das Filtrat am Rotationsverdampfer bis zur Trockene eingeengt. Der Rückstand wurde 1 Tag bei 50°C im Feinvakuum getrocknet. Man erhielt 2,6 g Produkt als roten Feststoff.
IR (in Substanz, $cm^{-1}$): 2939, 2863, 1724, 1610, 1573, 1538, 1480, 1402, 1364, 1308, 1256, 1219, 1195, 1101, 1079, 1060, 1016, 959, 920, 892, 858, 811, 764, 724, 692.
Elementaranalyse: C 51.62%, H 6.23%, N 5.90%
Fe-Gehalt: 8.2% [m/m].

**Beispiel 31**

**Tris-(3-acetyl-1-methylpiperidin-2-on)-eisen(III)-Komplex**

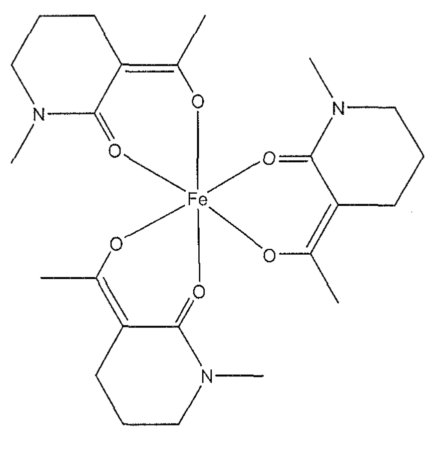

0,34 g (2,10 mmol) Eisen(III)chlorid wurden in 100ml Ethanol gelöst und 1,34 g (6,30 mmol) 3-Acetyl-1-methylpiperidin-2-on zugegeben. 1,33 g 25%ige Natriummethanolat-Lösung (6,13 mmol) wurden mit 10ml Ethanol verdünnt und zur Reaktionslösung getropft. Die Reaktionslösung wurde noch für 30 min gerührt und anschliessend abfiltriert. Das Filtrat wurde am Rotationsverdampfer bis zur Trockene eingeengt und der Rückstand in 50 ml Dichlormethan aufgenommen. Die Lösung wurde nochmals filtriert, das Dichlormethan am Rotationsverdampfer entfernt und der Rückstand 2 Tage bei 50°C im Feinvakuum getrocknet. Man erhielt 1,2 g Produkt als dunkelroten Feststoff.
IR (in Substanz, $cm^{-1}$): 2927, 2855, 1559, 1462, 1399, 1303, 1254, 1202, 1181, 1082,992,921,887,856,757,707,626.
Elementaranalyse: C 49.63%, H 6.43%, N 7.23%
Fe-Gehalt: 9.80% [m/m].

**Beispiel 32**

**Tris-(ethyl 4-(dimethylamino)-2,4-dioxobutanoat)-eisen(III)-Komplex**

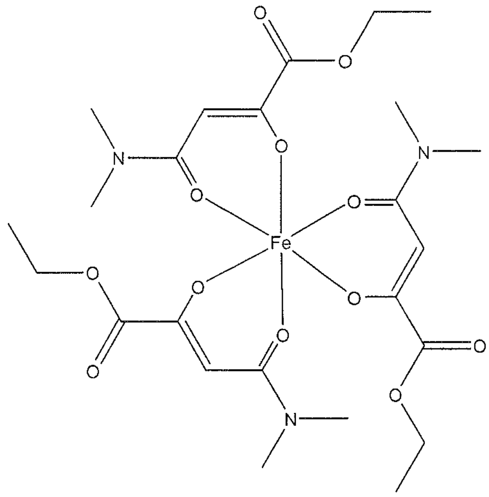

0,58 g (3,56 mmol) Eisen(III)chlorid wurden in 80 ml THF gelöst und 2,00 g (10,69 mmol) Ethyl-4-(dimethylamino)-2,4-dioxobutanoat zugegeben. Nach 40min Rührzeit gab man 1,07 g (10,69 mmol) Triethylamin zu und liess für weitere 60 min bei Raumtemperatur rühren. Es wurden anschliessend 2,31 g 25%ige Natriummethanolat-Lösung (10,69 mmol) zugetropft und die Lösung für 2 Stunden weiter gerührt. Das ausgefallene Salz wurde abfiltriert und das Filtrat am Rotationsverdampfer bis zur Trockene eingeengt. Der Rückstand wurde 1 Tag bei 50°C im Feinvakuum getrocknet. Man erhielt 2,0 g Produkt als roten Feststoff. IR (in Substanz, $cm^{-1}$): 2927, 1719, 1616, 1575, 1501, 1433, 1404, 1362, 1235, 1174, 1136, 1019, 944, 924, 767, 742, 652.
Elementaranalyse: C 45.83%, H 5.82%, N 6.83%
Fe-Gehalt: 8.54% [m/m].

**Beispiel 33**

**Tris-(1-(morpholin-4-yl)butan-1,3-dion)-eisen(III)-Komplex**

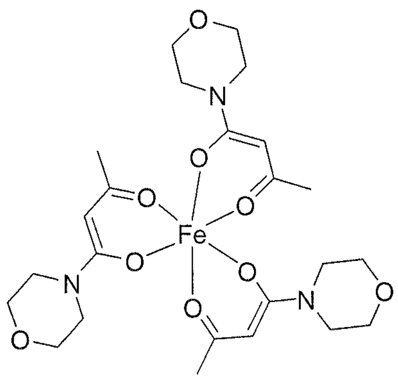

Zu einer Lösung von 3,17 g (18,50 mmol) 1-(Morpholin-4-yl)butan-1,3-dion in 40 ml Ethanol wurde eine Lösung von 1,00 g (6,17 mmol) Eisen(III)chlorid wasserfrei in 10 ml Ethanol tropfenweise zugegeben. Dann wurden 6,22 g (74,00 mmol) Natriumbicarbonat portionsweise zugegeben. Nach 1.5 h Rühren bei Raumtemperatur wurde das Reaktionsgemisch abfiltriert, mit 10 ml Ethanol gewaschen, das Filtrat abrotiert und der Rückstand über Nacht im Trockenschrank bei 50°C getrocknet. Man erhielt 3,50 g braunen Feststoff.
IR (in Substanz, $cm^{-1}$): 2961, 2896, 2854, 1595, 1553, 1509, 1476, 1443, 1362, 1299, 1273, 1244, 1193, 1110.

Fe-Gehalt: 9.62 % [m/m].

**Beispiel 34**

**Tris-(N-(2-methoxyethyl)-3-oxobutanamid)-eisen(III)-Komplex**

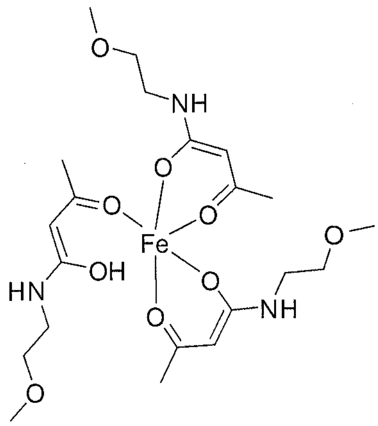

Zu einer Lösung von 1,47 g (9,25 mmol) N-(2-Methoxyethyl)-3-oxobutanamid in 15 ml Ethanol wurde eine Lösung von 0,5 g (3,08 mmol) Eisen(III)chlorid wasserfrei in 5 ml Ethanol tropfenweise zugegeben. Dann wurden 1,56 g (18,50 mmol) Natriumbicarbonat portionsweise zugegeben. Nach 1 h Rühren bei Raumtemperatur wurde das Reaktionsgemisch abfiltriert, mit 10 ml Ethanol gewaschen, das Filtrat abrotiert und der Rückstand über Nacht im Trockenschrank bei 50°C getrocknet. Man erhielt 1,51 g braunes Öl.
IR (in Substanz, $cm^{-1}$): 3269, 2980, 2928, 2882, 2830, 1650, 1550, 1499, 1451, 1410, 1275, 1192, 1117, 1092, 1017, 958.
Fe-Gehalt: 9.68 % [m/m].

**Beispiel 35**

**Tris-(N-Cyclopropyl-3-oxobutanamid)-eisen(III)-Komplex**

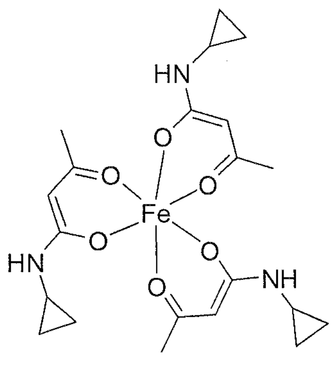

Zu einer Lösung von 2,61 g (18,50 mmol) N-Cyclopropyl-3-oxobutanamid in 30 ml Ethanol wurde eine Lösung von 1,00 g (6,17 mmol) Eisen(III)chlorid wasserfrei in 10 ml Ethanol tropfenweise zugegeben. Dann wurden 3,11 g (37,00 mmol) Natriumbicarbonat portionsweise zugegeben. Nach 1 h Rühren bei Raumtemperatur wurde das Reaktionsgemisch abfiltriert, mit 10 ml Ethanol gewaschen, das Filtrat abrotiert und der Rückstand über Nacht im Trockenschrank bei 50°C getrocknet. Man erhielt 2,57 g braunen Feststoff.
IR (in Substanz, $cm^{-1}$): 3245, 3088, 3008, 1549, 1493, 1453, 1405, 1334, 1275, 1219, 1189, 1060, 1023, 981, 937.
Fe-Gehalt: 11.00 % [m/m].

**Beispiel 36**

**Tris-(3-oxo-N-(propan-2-yl)butanamid)-eisen(III)-Komplex**

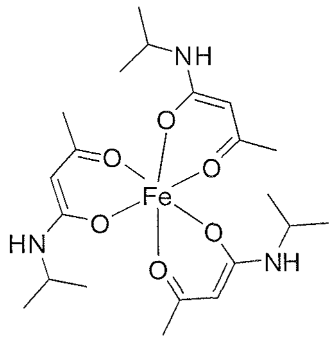

Zu einer Lösung von 1,32 g (9,25 mmol) 3-Oxo-N-(propan-2-yl)butanamid in 15 ml Ethanol wurde eine Lösung von 0,5 g (3,08 mmol) Eisen(III)chlorid wasserfrei in 5 ml Ethanol tropfenweise zugegeben. Dann wurden 1,56 g (18,50 mmol) Natriumbicarbonat portionsweise zugegeben. Nach 2 h Rühren bei Raumtemperatur wurde das Reaktionsgemisch abfiltriert, mit 10 ml Ethanol gewaschen, das Filtrat abrotiert und der Rückstand über Nacht im Trockenschrank bei 50°C getrocknet. Man erhielt 1,30 g braunen Feststoff.
IR (in Substanz, $cm^{-1}$): 3248, 3101, 2972, 2936, 2873, 1544, 1492, 1468, 1447, 1365, 1321, 1265, 1187, 1171, 1129, 998, 968.
Fe-Gehalt: 11.22 % [m/m].

**Beispiel 37**

**Tris-(N-(2-hydroxyethyl)-3-oxobutanamid)-eisen(III)-Komplex**

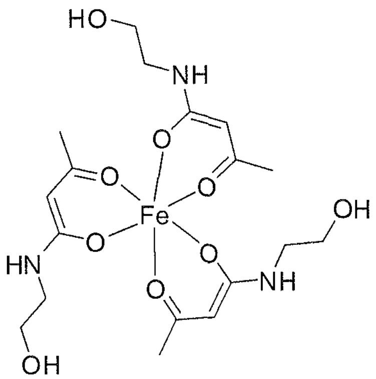

Zu einer Lösung von 2,69 g (18,50 mmol) N-(2-Hydroxyethyl)-3-oxobutanamid in 40 ml Ethanol wurde eine Lösung von 1,00 g (6,17 mmol) Eisen(III)chlorid wasserfrei in 10 ml Ethanol tropfenweise zugegeben. Dann wurden 6,22 g (74,00 mmol) Natriumbicarbonat portionsweise zugegeben. Nach 2 h Rühren bei Raumtemperatur wurde das Reaktionsgemisch abfiltriert, mit 10 ml Ethanol gewaschen, das Filtrat abrotiert und der Rückstand über Nacht im Trockenschrank bei 50°C getrocknet. Man erhielt 2,72 g braunen Feststoff.
IR (in Substanz, $cm^{-1}$): 3259, 3110, 2946, 2875, 1552, 1498, 1451, 1405, 1360, 1273, 1188, 1059, 1018. 955, 778.
Fe-Gehalt: 11.30 % [m/m].

**Beispiel 38**

**Tris-(N-(3-hydroxypropyl)-3-oxobutanamid)-eisen(III)-Komplex**

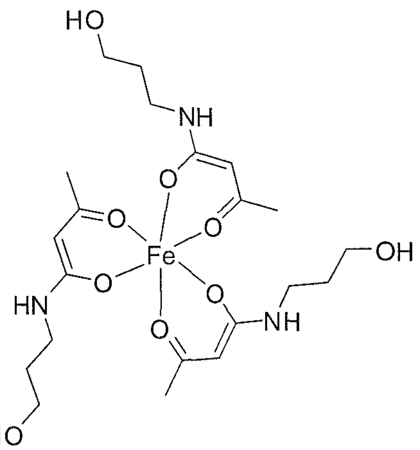

Zu einer Lösung von 1,47 g (9,25 mmol) N-(3-Hydroxypropyl)-3-oxobutanamid in 20 ml Ethanol wurde eine Lösung von 0,5 g (3,08 mmol) Eisen(III)chlorid wasserfrei in 5 ml Ethanol tropfenweise zugegeben. Dann wurden 1,56 g (18,50 mmol) Natriumbicarbonat portionsweise zugegeben. Nach 2 h Rühren bei Raumtemperatur wurde das Reaktionsgemisch abfiltriert, mit 10 ml Ethanol gewaschen, das Filtrat abrotiert und der Rückstand über Nacht im Trockenschrank bei 50°C getrocknet. Man erhielt 1,80 g braunen Feststoff.
IR (in Substanz, $cm^{-1}$): 3270, 2939, 1551, 1500, 1407, 1270, 1188, 1008, 973, 947, 777.
Fe-Gehalt: 10.05 % [m/m].

**Beispiel 39**

**Tris-(N-(4-hydroxybutyl)-3-oxobutanamid)-eisen(III)-Komplex**

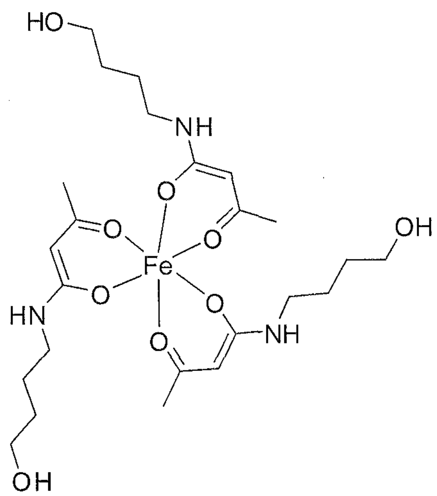

Zu einer Lösung von 2,94 g (18,50 mmol) N-(4-Hydroxybutyl)-3-oxobutanamid in 40 ml Ethanol wurde eine Lösung von 1,00 g (6,17 mmol) Eisen(III)chlorid wasserfrei in 10 ml Ethanol tropfenweise zugegeben. Dann wurden 3,11 g (37,00 mmol) Natriumbicarbonat portionsweise zugegeben. Nach 2 h Rühren bei Raumtemperatur wurde das Reaktionsgemisch abfiltriert, mit 10 ml Ethanol gewaschen, das Filtrat abrotiert und der Rückstand über Nacht im Trockenschrank bei 50°C getrocknet. Man erhielt 2,72 g braunen Feststoff.

IR (in Substanz, $cm^{-1}$): 3273, 2934, 2867, 1649, 1555, 1502, 1436, 1409, 1272, 1188, 1053, 1027, 946, 778.
Fe-Gehalt: 9.36 % [m/m].

**Beispiel 40**

**Tris-(N-(5-hydroxypentyl)-3-oxobutanamid)-eisen(III)-Komplex**

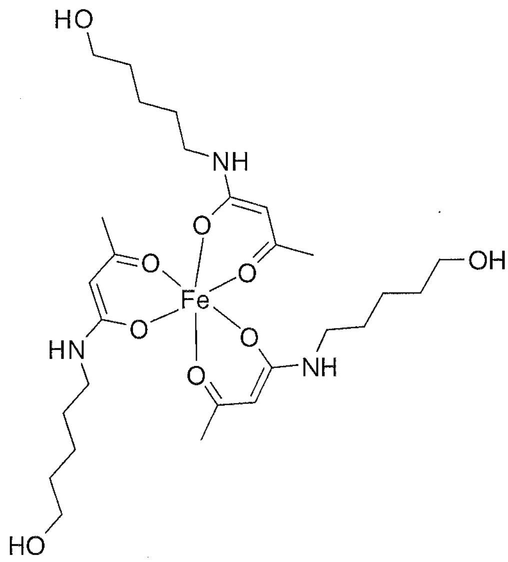

Zu einer Lösung von 3,46 g (18,50 mmol) N-(5-Hydroxypentyl)-3-oxobutanamid in 40 ml Ethanol wurde eine Lösung von 1,00 g (6,17 mmol) Eisen(III)chlorid wasserfrei in 10 ml Ethanol tropfenweise zugegeben. Dann wurden 3,11 g (37,00 mmol) Natriumbicarbonat portionsweise zugegeben. Nach 1 h Rühren bei Raumtemperatur wurde das Reaktionsgemisch abfiltriert, mit 10 ml Ethanol gewaschen, das Filtrat abrotiert und der Rückstand über Nacht im Trockenschrank bei 50°C getrocknet. Man erhielt 3,51 g braunen Feststoff.
IR (in Substanz, $cm^{-1}$): 3255, 3109, 2932, 2862, 1558, 1500, 1433, 1407, 1274, 1187, 1021, 949, 779.
Fe-Gehalt: 8.85 % [m/m].

**Beispiel 41**

**Tris-(N-(1-hydroxy-2-methylpropan-2-yl)-3-oxobutanamid)-eisen(III)-Komplex**

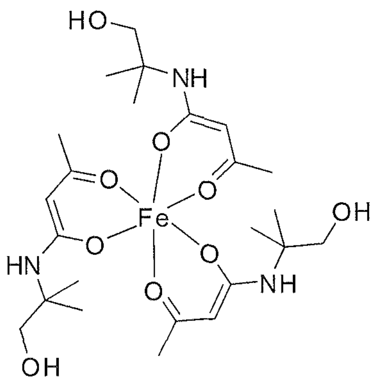

Zu einer Lösung von 3,20 g (18,50 mmol) N-(1-Hydroxy-2-methylpropan-2-yl)-3-oxobutanamid in 40 ml Ethanol wurde eine Lösung von 1,00 g (6,17 mmol) Eisen(III)chlorid wasserfrei in 10 ml Ethanol tropfenweise zugegeben. Dann

wurden 3,11 g (37,00 mmol) Natriumbicarbonat portionsweise zugegeben. Nach 1 h Rühren bei Raumtemperatur wurde das Reaktionsgemisch abfiltriert, mit 10 ml Ethanol gewaschen, das Filtrat abrotiert und der Rückstand über Nacht im Trockenschrank bei 50°C getrocknet. Man erhielt 3,42 g braunen Feststoff.
IR (in Substanz, $cm^{-1}$): 3286, 2972, 2927, 1593, 1550, 1498, 1446, 1411, 1363, 1287, 1225, 1189, 1050, 962.
Fe-Gehalt: 9.17 % [m/m].

**Beispiel 42**

**Tris-(N-(2-hydroxyethyl)-N-methyl-3-oxobutanamid)-eisen(III)-Komplex**

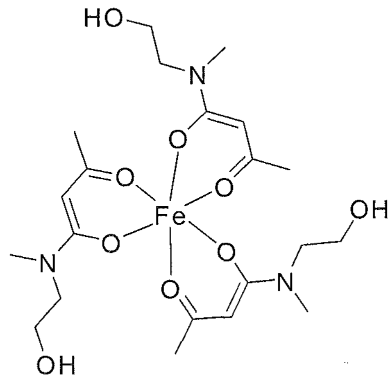

Zu einer Lösung von 2,94 g (18,50 mmol) N-(2-Hydroxyethyl)-N-methyl-3-oxobutanamid in 30 ml Ethanol wurde eine Lösung von 1,00 g (6,17 mmol) Eisen(III)chlorid wasserfrei in 10 ml Ethanol tropfenweise zugegeben. Dann wurden 3,11 g (37,00 mmol) Natriumbicarbonat portionsweise zugegeben. Nach 1 h Rühren bei Raumtemperatur wurde das Reaktionsgemisch abfiltriert, mit 10 ml Ethanol gewaschen, das Filtrat abrotiert und der Rückstand über Nacht im Trockenschrank bei 50°C getrocknet. Man erhielt 3,11 g braunen Feststoff.
IR (in Substanz, $cm^{-1}$): 3371, 2975, 2930, 2884, 1638, 1561, 1517, 1494, 1436, 1351, 1301, 1205, 1170, 1051, 958.
Fe-Gehalt: 9.61 % [m/m].

**Beispiel 43**

**Tris-(N-(2-hydroxypropyl)-3-oxobutanamid)-eisen(III)-Komplex**

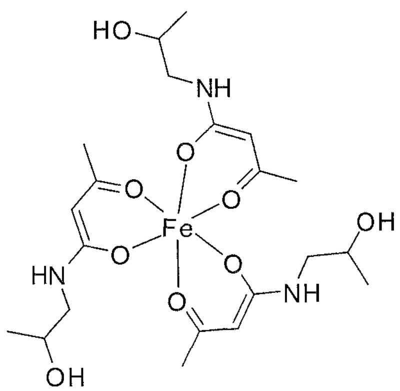

Zu einer Lösung von 2,94 g (18,50 mmol) N-(2-Hydroxypropyl)-3-oxobutanamid in 30 ml Ethanol wurde eine Lösung von 1,00 g (6,17 mmol) Eisen(III)chlorid wasserfrei in 10 ml Ethanol tropfenweise zugegeben. Dann wurden 6,22 g (74,00 mmol) Natriumbicarbonat portionsweise zugegeben. Nach 2 h Rühren bei Raumtemperatur wurde das Reaktionsgemisch abfiltriert, mit 10 ml Ethanol gewaschen, das Filtrat abrotiert und der Rückstand über Nacht im

Trockenschrank bei 50°C getrocknet. Man erhielt 3,11 g braunen Feststoff.
IR (in Substanz, $cm^{-1}$): 3281, 2973, 2925, 1552, 1501, 1454, 1409, 1377, 1272, 1190, 1079, 1053, 951, 777.
Fe-Gehalt: 9.84 % [m/m].

**Beispiel 44**

**Tris-(N-(1-hydroxypropan-2-yl)-3-oxobutanamid)-eisen(III)-Komplex**

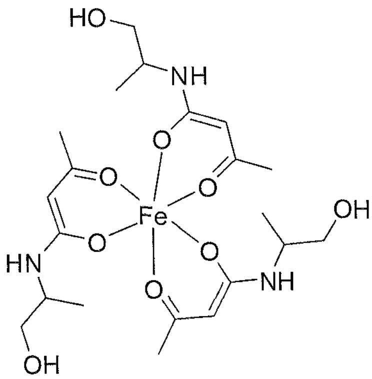

Zu einer Lösung von 2,94 g (18,50 mmol) N-(2-Hydroxypropyl)-3-oxobutanamid in 30 ml Ethanol wurde eine Lösung von 1,00 g (6,17 mmol) Eisen(III)chlorid wasserfrei in 10 ml Ethanol tropfenweise zugegeben. Dann wurden 6,22 g (74,00 mmol) Natriumbicarbonat portionsweise zugegeben. Nach 2 h Rühren bei Raumtemperatur wurde das Reaktionsgemisch abfiltriert, mit 10 ml Ethanol gewaschen, das Filtrat abrotiert und der Rückstand über Nacht im Trockenschrank bei 50°C getrocknet. Man erhielt 3,22 g braunen Feststoff.
IR (in Substanz, $cm^{-1}$): 3259, 2973, 1551, 1494, 1451, 1409, 1270, 1189, 1158, 1091, 1041, 962.
Fe-Gehalt: 9.57 % [m/m].

**Beispiel 45**

**Tris-(N-(1-hydroxybutan-2-yl)-3-oxobutanamid)-eisen(III)-Komplex**

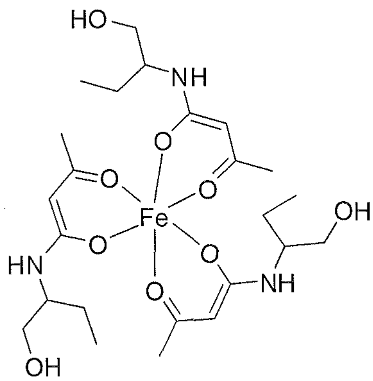

Zu einer Lösung von 3,21 g (18,50 mmol) N-(1-Hydroxybutan-2-yl)-3-oxobutanamid in 30 ml Ethanol wurde eine Lösung von 1,00 g (6,17 mmol) Eisen(III)chlorid wasserfrei in 10 ml Ethanol tropfenweise zugegeben. Dann wurden 6,22 g (74,00 mmol) Natriumbicarbonat portionsweise zugegeben. Nach 2 h Rühren bei Raumtemperatur wurde das Reaktionsgemisch abfiltriert, mit 10 ml Ethanol gewaschen, das Filtrat abrotiert und der Rückstand über Nacht im Trockenschrank bei 50°C getrocknet. Man erhielt 3,22 g braunen Feststoff.
IR (in Substanz, $cm^{-1}$): 3268, 2965, 2933, 2875, 1546, 1493, 1457, 1410, 1285, 1188, 1049, 1000, 959, 775.

Fe-Gehalt: 9.39 % [m/m].

**Beispiel 46**

**Tris-(N-(2,3-dihydroxypropyl)-3-oxobutanamid)-eisen(III)-Komplex**

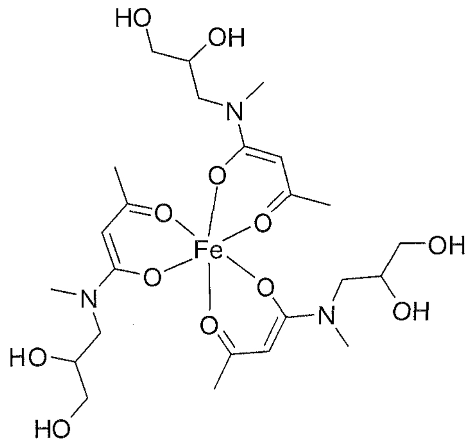

Zu einer Lösung von 1,75 g (9,25 mmol) N-(2,3-Dihydroxypropyl)-3-oxobutanamid in 20 ml Ethanol wurde eine Lösung von 0,5 g (3,08 mmol) Eisen(III)chlorid wasserfrei in 5 ml Ethanol tropfenweise zugegeben. Dann wurden 1,56 g (18,50 mmol) Natriumbicarbonat portionsweise zugegeben. Nach 1 h Rühren bei Raumtemperatur wurde das Reaktionsgemisch abfiltriert, mit 10 ml Ethanol gewaschen, das Filtrat abrotiert und der Rückstand über Nacht im Trockenschrank bei 50°C getrocknet. Man erhielt 1,80 g braunen Feststoff.
IR (in Substanz, $cm^{-1}$): 3349, 2929, 1635, 15559, 1519, 1493, 1353, 1210, 1157, 1099, 1044, 996, 956, 765.
Fe-Gehalt: 8.81 % [m/m].

**Beispiel 47**

**Tris-(1-(3-hydroxypiperidin-1-yl)butan-1,3-dion)-eisen(III)-Komplex**

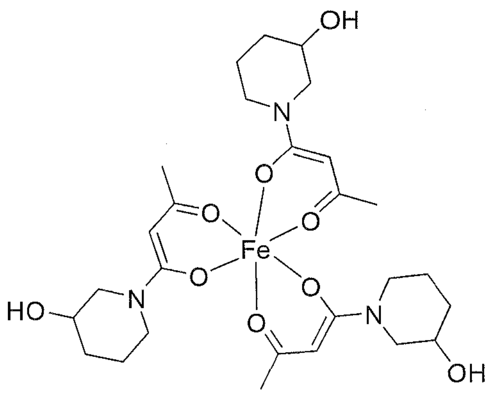

Zu einer Lösung von 3,43 g (18,50 mmol) 1-(3-Hydroxypiperidin-1-yl)butan-1,3-dion in 50 ml Ethanol wurde eine Lösung von 1,00 g (6,17 mmol) Eisen(III)chlorid wasserfrei in 10 ml Ethanol tropfenweise zugegeben. Dann wurden 1,56 g (18,50 mmol) Natriumbicarbonat portionsweise zugegeben. Nach 4 h Rühren bei Raumtemperatur wurde das Reaktionsgemisch abfiltriert, mit 10 ml Ethanol gewaschen, das Filtrat abrotiert und der Rückstand über Nacht im Trockenschrank bei 50°C getrocknet. Man erhielt 3,51 g braunen Feststoff.
IR (in Substanz, $cm^{-1}$): 3290, 2929, 2859, 1554, 1509, 1483, 1441, 1363, 1254, 1228, 1206, 1143, 1072, 997, 953, 858,

760.
Fe-Gehalt: 8.73 % [m/m].

**Beispiel 48**

**Tris-(1-[4-(hydroxymethyl)piperidin-1-yl]butan-1,3-dion)-eisen(III)-Komplex**

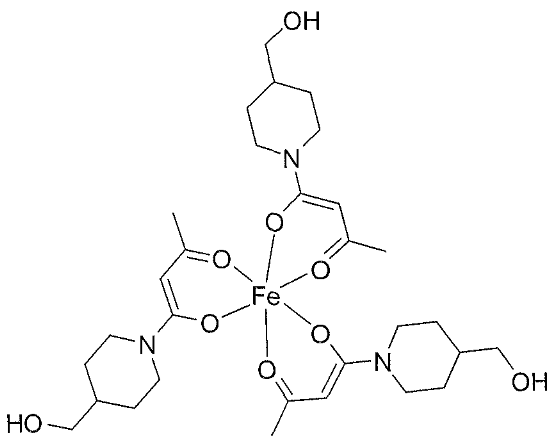

Zu einer Lösung von 3,68 g (18,50 mmol) 1-[4-(Hydroxymethyl)piperidin-1-yl]butan-1,3-dion in 50 ml Ethanol wurde eine Lösung von 1,00 g (6,17 mmol) Eisen(III)chlorid wasserfrei in 10 ml Ethanol tropfenweise zugegeben. Dann wurden 3,11 g (37,00 mmol) Natriumbicarbonat portionsweise zugegeben. Nach 2 h Rühren bei Raumtemperatur wurde das Reaktionsgemisch abfiltriert, mit 10 ml Ethanol gewaschen, das Filtrat abrotiert und der Rückstand über Nacht im Trockenschrank bei 50°C getrocknet. Man erhielt 3,81 g braunen Feststoff.
IR (in Substanz, $cm^{-1}$): 3334, 2916, 2858, 1555, 1511, 1485, 1445, 1366, 1268, 1247, 1217, 1088, 1033, 979, 953, 760.
Fe-Gehalt: 8.39 % [m/m].

**Beispiel 49**

**Tris-(1-[3-(hydroxymethyl)piperidin-1-yl]butan-1,3-dion)-eisen(III)-Komplex**

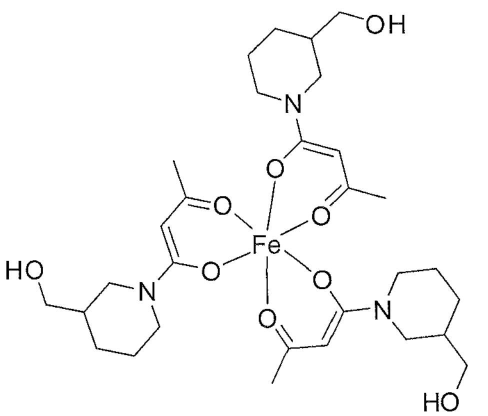

Zu einer Lösung von 3,68 g (18,50 mmol) 1-[3-(Hydroxymethyl)piperidin-1-yl]butan-1,3-dion in 50 ml Ethanol wurde eine Lösung von 1,00 g (6,17 mmol) Eisen(III)chlorid wasserfrei in 10 ml Ethanol tropfenweise zugegeben. Dann wurden 3,11 g (37,00 mmol) Natriumbicarbonat portionsweise zugegeben. Nach 1 h Rühren bei Raumtemperatur wurde das Reaktionsgemisch abfiltriert, mit 10 ml Ethanol gewaschen, das Filtrat abrotiert und der Rückstand über Nacht im Trockenschrank bei 50°C getrocknet. Man erhielt 3,92 g braunen Feststoff.

IR (in Substanz, $cm^{-1}$): 3350, 2922, 2858, 1555, 1510, 1485, 1440, 1366, 1259, 1088, 1039, 995, 953, 760.
Fe-Gehalt: 8.27 % [m/m].

**Beispiel 50**

**Tris-(1-[2-(hydroxymethyl)piperidin-1-yl]butan-1,3-dion)-eisen(III)-Komplex**

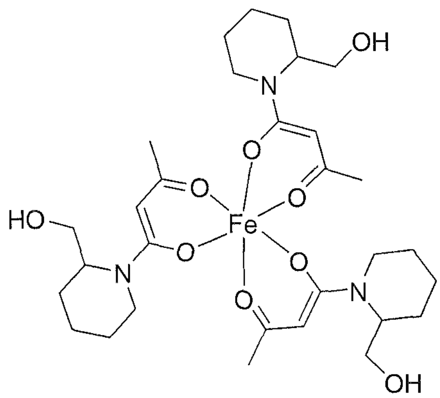

Zu einer Lösung von 3,68 g (18,50 mmol) 1-[2-(Hydroxymethyl)piperidin-1-yl]butan-1,3-dion in 50 ml Ethanol wurde eine Lösung von 1,00 g (6,17 mmol) Eisen(III)chlorid wasserfrei in 10 ml Ethanol tropfenweise zugegeben. Dann wurden 3,11 g (37,00 mmol) Natriumbicarbonat portionsweise zugegeben. Nach 2 h Rühren bei Raumtemperatur wurde das Reaktionsgemisch abfiltriert, mit 10 ml Ethanol gewaschen, das Filtrat abrotiert und der Rückstand über Nacht im Trockenschrank bei 50°C getrocknet. Man erhielt 3,88 g braunen Feststoff.
IR (in Substanz, $cm^{-1}$): 3358, 2914, 2854, 1554, 1509, 1484, 1445, 1370, 1311, 1269, 1245, 1217, 1088, 1035, 978, 953, 759.
Fe-Gehalt: 8.37 % [m/m].

**Beispiel 51**

**Tris-(1-[(3S)-3-hydroxypyrrolidin-1-yl]butan-1,3-dion)-eisen(III)-Komplex**

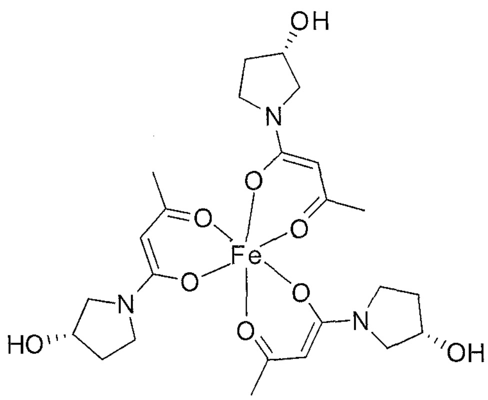

Zu einer Lösung von 3,17 g (18,50 mmol) 1-[(3S)-3-Hydroxypyrrolidin-1-yl]butan-1,3-dion in 50 ml Ethanol wurde eine Lösung von 1,00 g (6,17 mmol) Eisen(III)chlorid wasserfrei in 10 ml Ethanol tropfenweise zugegeben. Dann wurden 3,11 g (37,00 mmol) Natriumbicarbonat portionsweise zugegeben. Nach 2 h Rühren bei Raumtemperatur wurde das Reaktionsgemisch abfiltriert, mit 10 ml Ethanol gewaschen, das Filtrat abrotiert und der Rückstand über Nacht im Trockenschrank bei 50°C getrocknet. Man erhielt 3,23 g braunen Feststoff.
IR (in Substanz, $cm^{-1}$): 3315, 2946, 1558, 1514, 1472, 1350, 1206, 1103, 1054, 951, 875, 763.

Fe-Gehalt: 8.61 % [m/m].

**Beispiel 52**

**Tris-(1-[4-(2-hydroxyethyl)piperazin-1-yl]butan-1,3-dion)-eisen(III)-Komplex**

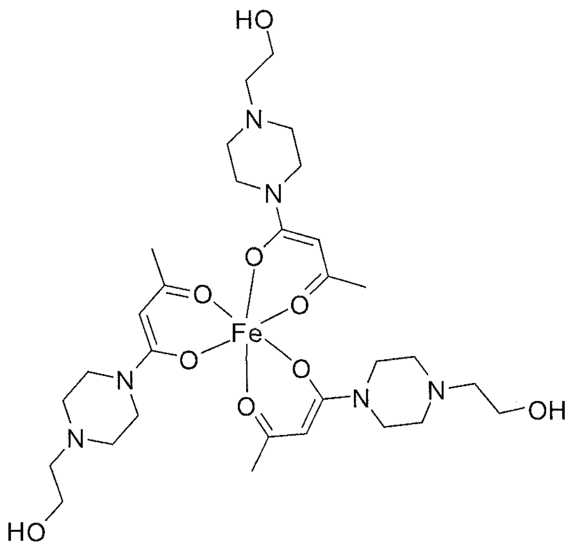

Zu einer Lösung von 3,96 g (18,50 mmol) 1-[4-(2-Hydroxyethyl)piperazin-1-yl]butan-1,3-dion in 80 ml MeOH wurde eine Lösung von 1,00 g (6,17 mmol) Eisen(III)chlorid wasserfrei in 20 ml MeOH tropfenweise zugegeben. Dann wurden 3,11 g (37,00 mmol) Natriumbicarbonat portionsweise zugegeben. Nach 2 h Rühren bei Raumtemperatur wurde das Reaktionsgemisch abrotiert, der Rückstand in 100 ml Dichlormethan aufgenommen, 15 min gerührt, abfiltriert, das Filtrat abrotiert, der Rückstand 50 ml EtOH aufgenommen, erneut abrotiert und der erhaltene Rückstand übernacht im Trockenschrank getrocknet. Man erhielt 4,10 g braunen Feststoff.
IR (in Substanz, $cm^{-1}$): 3365, 2914, 2809, 1591, 1554, 1509, 1480, 1443, 1369, 1290, 1249, 1138, 1050, 983, 960, 876, 759, 673.
Fe-Gehalt: 7.89 % [m/m].

**Beispiel 53**

**Tris-(1-(4-methylpiperazin-1-yl)butan-1,3-dion)-eisen(III)-Komplex**

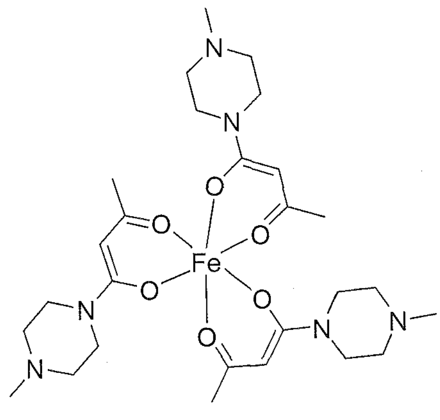

Zu einer Lösung von 3,41 g (18,50 mmol) 1-(4-Methylpiperazin-1-yl)butan-1,3-dion in 80 ml MeOH wurde eine

Lösung von 1,00 g (6,17 mmol) Eisen(III)chlorid wasserfrei in 20 ml MeOH tropfenweise zugegeben. Dann wurden 3,11 g (37,00 mmol) Natriumbicarbonat portionsweise zugegeben. Nach 2 h Rühren bei Raumtemperatur wurde das Reaktionsgemisch abrotiert, der Rückstand in 100 ml Dichlormethan aufgenommen, 15 min gerührt, abfiltriert, das Filtrat abrotiert, der Rückstand 50 ml EtOH aufgenommen, erneut abrotiert und der erhaltene Rückstand übernacht im Trockenschrank getrocknet. Man erhielt 3,52 g braunen Feststoff.
IR (in Substanz, $cm^{-1}$): 2934, 2845, 2789, 1554, 1507, 1478, 1445, 1370, 1291, 1257, 1235, 1142, 1092, 1072, 1000, 981, 959. 757.
Fe-Gehalt: 8.77 % [m/m].

**Beispiel 54**

**Tris-(N-(3-hydroxypropyl)-N-methyl-3-oxobutanamid)-eisen(III)-Komplex**

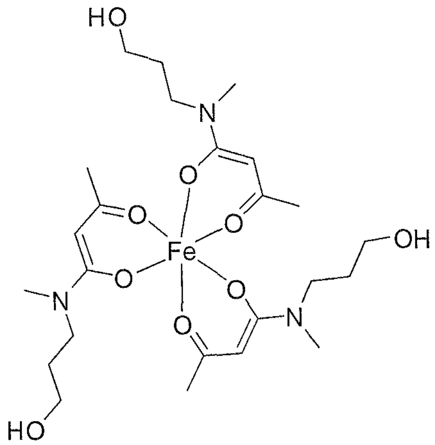

Zu einer Lösung von 3,20 g (18,50 mmol) N-(3-Hydroxypropyl)-N-methyl-3-oxobutanamid in 40 ml Ethanol wurde eine Lösung von 1,00 g (6,17 mmol) Eisen(III)chlorid wasserfrei in 10 ml Ethanol tropfenweise zugegeben. Dann wurden 3,11 g (37,00 mmol) Natriumbicarbonat portionsweise zugegeben. Nach 1 h Rühren bei Raumtemperatur wurde das Reaktionsgemisch abfiltriert, mit 10 ml Ethanol gewaschen, das Filtrat abrotiert und der Rückstand über Nacht im Trockenschrank bei 50°C getrocknet. Man erhielt 3,31 g braunes Öl.
IR (in Substanz, $cm^{-1}$): 3376, 2919, 1557, 1492, 1348, 1296, 1263, 1212, 1190, 1051, 989, 955, 763.
Fe-Gehalt: 9.08 % [m/m].

**Beispiel 55**

**Tris-(N-(trans-4-hydroxycyclohexyl)-3-oxobutanamid)-eisen(III)-Komplex**

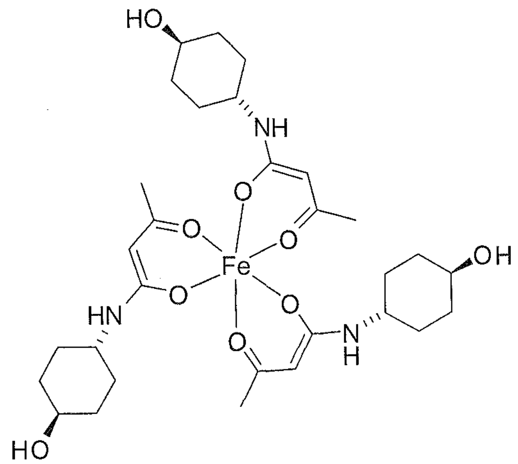

Zu einer Lösung von 3,68 g (18,50 mmol) N-(trans-4-Hydroxycyclohexyl)-3-oxobutanamid in 80 ml Ethanol wurde eine Lösung von 1,00 g (6,17 mmol) Eisen(III)chlorid wasserfrei in 20 ml Ethanol tropfenweise zugegeben. Dann wurden 6,22 g (74,00 mmol) Natriumbicarbonat portionsweise zugegeben. Nach 2 h Rühren bei Raumtemperatur wurde das Reaktionsgemisch abfiltriert, mit 10 ml Ethanol gewaschen, das Filtrat abrotiert und der Rückstand über Nacht im Trockenschrank bei 50°C getrocknet. Man erhielt 3,81 g braunen Feststoff.
IR (in Substanz, $cm^{-1}$): 3280, 2932, 2859, 1549, 1494, 1453, 1409, 1371, 1309, 1265, 1187, 1055, 1014, 961, 941, 776.
Fe-Gehalt: 7.84 % [m/m].

**Beispiel 56**

**Tris-(N-(3-hydroxy-2,2-dimethylpropyl)-3-oxobutanamid)-eisen(III)-Komplex**

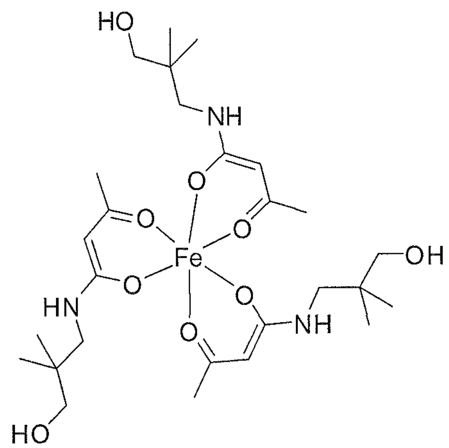

Zu einer Lösung von 3,46 g (18,50 mmol) N-(3-Hydroxy-2,2-dimethylpropyl)-3-oxobutanamid in 50 ml Ethanol wurde eine Lösung von 1,00 g (6,17 mmol) Eisen(III)chlorid wasserfrei in 15 ml Ethanol tropfenweise zugegeben. Dann wurden 6,22 g (74,00 mmol) Natriumbicarbonat portionsweise zugegeben. Nach 4 h Rühren bei Raumtemperatur wurde das Reaktionsgemisch abfiltriert, mit 10 ml Ethanol gewaschen, das Filtrat abrotiert und der Rückstand über Nacht im Trockenschrank bei 50°C getrocknet. Man erhielt 4,40 g braunen Feststoff.
IR (in Substanz, $cm^{-1}$): 3289, 2963, 2872, 1554, 1502, 1450, 1410, 1265, 1190, 1050, 1014, 948, 777.
Fe-Gehalt: 8.3 % [m/m].

**Beispiel 57**

**Tris-(1-[4-(dimethylamino)piperidin-1-yl]butan-1,3-dion)-eisen(III)-Komplex**

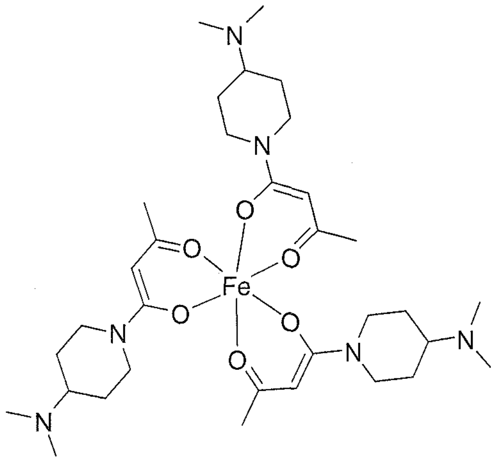

Zu einer Lösung von 3,93 g (18,50 mmol) 1-[4-(Dimethylamino)piperidin-1-yl]butan-1,3-dion in 80 ml MeOH wurde eine Lösung von 1,00 g (6,17 mmol) Eisen(III)chlorid wasserfrei in 10 ml MeOH tropfenweise zugegeben. Dann wurden 3,11 g (37,00 mmol) Natriumbicarbonat portionsweise zugegeben. Nach 2 h Rühren bei Raumtemperatur wurde das Reaktionsgemisch abrotiert, der Rückstand in 100 ml Dichlormethan aufgenommen, 15 min gerührt, abfiltriert, das Filtrat abrotiert, der Rückstand 50 ml EtOH aufgenommen, erneut abrotiert und der erhaltene Rückstand über Nacht im Trockenschrank getrocknet. Man erhielt 4,23 g braunen Feststoff.
IR (in Substanz, $cm^{-1}$): 2940, 2858, 2769, 1593, 1553, 1509, 1482, 1448, 1372, 1328, 1271, 1236, 1205, 1040, 957, 874, 758.
Fe-Gehalt: 7.97 % [m/m].

**Beispiel 58**

**Tris-(1-(4-methoxypiperidin-1-yl]butan-1,3-dion)-eisen(III)-Komplex**

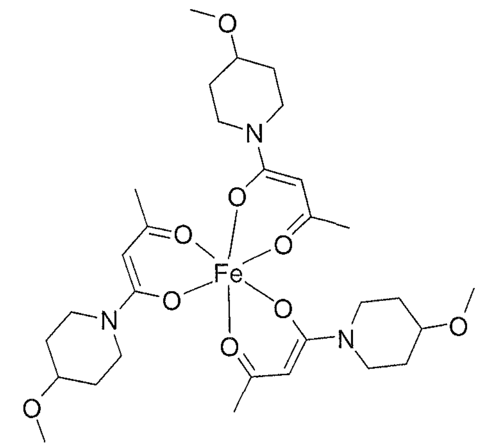

Zu einer Lösung von 3,69 g (18,50 mmol) 1-(4-Methoxypiperidin-1-yl]butan-1,3-dion in 80 ml MeOH wurde eine Lösung von 1,00 g (6,17 mmol) Eisen(III)chlorid wasserfrei in 10 ml MeOH tropfenweise zugegeben. Dann wurden 3,11 g (37,00 mmol) Natriumbicarbonat portionsweise zugegeben. Nach 1 h Rühren bei Raumtemperatur wurde das Reaktionsgemisch abrotiert, der Rückstand in 100 ml Dichlormethan aufgenommen, 15 min gerührt, abfiltriert, das Filtrat

abrotiert, der Rückstand 50 ml EtOH aufgenommen, erneut abrotiert und der erhaltene Rückstand übernacht im Trockenschrank getrocknet. Man erhielt 4,01 g braunen Feststoff.
IR (in Substanz, $cm^{-1}$): 2944, 2825, 1634, 1557, 1511, 1452, 1376, 1317, 1271, 1235, 1184, 1096, 1047, 1023, 959, 939, 759.
Fe-Gehalt: 8.42 % [m/m].

**Beispiel 59**

**Tris-(1-(2,6-dimethylmorpholin-4-yl]butan-1,3-dion)-eisen(III)-Komplex**

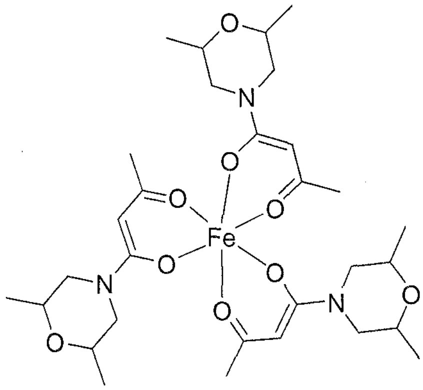

Zu einer Lösung von 3,69 g (18,50 mmol) 1-(2,6-Dimethylmorpholin-4-yl]butan-1,3-dion in 40 ml Ethanol wurde eine Lösung von 1,00 g (6,17 mmol) Eisen(III)chlorid wasserfrei in 10 ml Ethanol tropfenweise zugegeben. Dann wurden 3,11 g (37,00 mmol) Natriumbicarbonat portionsweise zugegeben. Nach 2 h Rühren bei Raumtemperatur wurde das Reaktionsgemisch abfiltriert, mit 10 ml Ethanol gewaschen, das Filtrat abrotiert und der Rückstand über Nacht im Trockenschrank bei 50°C getrocknet. Man erhielt 3,92 g braunen Feststoff.
IR (in Substanz, $cm^{-1}$): 2974, 2872, 1556, 1510, 1477, 1373, 1258, 1244, 1172, 1138, 1116, 1082, 1050, 1002, 958, 759.
Fe-Gehalt: 8.10 % [m/m].

**Beispiel 60**

**Tris-(N-(morpholin-4-yl)-3-oxobutanamid)-eisen(III)-Komplex**

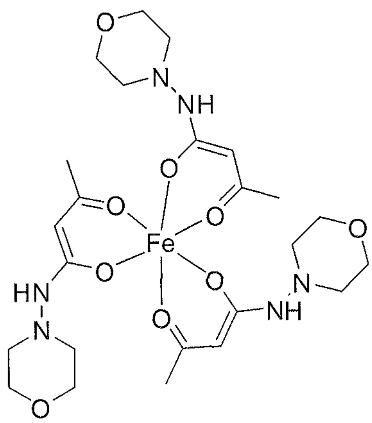

Zu einer Lösung von 3,44 g (18,50 mmol) N-(Morpholin-4-yl)-3-oxobutanamid in 50 ml Ethanol wurde eine Lösung von 1.00 g (6.17 mmol) Eisen(III)chlorid wasserfrei in 20 ml Ethanol tropfenweise zugegeben. Dann wurden 3,11 g (37,00 mmol) Natriumbicarbonat portionsweise zugegeben. Nach 2 h Rühren bei Raumtemperatur wurde das

Reaktionsgemisch abfiltriert, mit 10 ml Ethanol gewaschen, das Filtrat abrotiert und der Rückstand über Nacht im Trockenschrank bei 50°C getrocknet. Man erhielt 3.63 g braunen Feststoff.
IR (in Substanz, $cm^{-1}$):3209, 2854, 1569, 1518, 1430, 1366, 1335, 1265, 1204, 1108, 1072, 1044, 975, 952, 869, 778, 703.
Fe-Gehalt: 8.34 % [m/m].

**Beispiel 61**

**Tris-(1-(4-hydroxypiperidin-1-yl)pentan-1,3-dion)-eisen(III)-Komplex**

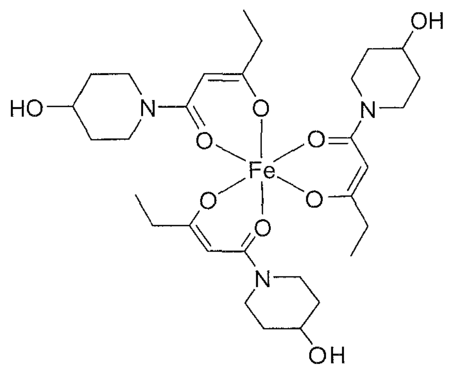

Zur Lösung von 5,54 g (27,8 mmol) 1-(4-Hydroxypiperidin-1-yl)pentan-1,3-dion in 45 ml wasserfreiem Ethanol wurde die Lösung von 1,51 g (9,31 mmol) wasserfreiem Eisen(III)chlorid in 10 ml wasserfreiem Ethanol gegeben, gefolgt von 4,67 g (55,6 mmol) Natriumhydrogencarbonat. Nach 6,5 h wurden der Reaktionsansatz filtriert und die Feststoffe verworfen. Das Filtrat wurde am Rotationsverdampfer vom Lösungsmittel befreit und der Rückstand 16 h im Vakuumtrockenschrank getrocknet. Man erhielt 5,56 g eines braunroten Feststoffs.
IR (in Substanz, $cm^{-1}$): 2932, 2870, 1588, 1551, 1509, 1444, 1382, 1366, 1314, 1265, 1224, 1119, 1074, 978, 924, 832, 801, 764, 705, 661.
Fe-Gehalt: 8.26 %

**Beispiel 62**

**Tris-(1-(morpholin-4-yl)pentan-1,3-dion)-eisen(III)-Komplex**

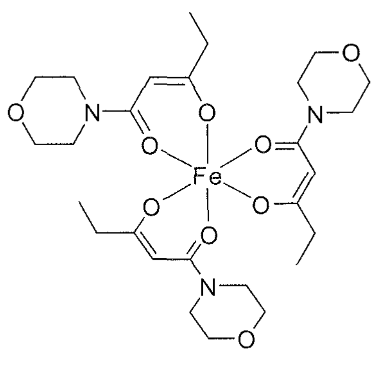

Zu einer Lösung von 3,18 g (17,2 mmol) 1-(Morpholin-4-yl)pentan-1,3-dion in wasserfreiem Ethanol wurde die Lösung von 0,997 g (6,15 mmol) wasserfreiem Eisen(III)chlorid in 11 ml wasserfreiem Ethanol gegeben. Dann wurden 3,11 g (37,0 mmol) Natriumhydrogencarbonat zugegeben und das Reaktionsgemisch 4 h gerührt. Die Feststoffe wurden abfiltriert und verworfen. Das Filtrat wurde am Rotationsverdampfer zur Trockne eingedampft und der Rückstand 16 h im Vakuumtrockenschrank getrocknet. Man erhielt 3,33 g rotbraunen Feststoff.
IR (in Substanz, $cm^{-1}$): 2964, 2851, 1591, 1551, 1510, 1473, 1439, 1380, 1315, 1300, 1274, 1241, 1189, 1112, 1063,

1003, 929, 857, 801, 763, 706, 664, 593.
Fe-Gehalt: 9.13 % [m/m/].

**Beispiel 63**

**Tris-(*N*-Butyl-3-oxoheptanamid)-eisen(III)-Komplex**

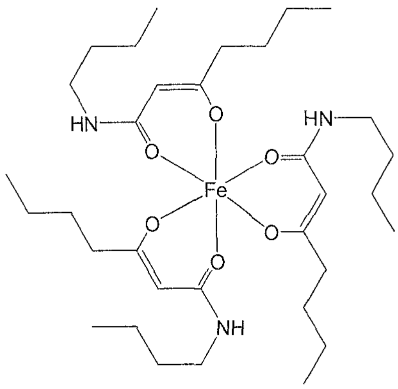

60 mmol (11,96 g) *N*-Butyl-3-oxoheptanamid und 20 mmol (3,24 g) $FeCl_3$ (wasserfrei) wurden in 110 ml Ethanol (wasserfrei) gelöst und 10.4 ml Natriummethylat-Lösung (30% m/m) zugegeben. Es wurde 0,5 h nachgerührt und die Reaktionslösung filtriert. Das Filtrat wurde am Rotationsverdampfer eingeengt und das Produkt getrocknet. Man erhielt 13,7 g der Titelverbindung.
IR (in Substanz, $cm^{-1}$): 2956, 2930, 2871, 1717, 1656, 1558, 1500, 1435, 1376, 1325, 1299, 1271, 1177, 1103, 1085, 1050, 994, 948, 894, 778, 682. CHN-Elementaranalyse: C, 59.26; H, 9.21; N, 6.12.
Fe-Gehalt: 7.39% [m/m]

**Beispiel 64**

**Tris-(1-(4-Hydroxypiperidin-1-yl)-4-methylpentan-1,3-dion)-eisen(III)-Komplex**

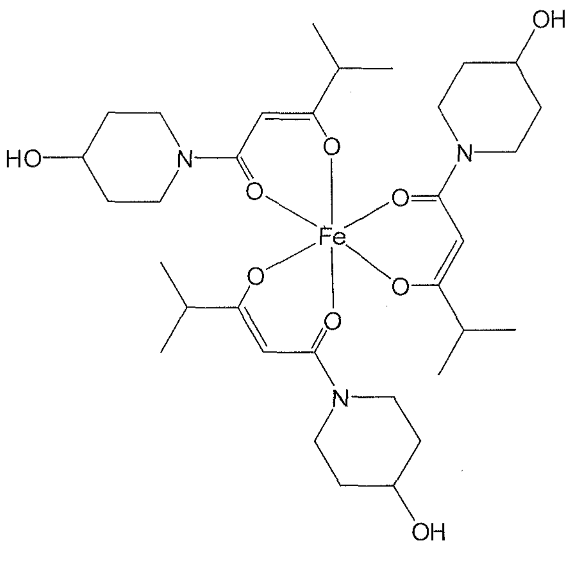

90 mmol (19,2 g) 1-(4-Hydroxypiperidin-1-yl)-4-methylpentan-1,3-dion und 30 mmol (4,87 g) $FeCl_3$ (wasserfrei) wurden in 150 ml Ethanol (wasserfrei) gelöst und 30 ml Natriumethylat-Lösung (21% m/m) zugegeben. Es wurde 0.5 h

nachgerührt und die Reaktionslösung filtriert. Das Filtrat wurde am Rotationsverdampfer eingeengt und das Produkt im Vakuumtrockenschrank bei 50°C getrocknet. Man erhielt 19,8 g der Titelverbindung.
IR (in Substanz, $cm^{-1}$): 2927, 2866, 1550, 1513, 1443, 1372, 1312, 1265, 1225, 1190, 1157, 1119, 1072, 1022, 980, 945, 882, 823, 808, 771, 714, 660, 646. CHN-Elementaranalyse: C, 52.00; H, 7.79; N, 5.99.
Fe-Gehalt: 7.21% [m/m]

**Beispiel 65**

**Tris-(*N*-(2-Hydroxyethyl)-*N*,4-dimethyl-3-oxopentanamid)-eisen(III)-Komplex**

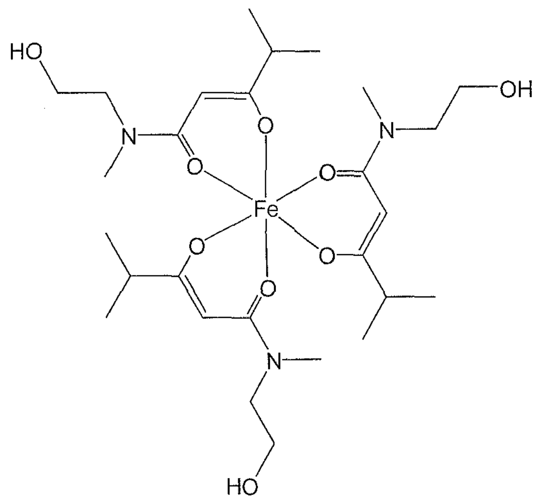

90 mmol (17,93 g) *N*-(2-Hydroxyethyl)-*N*,4-dimethyl-3-oxopentanamid und 30 mmol (4,87 g) $FeCl_3$ (wasserfrei) wurden in 150 ml Ethanol (wasserfrei) gelöst und 15 ml Natriummethylat-Lösung (30% m/m) zugegeben. Es wurde 1 h nachgerührt und die Reaktionslösung filtriert. Das Filtrat wurde am Rotationsverdampfer eingeengt und das Produkt im Vakuumtrockenschrank bei 50°C getrocknet. Man erhielt 20,7 g der Titelverbindung.
IR (in Substanz, $cm^{-1}$): 2966, 2926, 2859, 1714, 1627, 1550, 1517, 1479, 1460, 1439, 1384, 1371, 1358, 1301, 1273, 1246, 1190, 1159, 1114, 1087, 1065, 1051, 1019,991,947,927,884,850,774,717,682.
CHN-Elementaranalyse: C, 44.04; H, 6.97; N, 6.33.
Fe-Gehalt: 7.79% [m/m]

**Beispiel 66**

**Tris-(*N,N*-Dimethyl-2-oxocyclopentancarboxamid)-eisen(III)-Komplex**

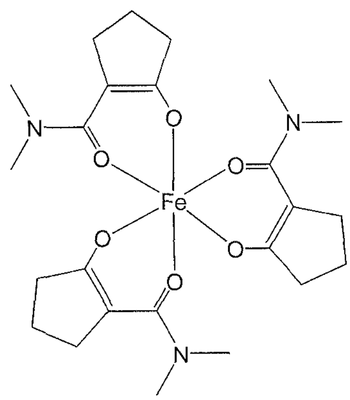

18 mmol (3,29 g) *N,N*-Dimethyl-2-oxocyclopentancarboxamid (Reinheit >85%) und 6 mmol (0,925 g) $FeCl_3$ (wasserfrei) wurden in 50 ml Ethanol (wasserfrei) gelöst und 3 ml Natriummethylat-Lösung (30% m/m) zugegeben. Es wurde 1 h nachgerührt und die Reaktionslösung filtriert. Das Filtrat wurde am Rotationsverdampfer eingeengt und das Produkt im Vakuumtrockenschrank bei 50°C getrocknet. Es wurden 3,9 g der Titelverbindung erhalten.
IR (in Substanz, $cm^{-1}$): 2945, 2882, 1735, 1635, 1555, 1491, 1466, 1451, 1411, 1395, 1362, 1347, 1303, 1262, 1215, 1186, 1169, 1141, 1099, 1050, 1020, 983, 946, 915, 903, 885, 834, 770, 754, 733, 694, 638.
Fe-Gehalt: 8.03% [m/m]

**Beispiel 67**

**Tris-(N-cyclopentyl-3-oxobutanamid)-eisen(III)-Komplex**

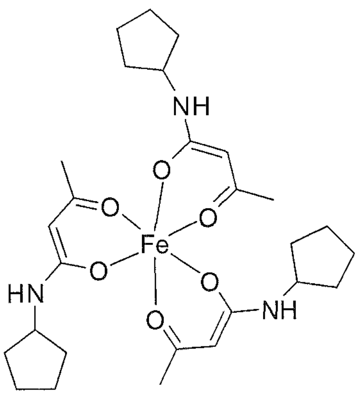

Zu einer Lösung von 31,3 g (18,50 mmol) N-Cyclopentyl-3-oxobutanamid in 40 ml Ethanol wurde eine Lösung von 1,00 g (6,17 mmol) Eisen(III)chlorid wasserfrei in 10 ml Ethanol tropfenweise zugegeben. Dann wurden 6,22 g (74,00 mmol) Natriumbicarbonat portionsweise zugegeben. Nach 2 h Rühren bei Raumtemperatur wurde das Reaktionsgemisch abfiltriert, mit 10 ml Ethanol gewaschen, das Filtrat abrotiert und der Rückstand über Nacht im Trockenschrank bei 50°C getrocknet. Man erhielt 3,74 g braunen Feststoff.
IR (in Substanz, $cm^{-1}$): 3256, 2956, 2869, 1550, 1491, 1450, 1409, 1357, 1265, 1185, 1042, 1015, 951, 780.
Fe-Gehalt: 9.17 % [m/m].

**Beispiel 68**

**Tris-(1-(pyrrolidin-1-yl)butan-1,3-dion)-eisen(III)-Komplex**

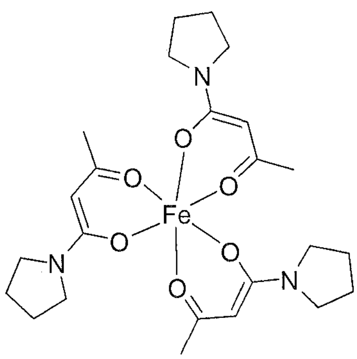

Zu einer Lösung von 2,87 g (18,50 mmol) 1-(Pyrrolidin-1-yl)butan-1,3-dion in 40 ml Ethanol wurde eine Lösung von 1.00 g (6.17 mmol) Eisen(III)chlorid wasserfrei in 10 ml Ethanol tropfenweise zugegeben. Dann wurden 6,22 g (74,00 mmol) Natriumbicarbonat portionsweise zugegeben. Nach 2 h Rühren bei Raumtemperatur wurde das Reaktionsgemisch abfiltriert, mit 10 ml Ethanol gewaschen, das Filtrat abrotiert und der Rückstand über Nacht im Trockenschrank bei 50°C getrocknet. Man erhielt 3,41 g braunen Feststoff.
IR (in Substanz, $cm^{-1}$): 2966, 2869, 1556, 1511, 1473, 1457, 1351, 1328, 1224, 1206, 1117, 1080, 996, 971, 953, 761.
Fe-Gehalt: 10.16 % [m/m].

**Beispiel 69**

Tris-(methyl-N-(3-oxobutanoyl)-L-serinat)-eisen(III)-Komplex

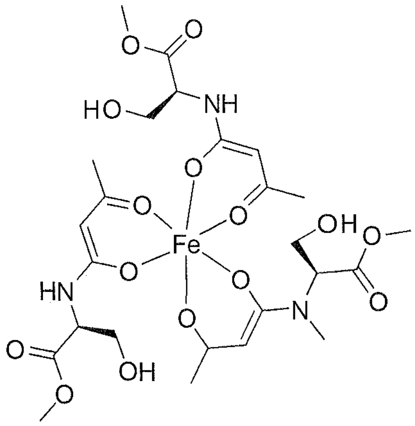

Zu einer Lösung von 3,75 g (18,50 mmol) Methyl-N-(3-oxobutanoyl)-L-serinat in 50 ml Ethanol wurde eine Lösung von 1,00 g (6,17 mmol) Eisen(III)chlorid wasserfrei in 10 ml Ethanol tropfenweise zugegeben. Dann wurden 3,11 g (37,00 mmol) Natriumbicarbonat portionsweise zugegeben. Nach 2 h Rühren bei Raumtemperatur wurde das Reaktionsgemisch abfiltriert, mit 10 ml Ethanol gewaschen, das Filtrat abrotiert und der Rückstand über Nacht im Trockenschrank bei 50°C getrocknet. Man erhielt 4,45 g braunen Feststoff.
IR (in Substanz, $cm^{-1}$): 3285, 2955, 1733, 1543, 1491, 1408, 1343, 1274, 1209, 1186, 1146, 1077, 1054, 1029, 958, 779.
Fe-Gehalt: 8.02 % [m/m].

**Beispiel 70**

Tris-(1-(4-acetylpiperazin-1-yl)butan-1,3-dion)-eisen(III)-Komplex

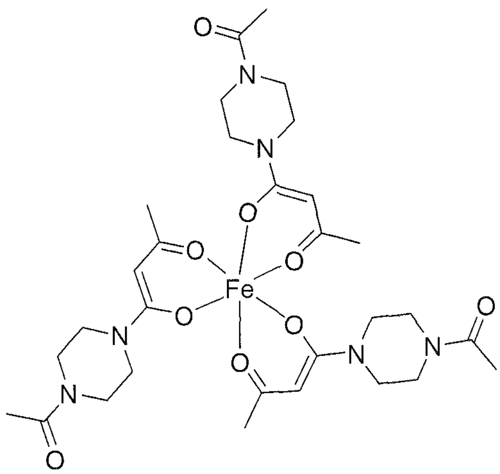

Zu einer Lösung von 3,92 g (18,50 mmol) 1-(4-Acetylpiperazin-1-yl)butan-1,3-dion in 80 ml MeOH wurde eine Lösung von 1,00 g (6,17 mmol) Eisen(III)chlorid wasserfrei in 20 ml MeOH tropfenweise zugegeben. Dann wurden 3,11

g (37,00 mmol) Natriumbicarbonat portionsweise zugegeben. Nach 1 h Rühren bei Raumtemperatur wurde das Reaktionsgemisch abrotiert, der Rückstand in 200 ml Dichlormethan aufgenommen, 15 min gerührt, abfiltriert, das Filtrat abrotiert, der Rückstand 50 ml EtOH aufgenommen, erneut abrotiert und der erhaltene Rückstand übernacht im Trockenschrank getrocknet. Man erhielt 4,1 g braunen Feststoff.
IR (in Substanz, $cm^{-1}$): 2914, 1637, 1555, 1511, 1468, 1421, 1367, 1284, 1239, 1172, 1046, 981, 958, 760.
Fe-Gehalt: 7.74 % [m/m].

**Beispiel 71**

**Tris-(N-cyclohexyl-3-oxobutanamid)-eisen(III)-Komplex**

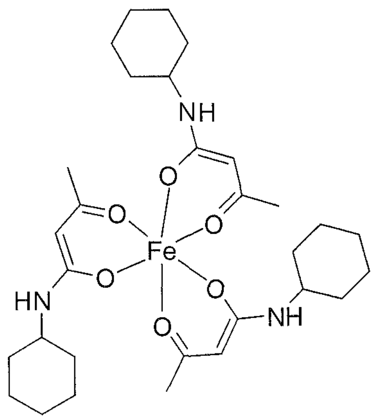

Zu einer Lösung von 3,39 g (18,50 mmol) N-Cyclohexyl-3-oxobutanamid in 40 ml Ethanol wurde eine Lösung von 1,00 g (6,17 mmol) Eisen(III)chlorid wasserfrei in 10 ml Ethanol tropfenweise zugegeben. Dann wurden 3,11 g (37,00 mmol) Natriumbicarbonat portionsweise zugegeben. Nach 2 h Rühren bei Raumtemperatur wurde das Reaktionsgemisch abfiltriert, mit 10 ml Ethanol gewaschen, das Filtrat abrotiert und der Rückstand über Nacht im Trockenschrank bei 50°C getrocknet. Man erhielt 3,8 g braunen Feststoff.
IR (in Substanz, $cm^{-1}$): 3282, 2928, 2854, 1552, 1488, 1448, 1408, 1315, 1278, 1256, 1187, 1150, 1109, 1041, 975, 942, 781.
Fe-Gehalt: 8.81 % [m/m].

**Beispiel 72**

**Tris-(methyl-N-(3-oxobutanoyl)glycinat)-eisen(III)-Komplex**

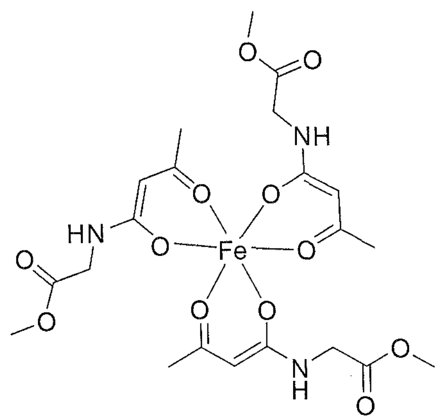

Zu einer Lösung von 3,21 g (18,50 mmol) Methyl-N-(3-oxobutanoyl)glycinat in 40 ml Ethanol wurde eine Lösung von 1,00 g (6,17 mmol) Eisen(III)chlorid wasserfrei in 10 ml Ethanol tropfenweise zugegeben. Dann wurden 3,11 g (37,00 mmol) Natriumbicarbonat portionsweise zugegeben. Nach 1 h Rühren bei Raumtemperatur wurde das Reaktionsgemisch abfiltriert, mit 10 ml Ethanol gewaschen, das Filtrat abrotiert und der Rückstand über Nacht im Trockenschrank bei 50°C getrocknet. Man erhielt 3,95 g braunes Öl.
IR (in Substanz, $cm^{-1}$):3343, 2955, 1739, 1543, 1495, 1409, 1365, 1284, 1179, 1051, 1011, 988, 779.
Fe-Gehalt: 8.95 % [m/m].

**Beispiel 73**

**Tris-(N-(2-methylpropyl)-3-oxobutanamid)-eisen(III)-Komplex**

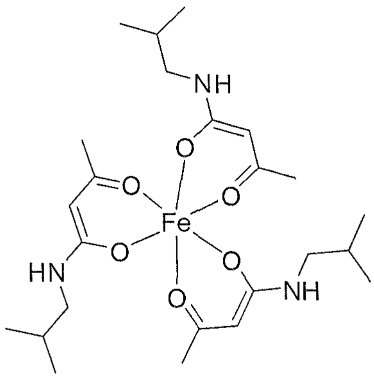

Zu einer Lösung von 2,90 g (18,50 mmol) N-(2-Methylpropyl)-3-oxobutanamid in 40 ml Ethanol wurde eine Lösung von 1,00 g (6,17 mmol) Eisen(III)chlorid wasserfrei in 10 ml Ethanol tropfenweise zugegeben. Dann wurden 3,11 g (37,00 mmol) Natriumbicarbonat portionsweise zugegeben. Nach 4 h Rühren bei Raumtemperatur wurde das Reaktionsgemisch abfiltriert, mit 10 ml Ethanol gewaschen, das Filtrat abrotiert und der Rückstand über Nacht im Trockenschrank bei 50°C getrocknet. Man erhielt 3,49 g braunen Feststoff.
IR (in Substanz, $cm^{-1}$): 3284, 3110, 2959, 2927, 2871, 1551, 1499, 1433, 1408, 1271, 1188, 1156, 1102, 1018, 954, 940, 777.
Fe-Gehalt: 9.70 % [m/m].

**Beispiel 74**

**Tris-(N-(cyclopropylmethyl)-3-oxobutanamid)-eisen(III)-Komplex**

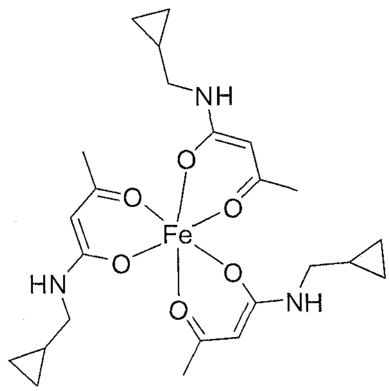

Zu einer Lösung von 1,44 g (9,25 mmol) N-(Cyclopropylmethyl)-3-oxobutanamid in 20 ml Ethanol wurde eine

Lösung von 0,5 g (3,08 mmol) Eisen(III)chlorid wasserfrei in 10 ml Ethanol tropfenweise zugegeben. Dann wurden 1,56 g (18,50 mmol) Natriumbicarbonat portionsweise zugegeben. Nach 4 h Rühren bei Raumtemperatur wurde das Reaktionsgemisch abfiltriert, mit 10 ml Ethanol gewaschen, das Filtrat abrotiert und der Rückstand über Nacht im Trockenschrank bei 50°C getrocknet. Man erhielt 1,75 g braunen Feststoff.
IR (in Substanz, $cm^{-1}$): 3270, 1549, 1498, 1431, 1407, 1265, 1187, 1165, 1096, 1023,951,830,778.
Fe-Gehalt: 9.89 % [m/m].

**Beispiel 75**

**Tris-(ethyl 4-(morpholin-4-yl)-2,4-dioxobutanoat)-eisen(III)-Komplex**

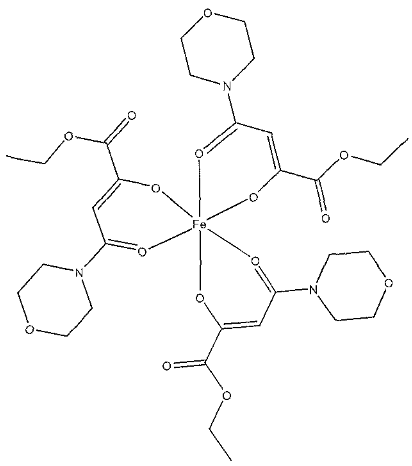

0,24 g (1,45 mmol) Eisen(III)chlorid wurden in 20 ml THF gelöst und 1,00 g (4,36 mmol) Ethyl 4-(morpholin-4-yl)-2,4-dioxobutanoat zugegeben. Nach 40 min Rührzeit gab man 0,44 g (4,36 mmol) Triethylamin zu und ließ für weitere 60 min bei Raumtemperatur rühren. Es wurden anschliessend 4,6 g 6%-ige Natriumethanolat-Lösung (4,4 mmol) zugetropft und die Lösung für 2 Stunden weiter gerührt. Das ausgefallene Salz wurde abfiltriert und das Filtrat am Rotationsverdampfer bis zur Trockene eingeengt. Der Rückstand wurde 1 Tag bei 50°C im Feinvakuum getrocknet. Man erhielt 0,8 g Produkt als orangen Feststoff.
IR (in Substanz, $cm^{-1}$): 2978, 2923, 2863, 1704, 1610, 1568, 1511, 1442, 1375, 1300, 1275, 1246, 1138, 1111, 1062, 1018, 946, 860, 759, 722, 651.
Elementaranalyse: C 48.54%, H 5.8%, N 5.48%
Fe-Gehalt: 6.9% [m/m].

**Beispiel 76**

**Tris-(ethyl-N-(3-oxobutanoyl)-L-alaninat)-eisen(III)-Komplex**

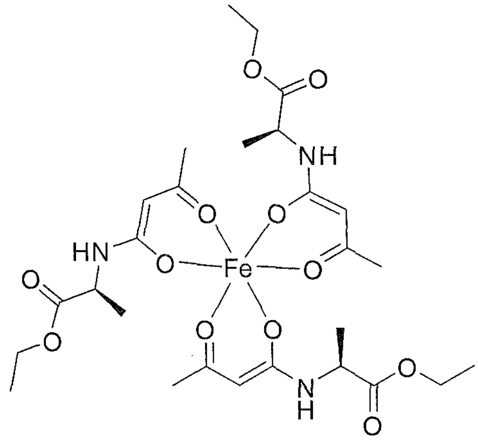

Zu einer Lösung von 3,72 g (18,50 mmol) Ethyl-N-(3-oxobutanoyl)-L-alaninat in 40 ml Ethanol wurde eine Lösung von 1,00 g (6,17 mmol) Eisen(III)chlorid wasserfrei in 10 ml Ethanol tropfenweise zugegeben. Dann wurden 3,11 g (37,00 mmol) Natriumbicarbonat portionsweise zugegeben. Nach 2 h Rühren bei Raumtemperatur wurde das Reaktionsgemisch abfiltriert, mit 10 ml Ethanol gewaschen, das Filtrat abrotiert und der Rückstand über Nacht im Trockenschrank bei 50°C getrocknet. Man erhielt 4,00 g braunes Öl.
IR (in Substanz, $cm^{-1}$): 3348, 2981, 1733, 1658, 1589, 1539, 1490, 1448, 1411, 1300, 1206, 1186, 1155, 1051, 965, 777.
Fe-Gehalt: 7.80 % [m/m].

**Beispiel 77**

**Tris-(N-cyclobutyl-3-oxobutanamid)-eisen(III)-Komplex**

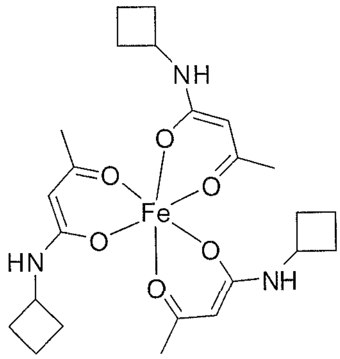

Zu einer Lösung von 1,43 g (9,25 mmol) N-Cyclobutyl-3-oxobutanamid in 20 ml Ethanol wurde eine Lösung von 0,5 g (3,08 mmol) Eisen(III)chlorid wasserfrei in 10 ml Ethanol tropfenweise zugegeben. Dann wurden 1,56 g (18,50 mmol) Natriumbicarbonat portionsweise zugegeben. Nach 2 h Rühren bei Raumtemperatur wurde das Reaktionsgemisch abfiltriert, mit 10 ml Ethanol gewaschen, das Filtrat abrotiert und der Rückstand über Nacht im Trockenschrank bei 50°C getrocknet. Man erhielt 1,71 g braunen Feststoff.
IR (in Substanz, $cm^{-1}$): 3280, 2978, 2943, 1546, 1488, 1408, 1274, 1187, 1156, 1031, 970, 946, 779, 757.
Fe-Gehalt: 9.98 % [m/m].

**Beispiel 78**

**Tris-(1-(azetidin-1-yl)butan-1,3-dion)-eisen(III)-Komplex**

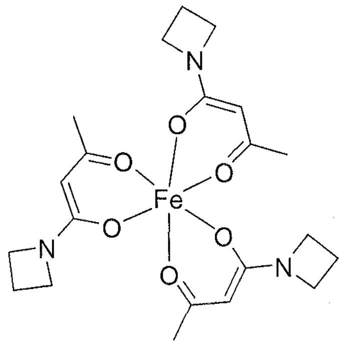

Zu einer Lösung von 1,30 g (9,25 mmol) 1-(Azetidin-1-yl)-butan-1,3-dion in 20 ml Ethanol wurde eine Lösung von 0,5 g (3,08 mmol) Eisen(III)chlorid wasserfrei in 10 ml Ethanol tropfenweise zugegeben. Dann wurden 3,11 g (37,00 mmol) Natriumbicarbonat portionsweise zugegeben. Nach 2 h Rühren bei Raumtemperatur wurde das Reaktionsgemisch abfiltriert, mit 10 ml Ethanol gewaschen, das Filtrat abrotiert und der Rückstand über Nacht im Trockenschrank bei 50°C getrocknet. Man erhielt 1,58 g braunen Feststoff.
IR (in Substanz, $cm^{-1}$): 2945, 2878, 1558, 1510, 1473, 1344, 1297, 1201, 1003, 948, 753.
Fe-Gehalt: 11.13% [m/m].

**Beispiel 79**

**Tris-(ethyl-1-(3-oxobutanoyl)-piperidin-4-carboxylat)-eisen(III)-Komplex**

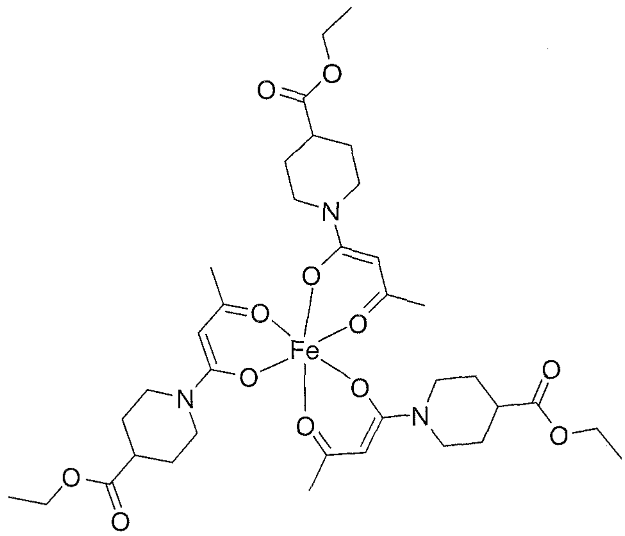

Zu einer Lösung von 2,23 g (9,25 mmol) Ethyl-1-(3-oxobutanoyl)-piperidin-4-carboxylat in 30 ml Ethanol wurde eine Lösung von 0,5 g (3,08 mmol) Eisen(III)chlorid wasserfrei in 10 ml Ethanol tropfenweise zugegeben. Dann wurden 3,11 g (37,00 mmol) Natriumbicarbonat portionsweise zugegeben. Nach 2 h Rühren bei Raumtemperatur wurde das Reaktionsgemisch abfiltriert, mit 10 ml Ethanol gewaschen, das Filtrat abrotiert und der Rückstand über Nacht im Trockenschrank bei 50°C getrocknet. Man erhielt 2,43 g braunen Feststoff.
IR (in Substanz, $cm^{-1}$): 2957, 2931, 2862, 1724, 1639, 1555, 1511, 1485, 1446, 1372, 1312, 1271, 1236, 1174, 1038, 973, 958, 760.
Fe-Gehalt: 6.67 % [m/m].

**Beispiel 80**

**Tris-(1-(4-Hydroxypiperidin-1-yl)-4-methoxybutan-1,3-dion)-eisen(III)-Komplex**

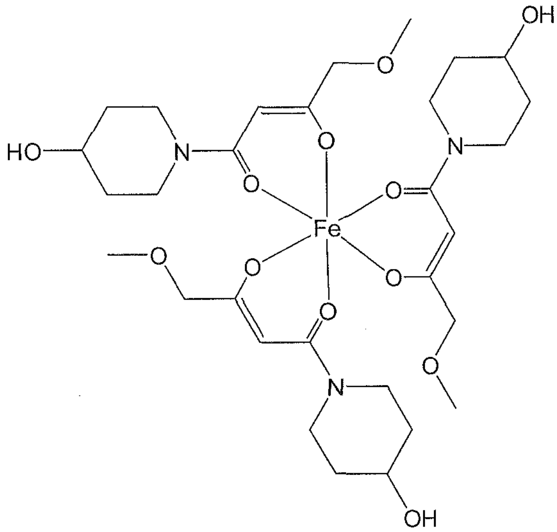

18 mmol (3,91 g) 1-(4-Hydroxypiperidin-1-yl)-4-methoxybutan-1,3-dion und 6 mmol (0,96 g) $FeCl_3$ (wasserfrei) wurden in 80 ml Ethanol (wasserfrei) gelöst und 4 ml Natriummethylat-Lösung (30% m/m) zugegeben. Es wurde 1 h nachgerührt und die Reaktionslösung filtriert. Das Filtrat wurde am Rotationsverdampfer eingeengt und das Produkt getrocknet. Man erhielt 4,36 g der Titelverbindung.
IR (in Substanz, $cm^{-1}$): 2928, 2824, 1727, 1597, 1564, 1513, 1491, 1445, 1385, 1331, 1265, 1227, 1197, 1109, 1074, 1051, 1025, 990, 957, 845, 807, 765, 723, 653.
Fe-Gehalt: 7.11% [m/m].

**Patentansprüche**

1. Eisen(III)-Komplexverbindungen enthaltend mindestens einen Liganden der Formel (I):

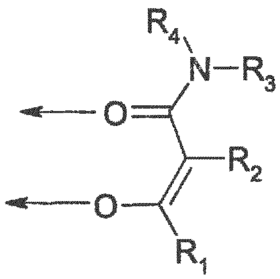

(I)

worin

die Pfeile jeweils eine koordinative Bindung zu einem oder verschiedenen Eisenatome darstellen,
$R_1$ ausgewählt wird aus der Gruppe, die besteht aus gegebenenfalls substituiertem Alkyl und gegebenenfalls substituiertem Alkoxycarbonyl,
$R_2$ ausgewählt wird aus der Gruppe, die besteht aus Wasserstoff, gegebenenfalls substituiertem Alkyl, Halogen, und Cyano, oder
$R_1$ und $R_2$ gemeinsam mit den Kohlenstoffatomen, an die sie gebunden sind, einen gegebenenfalls substituierten 5- oder 6-gliedrigen Ring bilden, der gegebenenfalls ein oder mehrere Heteroatome aufweisen kann,
$R_3$ und $R_4$ gleich oder verschieden sind, und jeweils ausgewählt werden aus der Gruppe, die besteht aus

Wasserstoff, gegebenenfalls substituiertem Amino und gegebenenfalls substituiertem Alkyl, oder $R_3$ und $R_4$ gemeinsam mit dem Stickstoffatom, an das sie gebunden sind, einen gegebenenfalls substituierten 3- bis 6-gliedrigen Ring bilden, der gegebenenfalls ein oder mehrere weitere Heteroatome aufweisen kann, oder

$R_2$ und $R_3$ gemeinsam einen gesättigten oder ungesättigten gegebenenfalls substituierten 5- oder 6-gliedrigen Ring unter Bildung eines Liganden der Formel (Ia) bilden:

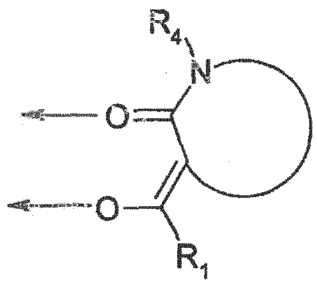

(Ia)

worin $R_1$ und $R_4$ wie zuvor definiert sind,

oder

$R_2$ und $R_3$ gemeinsam einen gesättigten oder ungesättigten gegebenenfalls substituierten 5- oder 6-gliedrigen Ring und $R_1$ und $R_2$ gemeinsam einen gesättigten oder ungesättigten gegebenenfalls substituierten 5- oder 6-gliedrigen Ring unter Bildung eines Liganden der Formel (Ib) bilden:

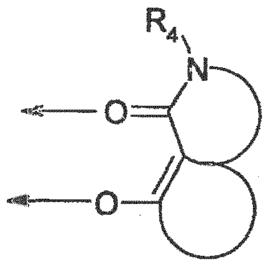

(Ib),

worin $R_4$ wie zuvor definiert ist,

oder pharmazeutisch verträgliche Salze davon, zur Verwendung in der Behandlung und Prophylaxe von Eisenmangelerscheinungen und Eisenmangelanämien sowie der damit einhergehenden Symptome.

2. Eisen(III)-Komplexverbindungen zur Verwendung nach Anspruch 1, enthaltend mindestens einen Liganden der Formel (I):

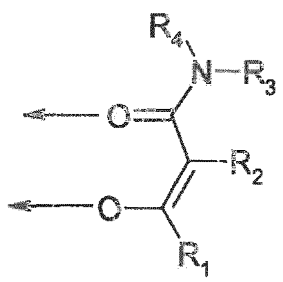

(I)

worin

die Pfeile jeweils eine koordinative Bindung zu einem oder verschiedenen Eisenatomen darstellen,

$R_1$ gegebenenfalls substituiertes Akyl ist,

$R_2$ ausgewählt wird aus der Gruppe, die besteht aus Wasserstoff, gegebenenfalls substituiertem Alkyl, Halogen, und Cyano, oder

$R_1$ und $R_2$ gemeinsam mit den Kohlenstoffatomen, an die sie gebunden sind, einen gegebenenfalls substituierten 5- oder 6-gliedrigen Ring bilden, der gegebenenfalls ein oder mehrere Heteroatome aufweisen kann,
$R_3$ und $R_4$ gleich oder verschieden sind, und jeweils ausgewählt werden aus der Gruppe, die besteht aus Wasserstoff und gegebenenfalls substituiertem Alkyl, oder
$R_3$ und $R_4$ gemeinsam mit dem Stickstoffatom, an das sie gebunden sind, einen gegebenenfalls substituieren 3- bis 6-gliedrigen Ring bilden, der gegebenenfalls ein oder mehrere weitere Heteroatome aufweisen kann, oder
$R_2$ und $R_3$ gemeinsam einen gesättigten oder ungesättigten gegebenenfalls substituierten 5- oder 6-gliedrigen Ring unter Bildung eines Liganden der Formel (Ia) bilden:

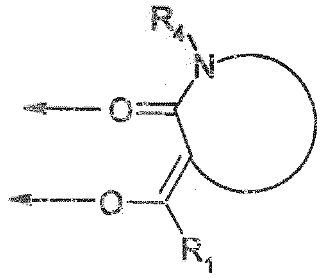

(Ia)

worin $R_1$ und $R_4$ wie zuvor definiert sind,
oder
$R_2$ und $R_3$ gemeinsam einen gesättigten oder ungesättigten gegebenenfalls substituierten 5- oder 6-gliedrigen Ring und $R_1$ und $R_2$ gemeinsam einen gesättigten oder ungesättigten gegebenenfalls substituierten 5- oder 6-gliedrigen Ring unter Bildung eines Liganden der Formel (Ib) bilden:

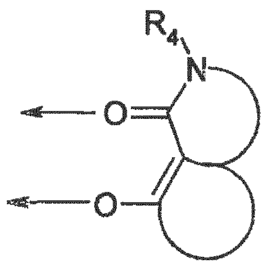

(Ib),

worin $R_4$ wie zuvor definiert ist,
oder pharmazeutisch verträgliche Salze davon.

**3.** Eiseni(III)-Komplexiverbindungen zur Verwendung nach Anspruch 1 oder 2, enthaltend mindestens einen Liganden der Formel (I):

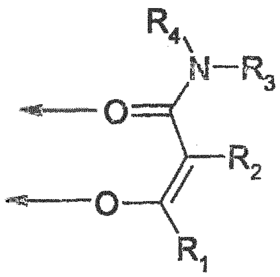

(I)

worin

die Pfeile jeweils eine koordinative Bindung zu einem oder verschiedenen Eisenatomen darstellen,
$R_1$ Alkyl ist, dass gegebenenfalls durch 1 bis 3 Substituenten substituiert sein kann, die aus der Gruppe ausgewählt sind, die besteht aus Hydroxy, Alkoxy, Halogen, Cyano und Alkoxycarbonyl,
$R_2$ ausgewählt wird aus der Gruppe, die besteht aus
Wasserstoff,
Alkyl, das gegebenenfalls durch 1 bis 3 Substituenten substituiert sein kann, die aus der Gruppe ausgewählt sind, die besteht aus Hydroxy, Alkoxy, Halogen, Cyano und Alkoxycarbonyl,
Halogen und
Cyano, oder
$R_1$ und $R_2$ gemeinsam mit den Kohlenstoffatomen, an die sie gebunden sind, einen gegebenenfalls substituierten 5- oder 6-gliedrigen Ring bilden, der gegebenenfalls ein oder mehrere Heteroatome aufweisen kann,
$R_3$ und $R_4$ gleich oder verschieden sind, und jeweils ausgewählt werden aus der Gruppe, die besteht aus Wasserstoff und Alkyl, dass gegebenenfalls durch 1 bis 3 Substituenten substituiert sein kann, die aus der Gruppe ausgewählt sind, die besteht aus Hydroxy, Alkoxy, Halogen, Cyano und Alkoxycarbonyl,
oder
$R_3$ und $R_4$ gemeinsam mit dem Stickstoffatom, an das sie gebunden sind, einen gegebenenfalls substituierten 3- bis 6-gliedrigen Ring bilden, der gegebenenfalls ein oder mehrere weitere Heteroatome aufweisen kann,
oder
$R_2$ und $R_3$ gemeinsam einen gesättigten oder ungesättigten gegebenenfalls substituierten 5- oder 6-gliedrigen Ring unter Bildung eines Liganden der Formel (Ia) bilden:

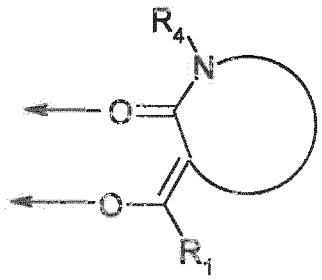
(Ia)

worin $R_1$ und $R_4$ wie zuvor definiert sind,
oder
$R_2$ und $R_3$ gemeinsam einen gesättigten oder ungesättigten gegebenenfalls substituierten 5- oder 6-gliedrigen Ring und $R_1$ und $R_2$ gemeinsam einen gesättigten oder ungesättigten gegebenenfalls substituierten 5- oder 6-gliedrigen Ring unter Bildung eines Liganden der Formel (Ib) bilden:

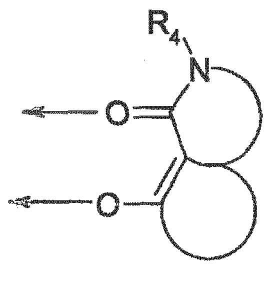
(Ib),

worin $R_4$ wie zuvor definiert ist,
oder pharmazeutisch verträgliche Salze davon.

**4.** Eisen(III)-Komplexverbindungen zur Verwendung nach einem der Ansprüche 1 bis 3 der Formel (II):

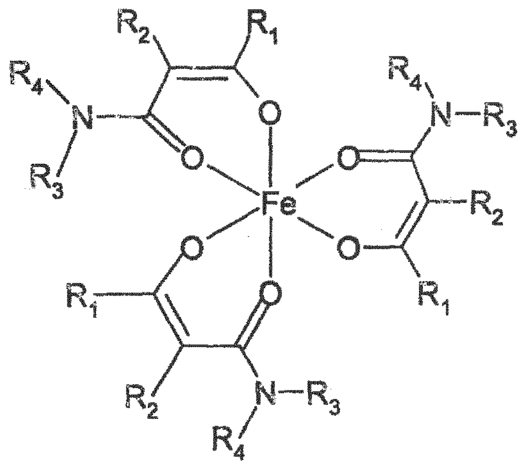

(II)

worin $R_1$, $R_2$, $R_3$ und $R_4$ wie oben definiert sind.

**5.** Eisen(III)-Komplexverbindungen zur Verwendung nach einem der Ansprüche 1 bis 4, worin $R_1$ ausgewählt wird aus der Gruppe, die besteht aus:

- $C_{1-6}$-Alkyl,
- $C_{3-6}$-Cycloalkyl,
- $C_{3-6}$-Cycloalkyl-$C_{1-4}$-alkyl,
- $C_{1-4}$-Alkoxy-$C_{1-4}$-alkyl,
- Hydroxy-$C_{1-4}$-alkyl, und
- Halogen $C_{1-4}$-alkyl.

**6.** Eisen(III) -Komplexverbindungen zur Verwendung nach einem der Ansprüche 1 bis 5, worin $R_2$ ausgewählt wird aus der Gruppe, die besteht aus:

- Wasserstoff,
- Halogen,
- $C_{1-6}$-Alkyl,
- $C_{3-6}$-Cycloalkyl,
- Halogen-$C_{1-4}$-alkyl, und
- Cyano.

**7.** Eisen(III) -Komplexverbindungen zur Verwendung nach einem der Ansprüche 1 bis 4, worin $R_1$ und $R_2$ zusammen eine Propylen(-$CH_2$-$CH_2$-$CH_2$-)- oder eine Butylen (-$CH_2$-$CH_2$-$CH_2$-$CH_2$-)-Gruppe bilden, in denen jeweils eine Methylengruppe (-$CH_2$-) durch -O-, -NH-, oder -$NR_5$- ersetzt sein kann, worin $R_5$ gegebenenfalls substituiertes Alkyl ist, und worin die von $R_1$ und $R_2$ gebildeten Gruppen weiterhin jeweils durch einen bis drei Substituenten, ausgewählt aus der Gruppe, die besteht aus Hydroxy, $C_{1-4}$-Alkoxy, Amino und Mono- oder Di($C_{1-4}$-alkyl)amino, substituiert sein können.

**8.** Eisen(III)-Komplexverbindungen zur Verwendung nach einem der Ansprüche 1 bis 7, worin $R_3$ und $R_4$ gleich oder verschieden sind, und jeweils ausgewählt werden aus der Gruppe, die besteht aus:

- Wasserstoff,
- $C_{1-6}$-Alkyl,
- $C_{3-6}$-Cycloalkyl,
- $C_{3-6}$-Cycloalkyl-$C_{1-4}$-alkyl,
- $C_{1-4}$-Alkoxy-$C_{1-4}$-alkyl,
- $C_{1-3}$-Alkoxycarbonyl-$C_{1-6}$-alkyl,
- Hydroxy-$C_{1-4}$-alkyl, und
- Halogen-$C_{1-4}$-alkyl, oder

$R_3$ und $R_4$ zusammen eine Ethylen(-$CH_2$-$CH_2$-)-, Propylen (-$CH_2$-$CH_2$-$CH_2$-)-, Isopropylen(-$CH_2$-$CH(CH_3)$-)-, Butylen (-$CH_2$-$CH_2$-$CH_2$-$CH_2$-)-, Isobutylen-, Pentylen(-$CH_2$-$CH_2$-$CH_2$-$CH_2$-$CH_2$-)-, oder Isopentylen-Gruppe bilden, worin jeweils eine Methylengruppe (-$CH_2$-) durch -O-, -NH-, oder -$NR_5$- ersetzt sein kann, worin $R_5$ gegebenenfalls substituiertes Alkyl ist, und worin die von $R_3$ und $R_4$ gebildeten Gruppen weiterhin jeweils durch einen bis drei Substituenten, ausgewählt aus der Gruppe, die besteht aus Hydroxy, $C_{1-4}$-Alkoxy, Amino und Mono- oder Di($C_{1-4}$-alkyl)amino, substituiert sein können.

**9.** Verfahren zur Herstellung der Eisen(III)-Komplexverbindungen nach einem der Ansprüche 1 bis 8, welches die Umsetzung eines Eisen(III)-Salzes mit dem entsprechenden β-Ketoamid umfasst.

**10.** Eisen(III)-Komplexverbindungen zur Verwendung nach einem der Ansprüche 1 bis 8, worin die Symptome einschließen: Ermüdung, Antriebslosigkeit, Konzentrationschwäche, geringe kognitive Effizienz, Schwierigkeiten beim Finden der richtige Worte, Vergesslichkeit, unnatürliche Blässe, Reizbarkeit, Beschleunigung der Herzfrequenz (Tachykardie), wunde oder geschwollene Zunge, vergrößerte Milz, Schwangerengelüste (Pica), Kopfschmerzen, Appetitlosigkeit, erhöhte Infektionsarrfälligkeit, depressive Verstimmungen.

**11.** Eisen(III)-Komplexverbindungen zur Verwendung nach einem der Ansprüche 1 bis 8 zur Behandlung der Eisenmangelanämie bei Schwangeren, der latenten Eisenmangelanämie bei Kindern und Heranmachsenden, der Eisenmangelanämie infolge gastrointestinaler Abnormalitäten, der Eisenmangelanämie infolge von Blutverlusten, wie durch gastrointestinale Blutungen (z.B. infolge von Geschwüren, Karzinomen, Hämorriden, entzündlichen Störungen, Einnahme von Acetylsalicylsäure), der Eisenmangelanämie infolge von Menstruation, der Eisenmangelanämie infolge von Verletzungen, der Eisenmangelanämie infolge von Psilosis (sprue), der Eisenmangelanämie infolge verminderter Eisenaufnahme bei der Ernährung, insbesondere bei selektiv essenden Kindern und Heranwachsenden, der Immunschwäche hervorgerufen durch Eisenmangelanämie, der Beeinträchtigung der Hirnleistung hervorgerufen durch Eisenmangelanämien, des Restless Leg Syndrom hervorgerufen durch Eisenmangelanämien, Eisenmangelanämien bei Krebs, Eisenmangelanämien ausgelöst durch Chemotherapien, Eisenmangelanämien ausgelöst durch Inflammation (AI), Eisenmangelanämien bei kongestiver Herzinsuffizienz (CHF;congestive heart failure), Eisenmangelanämien bei chronischer Niereninsuffizienz Stadium 3-5 (CKD 3-5; chronic kidney diseases stage 3-5), Eisenmangelanämien ausgelöst durch chronische Inflammation (ACD), Eisenmangelanämien bei rheumatischer Arthritis (RA; rheumatoid arthritis), Eisenmangelanämien bei systemischem Lupus erythemotodes (SLE; systemic lupus erythematosus) und Eisenmangelanämien bei inflammatorischen Darmerkrankungen (IBD; inflammatory bowel diseases).

**12.** Eisen(III)-Komplexverbindungen zur Verwendung nach einem der Ansprüche 1 bis 8 zur oralen Verabreichung.

**13.** Eiser(III)-Komplexverbindungen zur Verwendung nach Anspruch 12 zur Verabreichung als flüssige Formulierung, einschließlich einer trinkbaren Formulierung, wie Sirup, Elixier, Lösung, Suspension, Saft.

**14.** Arzneimittel, enthaltend Eisen(III)-Komplexverbindungen wie in einem der Ansprüche 1 bis 8 definiert sowie mindestens einen physiologisch verträglichen Träger bzw. Exzipienten zur Verwendung in der Behandlung und Prophylaxe von Eisenmangelerscheinungen und Eisenmangelanämien sowie der damit einhergehenden Symptome.

**15.** Zusammensetzung enthaltend Eisen(III)-Komplexverbindungen, wie in einem der Ansprüche bis 8 definiert, in Kombination mit mindestens einem weiteren Arzneimittel welches auf den Eisenmetabolismus wirkt, zur Verwendung in der Behandlung und Prophylaxe von Eisenmangelerscheinungen und Eisenmangelanämien sowie der damit einhergehenden Symptome.

## Claims

**1.** Iron(III) complex compounds comprising at least one ligand of the formula (I):

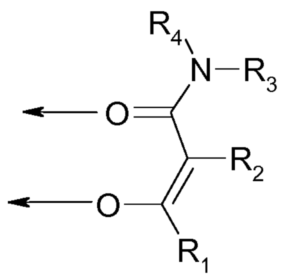

(I)

wherein

the arrows individually represent a coordinate bond to one or different iron atoms,
$R_1$ is selected from the group consisting of optionally substituted alkyl, and optionally substituted alkoxycarbonyl,
$R_2$ is selected from the group consisting of hydrogen, optionally substituted alkyl, halogen and cyano, or
$R_1$ and $R_2$, together with the carbon atoms to which they are bonded, form an optionally substituted 5- or 6-membered ring, which may optionally contain one or more heteroatoms,
$R_3$ and $R_4$ are the same or different and are individually selected from the group consisting of hydrogen, optionally substituted amino, and optionally substituted alkyl, or
$R_3$ and $R_4$, together with the nitrogen atom to which they are bonded, form an optionally substituted 3- to 6-membered ring, which may optionally contain one or more further heteroatoms,
or
$R_2$ and $R_3$ together form a saturated or unsaturated, optionally substituted 5- or 6-membered ring while forming a ligand of the formula (Ia):

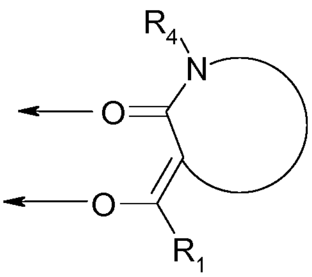

(Ia)

wherein $R_1$ and $R_4$ are defined as above,
or
$R_2$ and $R_3$ together form a saturated or unsaturated, optionally substituted 5- or 6-membered ring, and $R_1$ and $R_2$ together form a saturated or unsaturated, optionally substituted 5- or 6-membered ring, while forming a ligand of the formula (Ib):

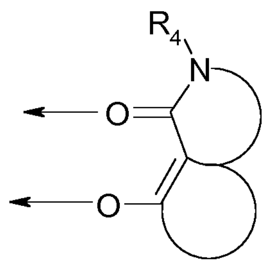

(Ib),

wherein $R_4$ is defined as above,
or pharmaceutically acceptable salts thereof,
for use in the treatment and prophylaxis of iron deficiency diseases and iron deficiency anemias and the symptoms associated therewith.

**2.** Iron(III) complex compounds for the use according to claim 1, comprising at least one ligand of the formula (I):

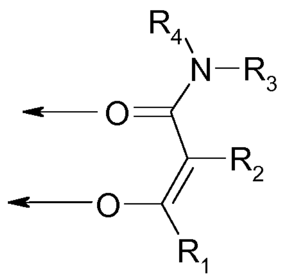

(I)

wherein

the arrows individually represent a coordinate bond to one or different iron atoms,
$R_1$ is optionally substituted alkyl,
$R_2$ is selected from the group consisting of hydrogen, optionally substituted alkyl, halogen and cyano, or
$R_1$ and $R_2$, together with the carbon atoms to which they are bonded, form an optionally substituted 5- or 6-membered ring, which may optionally contain one or more heteroatoms,
$R_3$ and $R_4$ are the same or different and are individually selected from the group consisting of hydrogen and optionally substituted alkyl, or
$R_3$ and $R_4$, together with the nitrogen atom to which they are bonded, form an optionally substituted 3- to 6-membered ring, which may optionally contain one or more further heteroatoms,
or
$R_2$ and $R_3$ together form a saturated or unsaturated, optionally substituted 5- or 6-membered ring while forming a ligand of the formula (Ia):

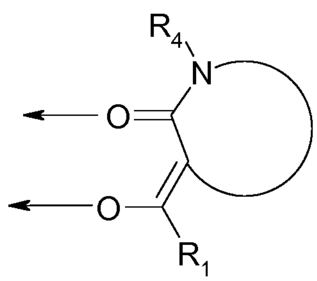

(Ia)

wherein $R_1$ and $R_4$ are defined as above,
or
$R_2$ and $R_3$ together form a saturated or unsaturated, optionally substituted 5- or 6-membered ring, and $R_1$ and $R_2$ together form a saturated or unsaturated, optionally substituted 5- or 6-membered ring, while forming a ligand of the formula (Ib):

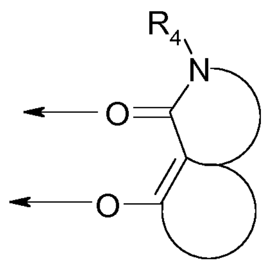

(Ib),

wherein $R_4$ is defined as above,
or pharmaceutically acceptable salts thereof.

**3.** Iron(III) complex compounds for the use according to claim 1 or 2, comprising at least one ligand of the formula (I):

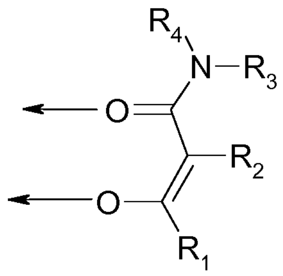

(I)

wherein

the arrows individually represent a coordinate bond to one or different iron atoms,
$R_1$ is alkyl, which can be optionally substituted with 1 to 3 substituents selected from the group consisting of hydroxy, alkoxy, halogen, cyano and alkoxycarbonyl,
$R_2$ is selected from the group consisting of
hydrogen,
alkyl, which can be optionally substituted with 1 to 3 substituents selected from the group consisting of hydroxy, alkoxy, halogen, cyano and alkoxycarbonyl,
halogen and
cyano or
$R_1$ and $R_2$, together with the carbon atoms to which they are bonded, form an optionally substituted 5- or 6-membered ring, which may optionally contain one or more heteroatoms,
$R_3$ and $R_4$ are the same or different and are individually selected from the group consisting of hydrogen and alkyl, which can be optionally substituted with 1 to 3 substituents selected from the group consisting of hydroxy, alkoxy, halogen, cyano and alkoxycarbonyl, or
$R_3$ and $R_4$, together with the nitrogen atom to which they are bonded, form an optionally substituted 3- to 6-membered ring, which may optionally contain one or more further heteroatoms,
or
$R_2$ and $R_3$ together form a saturated or unsaturated, optionally substituted 5- or 6-membered ring while forming a ligand of the formula (Ia):

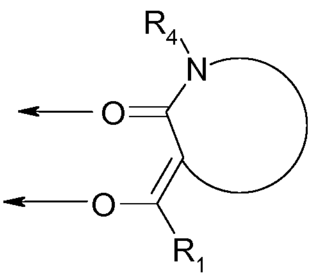

(Ia)

wherein $R_1$ and $R_4$ are defined as above,
or
$R_2$ and $R_3$ together form a saturated or unsaturated, optionally substituted 5- or 6-membered ring, and $R_1$ and $R_2$ together form a saturated or unsaturated, optionally substituted 5- or 6-membered ring, while forming a ligand of the formula (Ib):

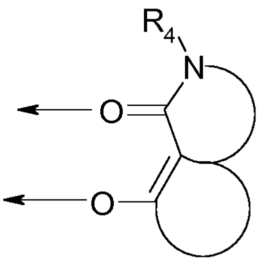

(Ib),

wherein $R_4$ is defined as above,
or pharmaceutically acceptable salts thereof.

**4.** Iron(III) complex compounds for the use according to any of claims 1 to 3 of the formula (II):

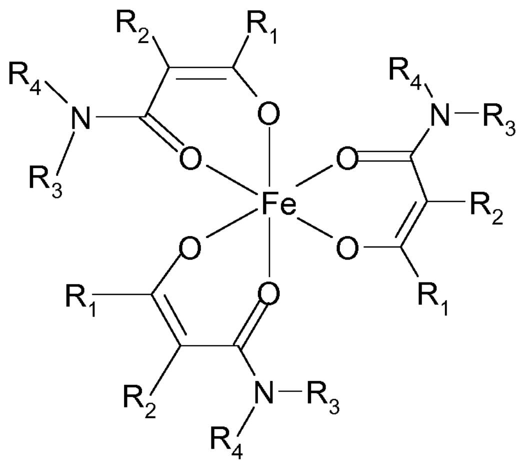

(II)

wherein $R_1$, $R_2$, $R_3$ and $R_4$ are defined as above.

**5.** Iron(III) complex compounds for the use according to any one of the claims 1 to 4, wherein $R_1$ is selected from the group consisting of:

- $C_{1-6}$-alkyl,
- $C_{3-6}$-cycloalkyl,
- $C_{3-6}$-cycloalkyl-$C_{1-4}$-alkyl,
- $C_{1-4}$-alkoxy-$C_{1-4}$-alkyl,
- hydroxy-$C_{1-4}$-alkyl, and
- halogen-$C_{1-4}$-alkyl.

**6.** Iron(III) complex compounds for the use according to any one of the claims 1 to 5, wherein $R_2$ is selected from the group consisting of:

- hydrogen,
- halogen,
- $C_{1-6}$-alkyl,
- $C_{3-6}$-cycloalkyl,
- halogen-$C_{1-4}$-alkyl, and
- cyano.

**7.** Iron(III) complex compounds for the use according to any one of the claims 1 to 4, wherein $R_1$ and $R_2$ together form a propylene ($-CH_2-CH_2-CH_2-$)- or a butylene ($-CH_2-CH_2-CH_2-CH_2-$)-group, in which one methylene group ($-CH_2-$), respectively, can be replaced with -O-, -NH-, or $-NR_5-$, wherein $R_5$ is optionally substituted alkyl, and wherein the groups formed by $R_1$ and $R_2$ can furthermore, respectively, be substituted by one to three substituents selected

from the group consisting of hydroxy, $C_{1-4}$-alkoxy, amino and mono- or di-($C_{1-4}$-alkyl)amino.

**8.** Iron(III) complex compounds for the use according to any one of the claims 1 to 7, wherein $R_3$ and $R_4$ are the same or different and are individually selected from the group consisting of:

- hydrogen,
- $C_{1-6}$-alkyl,
- $C_{3-6}$-cycloalkyl,
- $C_{3-6}$-cycloalkyl-$C_{1-4}$-alkyl,
- $C_{1-4}$-alkoxy-$C_{1-4}$-alkyl,
- $C_{1-3}$-alkoxycarbonyl-$C_{1-6}$-alkyl,
- hydroxy-$C_{1-4}$-alkyl, and
- halogen-$C_{1-4}$-alkyl, or

$R_3$ and $R_4$ together form an ethylene ($-CH_2-CH_2-$)-, propylene ($-CH_2-CH_2-CH_2-$)-, isopropylene ($-CH_2-CH(CH_3)-$)-, butylene ($-CH_2-CH_2-CH_2-CH_2-$)-, isobutylene , pentylene ($-CH_2-CH_2-CH_2-CH_2-CH_2-$)- or isopentylene group, in which one methylene group ($-CH_2-$), respectively, can be replaced with -O-, -NH-, or $-NR_5-$, wherein $R_5$ is optionally substituted alkyl, and wherein the groups formed by $R_3$ and $R_4$ can furthermore, respectively, be substituted by one to three substituents selected from the group consisting of hydroxy, $C_{1-4}$-alkoxy, amino and mono- or di($C_{1-4}$-alkyl)amino.

**9.** Method for the preparation of the iron(III) complex compounds according to any one of the claims 1 to 8, which comprises the reaction of an iron(III) salt with the corresponding β-ketoamide.

**10.** Iron(III) complex compounds for the use according to any one of the claims 1 to 8, wherein the symptoms include: fatigue, listlessness, lack of concentration, low cognitive efficiency, difficulties in finding the right words, forgetfulness, unnatural pallor, irritability, acceleration of heart rate (tachycardia), sore or swollen tongue, enlarged spleen, desire for strange foods (pica), headaches, lack of appetite, increased susceptibility to infections, depressive moods.

**11.** Iron(III) complex compounds for the use according to any one of the claims 1 to 8 for the treatment of iron deficiency anemia in pregnant women, latent iron deficiency anemia in children and adolescents, iron deficiency anemia caused by gastrointestinal abnormalities, iron deficiency anemia due to blood loss, such as gastrointestinal hemorrhage (e.g. due to ulcers, carcinoma, hemorrhoids, inflammatory disorders, taking of acetylsalicylic acid), iron deficiency anemia caused by menstruation, iron deficiency anemia caused by injuries, iron deficiency anemia due to sprue, iron deficiency anemia due to reduced dietary iron uptake, in particular in selectively eating children and adolescents, immunodeficiency caused by iron deficiency anemia, brain function impairment caused by iron deficiency anemias, restless leg syndrome caused by iron deficiency anemias, iron deficiency anemias in the case of cancer, iron deficiency anemias caused by chemotherapies, iron deficiency anemias triggered by inflammation (AI), iron deficiency anemias in the case of congestive cardiac insufficiency (CHF; congestive heart failure), iron deficiency anemias in the case of chronic renal insufficiency stage 3-5 (CKD 3-5; chronic kidney diseases stage 3-5), iron deficiency anemias triggered by chronic inflammation (ACD), iron deficiency anemias in the case of rheumatoid arthritis (RA), iron deficiency anemias in the case of systemic lupus erythematosus (SLE) and iron deficiency anemias in the case of inflammatory bowel diseases (IBD).

**12.** Iron(III) complex compounds for the use according to any one of the claims 1 to 8 for oral administration.

**13.** Iron(III) complex compounds for the use according to claim 12 for administration as a liquid formulation including a drinkable formulation such as a syrup, elixir, solution, suspension, juice.

**14.** Medicament, comprising iron(III) complex compounds as defined in any one of the claims 1 to 8 and at least one physiologically compatible carrier or excipient, for the use in the treatment and prophylaxis of iron deficiency diseases and iron deficiency anemias and the symptoms associated therewith.

**15.** Composition, comprising iron(III) complex compounds as defined in any one of the claims 1 to 8, in combination with at least one further medicament which acts on the iron metabolism, for the use in the treatment and prophylaxis of iron deficiency diseases and iron deficiency anemias and the symptoms associated therewith.

## Revendications

**1.** Composés complexes de fer (III) comprenant au moins un ligand de la formule (I) :

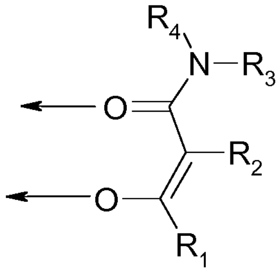

(I)

où

les flèches représentent individuellement une liaison de coordination avec un ou plusieurs atome(s) de fer,
$R_1$ est choisi dans le groupe constitué d'un alkyle facultativement substitué, et d'un alcoxycarbonyle facultativement substitué,
$R_2$ est choisi dans le groupe constitué d'un atome d'hydrogène, d'un alkyle facultativement substitué, d'un atome d'halogène et d'un cyano, ou
$R_1$ et $R_2$, ensemble avec les atomes de carbone auxquels ils sont liés, forment un noyau pentagonal ou hexagonal facultativement substitué, qui peut facultativement contenir un ou plusieurs hétéroatome(s),
$R_3$ et $R_4$ sont identiques ou différents et sont choisis individuellement dans le groupe constitué d'un atome d'hydrogène, d'un amino facultativement substitué et d'un alkyle facultativement substitué, ou
$R_3$ et $R_4$, ensemble avec l'atome d'azote auquel ils sont liés, forment un noyau tri- à hexagonal facultativement substitué, qui peut facultativement contenir un ou plusieurs autre(s) hétéroatome(s),
ou
$R_2$ et $R_3$ forment ensemble un noyau pentagonal ou hexagonal saturé ou insaturé, facultativement substitué, tout en formant un ligand de la formule (Ia) :

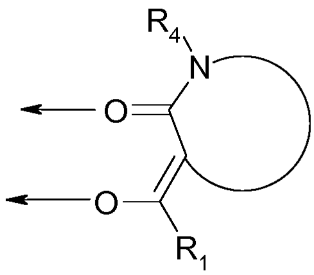

(Ia)

où $R_1$ et $R_4$ sont tels que définis ci-dessus,
ou
$R_2$ et $R_3$ forment ensemble un noyau pentagonal ou hexagonal saturé ou insaturé, facultativement substitué, et $R_1$ et $R_2$ forment ensemble un noyau pentagonal ou hexagonal saturé ou insaturé, facultativement substitué, tout en formant un ligand de la formule (Ib) :

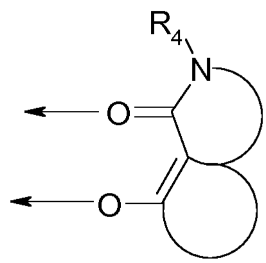

(Ib),

où $R_4$ est tel que défini ci-dessus,
ou des sels pharmaceutiquement acceptables de ceux-ci,
pour une utilisation dans le traitement et la prophylaxie de maladies ferriprives et d'anémies ferriprives et des symptômes associés à celles-ci.

**2.** Composés complexes de fer (III) pour l'utilisation selon la revendication 1, comprenant au moins un ligand de la formule (I) :

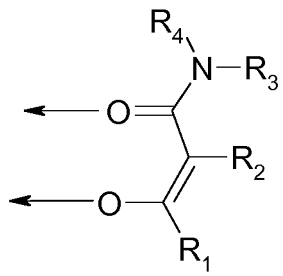

(I)

où

les flèches représentent individuellement une liaison de coordination avec un ou plusieurs atome(s) de fer,
$R_1$ est un alkyle facultativement substitué,
$R_2$ est choisi dans le groupe constitué d'un atome d'hydrogène, d'un alkyle facultativement substitué, d'un atome d'halogène et d'un cyano, ou
$R_1$ et $R_2$, ensemble avec les atomes de carbone auxquels ils sont liés, forment un noyau pentagonal ou hexagonal facultativement substitué, qui peut facultativement contenir un ou plusieurs hétéroatome(s),
$R_3$ et $R_4$ sont identiques ou différents et sont individuellement choisis dans le groupe constitué d'un atome d'hydrogène et d'un alkyle facultativement substitué, ou
$R_3$ et $R_4$, ensemble avec l'atome d'azote auquel ils sont liés, forment un noyau tri- à hexagonal facultativement substitué, qui peut facultativement contenir un ou plusieurs autre(s) hétéroatome(s),
ou
$R_2$ et $R_3$ forment ensemble un noyau pentagonal ou hexagonal saturé ou insaturé, facultativement substitué, tout en formant un ligand de la formule (Ia) :

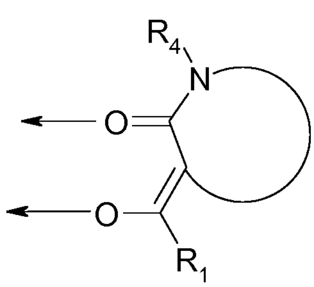

(Ia)

où $R_1$ et $R_4$ sont tels que définis ci-dessus,

ou
$R_2$ et $R_3$ forment ensemble un noyau pentagonal ou hexagonal saturé ou insaturé, facultativement substitué, et $R_1$ et $R_2$ forment ensemble un noyau pentagonal ou hexagonal saturé ou insaturé, facultativement substitué, tout en formant un ligand de la formule (Ib) :

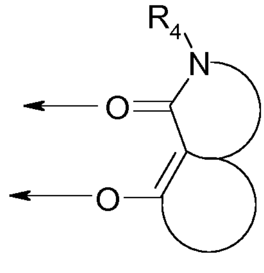

(Ib),

où $R_4$ est tel que défini ci-dessus,
ou des sels pharmaceutiquement acceptables de ceux-ci.

**3.** Composés complexes de fer (III) pour l'utilisation selon la revendication 1 ou 2, comprenant au moins un ligand de la formule (I) :

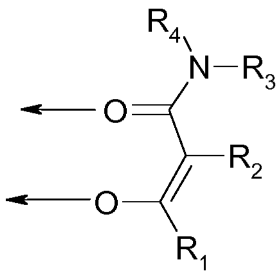

(I)

où

les flèches représentent individuellement une liaison de coordination avec un ou plusieurs atome(s) de fer,
$R_1$ est un alkyle, qui peut être facultativement substitué par 1 à 3 substituant(s) choisi(s) dans le groupe constitué d'un hydroxy, d'un alcoxy, d'un atome d'halogène, d'un cyano et d'un alcoxycarbonyle,
$R_2$ est choisi dans le groupe constitué
d'un atone d'hydrogène,
d'un alkyle, qui peut être facultativement substitué par 1 à 3 substituant(s) choisi(s) dans le groupe constitué d'un hydroxy, d'un alcoxy, d'un atome d'halogène, d'un cyano et d'un alcoxycarbonyle,
d'un atome d'halogène et
d'un cyano ou
$R_1$ et $R_2$, ensemble avec les atomes de carbone auxquels ils sont liés, forment un noyau pentagonal ou hexagonal facultativement substitué, qui peut facultativement contenir un ou plusieurs hétéroatome(s),
$R_3$ et $R_4$ sont identiques ou différents et sont choisis individuellement dans le groupe constitué d'un atome d'hydrogène et d'un alkyle, qui peut être facultativement substitué par 1 à 3 substituant(s) choisi(s) dans le groupe constitué d'un hydroxy, d'un alcoxy, d'un atome d'halogène, d'un cyano et d'un alcoxycarbonyle, ou
$R_3$ et $R_4$, ensemble avec l'atome d'azote auquel ils sont liés, forment un noyau tri- à hexagonal facultativement substitué, qui peut facultativement contenir un ou plusieurs autre(s) hétéroatome(s),

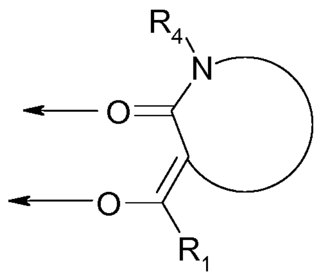

ou

$R_2$ et $R_3$ ensemble forment un noyau pentagonal ou hexagonal saturé ou insaturé, facultativement substitué, tout en formant un ligand de la formule (Ia):

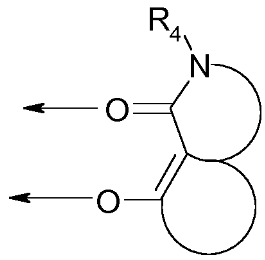

(Ia)

où $R_1$ et $R_4$ sont tels que définis ci-dessus,

ou

$R_2$ et $R_3$ forment ensemble un noyau pentagonal ou hexagonal saturé ou insaturé, facultativement substitué, et $R_1$ et $R_2$ forment ensemble un noyau pentagonal ou hexagonal saturé ou insaturé, facultativement substitué, tout en formant un ligand de la formule (Ib):

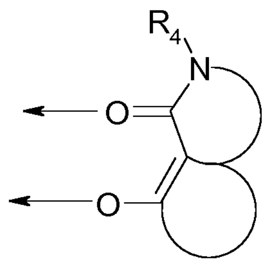

(Ib)

où $R_4$ est tel que défini ci-dessus,

ou des sels pharmaceutiquement acceptables de ceux-ci.

**4.** Composés complexes de fer (III) pour l'utilisation selon l'une des revendications 1 à 3 de la formule (II) :

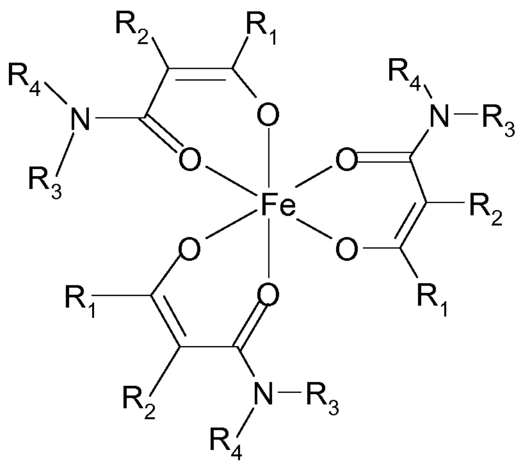

(II)

où $R_1$, $R_2$, $R_3$ et $R_4$ sont tels que définis ci-dessus.

**5.** Composés complexes de fer (III) pour l'utilisation selon l'une quelconque des revendications 1 à 4, dans lesquels $R_1$ est choisi dans le groupe constitué :

- d'un alkyle en $C_1$ à $C_6$,
- d'un cycloalkyle en $C_3$ à $C_6$,
- d'un cycloalkyle en $C_3$ à $C_6$-alkyle en $C_1$ à $C_4$,
- d'un alcoxy en $C_1$ à $C_4$-alkyle en $C_1$ à $C_4$,
- d'un hydroxyalkyle en $C_1$ à $C_4$, et
- d'un halogénoalkyle en $C_1$ à $C_4$.

**6.** Composés complexes de fer (III) pour l'utilisation selon l'une quelconque des revendications 1 à 5, dans lesquels $R_2$ est choisi dans le groupe constitué :

- d'un atome d'hydrogène,
- d'un atome d'halogène,
- d'un alkyle en $C_1$ à $C_6$,
- d'un cycloalkyle en $C_3$ à $C_6$,
- d'un halogénoalkyle en $C_1$ à $C_4$, et
- d'un cyano.

**7.** Composés complexes de fer (III) pour l'utilisation selon l'une quelconque des revendications 1 à 4, dans lesquels $R_1$ et $R_2$ forment ensemble un groupe propylène ($-CH_2-CH_2-CH_2-$) ou butylène ($-CH_2-CH_2-CH_2-CH_2-$), dans lequel un groupe méthylène ($-CH_2-$), respectivement, peut être remplacé par -O-, -NH- ou $-NR_5-$, où $R_5$ est un alkyle facultativement substitué, et où les groupes formés par $R_1$ et $R_2$ peuvent en outre, respectivement, être substitués par un à trois substituant(s) choisi(s) dans le groupe constitué d'hydroxy, d'alcoxy en $C_1$ à $C_4$, d'amino et de mono- ou di-(alkyle en $C_1$ à $C_4$)amino.

**8.** Composés complexes de fer (III) pour l'utilisation selon l'une quelconque des revendications 1 à 7, dans lesquels $R_3$ et $R_4$ sont identiques ou différents et sont choisis individuellement dans le groupe constitué :

- d'un atome d'hydrogène
- d'un alkyle en $C_1$ à $C_6$,
- d'un cycloalkyle en $C_3$ à $C_6$,
- d'un cycloalkyle en $C_3$ à $C_6$-alkyle en $C_1$ à $C_4$,
- d'un alcoxy en $C_1$ à $C_4$-alkyle en $C_1$ à $C_4$,
- d'un alcoxycarbonyle en $C_1$ à $C_3$-alkyle en $C_1$ à $C_6$,
- d'un hydroxyalkyle en $C_1$ à $C_4$, et
- d'un halogénoalkyle en $C_1$ à $C_4$, ou

$R_3$ et $R_4$ forment ensemble un groupe éthylène ($-CH_2-CH_2-$)-, propylène ($-CH_2-CH_2-CH_2-$)-, isopropylène ($-CH_2-CH(CH_3)-$)-, butylène ($-CH_2-CH_2-CH_2-CH_2-$)-, isobutylène, pentylène ($-CH_2-CH_2-CH_2-CH_2-CH_2-$)- ou isopentylène, dans lequel un groupe méthylène ($-CH_2-$), respectivement, peut être remplacé par -O-, -NH- ou $-NR_5-$, où $R_5$ est un alkyle facultativement substitué, et où les groupes formés par $R_3$ et $R_4$ peuvent en outre, respectivement, être substitués par un à trois substituant(s) choisi(s) dans le groupe constitué d'hydroxy, d'alcoxy en $C_1$ à $C_4$, d'amino et de mono- ou di(alkyle en $C_1$ à $C_4$)amino.

**9.** Procédé pour la préparation des composés complexes de fer (III) selon l'une quelconque des revendications 1 à 8, qui comprend la réaction d'un sel de fer (III) avec le β-cétoamide correspondant.

**10.** Composés complexes de fer (III) pour l'utilisation selon l'une quelconque des revendications 1 à 8, dans lesquels les symptômes incluent : la fatigue, l'apathie, le manque de concentration, une faible efficacité cognitive, des difficultés à trouver les mots justes, la perte de mémoire, une pâleur anormale, l'irritabilité, l'accélération de la fréquence cardiaque (tachycardie), une langue irritée ou gonflée, la splénomégalie, l'envie d'aliments étranges (pica), des maux de tête, le manque d'appétit, une susceptibilité accrue aux infections, des humeurs dépressives.

**11.** Composés complexes de fer (III) pour l'utilisation selon l'une quelconque des revendications 1 à 8 pour le traitement de l'anémie ferriprive chez les femmes enceintes, de l'anémie ferriprive latente chez les enfants et les adolescents, de l'anémie ferriprive causée par des anomalies gastro-intestinales, de l'anémie ferriprive due à une perte de sang, telle qu'une hémorragie gastro-intestinale (par exemple, due à des ulcères, à un carcinome, à des hémorroïdes, à des troubles inflammatoires, à la prise d'acide acétylsalicylique), de l'anémie ferriprive causée par la menstruation, de l'anémie ferriprive causée par des blessures, de l'anémie ferriprive due à la sprue, de l'anémie ferriprive due à un apport alimentaire en fer réduit, en particulier chez des enfants et des adolescents à alimentation sélective, de l'immunodéficience causée par l'anémie ferriprive, d'une déficience de la fonction cérébrale causée par des anémies ferriprives, du syndrome des jambes sans repos causé par des anémies ferriprives, des anémies ferriprives dans le cas de cancer, des anémies ferriprives causées par des chimiothérapies, des anémies ferriprives déclenchées par inflammation (AI), des anémies ferriprives dans le cas d'insuffisance cardiaque congestive (ICC ; défaillance cardiaque congestive), des anémies ferriprives dans le cas d'insuffisance rénale chronique stade 3-5 (CKD 3-5 ; maladies rénales chroniques stade 3-5), des anémies ferriprives déclenchées par une inflammation chronique (ACD), des anémies ferriprives dans le cas de la polyarthrite rhumatoïde (PAR), des anémies ferriprives dans le cas du lupus érythémateux disséminé (LED) et des anémies ferriprives dans le cas de maladies inflammatoires intestinales (MII).

**12.** Composés complexes de fer (III) pour l'utilisation selon l'une quelconque des revendications 1 à 8 pour une administration orale.

**13.** Composés complexes de fer (III) pour l'utilisation selon la revendication 12 pour une administration en tant que formulation liquide comportant une formulation buvable telle qu'un sirop, un élixir, une solution, une suspension, un jus.

**14.** Médicament, comprenant des composés complexes de fer (III) tels que définis dans l'une quelconque des revendications 1 à 8 et au moins un support ou excipient physiologiquement compatible, pour l'utilisation dans le traitement et la prophylaxie de maladies ferriprives et d'anémies ferriprives et des symptômes associés à celles-ci.

**15.** Composition, comprenant des composés complexes de fer (III) tels que définis dans l'une quelconque des revendications 1 à 8, en combinaison avec au moins un autre médicament qui agit sur le métabolisme du fer, pour l'utilisation dans le traitement et la prophylaxie de maladies ferriprives et d'anémies ferriprives et des symptômes associés à celles-ci.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 20081455586 A
- WO 2007062546 A
- WO 20040437865 A
- US 2003236224 A
- EP 150085 A
- CN 101481404
- EP 939083 A
- JP 02083400 A
- US 474670 A
- CN 1687089
- US 2009035385 A
- EP 308362 A
- ES 2044777
- FR 19671016
- EP 159194 A
- EP 138420 A
- EP 107458 A
- WO 2006037449 A
- GB 842637 A
- US 20050192315 A


### In der Beschreibung aufgeführte Nicht-Patentliteratur


- **M.W. HENTZE.** Balancing acts: molecular control of mammalian iron metabolism. *Cell,* 2004, vol. 117, 285-297
- *Biometals,* 2009, vol. 22, 701-710
- *JACS,* 2008, vol. 130, 2124-2125
- *Inorg.chem.,* 2006, vol. 45, 6028-6033
- *Inorg. chem.,* 1990, vol. 29, 4096-9
- *Journal of the Chemical Society of Pakistan,* 1991, vol. 13, 79-83
- *Indian Journal of Chemistry,* 1981, vol. 20A, 372-4
- *Journal of Inorganic and Nuclear Chemistry,* 1973, vol. 35, 1397-400
- *Can J Chem,* 1969, vol. 47, 1693-6
- *Indian Journal of Chemistry,* 1968, vol. 6, 516-20
- *Journal of Inorganic and Nuclear Chemistry,* 1967, vol. 29, 2484-6
- *Polyhedron,* 1995, vol. 14, 1397-9
- **SYAMAL, A.** Ferric benzoylacetanilides. *Canadian Journal of Chemistry,* 1969, vol. 47 (10), 1693-6
- **KORTE et al.** *Chemische Berichte,* vol. 95, 2424
- **WAMHOFF et al.** *Liebigs Ann. Chem.,* 1968, vol. 715, 23-34